# EUROPEAN PATENT APPLICATION

(11) **EP 2 441 755 A1**
(43) Date of publication of application: **18.04.2012**
(21) Application number: 10382260.7
(22) Date of filing: 30.09.2010
(51) Int. Cl.: C07D 213/74, C07D 401/12, C07D 401/14, C07D 405/14, C07D 487/08, A61K 31/444, A61K 31/4709, A61K 31/496, A61K 31/497, A61K 31/4995, A61P 29/00

(54) **Pyridine- and isoquinoline-derivatives as Syk and JAK kinase inhibitors**

(71) Applicant: Almirall, S.A., 08022 Barcelona (ES)
(72) Inventor: Vidal Juan, Bernat, 08980, Sant Feliu de Llobregat (ES); Forns Berenguel, Maria Pilar, 08980, Sant Feliu de Llobregat (ES); Castillo Mcquade, Marcos, 08980, Sant Feliu de Llobregat (ES); Erra Sola, Montserrat, 08980, Sant Feliu de Llobregat (ES); Mir Cepeda, Marta, 08980, Sant Feliu de Llobregat (ES)
(74) Representative: Elzaburu Marquez, Alberto

(57) **Abstract**

The present invention relates to a compound of formula (I), to the process for preparing such compounds and to their use in the treatment of a pathological condition or disease susceptible to amelioration by inhibition of Syk kinase and/or Janus kinases.

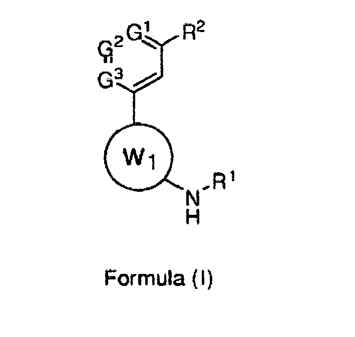

## Description

### FIELD OF THE INVENTION

The present invention is directed to novel compounds having Syk and/or JAK kinase inhibitory activity. The invention is also directed to pharmaceutical compositions comprising such compounds, methods of using such compounds to treat diseases associated with Syk and/or JAK activity and processes and intermediates useful for preparing such compounds.

### BACKGROUND OF THE INVENTION

Spleen tyrosine kinase (Syk) is a novel pharmaceutical target for treatment of allergic, autoimmune and neoplastic disorders. Syk is a non-receptor protein tyrosine kinase that plays an essential role in the signaling pathways of immune receptors on hematopoietic cells, mainly B-Cells, mast cells, macrophages and neutrophils. These immunoreceptors, including Fc receptors and B-Cell receptor, are important for both allergic diseases and antibody-mediated autoimmune diseases. Thus, pharmacological modulation of Syk activity can modulate the inflammatory response in immune-mediated disorders such as allergic disorders and autoimmune diseases. Syk belongs to the Syk/ZAP-70 family of tyrosine kinases predominantly expressed in hematopoietic cells. Syk is expressed in B cells, mast cells, basophils, eosinophils, neutrophils, macrophages, monocytes, NK cells, dendritic cells, platelets, erythrocytes and immature T cells. The contribution of Syk dependent processes to the pathology of allergic disorders, autoimmune disorders and oncology has been reviewed in Singh, R. and Matsuda, E.S. 2007, Annu. Rep. Med. Chem. 42, 379-391.
a) Asthma and allergic rhinitis: Allergic responses of asthma and rhinitis are characterized by hyperproduction of IgE antibodies directed to common environmental antigens (allergens). Allergen-specific IgE binds to the surface of mast cells and basophils through high affinity FcεRl receptors for IgE. Following subsequent allergen exposure, activation of mast cells and basophils through FcεRl occurs and causes mast cell degranulation and release of inflammatory mediators, either preformed or synthesized de novo. Upon FcεRl engagement Syk is rapidly recruited and activated following the initiation of FcεRl-mediated signaling resulting in cellular degranulation and mast cell mediators release. Thus, Syk mediates signaling pathways initiated by stimulation of FcεRl, leading to allergic inflammation. Similar mechanisms are involved in signaling mediated by the engagement of Fc receptors for IgG (FcγRI, FcγRIIA and FcγRIIIA) and IgA (FcaRI) that also recruit Syk to the proximal part of their signaling pathways. Syk is critically involved in the regulation of many cellular functions initiated by Fcγ receptor engagement. For example, in macrophages Syk is relevant for phagocytosis and in neutrophils Syk activation mediates cellular spreading and respiratory burst.
b) Rheumatoid arthritis (RA) and autoimmune diseases: The B cell receptor (BCR) controls a range of B cell responses including proliferation and differentiation into mature antibody producing cells. The BCR is a key regulator point for B cell activity and aberrant signaling can cause disregulated B cell proliferation and formation of pathogenic autoantibodies that lead to multiple auto-immune and/or inflammatory diseases. Syk is immediately downstream BCR and is essential for BCR signaling. Inhibition of Syk is likely to block the BCR signaling and therefore may have a therapeutic benefit blocking B-Cell mediated diseases processes. Moreover, Syk through FcγR-signaling play a critical role in the activation of neutrophils and macrophages elicited by these autoantiboides and their immune complexes. Thus, Syk inhibitors will also block the FcγR signaling through neutrophils and macrophages present in the synovial membrane. In addition, Syk has an additional benefit of inhibiting osteoclast maturation, thereby attenuating the bone erosion, joint destruction and osteopoenia associated with RA (Bajpai, M. et al.2008, Expert Opin. Investig. Drugs 17(5), 641-659).
c) Lymphoma: Besides the role of Syk in BCR cascade, Syk is over-expressed in a many B cell malignancies and is constitutively activated in several lymphomas, playing an essential role in BCR-mediated survival signals. Research at Harvard Medical School and the Max Planck Institute has shown that over activity of Syk kinase is an essential mechanism of survival in several types of B cell lymphoma. Preclinical studies showed that inhibition of Syk kinase inhibits the growth of B cell lymphoma driven by Syk kinase over activity. WO2006093247 and JP2008013499 describe aminopyridine compounds which inhibit Syk kinase. A combination of genomic and expression profiling has identified Syk as a therapeutic target in lymphoma. Overall, Syk appears to function as a proto-oncogene in lymphocytes and, therefore offers a potential target in the treatment of lymphoma.
d) Immune Thrombocytopenic Purpura (ITP): ITP is a disorder defined as a subnormal number of platelets in the circulating blood attributed to an immune-mediated platelet destruction. Once coated with IgG autoantibodies, platelets are prematured cleared through Fcγ receptors that are expressed by tissue macrophages, predominantly in the spleen and liver. Activation of Fcγ receptors and subsequent Syk-mediated signaling has been implicated in the immune destruction of platelets, a hallmark of ITP. The immune system attack and platelet destruction leads to an abnormally low platelet count with resulting bruising and bleeding. As Syk kinase is involved in macrophage Fcγ receptor function and antibody production, its inhibition should abrogate platelet destruction.

JAK kinases are a family of cytoplasmic protein tyrosine kinases including JAK-1, JAK-2, JAK-3 and Tyk-2. Coupled to STAT, the JAK-STAT pathway is universal and essential in cytokine receptor signaling. JAK family kinases are crucial for cytokines mediated differentiation and function of immune and hematopoietic cells. Almost 40 cytokine receptors signal through combinations of four JAK and seven STAT family members. Among Th2 cytokines, IL-4 and IL-13 are major players in asthma and therefore, JAK kinases that signal in IL-4 and IL-13 receptors constitute a target for asthma treatment. In fact, JAK inhibitor should block all effects of IL-4 and IL-13 signaling through type I and type II receptors (Khurana 2003, J. Allergy Clin. Immunol. 111:677 and Murray 2007, J. Immunol. 178:2623).

The role of JAK in asthma is also supported by the fact that JAK3 KO mice shows reduced airway inflammation and mucus production (Malaviya et al. 2000 J. Pharmacol. Exp. Ther. 912:912) and that the preferential JAK2/JAK3 inhibitor CP-690550 inhibits airway inflammation in an animal model of asthma (Kudlacz et al. 2008, Eur. J. Pharmacol. 582:154). Therefore, the four members of the JAK family are believed to play a role in the pathogenesis of the asthmatic response, chronic obstructive pulmonary disease, bronchitis and other related inflammatory diseases of the respiratory tract.

Inhibition of JAK kinases, especially JAK1 and JAK3, could give rise to potent immunosuppression which could be used therapeutically to prevent transplant rejection. In this regard, the JAK inhibitor Tasocitinib citrate (CP-690550) has shown efficacy in several animal models of transplantation (heretopic heart transplantation in mice, cardiac allografts implanted in the ear of mice, renal allotransplantation in cynomolgous monkeys, aorta and tracheal transplantation in rats) by prolonging the mean survival time of grafts (West K (2009). CP-690550, a JAK3 inhibitor as an immunosuppressant for the treatment of rheumatoid arthritis, transplant rejection, psoriasis and other immune-mediated disorders. Curr. Op. Invest. Drugs 10: 491).

In rheumatoid joints, an imbalance between pro and anti-inflammatory cytokine activities favours the induction of autoimmunity, followed by chronic inflammation and tissue destruction. In this regard, the pathogenic role of IL-6 in rheumatoid arthritis (RA) has been validated clinically by the use of the anti-IL-6R antibody tocilizumab. IL-6 activates the transcription factor STAT3, through the use of JAK1 binding to the gp130 receptor chain (Heinrich et al., (2003). Principles of interleukin (IL)-6-type cytokine signalling and its regulation. Biochem J. 374: 1). Constitutive STAT3 mediates the abnormal growth and survival properties of RA synoviocytes (Ivashkiv and Hu (2003). The JAK/STAT pathway in rheumatoid arthritis: pathogenic or protective? Arth & Rheum. 48:2092). Other cytokines that have been implicated in the pathogenesis of arthritis include IL-12 and IL-23, implicated in Th1 and Th17 cell proliferation, respectively; IL-15, and GM-CSF (McInnes and Schett, (2007). Cytokines in the pathogenesis of rheumatoid arthritis. Nature Rew Immunol. 7:429.). The receptors for these cytokines also utilize JAK proteins for signal transduction, making JAK inhibitors potential pleiotropic drugs in this pathology. Consequently, administration of several JAK inhibitors in animal models of murine collagen-induced arthritis and rat adjuvant-induced arthritis has shown to reduce inflammation, and tissue destruction (Milici et al., (2008). Cartilage preservation by inhibition of Janus kinase 3 in two rodent models of rheumatoid arthritis. Arth. Res. 10:R14).

Inhibition of JAK kinases could be an effective way to induce immunomodulation in respiratory and autoimmune diseases.

The present invention relates to novel aminopyridines and aminoisoquinolines derivatives which are inhibitors of Syk and/or JAK kinase activity. Such derivatives have potential therapeutic benefit in the treatment of disorders associated with inappropriate Syk and/or JAK activity, in particular in the treatment and prevention of allergic, autoimmune and neoplastic disorders including asthma, chronic obstructive pulmonary disease, allergic rhinitis, allergic conjunctivitis, allergic dermatitis, atopic dermatitis, seasonal allergies, rheumatoid arthritis, multiple sclerosis, systemic lupus erythematosus, B cell lymphoma and immune thrombocytopenic purpura.

### SUMMARY OF THE INVENTION

Thus, the present invention is directed to new compound of formula (I), or pharmaceutically acceptable salts or N-oxides or solvates or deuterated derivative thereof: 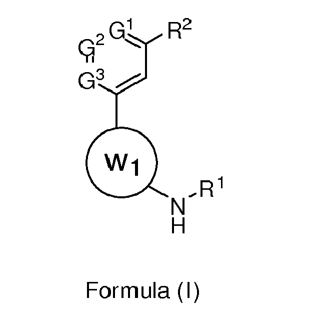 wherein:
- R¹ is a 5 to 6-membered monocyclic heteroaryl group comprising at least one N atom, wherein said heteroaryl group is optionally substituted by one or more substituents selected from the group consisting of a halogen atom, a linear or branched C₁-C₆ alkyl group, a -CN group, a -NR^{a}R^{b} group, a -CF₃ group, a C₁-C₄ alkoxy group and a (C₁-C₄ alkoxy)-(C₁-C₄ alkoxy) group;
- R² is selected from the group consisting of a -(CH₂)ₚ-CONR^{a}R^{b} group, a C₁-C₆ aminoalkyl group, a -NR^{a}-(CH₂)_{q}-NR^{b}R^{c} group, a -NR^{a}-(CH₂)_{q}-OH group, a -(CH₂)ₚ-COOH moiety, a N-Containing saturated or unsaturated 5 to 6-membered heterocyclyl group and a -NR^{d}R^{e} group, wherein
   o R^{d} represents a hydrogen atom or a C₁₋₆ alkyl group, and R^{e} represents a hydrogen atom, a C₁₋₆ alkyl group or a monocyclic or polycyclic C₃-C₁₀ cycloalkyl group optionally substituted by a hydroxy group; or
   o R^{d} and R^{e} together with the nitrogen atom to which they are attached form a 4-to 7-membered, mono- or poly-heterocyclic ring, optionally containing one additional nitrogen atom and optionally substituted by one or more substituents selected from the group consisting of a halogen atom, a hydroxy group, a -NH₂ group, a C₁-C₆ alkyl group, an oxo group, a -COR^{a} group, a -COOR^{a}group and a -CN group;
- W₁ represents a group of formula A, B, or C: 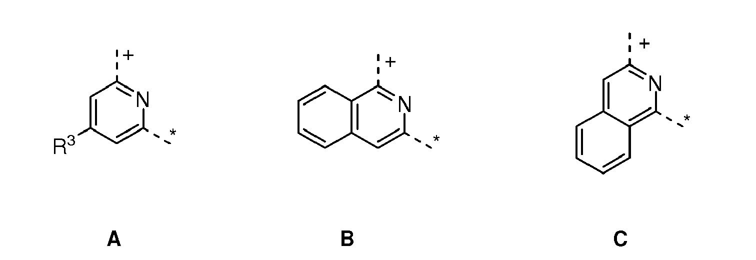 wherein the asterisk symbol indicates the bond that is linked to the -NH-R¹ moiety of Formula (I) and the plus symbol indicates the bond that is linked to the ring system of Formula (I);
- R³ is selected from the group consisting of a hydrogen atom, a -OMe group, a -CN group, a -NR^{a}R^{b} group, a C₆-C₁₀ aryl group, a 5 to 10-membered heteroaryl group, a 3 to 10-membered heterocyclyl group and a -L₁-L₂-W₂ group, wherein the aryl, heteroaryl and heterocyclyl groups are optionally substituted by one or more substituents selected from the group consisting of a halogen atom, a hydroxy group, a -NH₂ group, a C₁-C₆ alkyl group, an oxo group, a -COR^{a} group, a -COOR^{a}group and a -CN group;
- L₁ is selected from the group consisting of a -(CH₂)₀₋₁, NR^{a}CO- group, a -NR^{a}CO₂- group, a -NR^{a}CONR^{b}- group, a -NR^{a}SO₂- group, a -NR^{a}- group, a -CONR^{a}- group, a -CH₂O group and a -CO₂- group;
- L₂ is selected from the group consisting of a direct bond and a linear or branched C₁-C₆ alkylene group, wherein the alkylene group is optionally substituted by a carboxy group;
- W₂ is selected from the group consisting of a linear or branched C₁-C₆ alkyl group, a C₆-C₁₀ aryl group, a 5 to 10-membered heteroaryl group, a 3 to 10-membered heterocyclyl group, and a monocyclic or polycyclic C₄-C₁₂ cycloalkyl group, wherein the alkyl, aryl, heteroaryl, heterocyclyl and cycloalkyl groups are optionally substituted by one or more substituents selected from the group consisting of a halogen atom, a -CF₃ group, a -OCF₃ group, a hydroxy group, a C₁-C₆ alkyl group, a C₁-C₆ alkoxy group, a C₆-C₁₀ aryl-C₁-C₄ alkoxy group, a -COR^{a} group, a -COOR^{a} group, a C₁-C₆ alkyl-COOR^{a} group, a -CONR^{a}R^{b} group and a -NR^{a}R^{b} group, the phenyl moiety optionally has two substituents which are in ortho position to each other and form together a methylenedioxy or ethylenedioxy ring;
- each R^{a}, R^{b} and R^{c} is independently selected from the group consisting of a hydrogen atom and a C₁-C₆ alkyl group;
- each p is an integer independently selected from 0 to 4;
- each q is an integer independently selected from 1 to 4;
- G¹ and G³ are independently selected from the group consisting of a N atom and a CH group;
- G² is selected from the group consisting of a N atom and a C-R⁴ group; and
- R⁴ is selected from the group consisting of a hydrogen atom, a linear or branched C₁-C₆ alkyl group, a halogen atom, a oxo group and a hydroxy group.

The invention provides synthetic processes and intermediates described herein, which are useful for preparing compounds of the invention.

The invention also provides a pharmaceutical composition comprising a compound of the invention and a pharmaceutically-acceptable carrier. The invention further provides combinations comprising a compound of the invention and one or more other therapeutic agents and pharmaceutical compositions comprising such combinations.

The invention also provides a compound of the invention for use in the treatment of the human or animal body by therapy.

The invention also provides a method of treating a disease or condition susceptible to amelioration by inhibition of Syk kinase and/or Janus kinases in a mammal, in particular wherein the disease or condition is selected from a pulmonary disease, such as asthma or chronic obstructive pulmonary disease; allergic rhinitis, allergic conjunctivitis, allergic dermatitis, atopic dermatitis, seasonal allergies, rheumatoid arthritis, multiple sclerosis, systemic lupus erythematosus, B cell lymphoma and immune thrombocytopenic purpura; comprising such method administering to the mammal, a therapeutically effective amount of a compound of the invention. The invention further provides a method of treatment comprising administering a therapeutically effective amount of a combination of a compound of the invention together with one or more other therapeutic agents.

The invention also provides a compound as described herein for use in the treatment of a disease or condition susceptible to amelioration by inhibition of Syk kinase and/or Janus kinases in a mammal, in particular wherein the disease or condition is selected from a pulmonary disease, such as asthma or chronic obstructive pulmonary disease; allergic rhinitis, allergic conjunctivitis, allergic dermatitis, atopic dermatitis, seasonal allergies, rheumatoid arthritis, multiple sclerosis, systemic lupus erythematosus, B cell lymphoma and immune thrombocytopenic purpura.

The invention also provides the use of a compound of the invention in the manufacture of a formulation or medicament for treating a disease or condition susceptible to amelioration by inhibition of Syk kinase and/or Janus kinases in a mammal, in particular wherein the condition or disease is selected from a pulmonary disease, such as asthma or chronic obstructive pulmonary disease; allergic rhinitis, allergic conjunctivitis, allergic dermatitis, atopic dermatitis, seasonal allergies, rheumatoid arthritis, multiple sclerosis, systemic lupus erythematosus, B cell lymphoma and immune thrombocytopenic purpura.

The invention also provides a combination product comprising of (i) a compound of the invention as described herein; and (ii) one or more active ingredients selected from the group consisting of a β2-adrenergic agonist, a corticosteroid, an anti-Cholinergic agent, an anti-allergic agent, an anti-inflammatory agent, an immunosuppressant, a PDE4 inhibitor, a topically acting p38 inhibitor, an IKK2 inhibitor, an A_{2A} agonist, a Pl3Kδγnhibitor and an anti-infective agent, for simultaneous, separate or sequential use in the treatment of the human or animal body

### DETAILED DESCRIPTION OF THE INVENTION.

When describing the compounds, compositions and methods of the invention, the following terms have the following meanings, unless otherwise indicated.

As used herein the term C₁-₆ alkyl embraces linear or branched radicals having 1 to 6 carbon atoms. Examples include methyl, ethyl, n-propyl, i-propyl, n-butyl, sec-butyl, t-butyl, n-pentyl or neopentyl.

As used herein, the term C₁-₆ alkylene embraces divalent alkyl moieties typically having from 1 to 6 carbon atoms, preferably from 1 to 4 carbon atoms. Examples of C₁-₆ alkylene radicals include methylene, ethylene, propylene, butylene, pentylene and hexylene radicals.

As used herein, the term C₁-₆ alkoxy (or alkyloxy) embraces linear or branched oxy-Containing radicals each having alkyl portions of 1 to 6 carbon atoms. Examples of C₁-₆ alkoxy radicals include methoxy, ethoxy, n-propoxy, i-propoxy, n-butoxy, sec-butoxy, t-butoxy, n-pentoxy and n-hexyloxy.

As used herein the term C₁-₆ alkylamino embraces linear or branched radicals having 1 to 6 carbon atoms, said radical being substituted by one or more, typically one, amino group. Examples include -CH₂NH₂ and -CH₂CH₂NH₂.

As used herein, the term C₆-₁₀ aryl radical embraces typically a C₆-C₁₀ monocyclic or polycyclic aryl radical such as phenyl and naphthyl.

As used herein, the term 5- to 10- membered heteroaryl radical embraces typically a 5-to 10- membered ring system comprising at least one heteroaromatic ring and containing at least one heteroatom, and preferably one or two heteroatoms, selected from O, S and N. A 5- to 10- membered heteroaryl radical may be a single ring or two or more fused rings wherein at least one ring contains a heteroatom.

Examples include pyridyl, pyrazinyl, pyrimidinyl, pyridazinyl, furyl, benzofuranyl, oxadiazolyl, oxazolyl, isoxazolyl, benzoxazolyl, imidazolyl, benzimidazolyl, thiazolyl, thiadiazolyl, thienyl, pyrrolyl, pyridinyl, benzothiazolyl, indolyl, indazolyl, purinyl, quinolyl, isoquinolyl, phthalazinyl, naphthyridinyl, quinoxalinyl, quinazolinyl, quinolizinyl, cinnolinyl, triazolyl, indolizinyl, indolinyl, isoindolinyl, isoindolyl, imidazolidinyl, pteridinyl, thianthrenyl, pyrazolyl, 2H-pyrazolo[3,4-d]pyrimidinyl, 1 H-pyrazolo[3,4-d]pyrimidinyl, thieno[2,3-d] pyrimidnyl and the various pyrrolopyridyl radicals.

As used herein, the term 5 to 6-membered monocyclic heteroaryl group comprising at least one N atom embraces typically a 5- to 6- membered ring system comprising a single heteroaromatic ring and containing at least one, and preferably one or two, N atoms. Examples include pyridyl, pyrazinyl, pyrazolyl and pyrimininyl.

As used herein, the term C₃-₁₀ cycloalkyl embraces saturated carbocyclic radicals having from 3 to 10 carbon atoms. A C₃-₁₀ cycloalkyl moiety may be monocylic or polycyclic. Examples include cyclopropyl, cyclobutyl, cyclopentyl, cyclohexyl, cycloheptyl, cyclooctyl and bicyclo[2.2.1]heptan.

As used herein, the term 3 to 10-membered heterocyclyl radical embraces typically a non-aromatic, saturated or unsaturated C₃-C₁₀ carbocyclic ring system in which one or more carbon atoms are replaced by a heteroatom selected from N, O and S. A heterocyclic radical may be a single ring or two or more fused rings wherein at least one ring contains a heteroatom.

Examples of 3 to 10-membered heterocyclic radicals include piperidyl, pyrrolidyl, pyrrolinyl, piperazinyl, morpholinyl, thiomorpholinyl, pyrrolyl, pyrazolinyl, pirazolidinyl, quinuclidinyl, triazolyl, pyrazolyl, tetrazolyl, imidazolidinyl, imidazolyl, oxiranyl, 4,5-dihydro-oxazolyl, 2-benzofuran-1 (3H)-one, 1,3-dioxol-2-one and 3-aza-tetrahydrofuranyl.

As used herein, the term N-Containing saturated or unsaturated 5 to 6-membered heterocyclyl group embraces typically a non-aromatic, saturated or unsaturated C₅-C₆ carbocyclic ring system in which one or more, preferably one or two, carbon atoms is replaced by a nitrogen atom. Examples include piperidyl, pyrrolidyl, pyrrolinyl, piperazinyl and1 ,2,3,6-tetrahydropyridinyl.

As used herein, the 4-to 7-membered, mono- or poly-heterocyclic ring optionally containing one additional nitrogen atom which R^{d} and R^{e} may form together with the nitrogen atom to which they are attached embraces typically a non-aromatic, saturated or unsaturated C₄-C₇ carbocyclic ring system in which one or two carbon atom are replaced by nitrogen atoms. Examples include azetidyl, piperidyl, pyrrolidyl, pyrrolinyl, piperazinyl, 1,4-diazepanyl, 2,5-diazabicyclo[2.2.1 ]-heptanyl and 1,2,3,6-tetrahydropyridinyl.

When a substituent of W₂ is a (C₆-C₁₀ aryl)-(C₁-C₄-alkoxy) group, it is to be understood that the C₁-C₄-alkoxy portion is bonded to L₂ moiety of the molecule as depicted in formula (I).

As used herein, some of the atoms, radicals, moieties, chains and cycles present in the general structures of the invention are "optionally substituted". This means that these atoms, radicals, moieties, chains and cycles can be either unsubstituted or substituted in any position by one or more, for example 1, 2, 3 or 4, substituents, whereby the hydrogen atoms bound to the unsubstituted atoms, radicals, moieties, chains and cycles are replaced by chemically acceptable atoms, radicals, moieties, chains and cycles. When two or more substituents are present, each substituent may be the same or different. The substituents are typically themselves unsubstituted.

As used herein, the term halogen atom embraces chlorine, fluorine, bromine or iodine atoms typically a fluorine, chlorine or bromine atom. The term halo when used as a prefix has the same meaning.

The term "therapeutically effective amount" refers to an amount sufficient to effect treatment when administered to a patient in need of treatment.

The term "treatment" as used herein refers to the treatment of a disease or medical condition in a human patient which includes:
(a) preventing the disease or medical condition from occurring, i.e., prophylactic treatment of a patient;
(b) ameliorating the disease or medical condition, i.e., causing regression of the disease or medical condition in a patient;
(c) suppressing the disease or medical condition, i.e., slowing the development of the disease or medical condition in a patient; or
(d) alleviating the symptoms of the disease or medical condition in a patient.

The phrase "disease or condition susceptible to amelioration by inhibition of Syk kinase and/or Janus kinases" includes all disease states and/or conditions that are acknowledged now, or that are found in the future, to be associated with both Syk kinase and/or Janus kinases. Such disease states include, but are not limited to, pulmonary diseases, such as asthma and chronic obstructive pulmonary disease (including chronic bronchitis and emphysema), as well as allergic rhinitis, allergic conjunctivitis, allergic dermatitis, atopic dermatitis, seasonal allergies, rheumatoid arthritis, multiple sclerosis, systemic lupus erythematosus, B cell lymphoma and immune thrombocytopenic purpura.

The term "pharmaceutically-acceptable salt" refers to a salt prepared from a base or acid which is acceptable for administration to a patient, such as a mammal. Such salts can be derived from pharmaceutically-acceptable inorganic or organic bases and from pharmaceutically-acceptable inorganic or organic acids.

Salts derived from pharmaceutically-acceptable acids include acetic, benzenesulfonic, benzoic, camphosulfonic, citric, ethanesulfonic, fumaric, gluconic, glutamic, hydrobromic, hydrochloric, lactic, maleic, malic, mandelic, methanesulfonic, mucic, nitric, pantothenic, phosphoric, succinic, sulfuric, tartaric, p-toluenesulfonic, xinafoic (1-hydroxy-2-naphthoic acid), napadisilic (1,5-naphthalenedisulfonic acid) and the like. Particularly preferred are salts derived from fumaric, hydrobromic, hydrochloric, acetic, sulfuric, methanesulfonic, xinafoic, and tartaric acids.

Salts derived from pharmaceutically-acceptable inorganic bases include aluminum, ammonium, calcium, copper, ferric, ferrous, lithium, magnesium, manganic, manganous, potassium, sodium, zinc and the like. Particularly preferred are ammonium, calcium, magnesium, potassium and sodium salts.

Salts derived from pharmaceutically-acceptable organic bases include salts of primary, secondary and tertiary amines, including substituted amines, cyclic amines, naturally-occurring amines and the like, such as arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

As used herein, an N-oxide is formed from the tertiary basic amines or imines present in the molecule, using a convenient oxidising agent.

As used herein, the term solvate means a compound which further includes a stoichiometric or non-stoichiometric amount of solvent such as water, acetone, ethanol, methanol, dichloromethane, 2-propanol, or the like, bound by non-Covalent intermolecular forces. When the solvent is water, the term hydrate is used instead of solvate.

As used herein, the term deuterated derivative embraces compounds of the invention where in a particular position at least one hydrogen atom is replaced by deuterium. Deuterium (D or ²H) is a stable isotope of hydrogen which is present at a natural abundance of 0.015 molar %.

Hydrogen deuterium exchange (deuterium incorporation) -is a chemical reaction in which a covalently bonded hydrogen atom is replaced by a deuterium atom. Said exchange (incorporation) reaction can be total or partial.

Typically, a deuterated derivative of a compound of the invention has an isotopic enrichment factor (ratio between the isotopic abundance and the natural abundance of that isotope, i.e. the percentage of incorporation of deuterium at a given position in a molecule in the place of hydrogen) for each deuterium present at a site designated as a potential site of deuteration on the compound of at least 3500 (52.5% deuterium incorporation).

In a preferred embodiment, the isotopic enrichment factor is at least 5000 (75% deuterium). In a more preferred embodiment, the isotopic enrichment factor is at least 6333.3 (95% deuterium incorporation). In a most preferred embodiment, the isotopic enrichment factor is at least 6633.3 (99.5% deuterium incorporation). It is understood that the isotopic enrichment factor of each deuterium present at a site designated as a site of deuteration is independent from the other deuteration sites.

The isotopic enrichment factor can be determined using conventional analytical methods known too en ordinary skilled in the art, including mass spectrometry (MS) and nuclear magnetic resonance (NMR).

The compounds of the invention contain at least a chiral center. Accordingly, the invention includes racemic mixtures, enantiomers, and mixtures enriched in one or more stereoisomer. The scope of the invention as described and claimed encompasses the racemic forms of the compounds as well as the individual enantiomers, diastereomers, and stereoisomer-enriched mixtures.

Typically R¹ represents a pyrazin-2-yl group, a pyridin-2-yl group, a pyrimidin-4-yl group or a 1 *H-*pyrazol-3-yl group, wherein said heteroaryl groups are optionally substituted by one substituent selected from the group consisting of a halogen atom, a linear or branched C₁-C₆ alkyl group, a -CN group and a -CF₃ group. Preferably R¹ represents an unsubstituted pyrazin-2-yl group or a pyridin-2-yl group, wherein said pyridin-2-yl group is optionally substituted by one substituent selected from the group consisting of a methyl group and a -CF₃ group.

Typically R² represents an amino-C₁-C₆ alkyl group, a -NR^{a}-(CH₂)ₚ-NR^{b}R^{c} group, a N-Containg saturated or unsaturated 6-membered heterocyclyl group or a -NR^{d}R^{e} group, wherein
o R^{d} and R^{e} together with the nitrogen atom to which they are attached form a 6- to 7-membered, mono- or poly-heterocyclic ring, containing one additional nitrogen atom and optionally substituted by one or more substituents selected from the group consisting of a hydroxy group, a -NH₂ group, a C₁-₆ alkyl group, an oxo group, a -COR^{a} group, a -COOR^{a} group and a -CN group.

Preferably, R² represents an aminoethyl group, a -NH-(CH₂)₂₋₃-NH₂ group, a 1,2,3,6-tetrahydropyridin-4-yl group, a piperidin-4-yl group, or a -NR^{d}R^{e} group, wherein
o R^{d} and R^{e} together with the nitrogen atom to which they are attached form a 6- to 7-membered, mono- or poly-heterocyclic ring, selected from a piperazin-1-yl group, a 1,4-diazepan-1-yl group and a 2,5-diazabicyclo[2.2.1 ]heptan-2-yl group, wherein said ring is optionally substituted by one or two substituents selected from the group consisting of a methyl group, a fluorine and a -CN group.

Typically L₁ represents -NHCO-, -CONH- or NHCONH-.

Typically L₂ represents a direct bond or a linear or branched C₁-C₄ alkylene group, wherein the alkylene group is optionally substituted by a carboxy group.

Typically W₂ represents a phenyl group, a 5 to 6-membered heteroaryl group, a 6 to 10-membered heterocyclyl group or a cyclohexyl group, wherein the phenyl, heteroaryl, heterocyclyl and cyclohexyl groups are optionally substituted by one or two substituents selected from the group consisting of a a fluorine atom, a -CF₃ group, a methyl group, a methoxy group, a benzyloxy group, a -COR^{a} group, or a -COOR^{a} group, or wherein the phenyl group optionally has two substituents which are in ortho position to each other and form together a methylenedioxy or ethylenedioxy ring.

Typically, R³ represents a hydrogen atom, a -NR^{a}R^{b} group, a phenyl group, a 5 to 7-membered heterocyclyl group or a -L,-L₂-W₂ group, wherein the phenyl and heterocyclyl group are optionally substituted by a hydroxy group or a -COOR^{a} group. Preferably, L₁ represents -NHCO-, -CONH- or NHCONH- and W₂ represents a phenyl group, a 5 to 6-membered heteroaryl group, a 6 to 10-membered heterocyclyl group or a cyclohexyl group, wherein the phenyl, heteroaryl, heterocyclyl and cyclohexyl groups are optionally substituted by one or two substituents selected from the group consisting of a a fluorine atom, a -CF₃ group, a methyl group, a methoxy group, a benzyloxy group, a -COR^{a} group, a -COOR^{a} group or wherein the phenyl group optionally has two substituents which are in ortho position to each other and form together a methylenedioxy or ethylenedioxy ring.

Typically G³ represents a CH group. Typically G² is selected from a group consisting of a N atom and a CH group. Preferably G³ represents a CH group and G² is selected from a group consisting of a N atom and a CH group.

Typically, in a moiety of formula -COR^{a}, R^{a} represents C₁ to C₆ alkyl, preferably methyl.

Typically, when the ring which R^{d} and R^{e} may form together is substituted with an oxo group, then there is only one such oxo group substituent. Typically, when the aryl, heteroaryl or heterocyclyl moiety which R³ may represent is substituted with an oxo group, then there is only one such oxo group substituent.

Typically, when R^{d} and R^{e} together with the nitrogen atom to which they are attached form a 4-to 7-membered, mono- or poly-heterocyclic ring, the optional additional nitrogen atom in the ring is present as an -NH- moiety.

In a preferred embodiment of the present invention, compounds of the present invention are those of formula (I) wherein:
- R¹ represents an unsubstituted pyrazin-2-yl group or a pyridin-2-yl group, wherein said pyridin-2-yl group is optionally substituted by one substituent selected from the group consisting of a methyl group and a -CF₃ group;
- R² represents an aminoethyl group, a -NH-(CH₂)₂-₃-NH₂ group, a 1,2,3,6-tetrahydropyridin-4-yl group, a piperidin-4-yl group, or a -NR^{d}R^{e} group, wherein
   o R^{d} and R^{e} together with the nitrogen atom to which they are attached form a 6- to 7-membered, mono- or poly-heterocyclic ring, selected from a piperazin-1-yl group, a 1,4-diazepan-1-yl group and a 2,5-diazabicyclo[2.2.1 ]heptan-2-yl group, wherein said ring is optionally substituted by one or two substituents selected from the group consisting of a methyl group, a fluorine and a -CN group;

- R³ represents a hydrogen atom, a -NR^{a}R^{b} group, a phenyl group, a 5 to 7-membered heterocyclyl group or a-L₁-L₂₋W₂ group, wherein the phenyl and heterocyclyl group are optionally substituted by a hydroxy group or a -COOR^{a} group; and
- G³ represents a CH group and G² is selected from a group consisting of a N atom and a CH group

In one embodiment of the present invention, compounds of formula (I) are those wherein:
- R¹ represents a pyrazin-2-yl group, a pyridin-2-yl group, a pyrimidin-4-yl group or a 1 *H-*pyrazol-3-yl group, wherein said heteroaryl groups are optionally substituted by one substituent selected from the group consisting of a fluorine atom, a methyl group, an amino group, a -CN group, a -CF₃ group, a -C(CH₃)₃ group and a -O(CH₂)₂OCH₃ group;
- R² represents a -NH₂ group, a -(CH₂)₀₋₁CONH₂ group, a -(CH₂)₁₋₂NH₂ group, a -NH(CH₂)₂-₃NH₂ group, a -NH(CH₂)₂OH group, a -N(CH₃)(CH₂)₂₋₃N(CH₃)₂ group, a CH₂CH₂COOH group, a -NH(CH₂)₂OH group, an adamantylamino group, a bicyclo[2.2.1]heptan-2-amino group, or a 4 to 7-membered monocyclic or polycyclic ring selected from the group consisting of a piperazin-1-yl group, an azetidin-1-yl group, a pyrrolidin-1-yl group, a piperidin-4-yl group, a piperidin-1-yl group, a 1,4-diazepan-1-yl group, a 1 ,2,3,6-tetrahydropyridin-4-yl group and a 2,5-diazabicyclo[2.2.1]-heptan-2-yl group, and wherein the adamantylamino group, bicyclo[2.2.1]heptan-2-amino group, or 4 to 7-membered monocyclic or polycyclic ring is optionally substituted by one or two substitutents selected from the group consisting of a methyl group, a hydroxy group, an oxo group, a -COOH group, a -COCH₃ group and a -NH₂ group;
- R³ represents a hydrogen atom, an -OMe group, a -CN group, a NH₂ group, a -NHCH₃ group, a -NH(CH₂)₃CH₃ group, a -N(CH₃)₂ group, a phenyl group, a piperidin-1yl-4-ol group, a piperidin-1-yl-4-Carboxylic acid or a-L₁-L₂-W₂ group, wherein

o L₁ represents a -NHCO- group, a -NHCONH- group, a -NHSO₂ group, a -NH- group, a CH₂O group, a -CH₂NHCO- group or a -CONH- group;
o L₂ represents a direct bond or a linear or branched alkylene group selected from the group consisting of a -CH₂- group , a -(CH₂)₂- group , a -(CH₂)₃- group, a -CH(CH₃)-CH₂- group, a -CH₂-CH(CH₃)- group and a -CH₂CH(CH₃)CH₂ group, wherein said alkylene group is optionally substituted by a carboxy group;
o W₂ represents a methyl group, an ethyl group, an isopropyl group, a neopentyl group, a phenyl group, a pyridine group, a pyrimidine group, a quinoline group, a pyrazolyl group, a piperidinyl group, a 1,2,3,4-tetrahydroisoquinoline group, a cyclohexyl group, wherein said groups are optionally substituted by one or more, preferably one or two, substituents selected from the group consisting of a fluorine atom, a -CF₃ group, a -OCF₃ group, a methyl group, a methoxy group, a benzyloxy group, a -COCH₃ group, a -COOH group, a CH₂CH₂COOH group and a -CO₂CH₃ group, or wherein the phenyl group optionally has two substituents which are in ortho position to each other and form together a methylenedioxy ring;

Preferably in that embodiment G³ represents a CH group and G² represents an N atom or a CH group.

Particular individual compounds of the invention include:
*N*-{6-[3-(Aminomethyl)phenyl]pyridin-2-yl}-4-methylpyridin-2-amine;
*N*-{6-[3-(2-Aminoethyl)phenyl]pyridin-2-yl}-4-methylpyridin-2-amine;
*N*-[6-(3-Aminophenyl)pyridin-2-yl]-4-methylpyridin-2-amine;
3-{6-[(4-Methylpyridin-2-yl)amino]pyridin-2-yl}benzamide;
*N*-(3-{6-[(4-Methylpyridin-2-yl)amino]pyridin-2-yl}phenyl)ethane-1,2-diamine;
4-Methyl-*N*-{6-[3-(1,2,3,6-tetrahydropyridin-4-yl)phenyl]pyridin-2-yl}pyridin-2-amine;
4-Methyl-*N*-[6-(3-piperidin-4-ylphenyl)pyridin-2-yl]pyridin-2-amine;
*N*-{6-[3-(4-Aminopiperidin-1-yl)phenyl]pyridin-2-yl}-4-methylpyridin-2-amine;
*N*⁶-(4-Methylpyridin-2-yl)-2,4'-bipyridine-2',6-diamine;
*N*²-(2-Aminoethyl)-*N*⁶-(4-methylpyridin-2-yl)-2,4'-bipyridine-2',6-diamine;
2'-(3-Aminopyrrolidin-1-yl)-*N*-(4-methylpyridin-2-yl)-2,4'-bipyridin-6-amine;
2'-(4-Aminopiperidin-1-yl)-*N*-(4-methylpyridin-2-yl)-2,4'-bipyridin-6-amine;
*N*²'-(3-Aminopropyl)-*N*⁶-(4-methylpyridin-2-yl)-2,4'-bipyridine-2',6-diamine;
*N*-(4-Methylpyridin-2-yl)-2'-piperazin-1-yl-2,4'-bipyridin-6-amine;
2-({6-[(4-Methylpyridin-2-yl)amino]-2,4'-bipyridin-2'-yl}amino)ethanol;
2'-(1,4-Diazepan-1-yl)-*N*-(4-methylpyridin-2-yl)-2,4'-bipyridin-6-amine;
*N*²'-[2-(Dimethylamino)ethyl]-*N*²'-methyl-*N*⁶-(4-methylpyridin-2-yl)-2,4'-bipyridine-2',6-diamine;
*N*-{6-[3-(2-Aminoethyl)phenyl]pyridin-2-yl}-4-*tert*-butylpyridin-2-amine;
*N*²'-(2-Aminoethyl)-*N*⁶-(4-tert butylpyridin-2-yl)-2,4'-bipyridine-2',6-diamine;
*N*-{6-[3-(2-Aminoethyl)phenyl]pyridin-2-yl}-4-(trifluoromethyl)pyridin-2-amine;
*N*-{6-[3-(1,2,3,6-Tetrahydropyridin-4-yl)phenyl]pyridin-2-yl}-4-(trifluoromethyl) pyridin-2-amine;
*N*-[6-(3-Piperidin-4-ylphenyl)pyridin-2-yl]-4-(trifluoromethyl)pyridin-2-amine;
*N*-[6-(3-Piperazin-1-ylphenyl)pyridin-2-yl]-4-(trifluoromethyl)pyridin-2-amine;
2-[3-(6-{[4-(Trifluoromethyl)pyridin-2-yl]amino}pyridin-2-yl)phenyl]acetamide;
*N*²'-(2-Aminoethyl)-*N*⁶-[4-(trifluoromethyl)pyridin-2-yl]-2,4'-bipyridine-2',6-diamine;
2'-Piperazin-1-yl-*N*-[4-(trifluoromethyl)pyridin-2-yl]-2,4'-bipyridin-6-amine;
*N*-{6-[3-(2-Aminoethyl)phenyl]pyridin-2-yl}-4-(2-methoxyethoxy)pyridin-2-amine;
*N*-{6-[3-(2-Aminoethyl)phenyl]pyridin-2-yl}pyrazin-2-amine;
*N*-{6-[3-(1,2,3,6-Tetrahydropyridin-4-yl)phenyl]pyridin-2-yl}pyrazin-2-amine;
*N*-[6-(3-Piperidin-4-ylphenyl)pyridin-2-yl]pyrazin-2-amine;
2-{3-[6-(Pyrazin-2-ylamino)pyridin-2-yl]phenyl}acetamide;
*N*-[6-(3-Piperazin-1-ylphenyl)pyridin-2-yl]pyrazin-2-amine;
*N*²-(2-Aminoethyl)-*N*⁶-pyrazin-2-yl-2,4'-bipyridine-2',6-diamine;
2'-(4-Methylpiperazin-1-yl)-*N*-pyrazin-2-yl-2,4'-bipyridin-6-amine;
2'-Piperazin-1-yl-*N*-pyrazin-2-yl-2,4'-bipyridin-6-amine;
2'-(4-Acetylpiperazin-1-yl)-*N*-pyrazin-2-yl-2,4'-bipyridin-6-amine;
(4*r*,7*r*)-4-1[6-(Pyrazin-2-ylamino)-2,4'-bipyridin-2'-yl]aminoladamantan-1-ol;
*N*²-[(1 *S*,2*S*,4*R*)-Bicyclo[2.2.1]hept-2-yl]-*N*⁶-pyrazin-2-yl-2,4'-bipyridine-2',6-diamine;
(4*r*,7*r*)-4-({16-[(4-Methylpyridin-2-yl)amino]-2,4'-bipyridin-2'-yl}amino)adamantan-1-ol;
6-[3-(2-Aminoethyl)phenyl]-*N*-(5-methyl-1 *H*-pyrazol-3-yl)pyridin-2-amine;
*N*-(5-Methyl-1 *H-*pyrazol-3-yl)-6-[3-(1,2,3,6-tetrahydropyridin-4-yl)phenyl]pyridin-2-amine;
*N*-(5-Methyl-1 *H*-pyrazol-3-yl)-6-(3-piperidin-4-ylphenyl)pyridin-2-amine;
*N*-(5-Methyl-1 *H*-pyrazol-3-yl)-6-(3-piperazin-1-ylphenyl)pyridin-2-amine;
*N*-(5-Methyl-1 *H*-pyrazol-3-yl)-2'-piperazin-1-yl-2,4'-bipyridin-6-amine;
*N*-(2'-Piperazin-1-yl-2,4'-bipyridin-6-yl)pyrimidine-4,6-diamine;
3-[3-(2-Aminoethyl)phenyl]-*N*-[4-(trifluoromethyl)pyridin-2-yl]isoquinolin-1-amine;
3-[3-(2-Aminoethyl)phenyl]-*N*-pyrazin-2-ylisoquinolin-1-amine;
*N*-(4-Methylpyridin-2-yl)-1-[3-(1,2,3,6-tetrahydropyridin-4-yl)phenyl]isoquinolin-3-amine;
*N*-Pyrazin-2-yl-1-[3-(1,2,3,6-tetrahydropyridin-4-yl)phenyl]isoquinolin-3-amine;
*N*-(5-Methyl-1 *H-*pyrazol-3-yl)-1-[3-(1,2,3,6-tetrahydropyridin-4-yl)phenyl] isoquinolin-3-amine;
*N*-(5-Methyl-1 *H-*pyrazol-3-yl)-1-(3-piperidin-4-ylphenyl)isoquinolin-3-amine;
*N*-(4-Methylpyridin-2-yl)-1-(2-piperazin-1-ylpyridin-4-yl)isoquinolin-3-amine;
1-(2-Piperazin-1-ylpyridin-4-yl)-*N*-pyrazin-2-ylisoquinolin-3-amine;
1-(2-Piperazin-1-ylpyridin-4-yl)-*N*-pyrimidin-4-ylisoquinolin-3-amine;
*N*-(5-Fluoropyridin-2-yl)-1-(2-piperazin-1-ylpyridin-4-yl)isoquinolin-3-amine;
6-{[1-(2-Piperazin-1-ylpyridin-4-yl)isoquinolin-3-yl]amino}nicotinonitrile;
(±)-1-{2-[(3R,5S)-3,5-Dimethylpiperazin-1-yl]pyridin-4-yl}-N-pyrazin-2-ylisoquinolin-3-amine;
1-[2-(2-Methylpiperazin-1-yl)pyridin-4-yl]-*N*-pyrazin-2-ylisoquinolin-3-amine;
1-{2-[(1 *S*,4*S*)-2,5-Diazabicyclo[2.2.1 ]hept-2-yl]pyridin-4-yl)-*N*-pyrazin-2-ylisoquinolin-3-amine;
(±)-1-{2-[(2*S*,5*R*)-2,5-Dimethylpiperazin-1-yl]pyridin-4-yl}-*N*-pyrazin-2-ylisoquinolin-3-amine;
1-{4-[3-(Pyrazin-2-ylamino)isoquinolin-1-yl]pyridin-2-yl}azetidin-3-ol;
1-{4-[3-(Pyrazin-2-ylamino)isoquinolin-1-yl]pyridin-2-yl}pyrrolidin-3-ol;
1-{4-[3-(Pyrazin-2-ylamino)isoquinolin-1-yl]pyridin-2-yl}piperidin-4-ol;
1-{4-[3-(Pyrazin-2-ylamino)isoquinolin-1-yl]pyridin-2-yl}piperazine-2-Carboxylic acid;
4-{4-[3-(Pyrazin-2-ylamino)isoquinolin-1-yl]pyridin-2-yl}piperazin-2-one;
1-{4-[3-(Pyrazin-2-ylamino)isoquinolin-1-yl]pyridin-2-yl}piperazin-2-one;
2'-Piperazin-1-yl-6-(pyrazin-2-ylamino)-2,4'-bipyridine-4-Carbonitrile;
6-[3-(2-Aminoethyl)phenyl]-4-phenyl-*N*-[4-(trifluoromethyl)pyridin-2-yl]pyridin-2-amine;
*N*-{4-Phenyl-6-[3-(1,2,3,6-tetrahydropyridin-4-yl)phenyl]pyridin-2-yl}pyrazin-2-amine;
*N*-(5-Methyl-1 *H-*pyrazol-3-yl)-4-phenyl-6-[3-(1,2,3,6-tetrahydropyridin-4-yl)phenyl]pyridin-2-amine;
4-Phenyl-2'-piperazin-1-yl-*N*-pyrazin-2-yl-2,4'-bipyridin-6-amine;
*N*-12-(Pyrazin-2-ylamino)-6-[3-(1,2,3,6-tetrahydropyridin-4-yl)phenyl]pyridin-4-yl}benzamide;
3-[({2-(Pyrazin-2-ylamino)-6-[3-(1,2,3,6-tetrahydropyridin-4-yl)phenyl]pyridin-4-yl}amino)carbonyl]benzoic acid;
(2*S*)-2-Benzyl-4-oxo-4-{[6-(pyrazin-2-ylamino)-1 ",2",3",6"-tetrahydro-2,4':2',4"-terpyridin-4-yl]amino}butanoic acid;
*N*-[2'-Piperazin-1-yl-6-(pyrazin-2-ylamino)-2,4'-bipyridin-4-yl]benzamide;
*N*-[2'-Piperazin-1-yl-6-(pyrazin-2-ylamino)-2,4'-bipyridin-4-yl]-3-(trifluoromethyl) benzamide;
*N*-[2'-Piperazin-1-yl-6-(pyrazin-2-ylamino)-2,4'-bipyridin-4-yl]-1,3-benzodioxole-5-Carboxamide;
2-(3-Methoxyphenyl)-*N*-[2'-piperazin-1-yl-6-(pyrazin-2-ylamino)-2,4'-bipyridin-4-yl]acetam ide;
4-({[2'-Piperazin-1-yl-6-(pyrazin-2-ylamino)-2,4'-bipyridin-4-yl]amino}carbonyl) benzoic acid;
4-Fluoro-*N-[2'* -piperazin-1-yl-6-(pyrazin-2-ylamino)-2,4'-bipyridin-4-yl]benzamide;
4-Methoxy-*N*-[2'-piperazin-1-yl-6-(pyrazin-2-ylamino)-2,4'-bipyridin-4-yl]benzamide;
3-Phenyl-*N*-[2'-piperazin-1-yl-6-(pyrazin-2-ylamino)-2,4'-bipyridin-4-yl]propanamide;
1,3-Dimethyl-*N*-[2'-piperazin-1-yl-6-(pyrazin-2-ylamino)-2,4'-bipyridin-4-yl]-1 *H*-pyrazole-5-Carboxamide;
2-(2,4-Difluorophenyl)-*N*-[2'-piperazin-1-yl-6-(pyrazin-2-ylamino)-2,4'-bipyridin-4-yl]acetam ide;
*N*-[2'-Piperazin-1-yl-6-(pyrazin-2-ylamino)-2,4'-bipyridin-4-yl]nicotinamide;
*N*-[2'-Piperazin-1-yl-6-(pyrazin-2-ylamino)-2,4'-bipyridin-4-yl]quinoline-6-Carboxamide;
Methyl 3-({[2'-piperazin-1-yl-6-(pyrazin-2-ylamino)-2,4'-bipyridin-4-yl]amino} carbonyl)benzoate;
3-({[2'-Piperazin-1-yl-6-(pyrazin-2-ylamino)-2,4'-bipyridin-4-yl]amino}carbonyl) benzoic acid;
*N*-[2'-Piperazin-1-yl-6-(pyrazin-2-ylamino)-2,4'-bipyridin-4-yl]-2-pyridin-3-ylacetamide;
Methyl 4-(2-oxo-2-{[2'-piperazin-1-yl-6-(pyrazin-2-ylamino)-2,4'-bipyridin-4-yl]amino}ethyl)benzoate;
4-(2-Oxo-2-{[2'-piperazin-1-yl-6-(pyrazin-2-ylamino)-2,4'-bipyridin-4-yl]amino}ethyl)-benzoic acid;
*N*-[2'-Piperazin-1-yl-6-(pyrazin-2-ylamino)-2,4'-bipyridin-4-yl]piperidine-4-Carboxamide;
1-Acetyl-*N*-[2'-piperazin-1-yl-6-(pyrazin-2-ylamino)-2,4'-bipyridin-4-yl]piperidine-4-Carboxamide;
4-Oxo-4-{[2'-piperazin-1-yl-6-(pyrazin-2-ylamino)-2,4'-bipyridin-4-yl]amino} butanoic acid;
*N*-[2'-Piperazin-1-yl-6-(pyrazin-2-ylamino)-2,4'-bipyridin-4-yl]pyrimidine-5-Carboxamide;
5-({[2'-Piperazin-1-yl-6-(pyrazin-2-ylamino)-2,4'-bipyridin-4-yl]amino}carbonyl) pyridine-2-Carboxylic acid;
5-({[2'-Piperazin-1-yl-6-(pyrazin-2-ylamino)-2,4'-bipyridin-4-yl]amino}carbonyl) nicotinic acid;
(2*S*)-2-Benzyl-4-oxo-4-{[2'-piperazin-1-yl-6-(pyrazin-2-ylamino)-2,4'-bipyridin-4-yl]-amino}butanoic acid;
*trans*-4-({[2'-Piperazin-1-yl-6-(pyrazin-2-ylamino)-2,4'-bipyridin-4-yl]amino} carbonyl)-Cyclohexanecarboxylic acid;
(3*S*)-3-Benzyl-4-oxo-4-{[2'-piperazin-1-yl-6-(pyrazin-2-ylamino)-2,4'-bipyridin-4-yl]-amino}butanoic acid;
*N*-[2'-Piperazin-1-yl-6-(pyrazin-2-ylamino)-2,4'-bipyridin-4-yl]acetamide;
*N*-(2'-Piperazin-1-yl-6-{[4-(trifluoromethyl)pyridin-2-yl]amino}-2,4'-bipyridin-4-yl)-nicotinamide;
4-{[(2'-Piperazin-1-yl-6-{[4-(trifluoromethyl)pyridin-2-yl]amino}-2,4'-bipyridin-4-yl)amino]-Carbonyl}benzoic acid;
5-Oxo-3-phenyl-5-(2'-(piperazin-1-yl)-6-(pyrazin-2-ylamino)-2,4'-bipyridin-4-ylamino)-pentanoic acid;
(3*R*)-3-(Cyclohexylmethyl)-4-oxo-4-{[2'-piperazin-1-yl-6-(pyrazin-2-ylamino)-2,4'-bipyridin-4-yl]amino}butanoic acid;
2-({[2'-Piperazin-1-yl-6-(pyrazin-2-ylamino)-2,4'-bipyridin-4-yl]aminolcarbonyl)cyclohexanecarboxylic acid;
2-({[2'-Piperazin-1-yl-6-(pyrazin-2-ylamino)-2,4'-bipyridin-4-yl]amino}carbonyl)benzoic acid;
(3*R*)-3-Methyl-4-oxo-4-{[2'-piperazin-1-yl-6-(pyrazin-2-ylamino)-2,4'-bipyridin-4-yl]-amino}butanoic acid;
(3*S*)-4-Phenyl-3-{[3-({[2'-piperazin-1-yl-6-(pyrazin-2-ylamino)-2,4'-bipyridin-4-yl]amino}-Carbonyl)benzoyl]amino}butanoic acid;
3,3-Dimethyl-5-oxo-5-{[2'-piperazin-1-yl-6-(pyrazin-2-ylamino)-2,4'-bipyridin-4-yl]-amino}pentanoic acid;
2-Methyl-3-phenyl-*N*-[2'-piperazin-1-yl-6-(pyrazin-2-ylamino)-2,4'-bipyridin-4-yl]-propanamide;
5-Methyl-3-({[2'-piperazin-1-yl-6-(pyrazin-2-ylamino)-2,4'-bipyridin-4-yl]amino}-Carbonyl)hexanoic acid;
(3*R*)-4-Oxo-3-phenyl-4-{[2'-piperazin-1-yl-6-(pyrazin-2-ylamino)-2,4'-bipyridin-4-yl]-amino}butanoic acid;
(3*R*)-3-Benzyl-4-oxo-4-{[2'-piperazin-1-yl-6-(pyrazin-2-ylamino)-2,4'-bipyridin-4-yl]-amino}butanoic acid;
*N*-[2'-[(1*S*,4*S*)-2,5-Diazabicyclo[2.2.1]hept-2-yl]-6-(pyrazin-2-ylamino)-2,4'-bipyridin-4-yl]nicotinamide;
4-({[2'-[(1 *S*,4*S*)-2,5-Diazabicyclo[2.2.1]hept-2-yl]-6-(pyrazin-2-ylamino)-2,4'-bipyridin-4-yl]amino}carbonyl)benzoic acid;
*N*-(2'-[( 1*S*,4*S*)-2,5-Diazabicyclo[2.2.1 ]hept-2-yl]-6-{[4-(trifluoromethyl)pyridin-2-yl]-amino}-2,4'-bipyridin-4-yl)nicotinamide;
4-{[(2'-[(1 *S*,4*S*)-2,5-Diazabicyclo[2.2.1]hept-2-yl]-6-1[4-(trifluoromethyl)pyridin-2-yl]-amino}-2,4'-bipyridin-4-yl)amino]carbonyl}benzoic acid;
*N*-[2'-Piperazin-1-yl-6-(pyrazin-2-ylamino)-2,4'-bipyridin-4-yl]benzenesulfonamide
*N*-[2'-Piperazin-1-yl-6-(pyrazin-2-ylamino)-2,4'-bipyridin-4-yl]-3-(trifluoromethyl) benzenesulfonamide;
*N*-[2'-Piperazin-1-yl-6-(pyrazin-2-ylamino)-2,4'-bipyridin-4-yl]-3-(trifluoromethoxy) benzenesulfonamide;
(3S)-4-Phenyl-3-[({[2'-piperazin-1-yl-6-(pyrazin-2-ylamino)-2,4'-bipyridin-4-yl]amino}-Carbonyl)amino]butanoic acid;
*N*-[2'-Piperazin-1-yl-6-(pyrazin-2-ylamino)-2,4'-bipyridin-4-yl]-3,4-dihydroisoquinoline-2(1 *H*)-Carboxamide;
4-(Benzyloxy)-*N*-[2'-piperazin-1-yl-6-(pyrazin-2-ylamino)-2,4'-bipyridin-4-yl]piperidine-1-Carboxamide;
*N*⁶-( 4-Methylpyridin-2-yl)-2'-piperazin-1-yl-2,4'-bipyridine-4,6-diamine;
2'-[(1 *S*,4*S*)-2,5-Diazabicyclo[2.2.1]hept-2-yl]-*N*⁶-(4-methylpyridin-2-yl)-2,4'-bipyridine-4,6-diamine;
2'-Piperazin-1-yl-*N*⁶-pyrazin-2-yl-2,4'-bipyridine-4,6-diamine;
*N*⁴-Methyl-2'-(piperazin-1-yl)-*N*⁶-(pyrazin-2-yl)-2,4'-bipyridine-4,6-diamine;
*N*⁴,*N*⁴-Dimethyl-2' -piperazin-1-yl-*N*⁶-pyrazin-2-yl-2,4'-bipyridine-4,6-diamine;
1-(2'-(Piperazin-1-yl)-6-(pyrazin-2-ylamino)-2,4'-bipyridin-4-yl)piperidin-4-ol;
1-[2'-Piperazin-1-yl-6-(pyrazin-2-ylamino)-2,4'-bipyridin-4-yl]piperidine-4-Carboxylic acid;
N⁴-Butyl-2'-piperazin-1-yl-N⁶-pyrazin-2-yl-2,4'-bipyridine-4,6-diamine;
*trans*-4-{[2'-Piperazin-1-yl-6-(pyrazin-2-ylamino)-2,4'-bipyridin-4-yl]amino}cyclohexane-Carboxylic acid;
4-{[2'-Piperazin-1-yl-6-(pyrazin-2-ylamino)-2,4'-bipyridin-4-yl]amino}cyclohexanol;
N⁴-(3-Phenylpropyl)-2'-piperazin-1-yl-N⁶-pyrazin-2-yl-2,4'-bipyridine-4,6-diamine;
3-Benzyl-4-{[2'-piperazin-1-yl-6-(pyrazin-2-ylamino)-2,4'-bipyridin-4-yl]amino}butanoic acid;
4-{[2'-Piperazin-1-yl-6-(pyrazin-2-ylamino)-2,4'-bipyridin-4-yl]amino}butanoic acid;
5'-( Piperazin-1-yl)-*N*⁶-(pyrazin-2-yl)-2,3'-bipyridine-4,6-diamine;
3-{3-[4-Amino-6-(pyrazin-2-ylamino)pyridin-2-yl]phenyl}propanoic acid;
3-{3-[4-Amino-6-(pyrazin-2-ylamino)pyridin-2-yl]phenyl}propanamide;
(3S)-4-Phenyl-3-(2'-(piperazin-1-yl)-6-(pyrazin-2-ylamino)-2,4'-bipyridine-4-Carboxamido)butanoic acid;
2'-Piperazin-1-yl-6-(pyrazin-2-ylamino)-*N*-pyridin-3-yl-2,4'-bipyridine-4-Carboxamide;
N-{[2'-Piperazin-1-yl-6-(pyrazin-2-ylamino)-2,4'-bipyridin-4-yl]carbonyl}-L-phenylalanine;
(3*S*)-3-Benzyl-4-oxo-4-(1[2'-piperazin-1-yl-6-(pyrazin-2-ylamino)-2,4'-bipyridin-4-yl]methyl}amino)butanoic acid;
3-(4-{[2'-Piperazin-1-yl-6-(pyrazin-2-ylamino)-2,4'-bipyridin-4-yl]methoxy}phenyl)-propanoic acid; and
4-Methoxy-2'-piperazin-1-yl-*N*-pyrazin-2-yl-2,4'-bipyridin-6-amine.

Of particular interest are the compounds:
4-Methyl-N-{6-[3-(1,2,3,6-tetrahydropyridin-4-yl)phenyl]pyridin-2-yl}pyridin-2-amine;
N-(4-Methylpyridin-2-yl)-2'-piperazin-1-yl-2,4'-bipyridin-6-amine;
2'-(1,4-Diazepan-1-yl)-N-(4-methylpyridin-2-yl)-2,4'-bipyridin-6-amine;
N-{6-[3-(2-Aminoethyl)phenyl]pyridin-2-yl}-4-(trifluoromethyl)pyridin-2-amine;
N-[6-(3-Piperidin-4-ylphenyl)pyridin-2-yl]-4-(trifluoromethyl)pyridin-2-amine;
N-[6-(3-Piperazin-1-ylphenyl)pyridin-2-yl]-4-(trifluoromethyl)pyridin-2-amine;
N²-(2-Aminoethyl)-N6-[4-(trifluoromethyl)pyridin-2-yl]-2,4'-bipyridine-2',6-diamine;
2'-Piperazin-1-yl-N-[4-(trifluoromethyl)pyridin-2-yl]-2,4'-bipyridin-6-amine;
N-(6-[3-(1,2,3,6-Tetrahydropyridin-4-yl)phenyl]pyridin-2-yl}pyrazin-2-amine;
N-[6-(3-Piperazin-1-ylphenyl)pyridin-2-yl]pyrazin-2-amine;
N-(4-Methylpyridin-2-yl)-1-(2-piperazin-1-ylpyridin-4-yl)isoquinolin-3-amine;
1-(2-Piperazin-1-ylpyridin-4-yl)-N-pyrazin-2-ylisoquinolin-3-amine;
1-[2-(2-Methylpiperazin-1-yl)pyridin-4-yl]-N-pyrazin-2-ylisoquinolin-3-amine;
(±)-1-{2-[(2S,5R)-2,5-Dimethylpiperazin-1-yl]pyridin-4-yl}-N-pyrazin-2-ylisoquinolin-3-amine;
N-{2-(Pyrazin-2-ylamino)-6-[3-(1,2,3,6-tetrahydropyridin-4-yl)phenyl]pyridin-4-yl}-benzamide;
N-[2'-Piperazin-1-yl-6-(pyrazin-2-ylamino)-2,4'-bipyridin-4-yl]benzamide;
N-[2'-Piperazin-1-yl-6-(pyrazin-2-ylamino)-2,4'-bipyridin-4-yl]-3-(trifluoromethyl) benzamide;
N-[2'-Piperazin-1-yl-6-(pyrazin-2-ylamino)-2,4'-bipyridin-4-yl]-1,3-benzodioxole-5-Carboxamide;
2-(3-Methoxyphenyl)-N-[2'-piperazin-1-yl-6-(pyrazin-2-ylamino)-2,4'-bipyridin-4-yl]-acetamide;
4-({[2'-Piperazin-1-yl-6-(pyrazin-2-ylamino)-2,4'-bipyridin-4-yl]amino}carbonyl) benzoic acid;
4-Fluoro-N-[2'-piperazin-1-yl-6-(pyrazin-2-ylamino)-2,4'-bipyridin-4-yl]benzamide;
4-Methoxy-N-[2'-piperazin-1-yl-6-(pyrazin-2-ylamino)-2,4'-bipyridin-4-yl]benzamide;
3-Phenyl-N-[2'-piperazin-1-yl-6-(pyrazin-2-ylamino)-2,4'-bipyridin-4-yl]propanamide;
1,3-Dimethyl-N-[2'-piperazin-1-yl-6-(pyrazin-2-ylamino)-2,4'-bipyridin-4-yl]-1 H-pyrazole-5-Carboxamide;
2-(2,4-Difluorophenyl)-N-[2'-piperazin-1-yl-6-(pyrazin-2-ylamino)-2,4'-bipyridin-4-yl]-acetamide;
N-[2'-Piperazin-1-yl-6-(pyrazin-2-ylamino)-2,4'-bipyridin-4-yl]nicotinamide;
N-[2'-Piperazin-1-yl-6-(pyrazin-2-ylamino)-2,4'-bipyridin-4-yl]quinoline-6-Carboxamide;
Methyl 3-({[2'-piperazin-1-yl-6-(pyrazin-2-ylamino)-2,4'-bipyridin-4-yl]amino} carbonyl)-benzoate;
3-({[2'-Piperazin-1-yl-6-(pyrazin-2-ylamino)-2,4'-bipyridin-4-yl]amino}carbonyl) benzoic acid;
3-[({2-(Pyrazin-2-ylamino)-6-[3-(1,2,3,6-tetrahydropyridin-4-yl)phenyl]pyridin-4-yl}-amino)carbonyl]benzoic acid;
N-[2'-Piperazin-1-yl-6-(pyrazin-2-ylamino)-2,4'-bipyridin-4-yl]-2-pyridin-3-ylacetamide;
Methyl 4-(2-oxo-2-{[2'-piperazin-1-yl-6-(pyrazin-2-ylamino)-2,4'-bipyridin-4-yl]amino}-ethyl)benzoate;
1-Acetyl-N-[2'-piperazin-1-yl-6-(pyrazin-2-ylamino)-2,4'-bipyridin-4-yl]piperidine-4-Carboxamide;
4-Oxo-4-{[2'-piperazin-1-yl-6-(pyrazin-2-ylamino)-2,4'-bipyridin-4-yl]amino} butanoic acid;
N-[2'-Piperazin-1-yl-6-(pyrazin-2-ylamino)-2,4'-bipyridin-4-yl]pyrimidine-5-Carboxamide;
N-[2'-[(1 S,4S)-2,5-Diazabicyclo[2.2.1 ]hept-2-yl]-6-(pyrazin-2-ylamino)-2,4'-bipyridin-4-yl]nicotinamide;
5-({[2'-Piperazin-1-yl-6-(pyrazin-2-ylamino)-2,4'-bipyridin-4-yl]amino}carbonyl)pyridine-2-Carboxylic acid;
(2S)-2-Benzyl-4-oxo-4-{[2'-piperazin-1-yl-6-(pyrazin-2-ylamino)-2,4'-bipyridin-4-yl]amino}butanoic acid;
(2S)-2-Benzyl-4-oxo-4-{[6-(pyrazin-2-ylamino)-1",2",3",6"-tetrahydro-2,4':2',4"-terpyridin-4-yl]amino}butanoic acid;
4-{[(2'-(2,5-Diazabicyclo[2.2.1 ]hept-2-yl)-6-{[4-(trifluoromethyl)pyridin-2-yl]amino}-2,4'-bipyridin-4-yl)amino]carbonyl}benzoic acid;
(3S)-3-Benzyl-4-oxo-4-{[2'-piperazin-1-yl-6-(pyrazin-2-ylamino)-2,4'-bipyridin-4-yl]amino}butanoic acid;
2'-Piperazin-1-yl-*N*⁶-pyrazin-2-yl-2,4'-bipyridine-4,6-diamine;
N⁴,N⁴-Dimethyl-2'-piperazin-1-yl-N6-pyrazin-2-yl-2,4'-bipyridine-4,6-diamine;
N-[2'-Piperazin-1-yl-6-(pyrazin-2-ylamino)-2,4'-bipyridin-4-yl]acetamide;
N⁴-Methyl-2'-(piperazin-1-yl)-N6-(pyrazin-2-yl)-2,4'-bipyridine-4,6-diamine;
5'-(piperazin-1-yl)-N6-(pyrazin-2-yl)-2,3'-bipyridine-4,6-diamine;
1-(2'-(piperazin-1-yl)-6-(pyrazin-2-ylamino)-2,4'-bipyridin-4-yl)piperidin-4-ol;
2-({[2'-piperazin-1-yl-6-(pyrazin-2-ylamino)-2,4'-bipyridin-4-yl]amino}carbonyl)-Cyclohexanecarboxylic acid;
(3R)-3-methyl-4-oxo-4-{[2'-piperazin-1-yl-6-(pyrazin-2-ylamino)-2,4'-bipyridin-4-yl]amino}butanoic acid;
(3S)-4-phenyl-3-{[3-({[2'-piperazin-1-yl-6-(pyrazin-2-ylamino)-2,4'-bipyridin-4-yl]amino}carbonyl)benzoyl]amino}butanoic acid;
4-{[2'-Piperazin-1-yl-6-(pyrazin-2-ylamino)-2,4'-bipyridin-4-yl]amino}cyclohexanol;
N⁴-butyl-2'-piperazin-1-yl-N6-pyrazin-2-yl-2,4'-bipyridine-4,6-diamine; and 2-methyl-3-phenyl-N-[2'-piperazin-1-yl-6-(pyrazin-2-ylamino)-2,4'-bipyridin-4-yl]propanamide.

### GENERAL SYNTHETIC PROCEDURES

The compounds of the invention can be prepared using the methods and procedures described herein, or using similar methods and procedures. It will be appreciated that where typical or preferred process conditions (i.e., reaction temperatures, times, mole ratios of reactants, solvents, pressures, etc.) are given. Other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. The choice of a suitable protecting group for a particular functional group, as well as suitable conditions for protection and deprotection, are well known in the art. For example, numerous protecting groups, and their introduction and removal are described in T. W. Greene and G. M. Wuts, Protecting Groups in Organic Synthesis, Third Edition, Wiley, New York, 1999, and references cited therein.

Processes for preparing compounds of the invention are provided as further embodiments of the invention and are illustrated by the procedures below.

Compounds of general formula (IA) wherein R³ is not a -NR^{b}COR^{a} group, a - NR^{b}CONR^{a}R^{b}, a -NR^{b}CO₂R^{a}, a -NR^{b}SO₂R^{a} group, an amino group, a -NR^{a}R^{b} group, a -CONR^{a}R^{b} group, or a -COOR^{a} group may be prepared following the synthetic scheme illustrated in Figure 1.

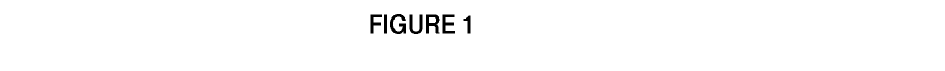

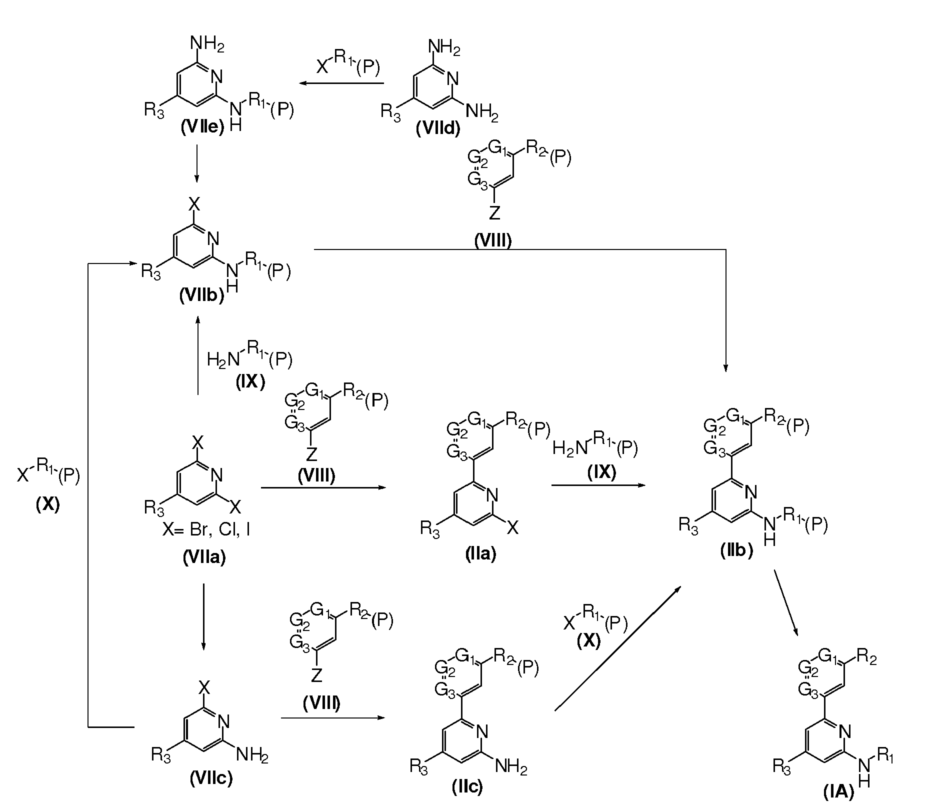

The compounds of formula (IIa), may be obtained by coupling a haloderivative of formula (VIIa), wherein R³ is as defined above and X represents a halogen atom such as bromine, chlorine or iodine, with the corresponding compounds of formula (VIII) wherein G¹, G², G³ and R² are as defined above, P represents a protecting group such as tert-butoxycarbonyl (BOC) and Z represents a boronic acid, boronate derivative or trialkyltin derivative using Suzuki reactions (Miyaura, N.; Suzuki, A. Chem. Rev. 1995, 95, 2457) in the case of the boronic acids (VIIIa) and boronates (VIIIj) (See figure 2) or using Stille coupling reactions (Milstein, D; Stille, J.K., J. Am. Chem. Soc. 1978, 100(11), 3636) in the case of the tin derivatives (Vllld) and (Vllle) (see figure 2). The Suzuki reactions may be catalized by a palladium catalyst such as [1,1'-bis(diphenylphosphino)-ferrocene] dichloropalladium (II) complex with dichloromethane (1:1), tetrakis(triphenylphosphine)-palladium(0), bis(triphenylphosphine)palladium(II) chloride or tris(dibenzylideneacetone)-dipalladium(0) in an organic solvent such as dioxane, toluene, dimethylformamide, ethanol, 1,2-dimethoxyethane or mixtures of them and in the presence of a base such as cesium carbonate, sodium carbonate or potassium phosphate at a temperature from 80ºC to 140ºC, using conventional heating or microwave reactor. The Stille reactions may be carried on in the presence of palladium catalysts such as tetrakis(triphenylphosphine)palladium (0) in solvents such as xylene or dimethylformamide at a temperature between 25ºC to 200ºC

The compounds of formula (IIb) may be obtained by *N*-arylation of the haloderivative of formula (IIa) with the corresponding amine of formula (IX), optionally protected (P) with a group such as *tert-*butyl or BOC. These reactions may be catalized by a palladium catalyst such as tris(dibenzylideneacetone)-dipalladium(0) or palladium(II) acetate and a ligand such as 4,5-bis(diphenylphosphino)-9,9-dimethylxanthene or (±)-2,2'-bis(diphenylphosphino)-1,1'-binaphthalene in an organic solvent such as dioxane, toluene, DMF, THF, benzene or 1,2-dimethoxyethane and in the presence of a base such as cesium carbonate, sodium carbonate, potassium phosphate or potassium *tert-*butoxide at a temperature from 90ºC to 150ºC, using conventional heating or microwave reactor.

The intermediate of formula (IIb) may be also obtained by nucleophilic substitution of an amine of formula (IX), optionally protected (P) with a group such as *tert-*butyl or BOC, over the haloderivative of formula (IIa) in the presence of a base such as potassium *tert-*butoxide in an inert solvent such as toluene or benzene, at a temperature from 80ºC to the boiling point of the solvent.

Additionally, compounds of formula (IIb) may be also obtained by *N*-arylation or by nucheophilic addition of the haloderivative of formula (VIIa) with the corresponding amine of formula (IX), optionally protected (P) with a group such as *tert-*butyl or BOC, to yields intermediates of formula (VIIb). This reaction is carried out following the same conditions described for the synthesis of compounds of formula (IIb) synthesized from compounds of formula (IIa) to yield compounds of formula (VIIb). Subsequent coupling reaction of compounds of formula (Vllb) with the corresponding compounds of formula (VIII) using Suzuki or Stille conditions described for the synthesis of compounds of formula (IIa) yields compounds of formula (IIb).

Additionally, compounds of formula (IIb) may be also obtained by reacting intermediates of formula (VIIa) with ammonia in methanol to yield compounds of formula (VIIc). Subsequent coupling reaction of compounds of formula (Vllc) with the corresponding derivatives of formula (VIII) using Suzuki or Stille conditions described for the synthesis of compounds of formula (IIa) yields compounds of formula (IIc). Subsequent nucheophilic addition of the amine of formula (IIc) with the corresponding haloderivative of formula (X), optionally protected (P) with a group such as *tert-*butyl or BOC, following the same conditions described for the synthesis of compounds of formula (IIb) synthesized from compounds of formula (IIa), yields compounds of formula (IIb).

Intermediates of formula (Vllb) may also be obtained by nucheophilic addition of the amine of formula (Vllc) with the corresponding haloderivative of formula (X), optionally protected (P) with a group such as *tert-*butyl or BOC, following the same conditions described for the synthesis of compounds of formula (IIb) synthesized from compounds of formula (IIa).

Additionally, compounds of formula (Vllb) may also be obtained by nucheophilic addition of the amine of formula (Vlld) with the corresponding haloderivative of formula (X), optionally protected (P) with a group such as *tert-*butyl or BOC, following the same conditions described for the synthesis of compounds of formula (IIb) synthesized from compounds of formula (IIa), to yield compounds of formula (VIIe). Subsequent Sandmeyer reaction of intermediates of formula (VIIe) yields compounds of formula (Vllb) using Sandmeyer conditions such as addition of sodium nitrite in hydrochloric acid and water followed by addition of copper chloride in hydrochloric acid at a temperature from -5 ºC to room temperature.

The deprotection of intermediates of formula (IIb) giving the target compounds (IA) may be carried out by treating of intermediates of formula (IIb) with an acid such as trifluoroacetic acid or hydrochloric acid in a solvent such as dichloromethane, water or dioxane, at a temperature from room temperature to 90ºC.

In the particular case where R₂ is an ethane-1,2-diamine, compounds of formula (IAa) can be prepared by treating compounds of formula (IA) wherein R₂ is an amino group with *tert-*butyl (2-oxoethyl)carbamate in the presence of acetic acid in a solvent such as methanol, followed by addition of sodium cyanoborohydride to yield the protected derivative. Subsequent deprotection of intermediate giving the desired compound may be carried out using the conditions described for the synthesis of compounds of formula (IA) (Figure 1).

In the particular case where R₂ is a 4-piperidine compounds of formula (lAb) can be prepared by reduction of compounds of formula (IIb) wherein R₂ is a tert-butyl 5,6-dihydropyridine-1 (2*H*)-Carboxylate with hydrogen and a hydrogenation catalyst such as palladium on activated carbon in a solvent such as methanol and in a slightly acidic media at room temperature, followed by deprotection of intermediate giving the desired compound using the conditions described for the synthesis of compounds of formula (IA) (Figure 1).

The compounds of general formula (VIII), where G₁ and G₂ are independently N and/or CH and G₃ is CH, and the compounds of general formula (XVIII), where G₁ is N or CH, G₂ is CR₄ and G₃ is N, optionally protected (P) with a group such as BOC, may be prepared following the synthetic scheme illustrated in Figure 2.

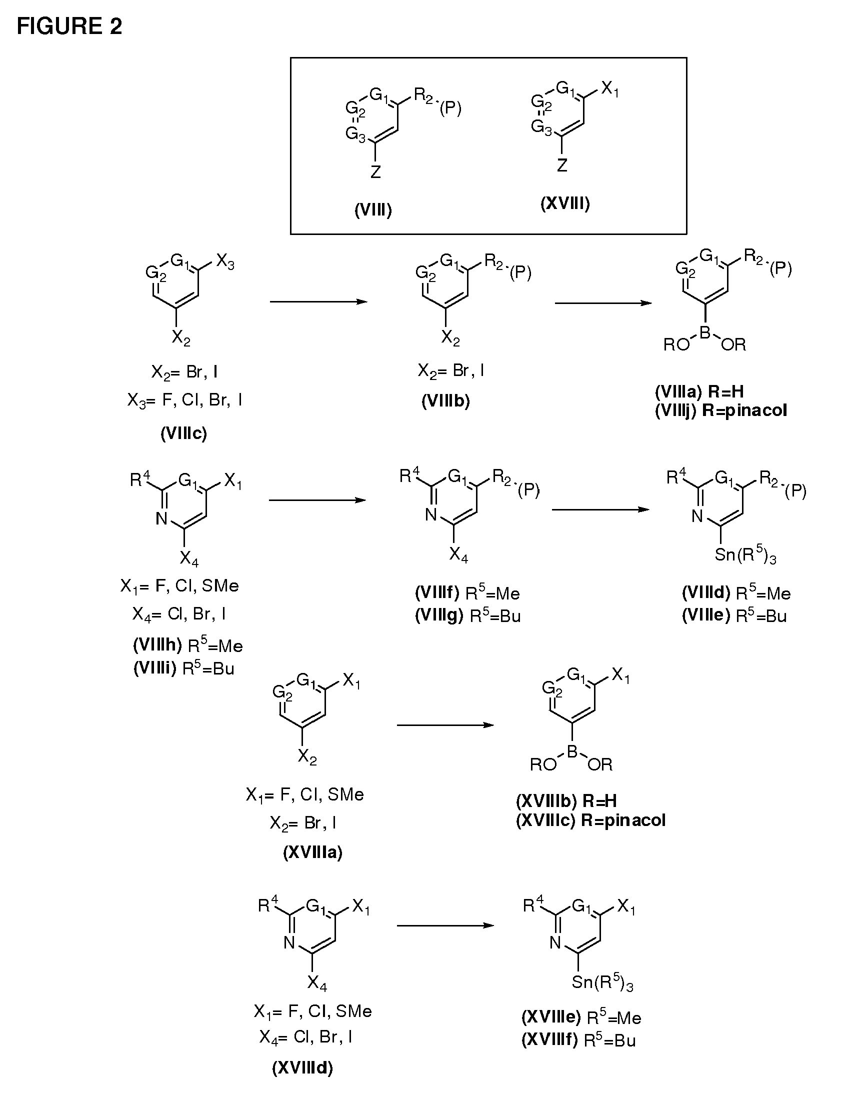

The compounds of formula (Vlllb) may be obtained by *N*-arylation or by nucheophilic addition of the haloderivative of formula (Vlllc) with the corresponding reactant, optionally protected (P) with a group such as BOC, following the conditions described for the synthesis of compounds of formula (IIb) synthesized from compounds of formula (IIa).

The boronic acids of general formula (VIIIa) and the boronates of general formula (VIIIj) may be prepared by treatment of the corresponding haloderivatives (VIIIb), with a suitable reagent such as bis(pinacolato)diboron in the presence of a suitable base such as potassium acetate and a palladium catalyst such as [1,1'-bis(diphenylphosphino)ferrocene]dichloro-palladium(II) complex with dichloromethane (1:1) in an aprotic organic solvent such as dioxane, *N*,*N*'-dimethylformamide or dimethylsulphoxide at a temperature ranging from 80 to 120 ºC.

The compounds of formula (Vlllf) and (Vlllg) may be obtained by *N*-arylation or by nucheophilic addition of the haloderivative of formula (Vlllh) or (VIIIi) with the corresponding reactant, optionally protected (P) with a group such as BOC, following the conditions described for the synthesis of compounds of formula (IIb) synthesized from compounds of formula (IIa).

The tin derivatives of general formula (Vllld) and (Vllle) may be prepared by treatment of the corresponding haloderivatives (VIIIf) and (VIIIg) respectively, prepared according to Sandosham J.; Undheim, K. Tetrahedron 1994, 50 (1), 275-284.

Alternatively, compounds of general formula (IA) wherein R³ is not a -NR^{b}COR^{a} group, a -NR^{b}CO₂R^{a}, a -NR^{b}SO₂R^{a} group, an amino group, a -NR^{a}R^{b} group, a -CONR^{a}R^{b} group, a -NR^{b}CONR^{a}R^{b} or a -COOR^{a} group may be prepared following the synthetic scheme illustrated in Figure 3.

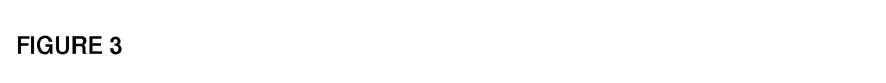

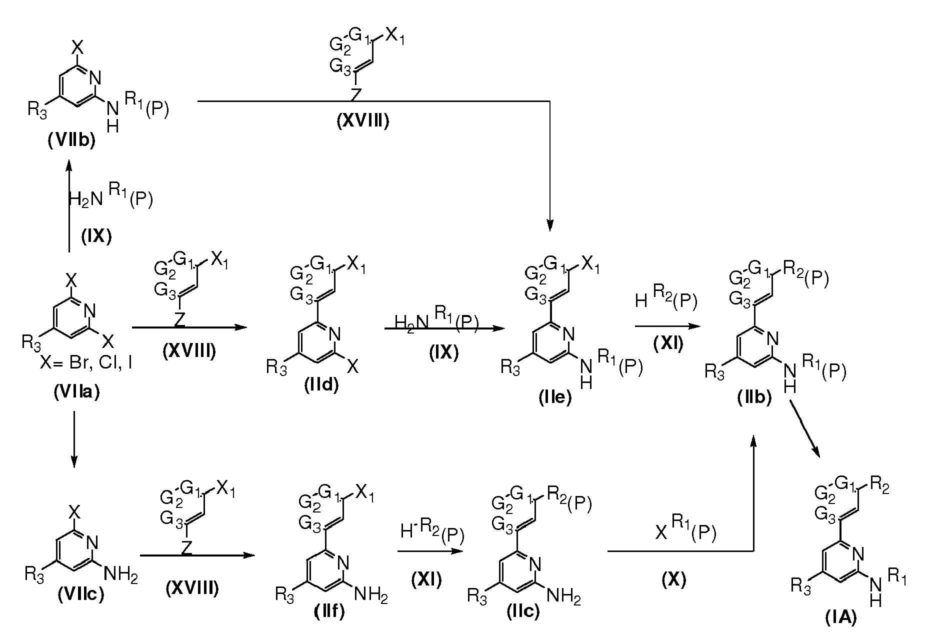

The compounds of formula (IId) may be obtained by coupling of the haloderivative of formula (VIIa) with the corresponding compounds of formula (XVIII) using Suzuki or Stille conditions described for the synthesis of compounds of formula (IIa).

The compounds of formula (IIe) may be obtained by *N*-arylation of the haloderivative of formula (IId) with the corresponding amine of formula (IX), optionally protected (P) with a group such as *tert-*butyl or BOC, using the conditions described for the synthesis of compounds of formula (IIb) synthesized from compounds of formula (IIa).

Additionally, compounds of formula (IIe) may be also obtained by coupling of compounds of formula (Vllb) with the corresponding compounds of formula (XVIII) using Suzuki or Stille conditions described for the synthesis of compounds of formula (IIa).

The intermediate of formula (IIb) may be obtained by nucleophilic substitution of an reactant of formula (XI), optionally protected (P) with a group such as BOC, over the haloderivative of formula (IIe) in the presence of a base such as potassium carbonate in an inert solvent such as dimethylsulphoxide, at a temperature from 80 ºC to 140 ºC.

Additionally, compounds of formula (IIb) may be also obtained starting with a coupling of compounds of formula (Vllc) with the corresponding compounds of formula (XVIII) using Suzuki or Stille conditions described for the synthesis of compounds of formula (IIa) to yield compounds of formula (IIf). Subsequent nucleophilic substitution of a reactant of formula (XI), optionally protected (P) with a group such as BOC, over the haloderivative of formula (IIf), using the conditions described for the synthesis of compounds of formula (IIb) synthesized from compounds of formula (IIe) yields compounds of formula (IIc). Subsequent nucleophilic substitution of an haloderivative of formula (X) over the amine of formula (IIc), using the conditions described for the synthesis of compounds of formula (IIb) synthesized from compounds of formula (IIa) yields compounds of formula (IIb).

According to a further feature of the present invention, compounds of general formula (II) may also be prepared following the synthetic scheme illustrated in Figure 4.1 and 4.2.

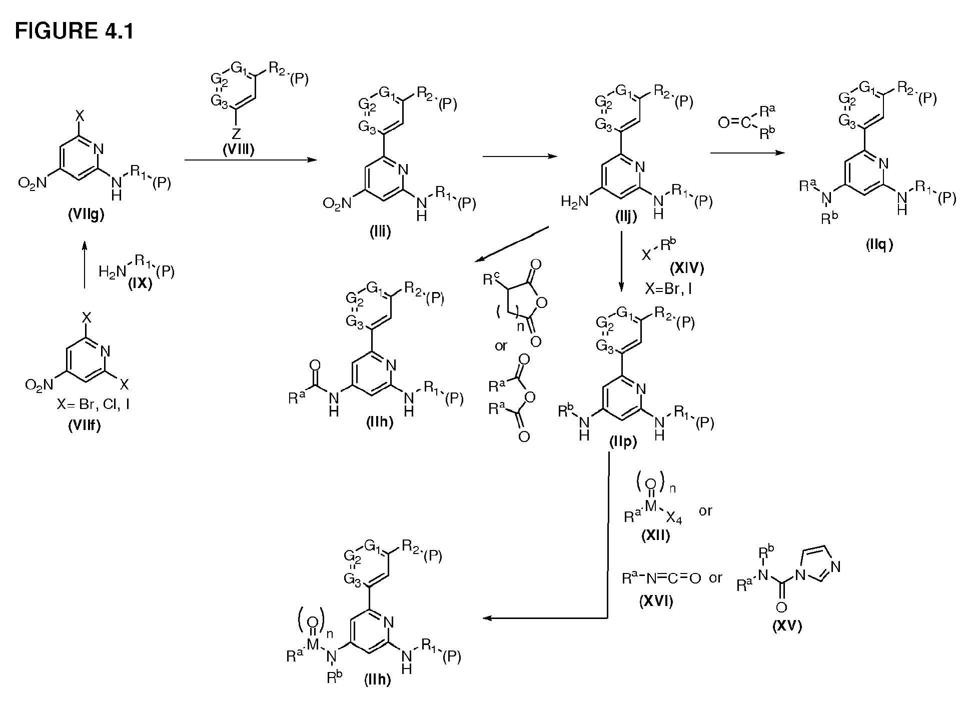

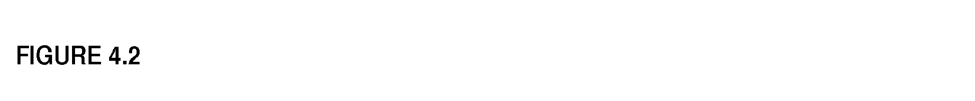

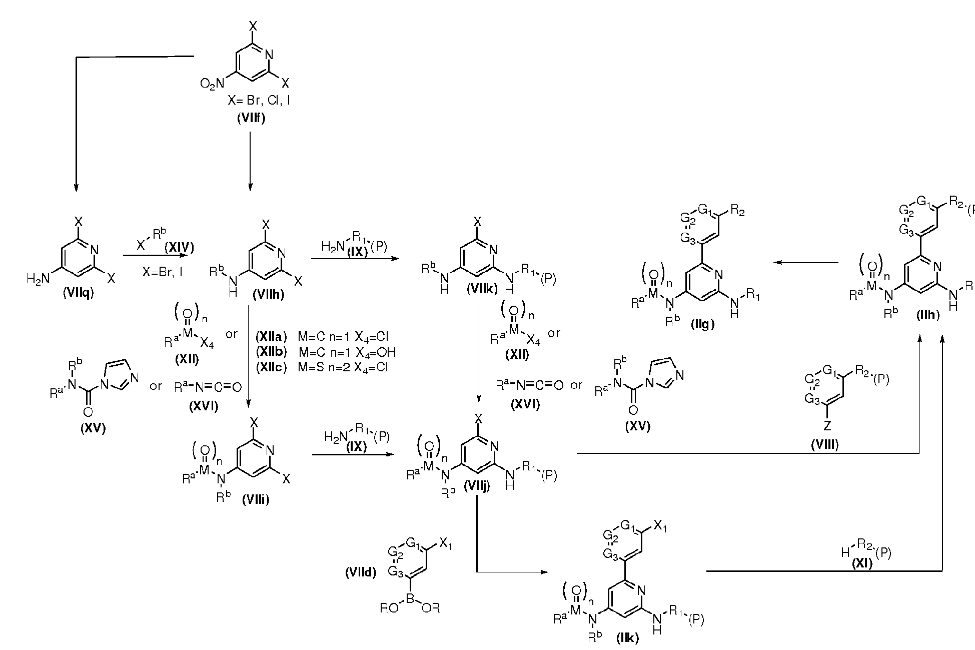

It is also possible to obtain compounds of formula (VIIi) and (Vllj) by reaction of amino derivatives of formula (Vllh) and (Vllk) with anhydrides in the presence of a base such as triethylamine.

The compounds of formula (Vllg) may be obtained by *N*-arylation or by nucheophilic addition of the haloderivative of formula (Vllf) with the corresponding amine of formula (IX), optionally protected (P) with a group such as BOC, following the conditions described for the synthesis of compounds of formula (IIb) synthesized from compounds of formula (IIa).

The compounds of formula (IIi) may be obtained by coupling of the haloderivative of formula (Vllg) with the corresponding compounds of formula (VIII) using Suzuki or Stille conditions described for the synthesis of compounds of formula (IIa).

The nitro group of the intermediate of formula (IIi) may be reduced with a reducing agent such as iron in a protic solvent such as ethanol at temperature from room temperature to the boiling point of the solvent to yield the aminoderivative of formula (IIj).

Reaction of the aminoderivative of formula (IIj) with an alkyl halide (XIV) in the presence of a base such as potassium carbonate using a solvent such as N,N'-dimethylformamide at room temperature or with the corresponding aldehydes in conditions of reductive amination using acetic acid in a solvent such as methanol, followed by addition of sodium cyanoborohydride, yields compounds of formula (IIp). This compounds (IIp) react with an acyl chloride (Xlla), an isocianate (XVI) or a *N*,*N'-*dialkyl-1*H*-imidazole-1-Carboxamide (XV) in the presence of a base such as triethylamine using a solvent such as tetrahydrofurane at a temperature from 0ºC to room temperature, or with an acid (Xllb) in the presence of coupling reagent such as *N-*[(dimethylamino)(3*H-*[1,2,3]triazolo[4,5-b]pyridin-3-yloxy)methylene]-*N-*methylmethanaminium hexafluorophosphate and a base such as *N*-ethyl-*N-*isopropylpropan-2-amine using a solvent such as dimethylformamide at a temperature from 0ºC to room temperature, or with a sulfonyl chloride (Xllc) in the presence of a base such as pyridine using a solvent such as tetrahydrofurane at a temperature from 0ºC to room temperature to yield compound (IIh).

Reaction of aminoderivative (IIj) with aldehydes or ketones under reductive amination conditions using reductive agents such as sodium cyanoborohydride or sodium triacetoxyborohydride provides compounds of general formula (IIq).

Additionally, compounds of formula (IIh) may be also obtained by another synthetic route. The nitro group of the intermediate of formula (Vllf) may be reduced using conditions described for the synthesis of compounds of formula (IIj) to yield the aminoderivative of formula (Vllq), which can be alkylated using the conditions described for the synthesis of compound of formula (IIp) to give compounds (VIIh).

Reaction of the aminoderivative of formula (Vllh) with an acyl chloride (Xlla), or with an acid (Xllb), or with a sulfonyl chloride (Xllc), or with an isocianate (XVI) or with a *N*,*N-*dialkyl-1 *H*-imidazole-1-Carboxamide (XV) using conditions described for the synthesis of compounds of formula (IIh) yields the derivative of formula (VIIi).

The intermediate of formula (Vllj) may be obtained by *N*-arylation or nucheophilic addition of the haloderivative of formula (VIIi) with the corresponding amine of formula (IX), optionally protected (P) with a group such as *tert-*butyl or BOC, using the conditions described for the synthesis of compounds of formula (IIb) synthesized from compounds of formula (IIa).

Additionally, compounds of formula (Vllj) may be also obtained by *N*-arylation or nucheophilic addition of the haloderivative of formula (Vllh) with the corresponding amine of formula (IX), optionally protected (P) with a group such as *tert-*butyl or BOC, using the conditions described for the synthesis of compounds of formula (IIb) synthesized from compounds of formula (IIa) to yield the derivative of formula (VIIk). Subsequent reaction of this aminoderivative of formula (Vllk) with an acyl chloride (Xlla), or with an acid (Xllb), or with a sulfonyl chloride (Xllc), or with an isocianate (XVI) or with a *N*,*N'*-dialkyl-1*H-*imidazole-1-Carboxamide (XV) using conditions described for the synthesis of compounds of formula (IIh) yield the derivative of formula (VIIj).

The intermediate of formula (IIh) may be also obtained by coupling of compounds of formula (Vllj) with the corresponding compounds of formula (VIII) using Suzuki or Stille conditions described for the synthesis of compounds of formula (IIa).

Additionally, compounds of formula (IIh) may be also obtained by coupling of compounds of formula (Vllj) with the corresponding compounds of formula (VIII) using Suzuki or Stille conditions described for the synthesis of compounds of formula (IIa) to yield the derivative of formula (Ilk). Subsequent nucleophilic substitution of a reactant of formula (XI), optionally protected (P) with a group such as BOC, over the haloderivative of formula (Ilk), using the conditions described for the synthesis of compounds of formula (IIb) synthesized from compounds of formula (IIe) (Figure 3) yields compounds of formula (IIh).

The deprotection of intermediates of formula (IIh) giving the target compounds (IIg) may be carried out using the conditions described for the synthesis of compounds of formula (IA) (Figure 1).

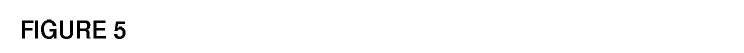

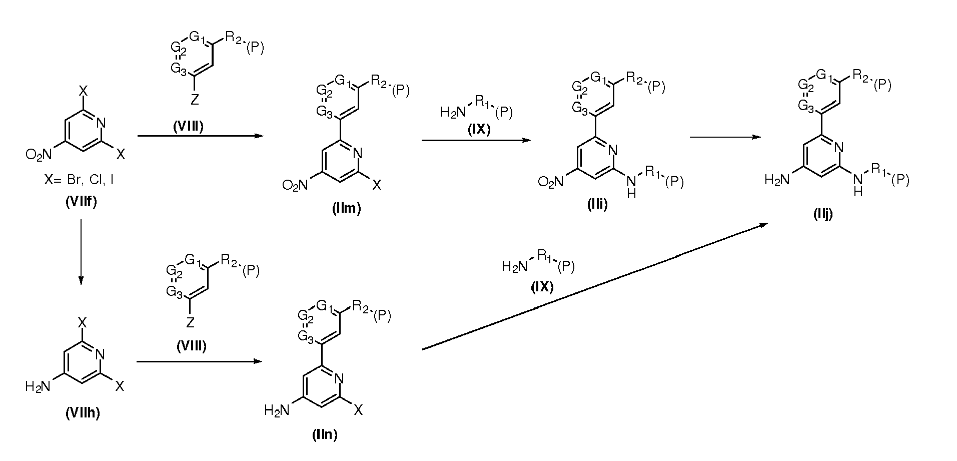

Compounds of formula (IIj) may be also obtained by coupling reaction of compounds of formula (Vllf) with the corresponding derivatives of formula (VIII) using Suzuki or Stille conditions described for the synthesis of compounds of formula (IIa) to yield the derivative of formula (IIm). Subsequent *N*-arylation or nucheophilic addition of the haloderivative of formula (IIm) with the corresponding amine of formula (IX), optionally protected (P) with a group such as BOC, following the conditions described for the synthesis of compounds of formula (IIb) synthesized from compounds of formula (IIa) (Figure 1) yields compounds of formula (IIi).

Additionally, compounds of formula (IIj) may be also obtained by coupling of compounds of formula (Vllh) with the corresponding compounds of formula (VIII) using Suzuki or Stille conditions described for the synthesis of compounds of formula (IIa) to yield the derivative of formula (IIn). Subsequent *N*-arylation or by nucheophilic addition of the haloderivative of formula (IIn) with the corresponding amine of formula (IX), optionally protected (P) with a group such as BOC, following the conditions described for the synthesis of compounds of formula (IIb) synthesized from compounds of formula (IIa) (Figure 1) yields compounds of formula (IIj).

According to a further feature of the present invention, compounds of general formula (II) wherein R³ is a -CONR^{b}R^{a} group or a -COOR^{a} group (IIo) may be prepared following the synthetic scheme illustrated in Figure 6.

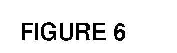

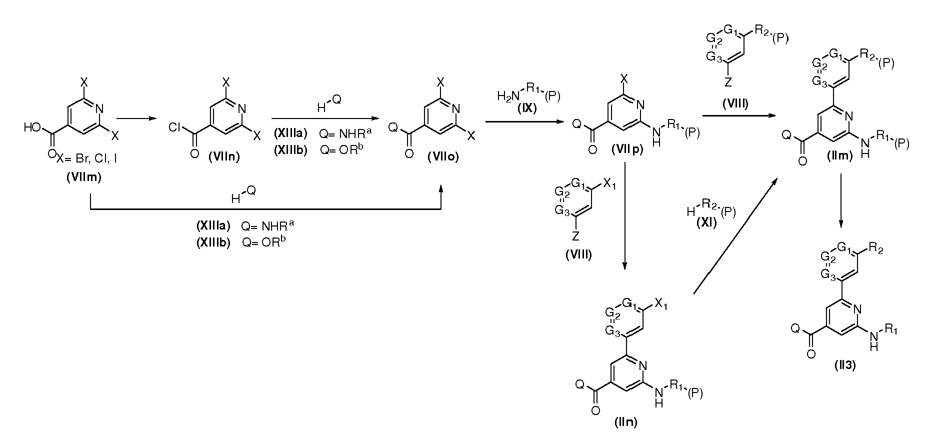

Reaction of the haloderivative of formula (VIIm) with thionyl dichloride or with phosphorous trichloride at a temperature from 80ºC to 110ºC yields the derivative of formula (VIIn).

Compounds of formula (Vllo) may be obtained by reaction of compounds of formula (VIIn) with the corresponding aminoderivative of formula (Xllla) o with the corresponding alcohol derivative of formula (Xlllb) in the presence of a base such as pyridine, triethylamine, ethyl(diisopropyl)amine or sodium hydride using a solvent such as tetrahydrofurane, dichloromethane or *N*,*N*-dimethylformamide at a temperature from 0ºC to room temperature.

Additionally, compounds of formula (Vllo) may be also obtained by coupling reaction of compounds of formula (VIIm) with the corresponding aminoderivative of formula (XIIIa) o with the corresponding alcohol derivative of formula (Xlllb) in the presence of a coupling reagent such as 3-{[(ethylimino)methylene]amino}-*N*,*N*-dimethylpropan-1-aminium chloride or *N*-[(dimethylamino)(3*H*-[1,2,3]triazolo[4,5-b]pyridin-3-yloxy)methylene]-*N*-methylmethanaminium hexafluorophosphate and a base such as *N*,*N*-dimethylpyridin-4-amine or *N*-ethyl-*N*-isopropylpropan-2-amine using a solvent such as dichloromethane, acetonitrile or *N*,*N'*-dimethylformamide at a temperature from 0ºC to room temperature.

Compounds of formula (Vllp) may be obtained by *N*-arylation or by nucheophilic addition of the haloderivative of formula (Vllo) with the corresponding amine of formula (IX), optionally protected (P) with a group such as BOC, following the conditions described for the synthesis of compounds of formula (IIb) synthesized from compounds of formula (IIa) (Figure 1).

Compounds of formula (IIm) may be obtained by coupling of compounds of formula (Vllp) with the corresponding compounds of formula (VIII) using Suzuki or Stille conditions described for the synthesis of compounds of formula (IIa).

Additionally, compounds of formula (IIm) may be also obtained by coupling of compounds of formula (Vllp) with the corresponding derivatives of formula (VIII) using Suzuki or Stille conditions described for the synthesis of compounds of formula (IIa) to yield compounds of formula (IIn). Subsequent nucleophilic substitution of a reactant of formula (XI), optionally protected (P) with a group such as BOC, over the haloderivative of formula (IIn), using the conditions described for the synthesis of compounds of formula (IIb) synthesized from compounds of formula (IIe) (Figure 3) yields compounds of formula (IIm).

The deprotection of intermediates of formula (IIm) giving the target compounds (II3) may be carried out using the conditions described for the synthesis of compounds of formula (IA) (Figure 1).

According to a further feature of the present invention, compounds of general formula (IB) may be prepared following the synthetic scheme illustrated in Figure 7.

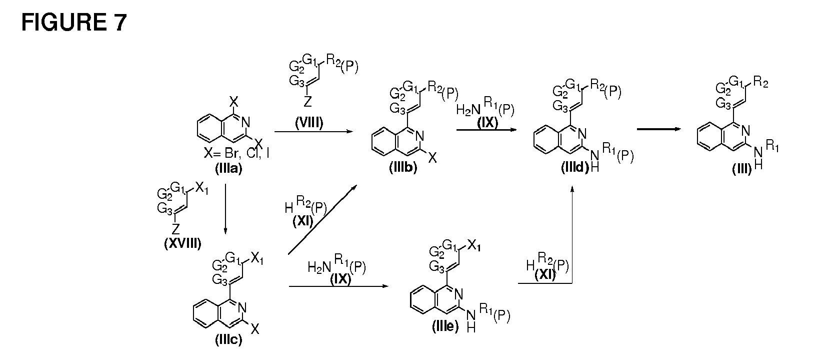

Compounds of formula (IIIb) may be obtained by coupling of compounds of formula (IIIa) with the corresponding compounds of formula (VIII) using Suzuki or Stille conditions described for the synthesis of compounds of formula (IIa).

Additionally, compounds of formula (IIIb) may be also obtained by coupling of compounds of formula (IIIa) with the corresponding compounds of formula (XVIII) using Suzuki or Stille conditions described for the synthesis of compounds of formula (IIa) to yield compounds of formula (IIIc). Subsequent nucleophilic substitution of a reactant of formula (XI), optionally protected (P) with a group such as BOC, over the haloderivative of formula (IIIc), using the conditions described for the synthesis of compounds of formula (IIb) synthesized from compounds of formula (IIe) (Figure 3) yields compounds of formula (IIIb).

Compounds of formula (IIId) may be obtained by *N*-arylation or by nucheophilic addition of the haloderivative of formula (IIIb) with the corresponding amine of formula (IX), optionally protected (P) with a group such as BOC, following the conditions described for the synthesis of compounds of formula (IIb) synthesized from compounds of formula (IIa) (Figure 1).

Additionally, compounds of formula (IIId) may be also obtained by *N*-arylation or by nucheophilic addition of the haloderivative of formula (IIIb) with the corresponding amine of formula (IX), optionally protected (P) with a group such as BOC, following the conditions described for the synthesis of compounds of formula (IIb) synthesized from compounds of formula (IIa) (Figure 1) to yield compounds of formula (IIIe). Subsequent nucleophilic substitution of a reactant of formula (XI), optionally protected (P) with a group such as BOC, over the haloderivative of formula (IIIe), using the conditions described for the synthesis of compounds of formula (IIb) synthesized from compounds of formula (IIe) (Figure 3) yields compounds of formula (IIId).

The deprotection of intermediates of formula (IIId) giving the target compounds (III) may be carried out using the conditions described for the synthesis of compounds of formula (IA) (Figure 1).

In the particular case where R₂ is a 4-piperidine compounds of formula (IB) can be prepared by reduction of compounds of formula (IIIb) wherein R₂ is a *tert-*butyl 5,6-dihydropyridine-1 (2H)-Carboxylate with hydrogen and a hydrogenation catalyst such as palladium on activated carbon in a solvent such as methanol and in a slightly acidic media at room temperature, followed by deprotection of intermediate giving the desired compound using the conditions described for the synthesis of compounds of formula (IA) (Figure 1).

According to a further feature of the present invention, compounds of general formula (IC) may be prepared following the synthetic scheme illustrated in Figure 8. 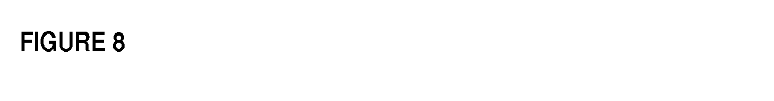 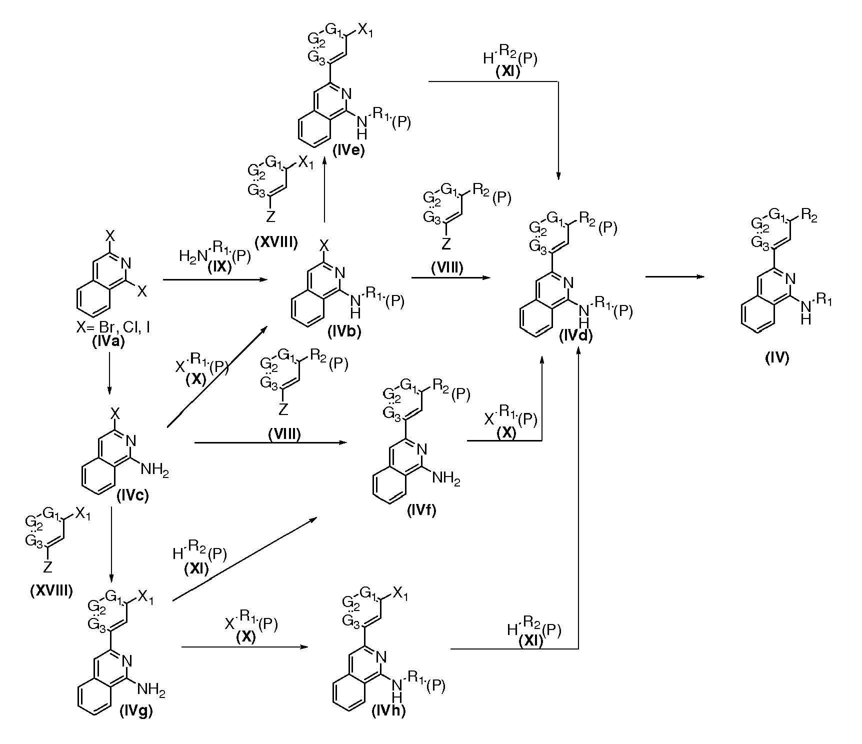 Compounds of formula (IVb) may be obtained by *N*-arylation or by nucheophilic addition of the haloderivative of formula (IVa) with the corresponding amine of formula (IX), optionally protected (P) with a group such as BOC, following the conditions described for the synthesis of compounds of formula (IIb) synthesized from compounds of formula (IIa) (Figure 1).

Additionally, compounds of formula (IVb) may be also obtained by reacting intermediates of formula (IVa) with ammonia in methanol to yield compounds of formula (VIIc). Subsequent nucheophilic addition of the amine of formula (Vllc) with the corresponding haloderivative of formula (X), optionally protected (P) with a group such as *tert-*butyl or BOC, following the conditions described for the synthesis of compounds of formula (IIb) synthesized from compounds of formula (IIa) (Figure 1) yields compounds of formula (IVb).

Compounds of formula (IVd) may be obtained by coupling of compounds of formula (IVb) with the corresponding compounds of formula (VIII) using Suzuki conditions described for the synthesis of compounds of formula (IIa).

Additionally, compounds of formula (IVd) may be also obtained by coupling of compounds of formula (IVb) with the corresponding derivatives of formula (XVIII) using Suzuki or Stille conditions described for the synthesis of compounds of formula (IIa) to yield compounds of formula (IVe). Subsequent nucleophilic substitution of a reactant of formula (XI), optionally protected (P) with a group such as BOC, over the haloderivative of formula (IVe), using the conditions described for the synthesis of compounds of formula (IIb) synthesized from compounds of formula (IIe) (Figure 3) yields compounds of formula (IVd).

Additionally, compounds of formula (IVd) may be also obtained by coupling of compounds of formula (IVc) with the corresponding compounds of formula (VIII) using Suzuki or Stille conditions described for the synthesis of compounds of formula (IIa) to yield compounds of formula (IVf). Subsequent nucheophilic addition of the amine of formula (Vllf) with the corresponding haloderivative of formula (X), optionally protected (P) with a group such as *tert*-butyl or BOC, following the conditions described for the synthesis of compounds of formula (IIb) synthesized from compounds of formula (IIa) (Figure 1) yields compounds of formula (IVd).

Additionally, compounds of formula (IVf) may be also obtained by coupling of compounds of formula (IVc) with the corresponding derivatives of formula (VIIId) using Suzuki or Stille conditions described for the synthesis of compounds of formula (IIa) to yield compounds of formula (IVg). Subsequent nucleophilic substitution of a reactant of formula (XI), optionally protected (P) with a group such as BOC, over the haloderivative of formula (IVg), using the conditions described for the synthesis of compounds of formula (IIb) synthesized from compounds of formula (IIe) (Figure 3) yields compounds of formula (IVf).

Additionally, compounds of formula (IVd) may be also obtained by nucheophilic addition of the amine of formula (Vllg) with the corresponding haloderivative of formula (X), optionally protected (P) with a group such as *tert*-butyl or BOC, following the conditions described for the synthesis of compounds of formula (IIb) synthesized from compounds of formula (IIa) (Figure 1) to yield compounds of formula (IVh). Subsequent nucleophilic substitution of a reactant of formula (XI), optionally protected (P) with a group such as BOC, over the haloderivative of formula (IVh), using the conditions described for the synthesis of compounds of formula (IIb) synthesized from compounds of formula (IIe) (Figure 3) yields compounds of formula (IVd).

The deprotection of intermediates of formula (IVd) giving the target compounds (IV) may be carried out using the conditions described for the synthesis of compounds of formula (IA) (Figure 1).

### EXAMPLES

**General.** Reagents, starting materials, and solvents were purchased from commercial suppliers and used as received. Concentration refers to evaporation under vacuum using a Büchi rotatory evaporator. Reaction products were purified, when necessary, by flash chromatography on silica gel (40-63 µm) with the solvent system indicated.

Spectroscopic data were recorded on a Varian Gemini 300 spectrometer. HPLC-MS were performed on a Gilson instrument equipped with a Gilson piston pump 321, a Gilson 864 vacuum degasser, a Gilson liquid handler 215, a Gilson 189 injection module, a Gilson Valvemate 7000, a 1/1000 splitter, a Gilson 307 make-up pump, a Gilson 170 diode array detector, and a Thermoquest Finnigan aQa detector.

### PREPARATION 1

### 2,4'-Bipyridin-6-amine

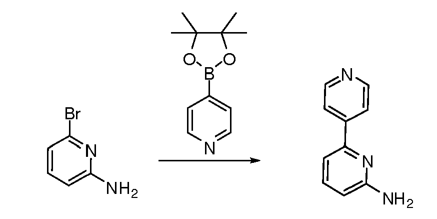

An oven dried resealable Schlenk tube was charged with 6-bromopyridin-2-amine (0.20 g, 1.15 mmol), 4-(4,4,5,5-tetramethyl-1,3,2-dioxaborolan-2-yl)pyridine (0.47 g, 2.30 mmol), sodium carbonate (2M, 2.30 mL, 4.60 mmol), ethanol (0.5 mL) and toluene (15 mL). The Schlenk tube was subjected to three cycles of evacuation-backfilling with argon, and 1,1'-bis(diphenylphosphino)ferrocene-palladium(II) dichloride dichloromethane complex (0.05 g, 0.06 mmol) was added. After three further cycles of evacuation-backfilling with argon, the Schlenk tube was capped and placed in an oil bath at 90 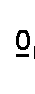 C. After 16h, the mixture was cooled and filtered. Ethyl acetate and water were added to the mixture and the organic layer was washed with satured aqueous sodium carbonate solution and brine, dried (Na₂SO₄) and evaporated to give the desired compound in quantitative yield. The crude material was used without further purification.

LRMS (m/z): 172 (M+1)⁺.

### PREPARATION 2

### N-(6-Bromopyridin-2-yl)-4-methylpyridin-2-amine

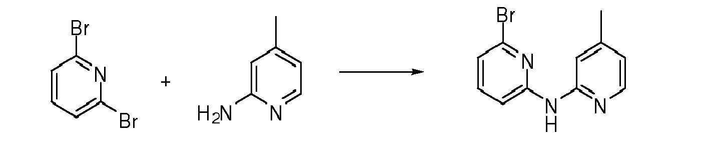

Potassium *tert-*butoxide (1.01 g, 9.00 mmol) was added to a stirred solution of 2,6-dibromopyridine (1.53 g, 6.33 mmol) and 4-methylpyridin-2-amine (0.49 g, 4.50 mmol) in benzene (15 mL) and the mixture was stirred at 80^{∘}C. After 30 minutes ethyl acetate and satured aqueous ammonium chloride solution were added to the mixture. The organic layer was washed with satured aqueous ammonium chloride solution and brine, dried (MgSO₄) and evaporated. Purification of the residue by flash chromatography (1:2 hexanes/ethyl acetate) gave the title compound (0.80 g, 67%) as a solid.

LRMS (m/z): 264/266 (M+1)⁺.

1 H NMR (500 MHz, CHLOROFORM-*d*) δ ppm 2.34 (s, 3 H) 6.72 (d, *J*=6.50 Hz, 1 H) 7.00 (d, *J*=9.00 Hz, 1 H) 7.07 (s, 1 H) 7.36 (br. s., 1 H) 7.44 (t, *J*=9.50 Hz, 1 H) 7.77 (d, *J*=9.50 Hz, 1 H) 8.13 (d, *J*=6.50 Hz, 1 H)

### PREPARATION 3

### (3-{2-[(tert-Butoxycarbonyl)amino]ethyl}phenyl)boronic acid

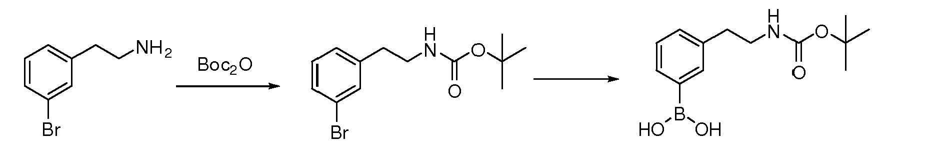

**a) *tert*-Butyl [2-(3-bromophenyl)ethyl]carbamate**
   Di-tert-butyl dicarbonate (0.30 g, 1.38 mmol) and triethylamine (0.44 mL, 3.13 mmol) were added to a stirred solution of [2-(3-bromophenyl)ethyl]amine (0.25 g, 1.25 mmol) in tetrahydrofuran (2 mL) and the mixture was stirred at room temperature. After 3 hours ethyl acetate and water were added to the mixture. The organic layer was washed with brine, dried (Na₂SO₄) and evaporated. Purification of the residue by flash chromatography (8:2 hexanes/ethyl acetate) gave the title compound (0.29 g, 77%) as a solid.
   LRMS (m/z): 300/302 (M+1)⁺.
   1 H NMR (400 MHz, CHLOROFORM-*d*) δ ppm 1.44 (s, 9 H) 2.75-2.80 (m, 2 H) 3.36-3.41 (m, 2 H) 4.56 (br. s., 1 H) 7.10-7.20 (m, 2 H) 7.35 - 7.40 (m, 2 H)
**b) (3-{2-(tert-Butoxycarbonyl)amino]ethyl}phenyl)boronic acid**
   An oven dried resealable Schlenk tube was charged with *tert*-butyl [2-(3-bromophenyl)ethyl]carbamate (preparation 3a, 0.29 g, 0.96 mmol), bis(pinacolato)diboron (0.27 g, 1.05 mmol), potassium acetate (0.28 g, 2.88 mmol) and dioxane (4 mL). The Schlenk tube was subjected to three cycles of evacuation-backfilling with argon, and 1,1'-bis(diphenylphosphino)ferrocene-palladium(II) dichloride dichloromethane complex (0.02 g, 0.03 mmol) and 1,1'-bis(diphenylphosphino)ferrocene (0.02 g, 0.03 mmol) were added. After three further cycles of evacuation-backfilling with argon, the Schlenk tube was capped and placed in an oil bath at 80 C. 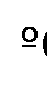 After 4h, the mixture was cooled and ethyl acetate and satured aqueous trimethylammonium chloride solution was added. The organic layer was washed with satured aqueous trimethylammonium chloride solution, dried (MgSO₄) and evaporated to give the desired compound in quantitative yield. The crude material was used without further purification.
   LRMS (m/z): 264 (M-1)⁻.

### PREPARATION 4

### N-(6-Bromopyridin-2-yl)pyrazin-2-amine

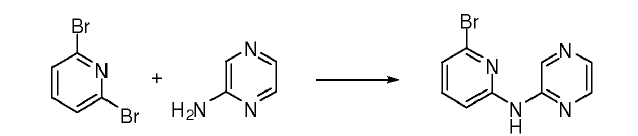

An oven dried resealable Schlenk tube was charged with 2,6-dibromopyridine (0.21 g, 0.88 mmol), pyrazin-2-amine (0.10 g, 1.05 mmol), cesium carbonate (1.14 g, 3.50 mmol) and 1,2-dimethoxyethane (6 mL). The Schlenk tube was subjected to three cycles of evacuation-backfilling with argon, and tris(dibenzylideneacetone)dipalladium(0) (0.02 g, 0.02 mmol) and 4,5-bis(diphenylphosphino)-9,9-dimethylxanthene (0.02 g, 0.04 mmol) were added. After three further cycles of evacuation-backfilling with argon, the Schlenk tube was capped and placed in an oil bath at 95 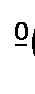 C. After 4h, the mixture was cooled and the solvent was removed in vacuo. The residue was purified by flash chromatography to give the title compound (0.18 g, 59%) as a solid.

LRMS (m/z): 251/253 (M+1)⁺.

1 H NMR (400 MHz, CHLOROFORM-*d*) δ ppm 7.10 (d, *J*=7.62 Hz, 1 H) 7.50 (t, *J*=7.62 Hz, 1 H) 7.62 (br. s., 1 H) 7.74 (d, *J*=8.41 Hz, 1 H) 8.15 (s, 1 H) 8.20 (s, 1 H) 8.74 (s, 1 H)

### PREPARATION 5

### tert-Butyl {2-[3-(6-aminopyridin-2-yl)phenyl]ethyllcarbamate

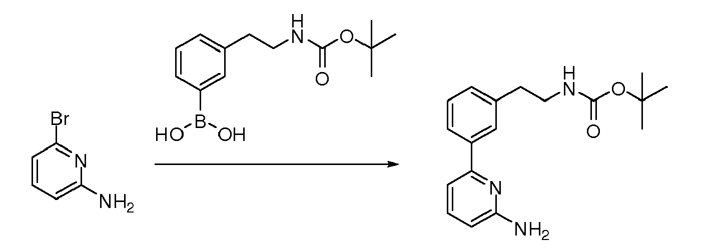

Obtained as a solid (54%) from 6-bromopyridin-2-amine and (3-{2-[(*tert-*butoxycarbonyl)amino]ethyl}phenyl)boronic acid (preparation 3b) following the experimental procedure as described in preparation 1 followed by purification by flash chromatography.

LRMS (m/z): 314 (M+1)⁺.

1 H NMR (400 MHz, CHLOROFORM-*d*) δ ppm 1.43 (s, 9 H) 2.02 (br. s., 1 H) 2.87 (t, *J*=6.80 Hz, 2 H) 3.40-3.48 (m, 2 H) 4.56 (br. s., 2 H) 6.46 (d, *J*=8.00 Hz, 1 H) 7.08 (d, *J*=7.60 Hz, 1 H) 7.21 (d, *J*=7.60 Hz, 1 H) 7.37 (t, *J*=7.60 Hz, 1 H) 7.50 (t, *J*=7.60 Hz, 1 H) 7.76 (d, *J*=8.00 Hz, 1 H) 7.78 (br. s., 1 H)

### PREPARATION 6

### 3-(6-Aminopyridin-2-yl)benzonitrile

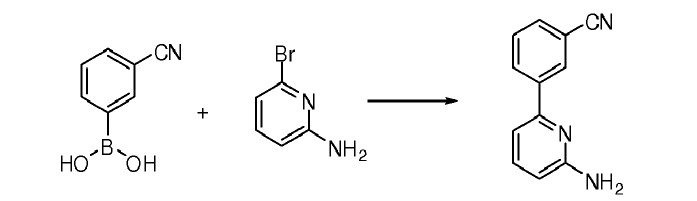

Obtained as a solid (63%) from (3-Cyanophenyl)boronic acid and 6-bromopyridin-2-amine following the experimental procedure as described in preparation 1 followed by purification by flash chromatography.

LRMS (m/z): 196 (M+1)⁺.

1 H NMR (400 MHz, CHLOROFORM-*d*) δ ppm 4.59 (br. s., 2 H) 6.52 (d, *J*=7.60 Hz, 1 H) 7.09 (d, *J*=8.00 Hz, 1 H) 7.51 - 7.56 (m, 2 H) 7.65 (d, *J*=8.80 Hz, 1 H) 8.17 (d, *J*=10.80 Hz, 1 H) 8.28 (s, 1 H)

### PREPARATION 7

### 2'-Fluoro-N-(4-methylpyridin-2-yl)-2,4'-bipyridin-6-amine

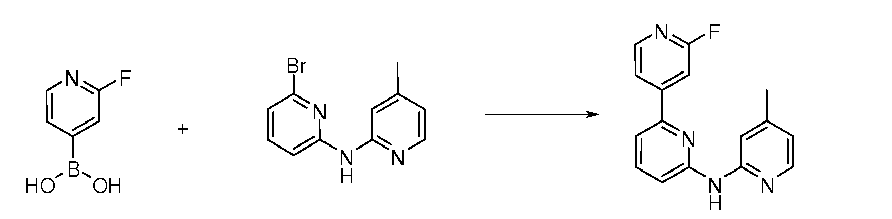

Obtained as a solid (86%) from (2-fluoropyridin-4-yl)boronic acid and *N*-(6-bromopyridin-2-yl)-4-methylpyridin-2-amine (preparation 2) following the experimental procedure as described in preparation 1 followed by purification by flash chromatography.

LRMS (m/z): 281 (M+1)⁺.

1 H NMR (400 MHz, CHLOROFORM-*d*) δ ppm 2.38 (s, 3 H) 6.76 (d, *J*=4.80 Hz, 1 H) 7.35 (br. s., 2 H) 7.49 (s, 1 H) 7.58 (s, 1 H) 7.63 (d, *J*=4.00 Hz, 1 H) 7.72 (d, *J*=7.60 Hz, 1 H) 7.75 - 7.77 (m, 1 H) 8.16 (d, *J*=5.20 Hz, 1 H) 8.31 (d, *J*=5.60 Hz, 1 H)

### PREPARATION 8

### 2'-Fluoro-N-pyrazin-2-yl-2,4'-bipyridin-6-amine

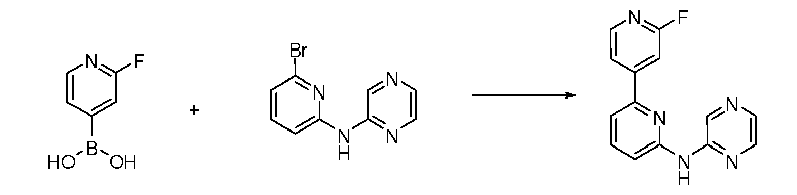

Obtained as a solid (42%) from (2-fluoropyridin-4-yl)boronic acid and *N*-(6-bromopyridin-2-yl)pyrazin-2-amine (preparation 4) following the experimental procedure as described in preparation 1 followed by purification by flash chromatography.

LRMS (m/z): 268 (M+1)⁺.

1 H NMR (400 MHz, CHLOROFORM-*d*) δ ppm 7.45 (d, *J*=6.80 Hz, 1 H) 7.45 (s, 1 H) 7.53 (s, 1 H) 7.65 (d, *J*=8.40 Hz, 1 H) 7.77 - 7.80 (m, 2 H) 8.18 (d, *J*=2.40 Hz, 1 H) 8.22 - 8.23 (m, 1 H) 8.33 (d, *J*=4.80 Hz, 1 H) 9.07 (s, 1 H)

### PREPARATION 9

### tert-Butyl 4-[3-(4,4,5-trimethyl-1,3,2-dioxaborolan-2-yl)phenyl]-3,6-dihydropyridine-1 (2H)-Carboxylate

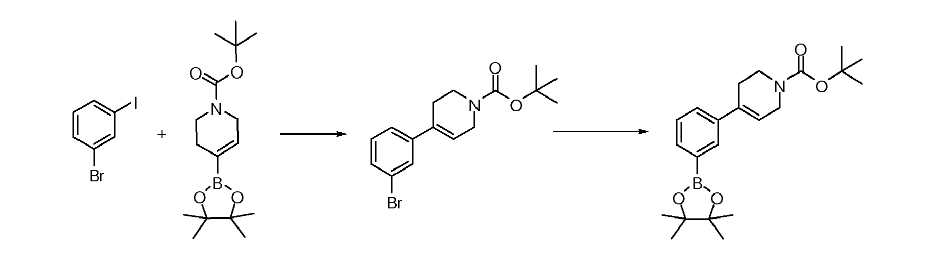

**a) *tert*-Butyl 4-(3-bromophenyl)-3,6-dihydropyridine-1(2*H*)-Carboxylate**
   Obtained as a yellow oil (81 %) from 1-bromo-3-iodobenzene and *tert-*butyl 4-(4,4,5,5-tetramethyl-1,3,2-dioxaborolan-2-yl)-3,6-dihydropyridine-1(2*H*)-Carboxylate following the experimental procedure as described in preparation 1.
   1 H NMR (300 MHz, CHLOROFORM-*d* δ ppm 1.49 (s, 9 H) 2.49 (br. s., 2 H) 3.63 (t, *J*=5.77 Hz, 2 H) 4.08 (d, *J*=2.75 Hz, 2 H) 6.05 (br. s., 1 H) 7.21 (t, *J*=7.69 Hz, 1 H) 7.27 - 7.31 (m, 1 H) 7.36 - 7.40 (m, 1 H) 7.51 (t, *J*=1.79 Hz, 1 H)
**b) *tert*-Butyl 4-[3-(4,4,5-trimethyl-1,3,2-dioxaborolan-2-yl)phenyl]-3,6-dihydropyridine-1 (2*H*)-Carboxylate**
   Obtained as a oil (59%) from *tert*-butyl 4-(3-bromophenyl)-3,6-dihydropyridine-1 (2*H*)-Carboxylate (preparation 9a) following the experimental procedure as described in preparation 3b.
   1 H NMR (300 MHz, CHLOROFORM-*d*) δ ppm 1.35 (s, 12 H) 1.49 (s, 9 H) 2.55 (br. s., 2 H) 3.63 (t, *J*=5.49 Hz, 2 H) 4.06 (br. s., 2 H) 6.06 (br. s., 1 H) 7.34 (t, *J*=7.55 Hz, 1 H) 7.43 - 7.49 (m, 1 H) 7.70 (d, *J*=7.14 Hz, 1 H) 7.82 (s, 1 H)

### PREPARATION 10

### 4-tert-Butyl-2-Chloropyridine

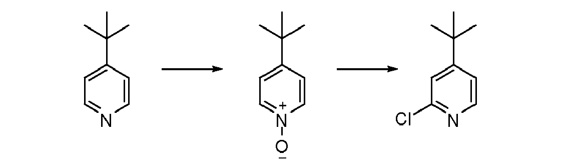

**a) 4-*tert*-Butylpyridine 1-oxide**
   Hydrogen peroxide (30% in water, 3.24 mL, 37 mmol) was added to a stirred solution of 4-*tert*-butylpyridine (5.00 g, 37 mmol) in glacial acetic acid (30 mL) and the mixture was stirred at 120 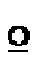 C. After 14 hours, the reaction mixture was concentrated in vacuo and the resulting oil was used without further purification.
   LRMS (m/z): 152 (M+1)⁺.
**b) 4-tert-Butyl-2-Chloropyridine**
   Phosphorus(V) oxychloride (8 mL) was added carefully to 4-*tert-*butylpyridine 1-oxide (preparation 10a, 2 g, 13.22 mmol), the reaction mixture was heated under reflux. After 16 hours, the reaction mixture was slow cooled, and the de excess of phosphorus(V) oxychloride was removed by distillation. Ethyl acetate and satured aqueous sodium carbonate solution were added to the crude. The organic layer was washed with brine, dried (Na₂SO₄), concentrated in vacuo and the resulting oil was used without further purification.
   LRMS (m/z): 170/172 (M+1)⁺.

### PREPARATION 11

### 2'-Fluoro-2,4'-bipyridin-6-amine

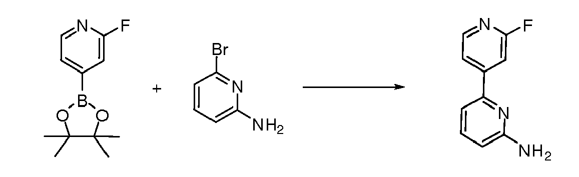

Obtained as a solid (63%) from 2-fluoro-4-(4,4,5,5-tetramethyl-1,3,2-dioxaborolan-2-yl)pyridine and 6-bromopyridin-2-amine following the experimental procedure as described in preparation 1 followed by purification by flash chromatography.

LRMS (m/z): 190 (M+1)⁺.

1H NMR (500 MHz, CHLOROFORM-*d*) δ ppm 4.58 (br. s., 2 H) 6.58 (d, *J*=10.50 Hz, 1 H) 7.16 (d, *J*=9.00 Hz, 1 H) 7.51 (s, 1 H) 7.56 (d, *J*=10.00 Hz, 1 H) 7.69 - 7.72 (m, 1 H) 8.26 (d, *J*=6.50 Hz, 1 H)

### PREPARATION 12

### tert-Butyl {1-[3-(4,4,5,5-tetramethyl-1,3,2-dioxaborolan-2-yl)phenyl]piperidin-4-yl}carbamate

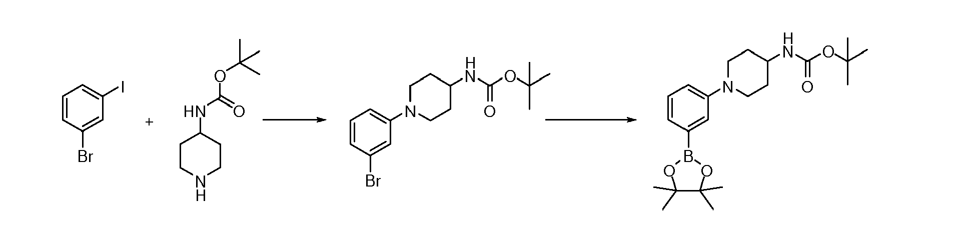

**a) *tert*-Butyl [1-(3-bromophenyl)piperidin-4-yl]carbamate**
   An oven dried resealable Schlenk tube was charged with 1-bromo-3-iodobenzene (0.06 ml, 0.50 mmol), *tert*-butyl piperidin-4-ylcarbamate (0.10 g, 0.50 mmol), sodium *tert-*butoxide (0.07 g, 1.40 mmol) and toluene (1 mL). The Schlenk tube was subjected to three cycles of evacuation-backfilling with argon, and tris(dibenzylideneacetone)dipalladium(0) (0.01 g, 0.02 mmol) and (±)-2,2'-bis(diphenylphosphino)-1,1'-binaphthalene (0.01 g, 0.04 mmol) were added. After three further cycles of evacuation-backfilling with argon, the Schlenk tube was capped and placed in an oil bath at 100 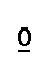 C. After 16h, the mixture was cooled and the solvent was removed in vacuo. The residue was purified by flash chromatography to give the title compound (0.03 g, 14%) as a solid.
   LRMS (m/z): 355/357 (M+1)⁺.
**b) *tert*-Butyl {1-[3-(4,4,5,5-tetramethyl-1,3,2-dioxaborolan-2-yl)phenyl]piperidin-4-yl}carbamate** Obtained as a oil from *tert-*butyl [1-(3-bromophenyl)piperidin-4-yl]carbamate (preparation 12a) following the experimental procedure as described in preparation 3b.
   LRMS (m/z): 403 (M+1)⁺.

### PREPARATION 13

### tert-Butyl 4-[3-(4,4,5,5-tetramethyl-1,3,2-dioxaborolan-2-yl)phenyl]piperazine-1-Carboxylate

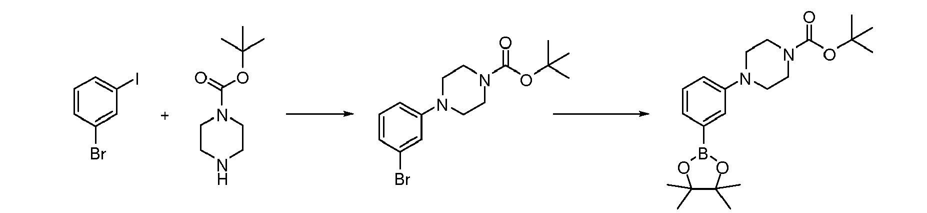

**a) *tert*-Butyl 4-(3-bromophenyl)piperazine-1-Carboxylate**
   Obtained as a solid (53%) from 1-bromo-3-iodobenzene and *tert*-butyl piperazine-1-Carboxylate following the experimental procedure as described in preparation 12a followed by purification by flash chromatography (100% hexanes to 4:1 hexanes/ethyl acetate).
   LRMS (m/z): 341/343 (M+1)⁺.
**b) *tert*-Butyl 4-[3-(4,4,5,5-tetramethyl-1,3,2-dioxaborolan-2-yl)phenyl]piperazine-**1-Carboxylate
   Obtained as a oil from *tert-*butyl 4-(3-bromophenyl)piperazine-l-Carboxylate (preparation 13a) following the experimental procedure as described in preparation 3b.
   LRMS (m/z): 389 (M+1)⁺.

### PREPARATION 14

### 2-Chloro-4-(2-methoxyethoxy)pyridine

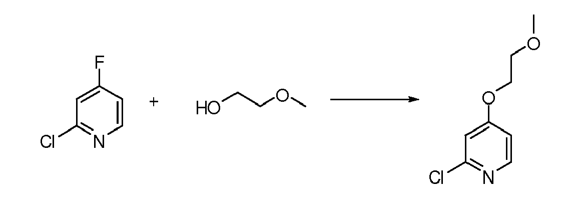

2-Chloro-4-fluoropyridine (0.11 mL, 1.05 mmol) was added to a stirred solution of 2-methoxyethanol (0.08 mL, 1.00 mmol) and potassium *tert-*butoxide (0.34 g, 3.00 mmol) in tetrahydrofurane (2.5 mL) at 0 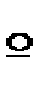 C and the mixture was stirred at room temperature. After 1 hour, ethyl acetate and water were added to the mixture. The organic layer was washed with brine, dried (Na₂SO₄) and evaporated to give the desired compound (0.15 g, 80%).
LRMS (m/z): 188/190 (M+1)⁺.

### PREPARATION 15

### N-(6-Bromopyridin-2-yl)-4-(trifluoromethyl)pyridin-2-amine

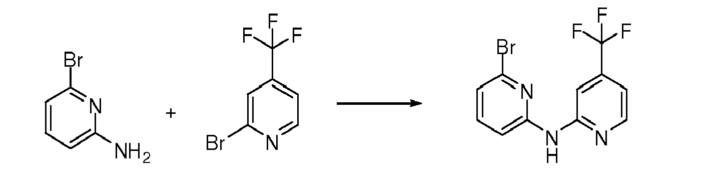

Obtained (56%) from 6-bromopyridin-2-amine and 2-bromo-4-(trifluoromethyl)pyridine following the experimental procedure as described in preparation 4 using dioxane as a solvent, followed by purification of the crude product by flash chromatography (1:1 hexanes/ethyl acetate).
LRMS (m/z): 318/320 (M+1)⁺.

### PREPARATION 16

### tert-Butyl {2-[3-(6-bromopyridin-2-yl)phenyl]ethyl}carbamate

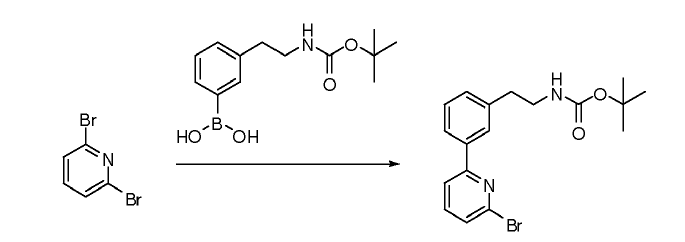

Obtained as a solid (39%) from 2,6-dibromopyridine and (3-12-[(*tert-*butoxycarbonyl)amino]ethyl}phenyl)boronic acid (preparation 3b) following the experimental procedure as described in preparation 1 followed by purification by flash chromatography.
LRMS (m/z): 377/379 (M+1)⁺.

### PREPARATION 17

### 6-Bromo-N-(1-tert-butyl-5-methyl-1H-pyrazol-3-yl)pyridin-2-amine

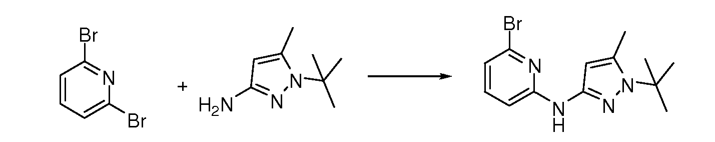

Obtained as a solid (55%) from 2,6-dibromopyridine and 1-*tert-*butyl-5-methyl-1 *H* pyrazol-3-amine following the experimental procedure as described in preparation 4 followed by purification by flash chromatography (7:93 hexanes/ethyl acetate to 2:3 hexanes/ethyl acetate).
LRMS (m/z): 309/311 (M+1)⁺

### PREPARATION 18

### 6-Bromo-2'-fluoro-2,4'-bipyridine

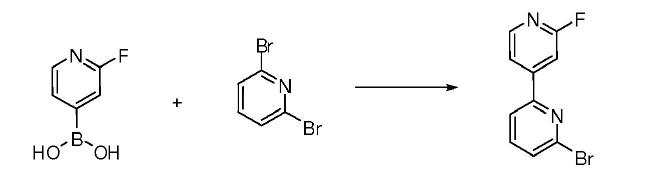

Obtained (59%) from (2-fluoropyridin-4-yl)boronic acid and 2,6-dibromopyridine following the experimental procedure as described in preparation 1 followed by purification by flash chromatography.
LRMS (m/z): 253/255 (M+1)⁺

### PREPARATION 19

### tert-Butyl 3-amino-5-methyl-1 H-pyrazole-1-Carboxylate

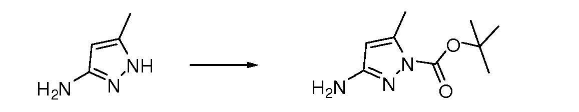

Sodium hydride (60% dispersion in mineral oil, 0.81 g, 33.96 mmol) was added slowly to a stirred suspension of 5-methyl-1*H*-pyrazol-3-amine (3.00 g, 30.90 mmol) in tetrahydrofuran (150 mL) at 0 °C. The mixture was stirred for 30 minutes, then di-tert-butyl dicarbonate (7.40 g, 33.96 mmol) was added to the reaction. The mixture was stirred and warmed to room temperature. After 2 hours, saturated aqueous sodium hydrogencarbonate solution was added to the reaction and the mixture was extracted with chloroform. The organic layer was washed with brine, dried (Na₂SO₄) and evaporated. Followed by purification of the crude product by flash chromatography (2.44 g, 40%).
LRMS (m/z): 198 (M+1)⁺.
1 H NMR (400 MHz, METHANOL-*d*₄) δ ppm 1.61 (s, 9 H) 2.10 (s, 3 H) 4.85 (s, 2 H) 5.24 (s, 1 H)

### PREPARATION 20

### 3-Bromoisoquinolin-1-amine

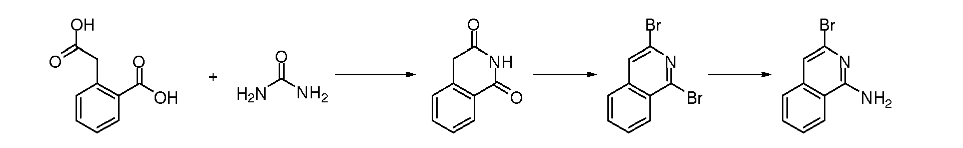

**a) Isoquinoline-1,3(2*H*,4*H*)-dione**
   A solution of 2-(carboxymethyl)benzoic acid (1.00 g, 5.55 mmol) and urea (0.37 g, 6.10 mmol) in ethylene glycol anhydrous (20 mL) was heated in a sealed tube at 175 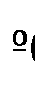 C with stirring. After stirring overnight the mixture was cooled, poured onto water and 1 N aqueous hydrochloric acid was added until the pH was 4-5. The aqueous layer was extracted with ethyl acetate, dried (Na₂SO₄) and evaporated. Methanol and few drops of hexanes were added to the crude and the precipitate was filtered, washed whith hexanes and dried to give the desired compound (0.43 g, 47%) as a solid.
   LRMS (m/z): 162 (M+1)⁺.
**b) 1,3-Dibromoisoquinoline**
   A mixture of isoquinoline-1,3(2*H*,4*H*)-dione (preparation 20a, 0.65 g, 4.02 mmol), phosphorous tribromide (1.51 mL, 16.05 mmol) and phosphoric tribromide (1.04 g, 3.61 mmol) was stirred at 180 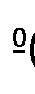 C. After 2 hours, the mixture was cooled, poured onto ice-water and 2N aqueous potassium hydroxide was added until the pH was 8-9. The aqueous layer was extracted with toluene, and the organic layer was filtered through neutral alumina and the filtrate was evaporated to give the title compound (0.58 g, 56%) as a white solid.
   LRMS (m/z): 286/288/290 (M+1)⁺.
   1 H NMR (400 MHz, CHLOROFORM-*d*) δ ppm 7.67 - 7.76 (m, 3 H) 7.84 (s, 1 H) 8.24 (d, *J*=8.40 Hz, 1 H)
**c) 3-Bromoisoquinolin-1-amine**
   A solution of 1,3-dibromoisoquinoline (preparation 20b, 0.10 g, 0.35 mmol) and a
   solution of 7M ammonium hydroxide in methanol (1.5 mL) was heated in a sealed tube at 150 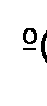 C with stirring. After heating overnight the mixture was cooled and water was added. The aqueous layer was extracted with diethyl ether, dried (Na₂SO₄) and evaporated to give the desired compound (0.77 g, 98%) as a yellow solid.
   LRMS (m/z): 223/225 (M+1)⁺.

### PREPARATION 21

### {2-[4-(tert-Butoxycarbonyl)-2-oxopiperazin-1-yl]pyridin-4-yl}boronic acid

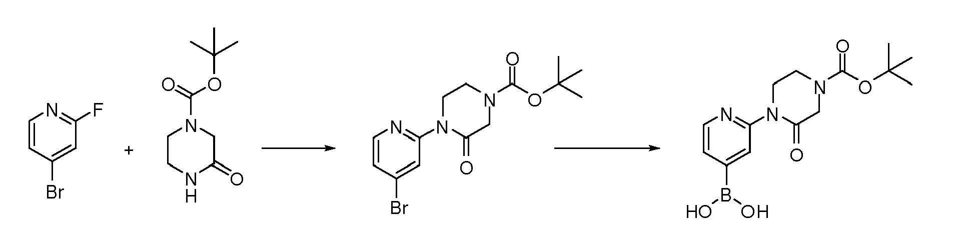

**a) *tert*-Butyl 4-(4-bromopyridin-2-yl)-3-oxopiperazine-1-Carboxylate**
   A suspension of 4-bromo-2-fluoropyridine (0.44 g, 2.50 mmol), *tert-*butyl 3-oxopiperazine-1-Carboxylate (0.75 g, 3.75 mmol) and potassium *tert*-butoxide (0.42 g, 3.75 mmol) in toluene (6 mL) was heated at 110 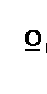 C. After stirring for 3 hours the mixture was cooled and the solvent was evaporated. Purification of the residue by flash chromatography (9:1 hexanes/ethyl acetate to 3:7 hexanes/ethyl acetate) gave the title compound (0.89 g, 56%).
   LRMS (m/z): 356/358 (M+1)⁺.
**b) {2-[4-(*tert*-Butoxycarbonyl)-2-oxopiperazin-1-yl]pyridin-4-yl}boronic acid**
   Obtained as a oil from *tert-*butyl \4-(4-bromopyridin-2-yl)-3-oxopiperazine-1-Carboxylate (preparation 21 a) following the experimental procedure as described in preparation 3b.
   LRMS (m/z): 322 (M+1)⁺.

### PREPARATION 22

### 2,6-Dibromo-4-phenylpyridine

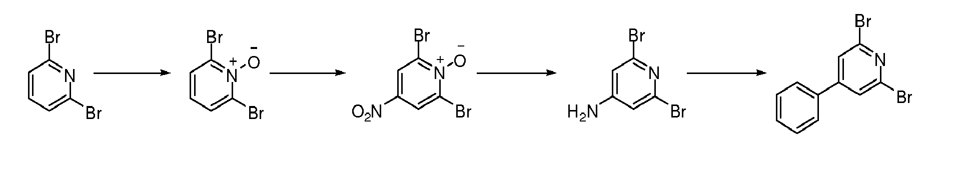

**a) 2,6-Dibromopyridine 1-oxide**
   Hydrogen peroxide (30% in water, 1 mL) was added dropwise to a stirred solution of 2,6-dibromopyridine (1.00 g, 4.22 mmol) in trifluoroacetic acid (5 mL) at 40 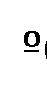 C, and the mixture was stirred at 95 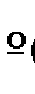 C. After 4 hours, the reaction mixture was cooled and poured onto water (25 mL). The resultant precipitate was filtered and the solid was washed with water to give the title compound (0.96 g, 90%) as an off-white solid.
   LRMS (m/z): 252/254/256 (M+1)⁺.
**b) 2,6-Dibromo-4-nitropyridine 1-oxide**
   2,6-Dibromopyridine 1-oxide (preparation 22a, 1.85 g, 7.31 mmol) was added slowly to a stirred solution of concentrated sulfuric acid (7 mL) and fuming nitric acid (3 mL) at 0 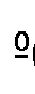 C, and the mixture was stirred at 60 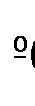 C. After overnight, the reaction mixture was cooled and poured onto ice-water. The resultant precipitate was filtered and the solid was washed with water to give the title compound (1.68 g, 77%) as a yellow solid.
   LRMS (m/z): 297/299/301 (M+1)⁺.
**c) 2,6-Dibromopyridin-4-amine**
   Iron (1.26 g, 22.56 mmol) was added slowly to a stirred solution of 2,6-dibromo-4-nitropyridine 1-oxide (preparation 22b, 1.68 g, 5.64 mmol) in acetic acid (16 mL) at 0 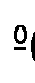 C, and the mixture was stirred at room temperature. After 1 hour, water and ethyl acetate were added to the reaction mixture. The organic layer was washed with water, satured aqueous potassium carbonate solution and brine, and dried (MgSO₄ and evaporated to give the title compound (1.35 g, 96%) as a white solid.
   LRMS (m/z): 251/253/255 (M+1)⁺.
   1 H NMR (400 MHz, CHLOROFORM-*d*) δ ppm 4.33 (br. s., 2 H) 6.68 (s, 2 H)
**d) 2,6-Dibromo-4-phenylpyridine**
   3-Methylbutyl nitrite (0.50 mL, 3.57 mmol) was added dropwise to a stirred solution of 2,6-dibromopyridin-4-amine (preparation 22c, 0.50 g, 1.98 mmol) in benzene (24 mL) and trifluoroacetic acid (4 mL) at room temperature, after 20 minutes the mixture was heated at 85 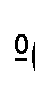 C. After 3 hours, water and ethyl acetate were added to the reaction mixture. The organic layer was washed with water and 5% aqueous sodium carbonate solution, dried (MgSO₄) and evaporated. Purification of the residue by flash chromatography (100% hexanes to 1:1 hexanes/ethyl acetate) gave the title compound (0.33 g, 53%) as a solid.
   1 H NMR (400 MHz, CHLOROFORM-*d*) δ ppm 7.49 - 7.51 (m, 3 H) 7.57 - 7.59 (m, 2 H) 7.66 (s, 2 H)

### PREPARATION 23

### N-(2,6-Dibromopyridin-4-yl)benzamide

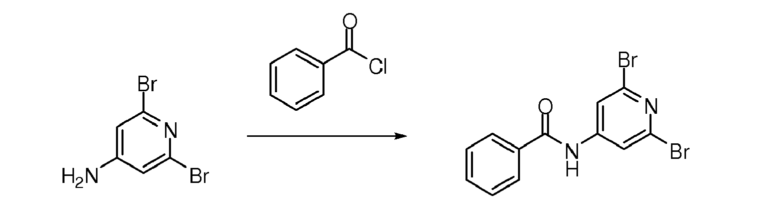

A solution of 2,6-dibromopyridin-4-amine (0.15 g, 0.59 mmol), benzoyl chloride (0.21 mL, 1.78 mmol) and pyridine (0.14 mL, 1.73 mmol) in dichloromethane (3.7 mL) was stirred at room temperature. After overnight, water and dichloromethare were added to the mixture, the organic layer washed with water, dried (Na₂SO₄) and evaporated. Purification of the residue by flash chromatography (100% hexanes to 1:1 hexanes/ethyl acetate) gave the title compound (0.21 g, 98%) as a white solid.
LRMS (m/z): 355/357/359 (M+1)⁺.

### PREPARATION 24

### {2-[4-(tert-Butoxycarbonyl)piperazin-1-yl]pyridin-4-yl}boronic acid

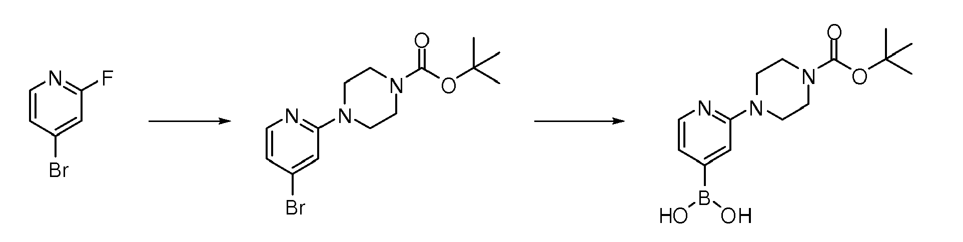

**a) *tert*-Butyl 4-(4-bromopyridin-2-yl)piperazine-1-Carboxylate**
   A suspension of 4-bromo-2-fluoropyridine (1.55 g, 8.81 mmol), *tert-*butyl piperazine-1-Carboxylate (2.46 g, 13.22 mmol) and potassium carbonate (3.65 g, 26.41 mmol) in dimethylsulphoxide (10 mL) was heated in a sealed tube at 100 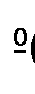 C with stirring. After stirring overnight the mixture was cooled and ethyl acetate and water were added. The organic layer was washed with brine, dried (MgSO₄) and evaporated. Purification of the residue by flash chromatography (100% hexanes to 1:4 hexanes /ethyl acetate) gave the title compound (2.33 g, 78%) as a white solid.
   LRMS (m/z): 342/344 (M+1)⁺.
**b) {2-[4-(*tert*-Butoxycarbonyl)piperazin-1-yl]pyridin-4-yl}boronic acid**
   Obtained as a oil from *tert*-butyl 4-(4-bromopyridin-2-yl)piperazine-1-Carboxylate (preparation 24a) following the experimental procedure as described in preparation 3b, heating at 80 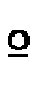 C for 46 hours, in quantitative yield.
   LRMS (m/z): 308 (M+1)⁺.

### PREPARATION 25

### tert-Butyl (1S,4S)-5-[4-(4,4,5,5-tetramethyl-1,3,2-dioxaborolan-2-yl)pyridin-2-yl]-2,5-diazabicyclo[2.2.1]heptane-2-Carboxylate

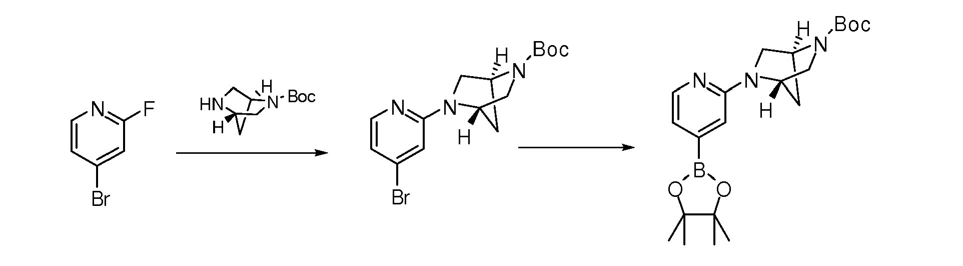

**a) *tert*-Butyl (1*S*,4*S*)-5-(4-bromopyridin-2-yl)-2,5-diazabicyclo[2.2.1]heptane-2-Carboxylate**
   Obtained (72%) from 4-bromo-2-fluoropyridine and *tert*-butyl (1*S*,4*S*)-2,5-diazabicyclo[2.2.1]heptane-2-Carboxylate following the experimental procedure as described in preparation 24a followed by purification by flash chromatography.
   LRMS (m/z): 354/356 (M+1)⁺.
**b) *tert*-Butyl (1*S*,4*S*)-5-[4-(4,4,5,5-tetramethyl-1,3,2-dioxaborolan-2-yl)pyridin-2-yl]-2,5-diazabicyclo[2.2.1]heptane-2-Carboxylate**
   Obtained from *tert-*butyl (1*S*,4*S*)-5-(4-bromopyridin-2-yl)-2,5-diazabicyclo[2.2.1]heptane-2-Carboxylate (preparation 25a) following the experimental procedure as described in preparation 3b.
   LRMS (m/z): 320 (M+1)⁺ (boronic acid).

### PREPARATION 26

### tert-Butyl 4-[4-amino-6-(pyrazin-2-ylamino)-2,4'-bipyridin-2'-yl]piperazine-1-Carboxylate

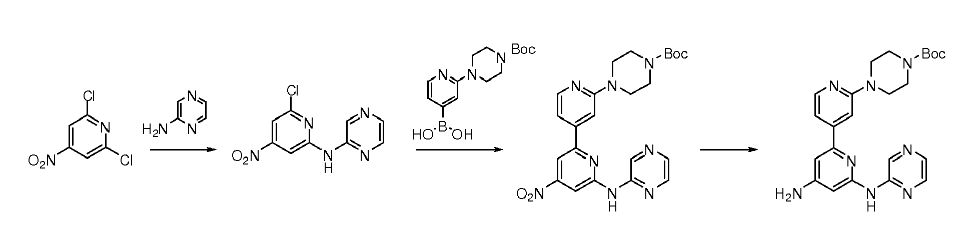

**a) *N-*(6-Chloro-4-nitropyridin-2-yl)pyrazin-2-amine**
   Obtained (63%) from 2,6-dichloro-4-nitropyridine and pyrazin-2-amine following the experimental procedure as described in preparation 4 using dioxane as a solvent, followed by purification by flash chromatography (100% hexanes to 1:4 hexanes/ethyl acetate).
   LRMS (m/z): 252 (M+1)⁺.
**b) *tert*-Butyl 4-[4-nitro-6-(pyrazin-2-ylamino)-2,4'-bipyridin-2'-yl]piperazine-1-Carboxylate**
   Obtained (42%) from *N*-(6-Chloro-4-nitropyridin-2-yl)pyrazin-2-amine (preparation 26a) and {2-[4-(*tert*-butoxycarbonyl)piperazin-1-yl]pyridin-4-yl}boronic acid (preparation 24b) following the experimental procedure as described in preparation 1, followed by purification by flash chromatography (100% hexanes to 100% ethyl acetate).
   LRMS (m/z): 479 (M+1)⁺.
   1 H NMR (500 MHz, ACETONE) δ ppm 2.05 (s, 9 H) 3.52 - 3.57 (m, 4 H) 3.67 - 3.70 (m, 4 H) 7.40 (d, *J*=1.50 Hz, 1 H) 7.41 (d, *J*=1.50 Hz, 1 H) 7.60 (s, 1 H) 8.22 (d, *J*=2.00 Hz, 1 H) 8.24 (d, *J*=3.50 Hz, 1 H) 8.32 - 8.36 (m, 2 H) 8.76 (s, 1 H) 9.10 (s, 1 H)
**c) *tert*-Butyl 4-[4-amino-6-(pyrazin-2-ylamino)-2,4'-bipyridin-2'-yl]piperazine-1-Carboxylate**
   Iron (0.58 g, 10.38 mmol) was added to a stirred solution of *tert*-butyl 4-[4-nitro-6-(pyrazin-2-ylamino)-2,4'-bipyridin-2'-yl]piperazine-1-Carboxylate (example 69b, 1.24 g, 2.59 mmol) in satured aqueous ammonium chloride solution (4 mL) and ethanol (8 mL), and the mixture was stirred at 70 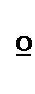 C. After 1 hour, the reaction mixture was filtered and evaporated. Water was added to the crude and satured aqueous potassium carbonate solution was added until the pH was 10. The aqueous layer was extracted with ethyl acetate. The organic layer was washed with brine, and dried (MgSO₄) and evaporated. Purification of the crude by flash chromatography (1:1 hexanes/ethyl acetate to 100% ethyl acetate) gave the title compound (0.81 g, 70%) as a white solid.
   LRMS (m/z): 449 (M+1)⁺.
   1 H NMR (500 MHz, METHANOL-*d*₄) δ ppm 1.50 (s, 9 H) 3.30 - 3.32 (m, 4 H) 3.59 (br. s., 4 H) 6.83 (d, *J*=2.00 Hz, 1 H) 6.86 (d, *J*=2.00 Hz, 1 H) 7.22 (dd, *J*=6.50, 1.50 Hz, 1 H) 7.43 (s, 1 H) 7.99 (d, *J*=3.50 Hz, 1 H) 8.16 (d, *J*=7.00 Hz, 1 H) 8.21 - 8.22 (m, 1 H) 9.12 (d, *J*=2.00 Hz, 1 H)

### PREPARATION 27

### tert-Butyl 4-(4-amino-6-{[4-(trifluoromethyl)pyridin-2-yl]amino}-2,4'-bipyridin-2'-yl)piperazine-1-Carboxylate

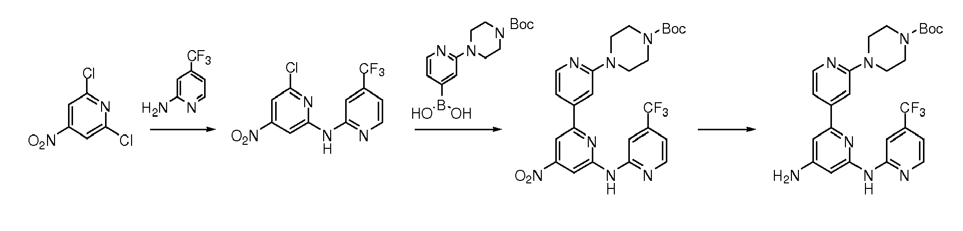

**a) 6-Chloro-4-nitro-*N*-[4-(trifluoromethyl)pyridin-2-yl]pyridin-2-amine**
   An oven dried resealable Schlenk tube was charged with 2,6-dichloro-4-nitropyridine (0.75 g, 3.89 mmol), 4-(trifluoromethyl)pyridin-2-amine (0.63 g, 3.89 mmol), cesium carbonate (1.52 g, 4.66 mmol) and 1,4-dioxane (9 mL). The Schlenk tube was subjected to three cycles of evacuation-backfilling with argon, and tris(dibenzylideneacetone)dipalladium(0) (0.07 g, 0.08 mmol) and 4,5-bis(diphenylphosphino)-9,9-dimethylxanthene (0.09 g, 0.15 mmol) were added. After three further cycles of evacuation-backfilling with argon, the Schlenk tube was capped and placed in an oil bath at 100 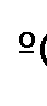 C. After 1 h, the mixture was cooled, filtered through Celite® and the solvent was removed in vacuo. The residue was purified by flash chromatography (gradient from 100% hexane to 80% ethyl acetate) to give the title compound (1.01 g, 84%) as a solid.
   LRMS (m/z): 319/321 (M+1)⁺.
   1 H NMR (400 MHz, CHLOROFORM-*d*) δ ppm 7.21 (d, *J*=4.80 Hz, 1 H) 7.52 (s, 1 H) 7.63 (s, 1 H) 7.86 (s, 1 H) 8.53 (d, *J*=4.80 Hz, 1 H) 8.63 (s, 1 H)
**b) *tert-*Butyl 4-(4-nitro-6-{[4-(trifluoromethyl)pyridin-2-yl]amino}-2,4'-bipyridin-2'-yl)piperazine-1-Carboxylate**
   Obtained (74%) from 6-Chloro-4-nitro-*N*-[4-(trifluoromethyl)pyridin-2-yl]pyridin-2-amine (preparation 27a) and {2-[4-(*tert*-butoxycarbonyl)piperazin-1-yl]pyridin-4-yl}boronic acid (preparation 24b) following the experimental procedure as described in preparation 1 using a mixture of toluene/ethanol (2:1) as solvent and followed by flash chromatography purification (gradient from 100% hexane to 100% ethyl acetate).
   LRMS (m/z): 546 (M+1)⁺.
**c) *tert*-Butyl 4-(4-amino-6-][4-(trifluoromethyl)pyridin-2-yl]amino}-2,4'-bipyridin-2'-yl)piperazine-1-Carboxylate**
   Obtained (45%) from *tert-*butyl 4-(4-nitro-6-{[4-(trifluoromethyl)pyridin-2-yl]amino}-2,4'-bipyridin-2'-yl)piperazine-1-Carboxylate (preparation 27b) following the experimental procedure as described in preparation 26c heating at 70 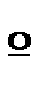 C for 3 h.
   LRMS (m/z): 516 (M+1)⁺.
   1 H NMR (400 MHz, METHANOL-*d*₄) δ ppm 1.49 (s, 9 H) 3.57 (s, 8 H) 6.64 (d, *J*=1.60 Hz, 1 H) 6.83 (d, *J*=1.60 Hz, 1 H) 7.06 (dd, *J*=5.20, 1.20 Hz, 1 H) 7.21 (dd, *J*=5.20, 1.20 Hz, 1 H) 7.41 (s, 1 H) 8.15 (d, *J*=5.20 Hz, 1 H) 8.37 (d, *J*=5.20 Hz, 1 H) 8.39 (s, 1 H)

### PREPARATION 28

### tert-Butyl (1S,4S)-5-(4-amino-6-{[4-(trifluoromethyl)pyridin-2-yl]amino}-2,4'-bipyridin-2'-yl)-2,5-diazabicyclo[2.2.1]heptane-2-Carboxylate

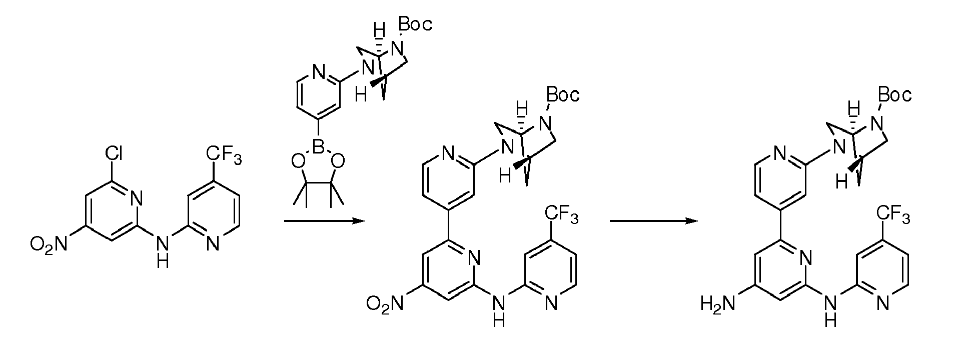

**a) *tert*-Butyl (1*S*,4*S*)-5-(4-nitro-6-1[4-(trifluoromethyl)pyridin-2-yl]aminol-2,4'-bipyridin-2'-yl)-2,5-diazabicyclo[2.2.1]heptane-2-Carboxylate**
   Obtained (68%) from 6-Chloro-4-nitro-*N*[4-(trifluoromethyl)pyridin-2-yl]pyridin-2-amine (preparation 27a) and *tert*-butyl (1*S*,4*S*)-5-[4-(4,4,5,5-tetramethyl-1,3,2-dioxaborolan-2-yl)pyridin-2-yl]-2,5-diazabicyclo[2.2.1]heptane-2-Carboxylate (preparation 25) following the experimental procedure as described in preparation 1 using a mixture of 2:1 toluene/ethanol as solvent and followed by purification by flash chromatography (gradient from 100% hexane to 100% ethyl acetate).
   LRMS (m/z): 558 (M+1)⁺.
**b) *tert*-Butyl(1*S*,4*S*)-5-(4-amino-6-{[4-(trifluoromethyl)pyridin-2-yl]amino}-2,4'-bipyridin-2'-yl)-2,5-diazabicyclo[2.2.1]heptane-2-Carboxylate**
   Obtained (62%) from *tert-*butyl (1*S*,4*S*)-5-(4-nitro-6-{[4-(trifluoromethyl)pyridin-2-yl]amino}-2,4'-bipyridin-2'-yl)-2,5-diazabicyclo[2.2.1]heptane-2-Carboxylate (preparation 28a) following the experimental procedure as described in preparation 26c heating at 70 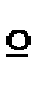 C for 2 h.
   LRMS (m/z): 528 (M+1)⁺.
   1 H NMR (400 MHz, METHANOL-*d*₄) δ ppm 1.41 (s, 4 H) 1.47 (s, 5 H) 2.02 (s, 3 H) 3.37-3.44 (m, 3 H) 3.64 (d, *J*=9.40 Hz, 1 H) 4.60 (s, 1 H) 6.71 (d, *J*=12.40 Hz, 1 H) 6.83 (s, 1 H) 7.05-7.09 (m, 2 H) 7.14 (d, *J*=5.20 Hz, 1 H) 8.08 (d, *J*=5.20 Hz, 1 H) 8.32 (d, *J*=7.60 Hz, 1 H) 8.37 (d, *J*=5.20 Hz, 1 H)

### PREPARATION 29

### 6-Chloro-N(4-methylpyridin-2-yl)-4-nitropyridin-2-amine

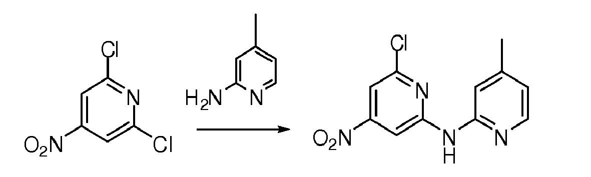

Obtained (80%) from 2,6-dichloro-4-nitropyridine and 4-methylpyridin-2-amine following the experimental procedure as described in preparation 27a.
LRMS (m/z): 265/267 (M+1)⁺.

### PREPARATION 30

### tert-Butyl 4-amino-6-(pyrazin-2-ylamino)-3",6"-dihydro-2,4':2',4"-terpyridine-1"(2" H)-Carboxylate

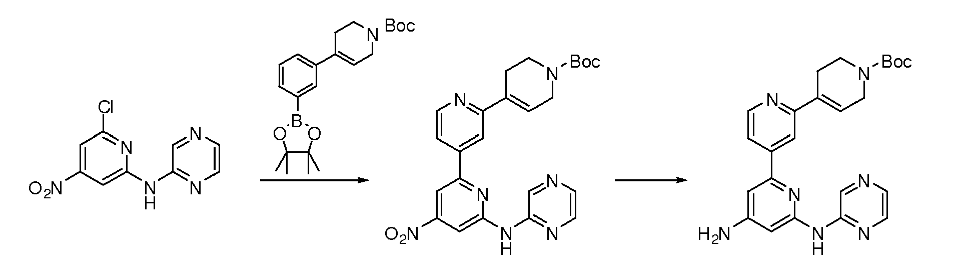

**a) *tert*-Butyl 4-nitro-6-(pyrazin-2-ylamino)-3",6"-dihydro-2,4':2',4"-terpyridine-1 "(2"*H*)-Carboxylate**
   Obtained (34%) from *N*-(6-Chloro-4-nitropyridin-2-yl)pyrazin-2-amine (preparation 26a) and *tert*-butyl 4-[3-(4,4,5-trimethyl-1,3,2-dioxaborolan-2-yl)phenyl]-3,6-dihydropyridine-1 (2*H*)-Carboxylate (preparation 9b) following the experimental procedure as described in preparation 1 using dioxane as reaction solvent.
   LRMS (m/z): 476 (M+1)⁺.
**b) *tert-*Butyl 4-amino-6-(pyrazin-2-ylamino)-3",6"-dihydro-2,4':2',4"-terpyridine-1 "(2"*H*)-Carboxylate**
   Obtained (26%) from *tert*-butyl 4-nitro-6-(pyrazin-2-ylamino)-3",6"-dihydro-2,4':2',4"-terpyridine-1"(2"*H*-Carboxylate (preparation 30a) following the experimental procedure as described in preparation 26c.
   LRMS (m/z): 446 (M+1)⁺.

### PREPARATION 31

### tert-Butyl 4-(5-(4,4,5,5-tetramethyl-1,3,2-dioxaborolan-2-yl)pyridin-3-yl)piperazine-1-Carboxylate

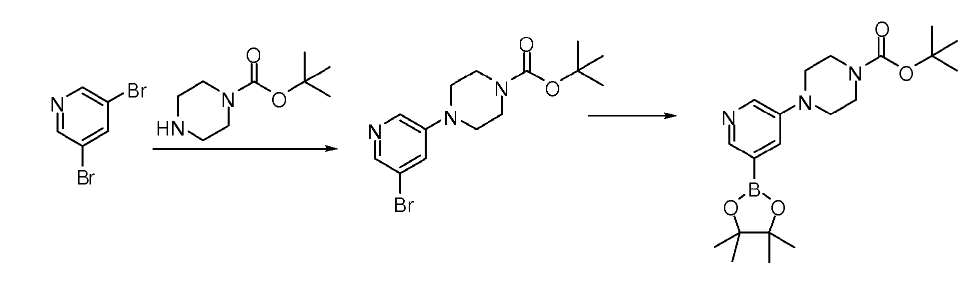

**a) *tert*-Butyl 4-(4-bromopyridin-2-yl)piperazine-1-Carboxylate**
   Obtained (65%) from 3,5-dibromopyridine and *tert-*butyl piperazine-1-Carboxylate following the experimental procedure as described in preparation 27a, using sodium *tert*-butoxide as base and toluene as solvent, heating at 100 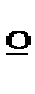 C for 6 hour.
   LRMS (m/z): 341/343 (M+1)⁺.
**b) *tert*-Butyl 4-(5-(4,4,5,5-tetramethyl-1,3,2-dioxaborolan-2-yl)pyridin-3-yl)piperazine-1-Carboxylate**
   Obtained as a solid (17%) from *tert*-butyl 4-(5-bromopyridin-3-yl)piperazine-1-Carboxylate (preparation 31 a) following the experimental procedure as described in preparation 3b, heating at 90 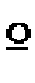 C overnight.
   LRMS (m/z): 390 (M+1)⁺.

### PREPARATION 32

### tert-butyl 2-Chloro-6-(pyrazin-2-ylamino)isonicotinate

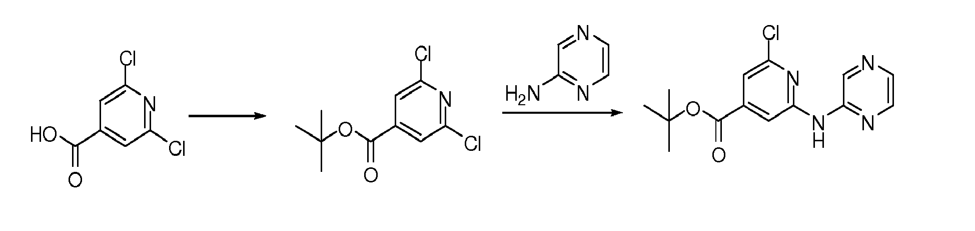

**a) *tert*-Butyl 2,6-dichloroisonicotinate**
   To a solution of 2,6-dichloroisonicotinic acid (2 g, 10.4 mmol) in toluene (50 mL) *N,N* dimethylformamide di-*tert*-butil acetal (15 mL, 62.5 mmol) was added under nitrogen atmosphere and mixture heated at 80 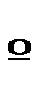 C overnight. Ethyl acetate was added and organic layer was washed with water and brine, dried (MgSO₄), filtered and concentrated to yield the title compound (2.34 g, 87%) as a solid.
   LRMS (m/z): 249 (M+1)⁺.
   ¹H NMR (400 MHz, CHLOROFORM-*d*) ppm 1.60 (s, 9 H) 7.74 (s, 2 H)
**b) *tert-*butyl 2-Chloro-6-(pyrazin-2-ylamino)isonicotinate**
   Obtained as a solid (38%) from *tert*-butyl 2,6-dichloroisonicotinate (preparation 32a) and pyrazin-2-amine following the experimental procedure as described in preparation 4, using cesium carbonate as base and dioxane as solvent.
   LRMS (m/z): 307 (M+1)⁺.

### PREPARATION 33

### tert-Butyl 3-[3-(4,4,5,5-tetramethyl-1,3,2-dioxaborolan-2-yl)phenyl]propanoate

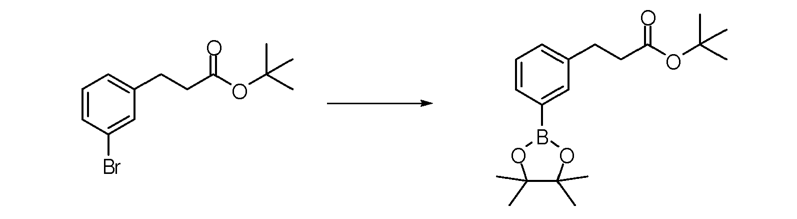

Obtained as a oil (65%) from *tert-*butyl 3-(3-bromophenyl)propanoate following the experimental procedure as described in preparation 3b, heating at 80C overnight.
1 H NMR (400 MHz, CHLOROFORM-*d*) δ ppm 1.34 (s, 12 H) 1.42 (s, 9 H) 2.55 (t, 2 H) 2.91 (t, *J*=7.82 Hz, 2 H) 7.29 (d, *J*=1.95 Hz, 1 H) 7.30 (d, *J*=1.17 Hz, 1 H) 7.63 (d, *J*=2.34 Hz, 1 H) 7.65 (s, 1 H)

### EXAMPLE 1

### N-{6-[3-(Aminomethyl)phenyl]pyridin-2-yl}-4-methylpyridin-2-amine

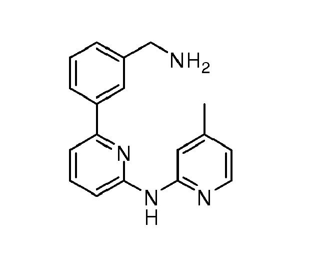

**a) 3-{6-[(4-Methylpyridin-2-yl)amino]pyridin-2-yl}benzonitrile**
   Obtained as a solid (50%) from *N-*(6-bromopyridin-2-yl)-4-methylpyridin-2-amine (preparation 2) and (3-Cyanophenyl)boronic acid following the experimental procedure as described in preparation 1 followed by purification by flash chromatography.
   LRMS (m/z): 287 (M+1)⁺.
   1 H NMR (400 MHz, CHLOROFORM-*d*) δ ppm 2.39 (s, 3 H) 6.75 (d, *J*=5.60 Hz, 1 H) 7.30 (d, *J*=8.00 Hz, 1 H) 7.33 (br. s., 1 H) 7.51 - 7.59 (m, 3 H) 7.67 - 7.73 (m, 2 H) 8.15 (d, *J*=5.20 Hz, 1 H) 8.22 (d, *J*=11.20 Hz, 1 H) 8.38 (br. s., 1 H)
**b) *N*-{6-[3-(Aminomethyl)phenyl]pyridin-2-yl}-4-methylpyridin-2-amine**
   A suspension of 3-{6-[(4-methylpyridin-2-yl)amino]pyridin-2-yl}benzonitrile (example 1 a, 0.05 g, 0.18 mmol) and 10% palladium on carbon (0.03 g, catalytic amount) in methanol (6 mL) and hydrochloric acid solution (0.2 mL) were stirred under an atmosphere of hydrogen. After 6 hour, the mixture was filtered through Celite® and the filter cake was washed with methanol. The combined filtrate and washings were evaporated to give the title compound (0.04 g, 75%) as a solid.
   LRMS (m/z): 291 (M+1)⁺.
   1 H NMR (400 MHz, CHLOROFORM-*d*) δ ppm 2.34 (s, 3 H) 3.95 (br. s., 2 H) 6.70 (d, *J*=4.40 Hz, 1 H) 7.28-7.42 (m, 6 H) 7.62-7.65 (m, 2 H) 7.86 (d, *J*=7.60 Hz, 1 H) 7.89 (br. s., 1 H) 8.01 (s, 1 H) 8.14 (br. s., 1 H)

### EXAMPLE 2

### N-{6-[3-(2-Aminoethyl)phenyl]pyridin-2-yl}-4-methylpyridin-2-amine

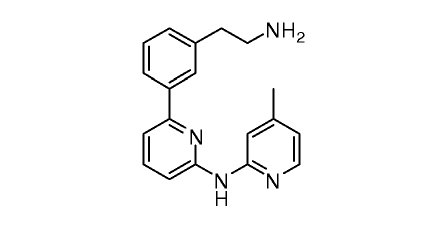

**a) *tert*-Butyl [2-(3-{6-[(4-methylpyridin-2-yl)amino]pyridin-2-yl}phenyl)ethyl] carbamate**
   Obtained as a solid (34%) from *N-*(6-bromopyridin-2-yl)-4-methylpyridin-2-amine (preparation 2) and (3-{2-[(*tert-*butoxycarbonyl)amino]ethyl}phenyl)boronic acid (preparation 3b) following the experimental procedure as described in preparation 1 followed by purification by flash chromatography.
   LRMS (m/z): 405 (M+1)⁺.
   1 H NMR (400 MHz, CHLOROFORM-*d*) δ ppm 1.43 (s, 9 H) 2.36 (s, 3 H) 2.90 (t, *J*=6.84 Hz, 2 H) 3.48 (m, 2 H) 4.58 (br. s., 1 H) 6.71 (d, *J*=5.20, 1 H) 7.29 (m, 2 H) 7.42 (m, 2 H) 7.60 (s, 1 H) 7.66 (t, *J*=8.08 Hz, 1 H) 7.86 (m, 2 H) 8.13 (d, *J*=5.20 Hz, 1 H)
**b) *N*-{6-[3-(2-Aminoethyl)phenyl]pyridin-2-yl}-4-methylpyridin-2-amine**
   2,2,2-Trifluoroacetic acid (1 mL) was added to a stirred solution of *tert*-butyl [2-(3-{6-[(4-methylpyridin-2-yl)amino]pyridin-2-yl}phenyl)ethyl]carbamate (example 2a, 0.15 g, 0.37 mmol) in dichloromethane (4 mL) and the mixture was stirred at room temperature. After 1 hour, satured aqueous sodium carbonate solution was added and the mixture was extracted with more dichloromethane. The organic layer was dried (MgSO₄) and evaporated to give the title compound (0.07 g, 65%) as a solid.
   LRMS (m/z): 305 (M+1)⁺.
   1 H NMR (400 MHz, CHLOROFORM-*d*) δ ppm 1.36 (br. s., 2 H) 2.36 (s, 3 H) 2.85 (t, *J*=6.84 Hz, 2 H) 3.04 (t, *J*=6.84 Hz, 2 H) 6.71 (dd, *J*=5.08, 0.78 Hz, 1 H) 7.24 (br. s., 1 H) 7.31 (dd, *J*=7.52, 0.68 Hz, 1 H) 7.38 - 7.44 (m, 3 H) 7.61 (br. s., 1 H) 7.65 (t, *J*=7.72 Hz, 1 H) 7.84 (dt, *J*=7.82, 1.47 Hz, 1 H) 7.89 (t, *J*=1.66 Hz, 1 H) 8.14 (d, *J*=5.08 Hz, 1 H)

### EXAMPLE 3

### N-{6-(3-Aminophenyl)pyridin-2-yl]-4-methylpyridin-2-amine

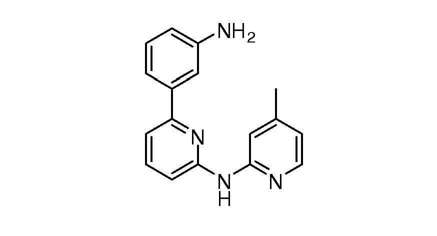

Obtained as a solid (85%) from *N-*(6-bromopyridin-2-yl)-4-methylpyridin-2-amine (preparation 2) and (3-aminophenyl)boronic acid hydrate following the experimental procedure as described in preparation 1 followed by purification by flash chromatography.
LRMS (m/z): 277 (M+1)⁺.
¹H NMR (400 MHz, CHLOROFORM-*d*) ppm 2.34 (s, 3 H) 6.69 (dd, *J*=4.79, 1.07 Hz, 1 H) 6.73 (ddd, *J*=7.82, 2.25, 1.07 Hz, 1 H) 7.18 - 7.29 (m, 2 H) 7.34 - 7.44 (m, 3 H) 7.55 (s, 1 H) 7.61 (t, 1 H) 7.67 (br. s., 1 H) 8.13 (d, *J*=5.08 Hz, 1 H)

### EXAMPLE 4

### 3-{6-[(4-Methylpyridin-2-yl)amino]pyridin-2-yl}benzamide

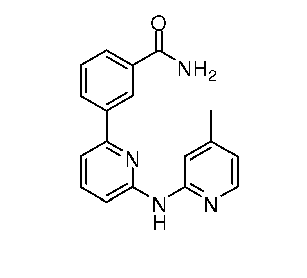

An oven dried resealable Schlenk tube was charged with 3-(6-aminopyridin-2-yl)benzonitrile (preparation 6, 0.06 g, 0.30 mmol), 2-bromo-4-methylpyridine (0.04 mL, 0.35 mmol), potassium *tert*-butoxide (0.13 g, 1.20 mmol) and benzene (2 mL). The Schlenk tube was subjected to three cycles of evacuation-backfilling with argon, and tris(dibenzylideneacetone)dipalladium(0) (0.01 g, catalytic amount) and (±)-2,2'-bis(diphenylphosphino)-1,1'-binaphthalene (0.01 g, catalytic amount) were added. After three further cycles of evacuation-backfilling with argon, the Schlenk tube was capped and placed in an oil bath at 150 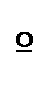 C. After 24h, the mixture was cooled, filtered and ethyl acetate was added. The organic layer was washed with satured aqueous ammonium chloride solution and water, dried (MgSO₄) and evaporated. Purification of the residue by flash chromatography (100% ethyl acetate) gave the title compound (0.02 g, 25%) as a solid.
LRMS (m/z): 305 (M+1)⁺.
1 H NMR (400 MHz, DMSO-*d*₆) δ ppm 2.32 (s, 3 H) 6.73 (d, *J*=4.69 Hz, 1 H) 7.38 - 7.63 (m, 4 H) 7.74 (t, *J*=7.91 Hz, 1 H) 7.86 - 7.96 (m, 2 H) 8.04 - 8.14 (m, 2 H) 8.19 (d, *J*=7.62 Hz, 1 H) 8.63 (s, 1 H) 9.66 (s, 1 H)

### EXAMPLE 5

### N-(3-{6-[(4-Methylpyridin-2-yl)amino]pyridin-2-yl}phenyl)ethane-1,2-diamine

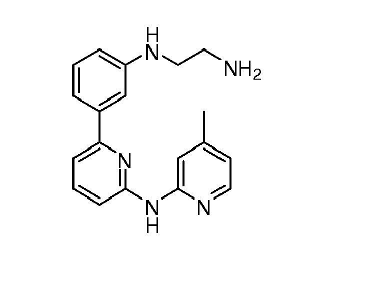

**a) *tert*-Butyl {2-[(3-]6-[(4-methylpyridin-2-yl)amino]pyridin-2-yl}phenyl)amino] ethyl}carbamate**
   Acetic acid (0.01 mL, 0.19 mmol) was added to a stirred solution of *N*-[6-(3-aminophenyl)pyridin-2-yl]-4-methylpyridin-2-amine (example 3, 0.05g, 0.19 mmol) and *tert-*butyl (2-oxoethyl)carbamate (0.05 g, 0.29 mmol) and freshly activated molecular sieves in methanol (2 mL). The mixture was stirred for 1 hour and sodium cyanoborohydride (0.01 g, 0.20 mmol) was added portionwise. After the addition, the mixture was stirred at room temperature for 18 hours. Satured aqueous sodium carbonate solution and dichloromethane were added to the reaction mixture and the organic layer was washed with brine, dried (MgSO₄) and the solvent was evaporated. Purification of the residue by flash chromatography gave the title compound (0.06 g, 80%) as a solid.
   LRMS (m/z): 420 (M+1)⁺.
   1 H NMR (400 MHz, CHLOROFORM-*d*) δ ppm 1.45 (s, 9 H) 2.32 (s, 3 H) 3.32 (br. s., 2 H) 3.38 (br. s., 2 H) 4.88 (br. s., 1 H) 6.64-6.69 (m, 2 H) 7.23-7.32 (m, 2 H) 7.05 (s, 3 H) 7.45 (d, *J*=8.00 Hz, 1 H) 7.50 (s, 1 H) 7.62 (t, *J*=8.20 Hz, 1 H) 7.75 (br. s., 1 H) 8.12 (d, *J*=8.00 Hz, 1 H)
**b) *N*-(3-{6-[(4-Methylpyridin-2-yl)amino]pyridin-2-yl}phenyl)ethane-1,2-diamine**
   Obtained as a solid (47%) from *tert-*butyl {2-[(3-16-[(4-methylpyridin-2-yl)amino]pyridin-2-yl}phenyl)amino]ethyl}carbamate (example 5a) following the experimental procedure as described in example 2b followed by purification of the crude product by flash chromatography (10:1 ethyl acetate/methanol).
   LRMS (m/z): 320 (M+1)⁺.
   1 H NMR (400 MHz, METHANOL-*d*₄) δ ppm 2.34 (s, 3 H) 2.89 (t, *J*=6.06 Hz, 2 H) 3.28 (t, *J*=6.06 Hz, 2 H) 6.68 - 6.77 (m, 2 H) 7.21 (t, *J*=7.82 Hz, 1 H) 7.26 - 7.31 (m, 2 H) 7.32 - 7.38 (m, 2 H) 7.64 (d, *J*=7.62 Hz, 1 H) 7.77 (s, 1 H) 8.03 (d, *J*=5.28 Hz, 1 H).

### EXAMPLE 6

### 4-Methyl-N-{6-[3-(1,2,3,6-tetrahydropyridin-4-yl)phenyl]pyridin-2-yl}pyridin-2-amine

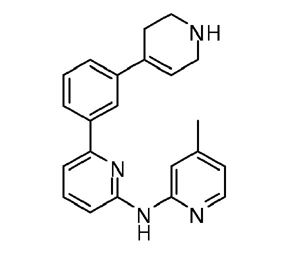

**a) *tert*-Butyl 4-(3-{6-[(4-methylpyridin-2-yl)amino]pyridin-2-yl}phenyl)-3,6-dihydropyridine-1 (2*H*)-Carboxylate**
   Obtained as a oil (51%) from *N*-(6-bromopyridin-2-yl)-4-methylpyridin-2-amine (preparation 2) and *tert*-butyl 4-[3-(4,4,5-trimethyl-1,3,2-dioxaborolan-2-yl)phenyl]-3,6-dihydropyridine-1(2*H*-Carboxylate (preparation 9b) following the experimental procedure as described in preparation 1 followed by purification of the crude product by flash chromatography (5:95 hexanes/ethyl acetate to 1:4 hexanes/ethyl acetate).
   LRMS (m/z): 443 (M+1)⁺.
**b) 4-Methyl-*N*-{6-[3-(1,2,3,6-tetrahydropyridin-4-yl)phenyl]pyridin-2-yl}pyridin-2-amine**
   Obtained as a solid (61%) from *tert*-butyl 4-(3-{6-[(4-methylpyridin-2-yl)amino]pyridin-2-yl}phenyl)-3,6-dihydropyridine-1(2*H*)-Carboxylate (example 6a) following the experimental procedure as described in example 2b.
   LRMS (m/z): 343 (M+1)⁺.
   1 H NMR (400 MHz, CHLOROFORM-*d*) δ ppm 2.35 (s, 3 H) 2.53 - 2.60 (m, 2 H) 3.15 (t, *J*=5.67 Hz, 2 H) 3.58 (q, *J*=2.80 Hz, 2 H) 6.20 - 6.26 (m, 1 H) 6.71 (dd, *J*=5.28, 0.78 Hz, 1 H) 7.31 (dd, *J*=7.62, 0.78 Hz, 1 H) 7.38 (dd, *J*=8.21, 0.78 Hz, 1 H) 7.41 - 7.44 (m, 2 H) 7.50 (br. s., 1 H) 7.62 - 7.68 (m, 2 H) 7.83 - 7.88 (m, 1 H) 8.09 (d, *J*=1.17 Hz, 1 H) 8.13 (d, *J*=5.08 Hz, 1 H).

### EXAMPLE 7

### 4-Methyl-N-[6-(3-piperidin-4-ylphenyl)pyridin-2-yl]pyridin-2-amine

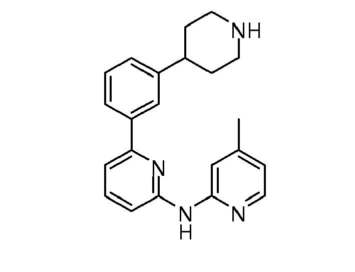

**a) *tert*-Butyl 4-(3-{6-[(4-methylpyridin-2-yl)amino]pyridin-2-yl}phenyl)piperidine-1-Carboxylate**
   Obtained as a solid (40%) from *tert*-butyl 4-(3-{6-[(4-methylpyridin-2-yl)amino]pyridin-2-yl}phenyl)-3,6-dihydropyridine-1 (2*H*)-Carboxylate (example 6a) following the experimental procedure as described in example 1 over 72 hours followed by purification of the crude product by flash chromatography (3:2 hexanes/ethyl acetate).
   LRMS (m/z): 445 (M+1)⁺.
**b) 4-Methyl-*N-*[6-(3-piperidin-4-ylphenyl)pyridin-2-yl]pyridin-2-amine**
   Obtained as a solid from *tert*-butyl 4-(3-{6-[(4-methylpyridin-2-yl)amino]pyridin-2-yl}phenyl)piperidine-1-Carboxylate (example 7a) following the experimental procedure as described in example 2b.
   LRMS (m/z): 345 (M+1)⁺.
   1 H NMR (400 MHz, CHLOROFORM-*d*) δ ppm 1.68 - 1.78 (m, 2 H) 1.84 - 1.92 (m, 2 H) 2.36 (s, 3 H) 2.69 - 2.81 (m, 2 H) 3.22 (d, *J*=12.00 Hz, 2 H) 6.71 (d, *J*=5.20 Hz, 1 H) 7.28 (t, *J*=7.20 Hz, 1 H) 7.36 - 7.43 (m, 2 H) 7.48 (br. s., 1 H) 7.63 - 7.67 (m, 2 H) 7.81 (d, *J*=7.60 Hz, 1 H) 7.94 (s, 1 H) 8.14 (d, *J*=5.20 Hz, 1 H).

### EXAMPLE 8

### N-{6-[3-(4-Aminopiperidin-1-yl)phenyl]pyridin-2-yl}-4-methylpyridin-2-amine

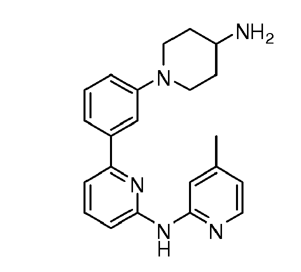

**a) *tert-*Butyl [1-(3-]6-[(4-methylpyridin-2-yl)amino]pyridin-2-yl}phenyl)piperidin-4-yl]carbamate**
   Obtained as a solid (62%) from *N-*(6-bromopyridin-2-yl)-4-methylpyridin-2-amine (preparation 2) and *tert-*butyl {1-[3-(4,4,5,5-tetramethyl-1,3,2-dioxaborolan-2-yl)phenyl]piperidin-4-yl}carbamate (preparation 12b) following the experimental procedure as described in preparation 1 followed by purification of the crude product by flash chromatography.
   LRMS (m/z): 460 (M+1)⁺.
**b) *N-*{6-[3-(4-Aminopiperidin-1-yl)phenyl]pyridin-2-yl}-4-methylpyridin-2-amine**
   Obtained as a solid from *tert-*butyl [1-(3-{6-[(4-methylpyridin-2-yl)amino]pyridin-2-yl}phenyl)piperidin-4-yl]carbamate (example 8a) following the experimental procedure as described in example 2b.
   LRMS (m/z): 360 (M+1)⁺.
   1H NMR (400 MHz, METHANOL-*d*₄) δ ppm 1.74 - 1.84 (m, 2 H) 2.11 (dd, *J*=12.40, 1.60 Hz, 2 H) 2.37 (s, 3 H) 2.86 - 2.93 (m, 2 H) 3.24 - 3.30 (m, 1 H) 3.88 (d, *J*=13.20 Hz, 2 H) 6.78 (d, *J*=5.20 Hz, 1 H) 7.63 (dd, *J*=8.00, 2.00 Hz, 1 H) 7.35 (t, *J*=8.00 Hz, 2 H) 7.50 (d, *J*=8.00 Hz, 1 H) 7.68 - 7.73 (m, 2 H) 7.81 (s, 1 H) 8.06 (d, *J*=4.80 Hz, 1 H) 8.52 (s, 1 H).

### EXAMPLE 9

### N⁶-(4-Methylpyridin-2-yl)-2,4'-bipyridine-2',6-diamine

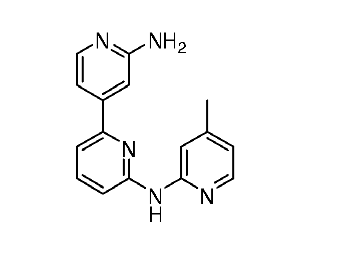

A suspension of 2'-fluoro-*N*-(4-methylpyridin-2-yl)-2,4'-bipyridin-6-amine (preparation 7, 0.04 g, 0.14 mmol) and aqueous ammonia (32%, 0.67 mL) in dioxane (0.2 mL) was heated in a sealed tube at 150 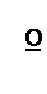 C with stirring. After stirring overnight the mixture was cooled and evaporated to give the desired compound (0.01 g, 33%) as a solid.
LRMS (m/z): 278 (M+1)⁺.
1 H NMR (400 MHz, DMSO-*d*₆) δ ppm 2.29 (s, 3 H) 5.98 (s, 2 H) 6.73 (d, *J*=5.08 Hz, 1 H) 7.04 - 7.11 (m, 2 H) 7.32 (d, *J*=6.84 Hz, 1 H) 7.64 - 7.68 (m, 1 H) 7.72 (d, *J*=7.42 Hz, 1 H) 7.74 - 7.78 (m, 1 H) 8.00 (d, *J*=5.28 Hz, 1 H) 8.08 (d, *J*=5.08 Hz, 1 H) 9.64 (s, 1 H)

### EXAMPLE 10

### N²-(2-Aminoethyl)-N⁶-(4-methylpyridin-2-yl)-2,4'-bipyridine-2',6-diamine

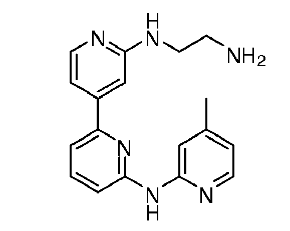

**a) *tert*-Butyl [2-({6-[(4-methy)pyridin-2-yl)amino]-2,4'-bipyridin-2'-yl}amino) ethyl] carbamate**
   A suspension of 2'-fluoro-*N*-(4-methylpyridin-2-yl)-2,4'-bipyridin-6-amine (preparation 7, 0.09 g, 0.32 mmol), *tert-*butyl (2-aminoethyl)carbamate (0.21 mL, 1.28 mmol) and potassium carbonate (0.14 g, 1.00 mmol) in dimethylsulphoxide (0.7 mL) was heated in a sealed tube at 100 C with stirring. After stirring overnight the mixture was cooled and ethyl acetate and water were added. The organic layer was washed with brine, dried (MgSO₄) and evaporated. Purification of the residue by flash chromatography (7:3 hexanes/ethyl acetate to 100% ethyl acetate) gave the title compound (0.09 g, 63%) as a solid.
   LRMS (m/z): 421 (M+1)⁺.
   1 H NMR (400 MHz, CHLOROFORM-*d*) δ ppm 1.42 (s, 9 H) 2.34 (s, 3 H) 3.38 (d, *J*=5.20 Hz, 2 H) 3.51 (d, *J*=5.20 Hz, 2 H) 5.03 (br. s., 1 H) 5.14 (br. s., 1 H) 6.70 (d, *J*=5.20 Hz, 1 H) 7.05 (s, 1 H) 7.13 (d, *J*=5.20 Hz, 1 H) 7.28 (s, 1 H) 7.46 (s, 1 H) 7.55 - 7.58 (m, 1 H) 7.65 (t, *J*=7.60 Hz, 1 H) 7.74 (br. s., 1 H) 8.12 - 8.17 (m, 2 H)
**b) *N*²-(2-Aminoethyl)-*N*⁶-(4-methylpyridin-2-yl)-2,4'-bipyridine-2',6-diamine**
   Obtained as a solid (73%) from *tert*-butyl [2-(16-[(4-methylpyridin-2-yl)amino]-2,4'-bipyridin-2'-yl}amino)ethyl]carbamate (example 10a) following the experimental procedure as described in example 2b.
   LRMS (m/z): 321 (M+1)⁺.
   ¹H NMR (400 MHz, METHANOL-*d*₄) ppm 2.36 (s, 3 H) 2.88 (t, *J*=6.25 Hz, 2 H) 3.24 - 3.35 (m, 3 H) 3.44 (t, *J*=6.25 Hz, 2 H) 6.77 (dd, *J*=5.18, 0.88 Hz, 1 H) 7.12 - 7.26 (m, 2 H) 7.38 (dd, *J*=7.52, 0.68 Hz, 1 H) 7.50 (dd, *J*=8.30, 0.68 Hz, 1 H) 7.63 - 7.77 (m, 2 H) 8.04 (dd, *J*=11.04, 4.98 Hz, 2 H)

### EXAMPLE 11

### 2'-(3-Aminopyrrolidin-1-yl)-N-(4-methy)pyridin-2-y)-2,4'-bipyridin-6-amine

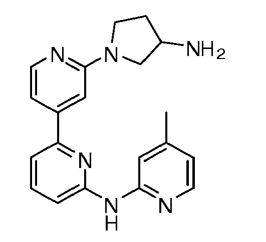

**a) *tert*-Butyl (1-{6-[(4-methylpyridin-2-yl)amino]-2,4'-bipyridin-2'-yl}pyrrolidin-3-yl)carbamate**
   Obtained as a solid (76%) from 2'-fluoro-*N-*(4-methylpyridin-2-yl)-2,4'-bipyridin-6-amine (preparation 7) and *tert*-butyl pyrrolidin-3-ylcarbamate following the experimental procedure as described in example 10a followed by purification of the crude product by flash chromatography (95:5 dichloromethane /methanol).
   LRMS (m/z): 447 (M+1)⁺.
   1 H NMR (400 MHz, METHANOL-*d*₄) δ ppm 1.46 (s, 9 H) 1.93 - 2.00 (m, 1 H) 2.19 - 2.27 (m, 1 H) 2.31 (s, 3 H) 3.28 - 3.30 (m, 1 H) 3.44 - 3.50 (m, 1 H) 3.56 - 3.61 (m, 1 H) 3.71 - 3.75 (m, 1 H) 4.26 - 4.26 (m, 1 H) 6.72 (d, *J=*5.60 Hz, 1 H) 7.07 (s, 1 H) 7.10 (d, *J*=5.20 Hz, 1 H) 7.33 - 7.36 (m, 2 H) 7.62 (t, *J*=7.60 Hz, 1 H) 7.75 (s, 1 H) 8.01 - 8.03 (m, 1 H).
**b) 2'-(3-Aminopyrro)idin-1-yl)-*N*-(4-methy)pyridin-2-yl)-2,4'-bipyridin-6-amine**
   Obtained as a solid from *tert*-butyl (1-{6-[(4-methylpyridin-2-yl)amino]-2,4'-bipyridin-2'-yl}pyrrolidin-3-yl)carbamate (example 11a) following the experimental procedure as described in example 2b.
   LRMS (m/z): 347 (M+1)⁺.
   ¹H NMR (400 MHz, DMSO-*d*₆) ppm 1.74 - 1.93 (m, 1 H) 2.06 - 2.23 (m, 1 H) 2.30 (s, 3 H) 3.41 - 3.54 (m, 2 H) 3.54 - 3.77 (m, 4 H) 6.75 (dd, *J*=5.08, 0.78 Hz, 1 H) 7.08 (s, 1 H) 7.16 (dd, *J*=5.37, 1.27 Hz, 1 H) 7.48 (d, *J*=7.03 Hz, 1 H) 7.58 (d, *J*=8.01 Hz, 1 H) 7.72 (t, 1 H) 7.86 (s, 1 H) 8.09 (d, *J*=5.08 Hz, 1 H) 8.15 (d, *J*=5.86 Hz, 1 H) 9.67 (s, 1 H)

### EXAMPLE 12

### 2'-(4-Aminopiperidin-1-yl)-N-(4-methylpyridin-2-yl)-2,4'-bipyridin-6-amine

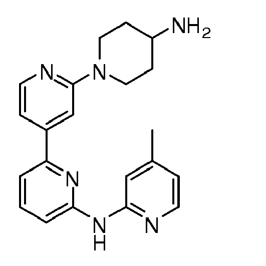

**a) *tert*-Butyl (1-{6-[(4-methylpyridin-2-yl)amino]-2,4'-bipyridin-2'-yl}piperidin-4-yl)carbamate**
   Obtained as a solid (59%) from 2'-fluoro-*N*-(4-methylpyridin-2-yl)-2,4'-bipyridin-6-amine (preparation 7) and *tert*-butyl piperidin-4-ylcarbamate following the experimental procedure as described in example 10a followed by purification of the crude product by flash chromatography (1:2 hexanes /ethyl acetate).
   LRMS (m/z): 461 (M+1)⁺.
   1 H NMR (400 MHz, CHLOROFORM-*d*) δ ppm 1.46 (br. s., 11 H) 2.07 (br. s., 2 H) 2.35 (s, 3 H) 3.05 (t, *J*=10.80 Hz, 2 H) 3.72 (br. s., 1 H) 4.34 (d, *J*=13.20 Hz, 2 H) 4.50 (d, *J*=7.60 Hz, 1 H) 6.73 (d, *J*=4.80 Hz, 1 H) 7.13 (d, *J*=5.20 Hz, 1 H) 7.31 (d, *J*=7.20 Hz, 1 H) 7.40 (s, 1 H) 7.47 (d, *J*=8.00 Hz, 1 H) 7.55 (br. s., 1 H) 7.58 (br. s., 1 H) 7.67 (t, *J*=7.60 Hz, 1 H) 8.14 (d, *J*=5.20 Hz, 1 H) 8.26 (d, *J*=5.20 Hz, 1 H)
**b) 2'-(4-Aminopiperidin-1-yl)-*N*-(4-methy)pyridin-2-yl-2,4'-bipyridin-6-amine**
   Obtained as a solid (86%) from *tert*-butyl (1-{6-[(4-methylpyridin-2-yl)amino]-2,4'-bipyridin-2'-yl}piperidin-4-yl)carbamate (example 12a) following the experimental procedure as described in example 2b.
   LRMS (m/z): 361 (M+1)⁺.
   1 H NMR (400 MHz, METHANOL-*d*₄) δ ppm 1.61 - 1.71 (m, 2 H) 2.09 (d, *J*=6.00 Hz, 2 H) 2.36 (s, 3 H) 3.02 (t, *J*=11.60 Hz, 2 H) 3.35 - 3.41 (m, 1 H) 4.48 (d, *J*=13.60 Hz, 2 H) 6.78 (d, *J*=4.80 Hz, 1 H) 7.29 (d, *J*=5.20 Hz, 1 H) 7.44 -7.50 (m, 2 H) 7.56 (s, 1 H) 7.73 (t, *J*=8.00 Hz, 1 H) 7.75 (br. s., 1 H) 8.07 (d, *J*=4.80 Hz, 1 H) 8.19 (d, *J*=5.20 Hz, 1 H) 8.55 (br. s., 1 H)

### EXAMPLE 13

### N²-(3-Aminopropyl)-N⁶-(4-methylpyridin-2-yl)-2,4'-bipyridine-2',6-diamine

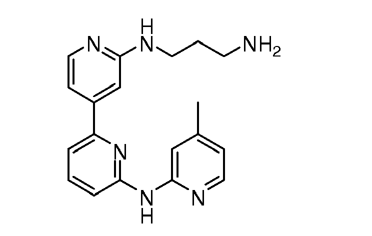

**a) *tert*-Butyl [3-({6-[(4-methylpyridin-2-yl)amino]-2,4'-bipyridin-2'-yl}amino) propyl] carbamate**
   Obtained as a solid (61 %) from 2'-fluoro-*N*-(4-methylpyridin-2-yl)-2,4'-bipyridin-6-amine (preparation 7) and *tert*-butyl (3-aminopropyl)carbamate following the experimental procedure as described in example 10a followed by purification of the crude product by flash chromatography.
   LRMS (m/z): 435 (M+1)⁺.
**b) *N*²-(3-Aminopropyl)-*N*⁶-(4-methylpyridin-2-yl)-2,4'-bipyridine-2',6-diamine**
   Obtained as a solid from *tert*-butyl [3-(16-[(4-methylpyridin-2-yl)amino]-2,4'-bipyridin-2'-yl}amino)propyl]carbamate (example 13a) following the experimental procedure as described in example 2b.
   LRMS (m/z): 335 (M+1)⁺.
   1 H NMR (400 MHz, METHANOL-*d*₄) δ ppm 1.78 - 1.91 (m, 2 H) 2.36 (s, 3 H) 2.84 (t, *J*=7.03 Hz, 2 H) 3.44 (t, *J*=6.64 Hz, 2 H) 6.77 (d, *J*=5.08 Hz, 1 H) 7.14 - 7.20 (m, 2 H) 7.37 (d, *J*=7.42 Hz, 1 H) 7.49 (d, *J*=8.40 Hz, 1 H) 7.71 (dd, *J*=8.30, 7.52 Hz, 1 H) 7.74 (d, *J*=0.78 Hz, 1 H) 8.03 (dd, *J*=5.47, 0.78 Hz, 1 H) 8.05 (d, *J*=5.08 Hz, 1 H).

### EXAMPLE 14

### N-(4-Methylpyridin-2-yl)-2'-piperazin-1-yl-2,4'-bipyridin-6-amine

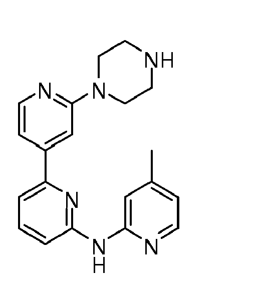

**a) *tert-*Butyl 4-{6-[(4-methylpyridin-2-yl)amino]-2,4'-bipyridin-2'-yl}piperazine-1-Carboxylate**
   Obtained as a solid (84%) from 2'-fluoro-*N*-(4-methylpyridin-2-yl)-2,4'-bipyridin-6-amine (preparation 7) and *tert*-butyl piperazine-1-Carboxylate following the experimental procedure as described in example 10a followed by purification of the crude product by flash chromatography.
   LRMS (m/z): 447 (M+1)⁺.
   1 H NMR (400 MHz, CHLOROFORM-*d*) δ ppm 1.50 (s, 9 H) 2.36 (s, 3 H) 3.58 - 3.65 (m, 8 H) 6.73 (d, *J*=4.80 Hz, 1 H) 7.18 (dd, *J*=5.20, 1.60 Hz, 1 H) 7.32 (d, *J*=7.60 Hz, 1 H) 7.34 (br. s., 1 H) 7.38 (s, 1 H) 7.52 - 7.54 (m, 2 H) 7.69 (t, *J*=7.60 Hz, 1 H) 8.15 (d, *J*=5.20 Hz, 1 H) 8.29 (d, *J*=5.60 Hz, 1 H)
**b) *N*-(4-Methy)pyridin-2-yl)-2'-piperazin-1-yl-2,4'-bipyridin-6-amine**
   Obtained as a solid (87%) from *tert*-butyl 4-16-[(4-methylpyridin-2-yl)amino]-2,4'-bipyridin-2'-yl}piperazine-1-Carboxylate (example 14a) following the experimental procedure as described in example 2b.
   LRMS (m/z): 347 (M+1)⁺.
   1 H NMR (400 MHz, METHANOL-*d*₄) δ ppm 2.35 (s, 3 H) 2.95 - 2.97 (m, 4 H) 3.56 - 3.58 (m, 4 H) 6.77 (d, *J*=4.80 Hz, 1 H) 7.28 (d, *J*=5.20 Hz, 1 H) 7.44 (t, *J*=6.80 Hz, 2 H) 7.51 (s, 1 H) 7.71 (t, *J*=8.40 Hz, 1 H) 7.80 (s, 1 H) 8.06 (d, *J*=5.20, 1 H) 8.17 (d, *J*=5.20 Hz, 1 H).

### EXAMPLE 15

### 2-({6-[(4-Methylpyridin-2-yl)amino]-2,4'-bipyridin-2'-yl}amino)ethanol

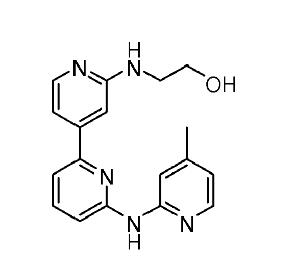

Obtained as a solid (54%) from 2'-fluoro-*N*-(4-methylpyridin-2-yl)-2,4'-bipyridin-6-amine (preparation 7) and 2-aminoethanol following the experimental procedure as described in example 10a followed by purification of the crude product by flash chromatography (9:1 ethyl acetate/methanol).
LRMS (m/z): 322 (M+1)⁺.
1 H NMR (400 MHz, METHANOL-*d*₄) δ ppm 2.35 (s, 3 H) 3.47 (t, *J*=5.67 Hz, 2 H) 3.75 (t, *J*=5.57 Hz, 2 H) 6.76 (dd, *J*=5.28, 0.78 Hz, 1 H) 7.18 (d, *J*=5.60 Hz, 1 H) 7.19 (s, 1 H) 7.37 (d, *J*=7.62 Hz, 1 H) 7.47 (d, *J*=8.21 Hz, 1 H) 7.69 (dd, *J*=8.30, 7.52 Hz, 1 H) 7.75 (br. s., 1 H) 8.01 (dd, *J*=5.28, 0.98 Hz, 1 H) 8.05 (d, *J*=5.28 Hz, 1 H)

### EXAMPLE 16

### 2'-(1,4-Diazepan-1-yl)-N-(4-methy)pyridin-2-yl)-2,4'-bipyridin-6-amine

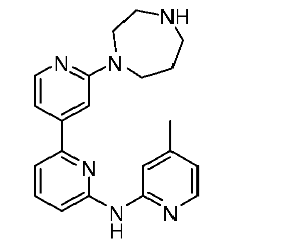

**a) *tert-*Butyl 4-{6-[(4-methylpyridin-2-yl)amino]-2,4'-bipyridin-2'-y1}-1,4-diazepane-1-Carboxylate**
   Obtained as a solid (62%) from 2'-fluoro-*N*-(4-methylpyridin-2-yl)-2,4'-bipyridin-6-amine (preparation 7) and *tert*-butyl 1,4-diazepane-1-Carboxylate following the experimental procedure as described in example 10a followed by purification of the crude product by flash chromatography.
   LRMS (m/z): 461 (M+1)⁺.
   1 H NMR (400 MHz, CHLOROFORM-*d*) δ ppm 1.42 (d, *J*=21.60 Hz, 9 H) 1.98 - 2.02 (m, 2 H) 2.33 (s, 3 H) 3.23 - 3.25 (m, 1 H) 3.33 - 3.36 (m, 1 H) 3.58 - 3.60 (m, 2 H) 3.71 - 3.75 (m, 2 H) 3.85 (br. s., 2 H) 6.71 (d, *J*=4.80 Hz, 1 H) 7.08 (d, *J*=5.20 Hz, 1 H) 7.18 (s, 1 H) 7.29 (d, *J*=7.20 Hz, 1 H) 7.48 (s, 1 H) 7.52 (d, *J*=8.00 Hz, 1 H) 7.67 (t, *J*=8.00 Hz, 1 H) 7.74 (br. s., 1 H) 8.15 (d, *J*=4.80 Hz, 1 H) 8.23 (d, *J*=5.20 Hz, 1 H)
**b) 2'-(1,4-Diazepan-1-yl)-*N*-(4-methylpyridin-2-yl)-2,4'-bipyridin-6-amine**
   Obtained as a solid (54%) from *tert*-butyl 4-{6-[(4-methylpyridin-2-yl)amino]-2,4'-bipyridin-2'-yl}-1,4-diazepane-1-Carboxylate (example 16a) following the experimental procedure as described in example 2b.
   LRMS (m/z): 361 (M+1)⁺.
   1 H NMR (400 MHz, CHLOROFORM-*d*) δ ppm 1.91 - 1.95 (m, 2 H) 2.34 (s, 3 H) 2.86 - 2.89 (m, 2 H) 3.05 - 3.08 (m, 2 H) 3.80 - 3.85 (m, 4 H) 6.71 (d, *J*=4.80 Hz, 1 H) 7.05 (d, *J*=5.20 Hz, 1 H) 7.18 (s, 1 H) 7.30 (d, *J*=7.60 Hz, 1 H) 7.45 (br. s., 1 H) 7.49 (d, *J*=8.40 Hz, 1 H) 7.53 (s, 1 H) 7.68 (t, *J*=7.20 Hz, 1 H) 8.14 (d, *J*=5.20 Hz, 1 H) 8.24 (d, *J*=5.20 Hz, 1 H).

### EXAMPLE 17

### N²-[2-(Dimethylamino)ethyl]-N²-methyl-N⁶-(4-methylpyridin-2-yl)-2,4'-bipyridine-2',6-diamine

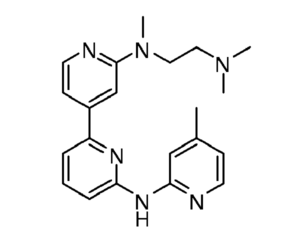

Obtained as a solid (28%) from 2'-fluoro-IV (4-methylpyridin-2-yl)-2,4'-bipyridin-6-amine (preparation 7) and *N,N,N*-trimethylethane-1,2-diamine following the experimental procedure as described in example 10a followed by purification of the crude product by flash chromatography (98:2 hexanes/ethyl acetate to 85:15 hexanes/ethyl acetate).
LRMS (m/z): 363 (M+1)⁺.
1 H NMR (400 MHz, CHLOROFORM-*d*) δ ppm 2.30 (s, 6 H) 2.35 (s, 3 H) 2.55 (t, *J*=7.42 Hz, 2 H) 3.16 (s, 3 H) 3.76 (t, *J*=7.42 Hz, 2 H) 6.71 (dd, *J*=5.08, 0.78 Hz, 1 H) 7.07 (dd, *J*=5.18, 1.47 Hz, 1 H) 7.17 (br. s., 1 H) 7.30 (dd, *J*=7.42, 0.78 Hz, 1 H) 7.49 (br. s., 1 H) 7.50 - 7.54 (m, 2 H) 7.67 (dd, *J*=8.21, 7.62 Hz, 1 H) 8.14 (d, *J*=5.28 Hz, 1 H) 8.24 (dd, *J*=5.18, 0.68 Hz, 1 H)

### EXAMPLE 18

### N-{6-[3-(2-Aminoethyl)phenyl]pyridin-2-yl}-4-tert-butylpyridin-2-amine

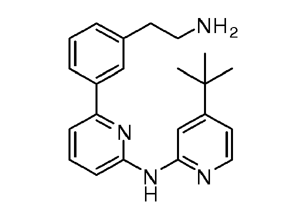

**a) *tert Butyl* [2-(3-{6-[(4-*tert*-butylpyridin-2-yl)amino]pyridin-2-yl}phenyl)ethyl] carbamate**
   Obtained as a oil (49%) from *tert-*butyl {2-[3-(6-aminopyridin-2-yl)phenyl]ethyl}carbamate (preparation 5) and 4-*tert* butyl-2-Chloropyridine (preparation 10b) following the experimental procedure as described in preparation 4 followed by purification by flash chromatography.
   LRMS (m/z): 447 (M+1)⁺.
**b) *N--*{6-[3-(2-Aminoethyl)phenyl]pyridin-2-yl}-4-*tert-*butylpyridin-2-amine**
   Obtained as a solid (22%) from *tert*-butyl [2-(3-{6-[(4-*tert*-butylpyridin-2-yl)amino]pyridin-2-yl}phenyl)ethyl]carbamate (example 18a) following the experimental procedure as described in example 2b.
   LRMS (m/z): 347 (M+1)⁺.
   1 H NMR (400 MHz, CHLOROFORM-*d*) δ ppm 1.35 (s, 9 H) 2.83 (t, J=6.84 Hz, 2 H) 3.01 (br. s., 2 H) 6.89 (dd, J=5.47, 1.76 Hz, 1 H) 7.24 (d, J=7.62 Hz, 1 H) 7.28 (dd, J=7.62, 0.59 Hz, 1 H) 7.34 - 7.41 (m, 2 H) 7.64 (t, J=7.62 Hz, 1 H) 7.84 - 7.90 (m, 3 H) 8.18 (d, J=5.47 Hz, 1 H).

### EXAMPLE 19

### N²-(2-Aminoethy))-N⁶-(4-tert-butylpyridin-2-y))-2,4'-bipyridine-2',6-diamine

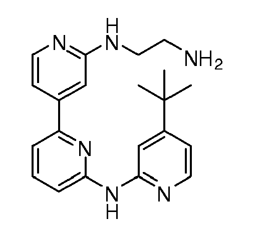

**a) *tert*-Butyl {2-[(6-amino-2,4'-bipyridin-2'-yl)amino]ethyl}carbamate**
   Obtained as a solid (33%) from 2'-fluoro-2,4'-bipyridin-6-amine (preparation 11) and *tert-*butyl (2-aminoethyl)carbamate following the experimental procedure as described in example 10a followed by purification by flash chromatography.
   LRMS (m/z): 330 (M+1)⁺.
**b) *tert-*Butyl [2-({6-[(4-*tert*-butylpyridin-2-yl)amino]-2,4'-bipyridin-2'-yl}amino)ethyl]carbamate**
   Obtained as a solid (14%) from *tert*-butyl {2-[(6-amino-2,4'-bipyridin-2'-yl)amino]ethyl}carbamate (example 19a) and 4-*tert-*butyl-2-Chloropyridine (preparation 10b) following the experimental procedure as described in preparation 12a, followed by purification of the crude product by flash chromatography (3:2 hexanes/ethyl acetate to 100% ethyl acetate).
   LRMS (m/z): 463 (M+1)⁺.
   ¹H NMR (400 MHz, CHLOROFORM-*d*) δ ppm 1.34 (s, 9 H) 2.97 (t, J=5.08 Hz, 2 H) 3.46 (q, J=5.73 Hz, 2 H) 4.90 - 5.03 (m, 1 H) 6.90 (dd, *J*=5.47, 1.56 Hz, 1 H) 7.07 (s, 1 H) 7.18 (dd, J=5.47, 1.37 Hz, 1 H) 7.27 (s, 1 H) 7.48 (d, J=8.01 Hz, 1 H) 7.65 (d, J=7.62 Hz, 1 H) 7.72 (br. s., 1 H) 7.76 (d, J=1.17 Hz, 1 H) 8.07 - 8.26 (m, 2 H)
**c) *N*²-(2-Aminoethyl)-*N*⁶-(4-*tert*-butylpyridin-2-yl)-2,4'-bipyridine-2',6-diamine**
   Obtained as a solid (67%) from *tert-*butyl [2-({6-[(4-*tert*-butylpyridin-2-yl)amino]-2,4'-bipyridin-2'-yl}amino)ethyl]carbamate (example 19b) following the experimental procedure as described in example 2b, followed by purification of the crude product by flash chromatography.
   LRMS (m/z): 363 (M+1)⁺.
   1 H NMR (500 MHz, CHLOROFORM-*d*) δ ppm 1.32 (s, 9 H) 2.98 (d, *J*=6.50 Hz, 2 H) 3.43 - 3.48 (m, 2 H) 4.98 (br. s., 1 H) 6.90 (dd, J=7.00, 2.00 Hz, 1 H) 7.07 (s, 1 H) 7.18 (dd, *J*=7.00, 2.00 Hz, 1 H) 7.27 (d, J=9.50 Hz, 1 H) 7.48 (d, J=10.00 Hz, 1 H) 7.66 (t, *J=1*0.00 Hz, 1 H) 7.72 (br. s., 1 H) 7.76 (br. s., 1 H) 8.16 - 8.19 (m, 2 H).

### EXAMPLE 20

### N-{6-[3-(2-Aminoethyl)phenyl]pyridin-2-yl}-4-(trifluoromethyl)pyridin-2-amine

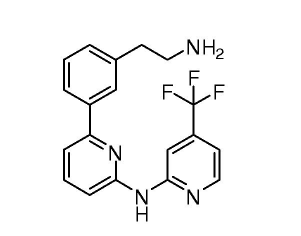

**a) *tert*-Butyl {2-[3-(6-{[4-(trifluoromethyl)pyridin-2-yl]amino}pyridin-2-yl)phenyl] ethyl}carbamate**
   Obtained as a solid (78%) from *tert*-butyl {2-[3-(6-aminopyridin-2-yl)phenyl]ethyl}carbamate (preparation 5) and 2-bromo-4-(trifluoromethyl)pyridine following the experimental procedure as described in preparation 4 followed by purification by flash chromatography (2:1 hexane/ethyl acetate).
   LRMS (m/z): 459 (M+1)⁺.
**b) *N*-{6-[3-(2-Aminoethyl)phenyl]pyridin-2-yl}-4-(trifluoromethyl)pyridin-2-amine**
   Obtained as a solid (83%) from *tert-*butyl {2-[3-(6-{[4-(trifluoromethyl)pyridin-2-yl]amino}pyridin-2-yl)phenyl]ethyl}carbamate (example 20a) following the experimental procedure as described in example 2b.
   LRMS (m/z): 359 (M+1)⁺.
   1 H NMR (400 MHz, CHLOROFORM-*d*) δ ppm 2.80 - 2.91 (m, 2 H) 2.95 - 3.16 (m, 2 H) 7.04 (d, J=7.62 Hz, 1 H) 7.08 (d, J=5.08 Hz, 1 H) 7.26 - 7.30 (m, 1 H) 7.35 - 7.47 (m, 2 H) 7.64 (br. s., 1 H) 7.68 (t, *J*=8.00 Hz, 1 H) 7.85 (d, J=8.00 Hz, 1 H) 7.93 (s, 1 H) 8.41 (d, *J*=5.28 Hz, 1 H) 8.64 (s, 1 H)

### EXAMPLE 21

### N-{6-[3-(1,2,3,6-Tetrahydropyridin-4-y)pheny)]pyridin-2-y)}-4-(trifluoromethy) pyridin-2-amine

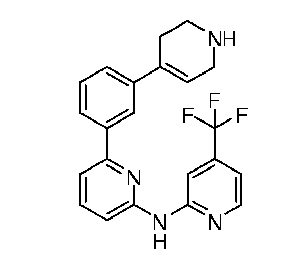

**a) *tert-Butyl* 4-[3-(6-aminopyridin-2-yl)phenyl]-3,6-dihydropyridine-1(*2H*)-Carboxylate**
   Obtained as a oil (60%) from 6-bromopyridin-2-amine and *tert*-butyl 4-[3-(4,4,5-trimethyl-1,3,2-dioxaborolan-2-yl)phenyl]-3,6-dihydropyridine-1 (*2H*)-Carboxylate (preparation 9b) following the experimental procedure as described in preparation 1 followed by purification of the crude product by flash chromatography.
   LRMS (m/z): 352 (M+1)⁺.
**b) *tert*-Butyl 4-[3-(6-{[4-(trifluoromethyl)pyridin-2-yl]amino}pyridin-2-yl)phenyl]-3,6-dihydropyridine-1 (2*H)*-Carboxylate**
   Obtained as a solid (78%) from *tert*-butyl 4-[3-(6-aminopyridin-2-yl)phenyl]-3,6-dihydropyridine-1 (2*H*)-Carboxylate (example 21 a) and 2-bromo-4-(trifluoromethyl)pyridine following the experimental procedure as described in preparation 4 using dioxane as a solvent, followed by purification of the crude product by flash chromatography (1:1 hexanes/ethyl acetate).
   LRMS (m/z): 497 (M+1)⁺.
**c) *N*-{6-[3-(1,2,3,6-Tetrahydropyridin-4-y))pheny)]pyridin-2-y}-4-(trifluoromethyl) pyridin-2-amine**
   Obtained as a solid (53%) from *tert*-butyl *tert*-butyl 4-[3-(6-{[4-(trifluoromethyl)pyridin-2-yl]aminolpyridin-2-yl)phenyl]-3,6-dihydropyridine-1 (2*H*)-Carboxylate (example 21 b) following the experimental procedure as described in example 2b.
   LRMS (m/z): 397 (M+1)⁺.
   1 H NMR (400 MHz, CHLOROFORM-*d*) δ ppm 2.53 (dd, J=4.49, 2.74 Hz, 2 H) 3.14 (t, J=5.67 Hz, 2 H) 3.56 (q, J=2.74 Hz, 2 H) 6.22 (br. s., 1 H) 7.07 (dd, J=5.08, 0.98 Hz, 1 H) 7.12 (d, *J*=7.62 Hz, 1 H) 7.39 (d, J=7.62 Hz, 1 H) 7.42 - 7.48 (m, 2 H) 7.61 (s, 1 H) 7.70 (t, J=7.91 Hz, 1 H) 7.85 - 7.92 (m, 1 H) 8.05 (d, *J=*1.17 Hz, 1 H) 8.41 (d, J=5.28 Hz, 1 H) 8.49 (s, 1 H).

### EXAMPLE 22

### N-[6-(3-Piperidin-4-ylphenyl)pyridin-2-yl]-4-(trifluoromethyl)pyridin-2-amine

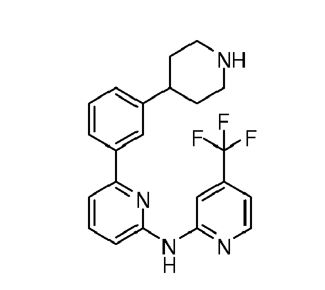

**a) *tert-*Butyl 4-[3-(6-aminopyridin-2-yl)phenyl]piperidine-1-Carboxylate**
   A suspension of *tert-*butyl 4-[3-(6-aminopyridin-2-yl)phenyl]-3,6-dihydropyridine-1(2*H*)-Carboxylate (example 21 a, 0.14 g, 0.40 mmol) and 10% palladium on carbon (0.02 g, catalytic amount) in methanol (6 mL) were hydrogenated at 40 psi at room temperature. After 2 hour, the mixture was filtered through Celite® and the filter cake was washed with methanol. The combined filtrate and washings were evaporated to give the title compound (0.12 g, 55%) as a solid.
   LRMS (m/z): 354 (M+1)⁺.
   1 H NMR (400 MHz, CHLOROFORM-*d*) δ ppm 1.48 (s, 9 H) 1.65 - 1.75 (m, 2 H) 1.86 (d, *J*=12.80 Hz, 2 H) 2.70 - 2.78 (m, 2 H) 2.80 - 2.88 (m, 2 H) 4.28 (br. s., 1 H) 4.54 (br. s., 1 H) 6.45 (d, J=8.00 Hz, 1 H) 7.07 (d, J=7.60 Hz, 1 H) 7.21 (d, *J*=7.60 Hz, 1 H) 7.36 (t, J=7.60 Hz, 1 H) 7.48 (t, J=8.00 Hz, 1 H) 7.73 (d, *J*=8.00 Hz, 1 H) 7.80 (s, 1 H)
**b) *tert*-Butyl 4-[3-(6-{[4-(trifluoromethyl)pyridin-2-yl]amino}pyridin-2-yl)phenyl]piperidine-1-Carboxylate**
   Obtained as a solid (45%) from *tert-*butyl 4-[3-(6-aminopyridin-2-yl)phenyl]piperidine-1-Carboxylate (example 22a) and 2-bromo-4-(trifluoromethyl)pyridine following the experimental procedure as described in preparation 4 using dioxane as a solvent, followed by purification of the crude product by flash chromatography (99:1 dichloromethane/methanol).
   LRMS (m/z): 499 (M+1)⁺.
**c) *N*-[6-(3-Piperidin-4-ylphenyl)pyridin-2-yl]-4-(trifluoromethyl)pyridin-2-amine**
   Obtained as a solid (89%) from *tert-*butyl 4-[3-(6-{[4-(trifluoromethyl)pyridin-2-yl]amino}pyridin-2-yl)phenyl]piperidine-1-Carboxylate (example 22b) following the experimental procedure as described in example 2b.
   LRMS (m/z): 399 (M+1)⁺.
   1 H NMR (400 MHz, CHLOROFORM-*d*) δ ppm 1.63 - 1.96 (m, 4 H) 2.78 (t, J=12.21 Hz, 3 H) 3.22 (d, *J*=12.31 Hz, 2 H) 7.06 (t, J=7.72 Hz, 2 H) 7.30 (d, J=7.42 Hz, 1 H) 7.38 (d, J=7.62 Hz, 1 H) 7.42 (t, J=7.62 Hz, 1 H) 7.68 (t, *J*=7.82 Hz, 1 H) 7.76 (br. s., 1 H) 7.82 (d, *J*=7.82 Hz, 1 H) 7.94 (s, 1 H) 8.41 (d, *J*=5.08 Hz, 1 H) 8.60 (s, 1 H)

### EXAMPLE 23

### N-[6-(3-Piperazin-1-ylphenyl)pyridin-2-yl]-4-(trifluoromethyl)pyridin-2-amine

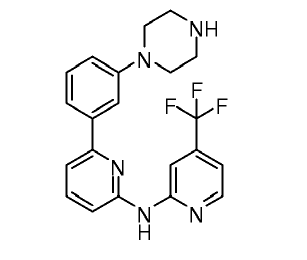

**a) *tert-*Butyl 4-[3-(6-aminopyridin-2-yl)phenyl]piperazine-1-Carboxylate**
   Obtained as a solid (62%) from 6-bromopyridin-2-amine and *tert-*butyl 4-[3-(4,4,5,5-tetramethyl-1,3,2-dioxaborolan-2-yl)phenyl]piperazine-1-Carboxylate (preparation 13b) following the experimental procedure as described in preparation 1 followed by purification of the crude product by flash chromatography.
   LRMS (m/z): 355 (M+1)⁺.
**b) *tert*-Butyl 4-[3-(6-{[4-(trifluoromethyl)pyridin-2-yl]amino}pyridin-2-yl)phenyl]piperazine-1-Carboxylate**
   Obtained as a solid (65%) from *tert-*butyl 4-[3-(6-aminopyridin-2-yl)phenyl]piperazine-1-Carboxylate (example 23a) and 2-bromo-4-(trifluoromethyl)pyridine following the experimental procedure as described in preparation 4 using dioxane as a solvent, followed by purification of the crude product by flash chromatography (7:3 hexanes/ethyl acetate).
   LRMS (m/z): 500 (M+1)⁺.
**c) N-[6-(3-Piperazin-1-ylphenyl)pyridin-2-yl]-4-(trifluoromethyl)pyridin-2-amine**
   Obtained as a solid (83%) from *tert-*butyl 4-[3-(6-{[4-(trifluoromethyl)pyridin-2-yl]aminolpyridin-2-yl)phenyl]piperazine-1-Carboxylate (example 23b) following the experimental procedure as described in example 2b.
   LRMS (m/z): 400 (M+1)⁺.
   1 H NMR (400 MHz, CHLOROFORM-*d*) δ ppm 3.03 - 3.10 (m, 4 H) 3.18 - 3.28 (m, 4 H) 7.00 (dd, *J*=8.21, 1.76 Hz, 1 H) 7.06 (dd, J=5.08, 0.98 Hz, 1 H) 7.12 (d, J=8.21 Hz, 1 H) 7.33 - 7.40 (m, 2 H) 7.43 - 7.49 (m, 1 H) 7.63 (t, J=2.15 Hz, 1 H) 7.68 (t, *J*=7.91 Hz, 1 H) 7.72 (br. s., 1 H) 8.40 (d, *J*=5.08 Hz, 1 H) 8.42 (s, 1 H)

### EXAMPLE 24

### 2-[3-(6-{[4-(Trifluoromethyl)pyridin-2-yl]amino}pyridin-2-yl)phenyl]acetamide

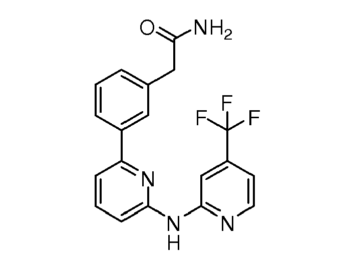

Obtained as a solid (10%) from *N*-(6-bromopyridin-2-yl)-4-(trifluoromethyl)pyridin-2-amine (preparation 15) and 2-(3-(4,4,5,5-tetramethyl-1,3,2-dioxaborolan-2-yl)phenyl)acetamide following the experimental procedure as described in preparation 1, followed by purification by flash chromatography and precipitation by methanol.
LRMS (m/z): 373 (M+1)⁺.
1 H NMR (200 MHz, DMSO-*d₆*) δ ppm 3.41 - 3.45 (m, 2 H) 6.91 (br. s., 1 H) 7.20 (d, *J*=5.20 Hz, 1 H) 7.33 (t, J=7.20 Hz, 1 H) 7.40 - 7.49 (m, 3 H) 7.79 (t, J=8.20 Hz, 1 H) 7.90 (d, J=8.80 Hz, 1 H) 7.92 (s, 1 H) 8.49 (d, J=5.00 Hz, 1 H) 8.62 (s, 1 H) 10.27 (s, 1 H)

### EXAMPLE 25

### N²-(2-Aminoethyl)-N⁶-[4-(trifluoromethyl)pyridin-2-yl]-2,4'-bipyridine-2',6-diamine

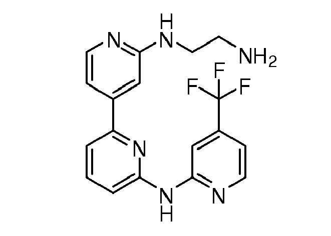

**a) *tert*-Butyl {2-[(6-{[4-(trifluoromethy))pyridin-2-y)]amino}-2,4'-bipyridin-2'-yl)amino]ethyl}carbamate**
   Obtained as a solid (67%) from *tert-*butyl {2-[(6-amino-2,4'-bipyridin-2'-yl)amino]ethyl}carbamate (example 19a) and 2-bromo-4-(trifluoromethyl)pyridine following the experimental procedure as described in preparation 4 followed by purification by flash chromatography (1:1 hexanes/ethyl acetate to 100% ethyl acetate).
   LRMS (m/z): 475 (M+1)⁺.

   1 H NMR (500 MHz, CHLOROFORM-*d*) δ ppm 1.44 (s, 9 H) 3.40 (d, J=6.50 Hz, 2 H) 3.55 (d, *J*=7.00 Hz, 2 H) 4.92 (br. s., 1 H) 5.12 (br. s., 1 H) 7.08 (s, 2 H) 7.13 (d, J=6.50 Hz, 1 H) 7.22 (d, *J*=10.50 Hz, 1 H) 7.37 (d, *J*=9.00 Hz, 1 H) 7.70 (t, *J*=10.00 Hz, 1 H) 7.80 (br. s., 1 H) 8.18 (d, J=6.50 Hz, 1 H) 8.41 (d, J=6.00 Hz, 1 H) 8.46 (s, 1 H)
**b) *N*²-(2-Aminoethyl)-*N*⁶-[4-(trifluoromethyl)pyridin-2-yl]-2,4'-bipyridine-2',6-diamine**
   Obtained as a solid from *tert-*butyl {2-[(6-{[4-(trifluoromethyl)pyridin-2-yl]amino}-2,4'-bipyridin-2'-yl)amino]ethyl}carbamate (example 25a) following the experimental procedure as described in example 2b.
   LRMS (m/z): 375 (M+1)⁺.
   ¹H NMR (400 MHz, METHANOL-*d*₄) δ ppm 3.34 (t, J=6.06 Hz, 2 H) 6.98 (dd, J=5.18, 1.07 Hz, 1 H) 7.01 (s, 1 H) 7.08 (dd, J=5.47, 1.37 Hz, 1 H) 7.29 (d, J=8.60 Hz, 2 H) 7.59 (t, 1 H) 7.90 (d, J=5.47 Hz, 1 H) 8.29 (d, J=5.28 Hz, 1 H) 8.39 (s, 1 H)

### EXAMPLE 26

### 2'-Piperazin-1-y)-N-[4-(trifluoromethy))pyridin-2-y)]-2,4'-bipyridin-6-amine

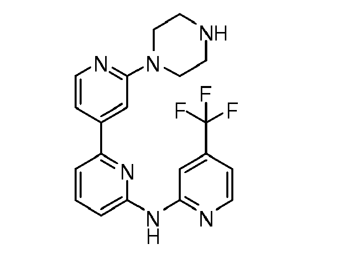

**a) *tert-*Butyl 4-(6-amino-2,4'-bipyridin-2'-yl)piperazine-1-Carboxylate**
   Obtained as a oil (77%) from *tert-*butyl piperazine-1-Carboxylate and 2'-fluoro-2,4'-bipyridin-6-amine (preparation 11) following the experimental procedure as described in example 10a followed by purification of the crude product by flash chromatography (3:2 hexanes/ethyl acetate to 100% ethyl acetate).
   LRMS (m/z): 356 (M+1)⁺.
**b) *tert*-Butyl 4-(6-{[4-(trifluoromethyl)pyridin-2-yl]amino}-2,4'-bipyridin-2'-yl)piperazine-1-Carboxylate**
   Obtained as a solid (82%) from *tert-*butyl 4-(6-amino-2,4'-bipyridin-2'-yl)piperazine-1-Carboxylate (example 26a) and 2-bromo-4-(trifluoromethyl)pyridine following the experimental procedure as described in preparation 4 using dioxane as a solvent, followed by purification of the crude product by flash chromatography (3:2 hexanes/ethyl acetate to 100% ethyl acetate).
   LRMS (m/z): 501 (M+1)⁺.
   1 H NMR (500 MHz, CHLOROFORM-*d*) δ ppm 1.50 (s, 9 H) 3.57 - 3.63 (m, 8 H) 7.09 (dd, *J*=6.50, 1.00 Hz, 1 H) 7.16 (dd, J=7.00, 1.50 Hz, 1 H) 7.21 (d, J=10.50 Hz, 1 H) 7.53 (br. s., 1 H) 7.39 (d, J=8.50 Hz, 1 H) 7.72 (t, J=10.00 Hz, 2 H) 8.29 (d, J=6.50 Hz, 1 H) 7.41 (br. s., 1 H) 8.42 (d, J=7.50 Hz, 1 H)
**c) 2'-Piperazin-1-y)-*N*-[4-(trif)uoromethy))pyridin-2-y)]-2,4'-bipyridin-6-amine**
   Obtained as a solid (98%) from *tert-*butyl 4-(6-{[4-(trifluoromethyl)pyridin-2-yl]amino}-2,4'-bipyridin-2'-yl)piperazine-1-Carboxylate (example 26b) following the experimental procedure as described in example 2b.
   LRMS (m/z): 401 (M+1)⁺.
   1 H NMR (400 MHz, CHLOROFORM-*d*) δ ppm 3.01 - 3.04 (m, 4 H) 3.59 - 3.62 (m, 4 H) 7.09 (dd, J=6.00, 1.5 Hz, 1 H) 7.14 (dd, J=6.50, 1.50 Hz, 1 H) 7.19 (d, *J*=10.00 Hz, 1 H) 7.35 (s, 1 H) 7.39 (d, *J*=9.00 Hz, 1 H) 7.66 (br. s., 1 H) 7.72 (t, J=9.50 Hz, 1 H) 8.29 (d, J=7.00 Hz, 1 H) 8.42 (d, J=2.50 Hz, 1 H) 8.42 (s, 1 H)

### EXAMPLE 27

### N-{6-[3-(2-Aminoethyl)phenyl]pyridin-2-yl}-4-(2-methoxyethoxy)pyridin-2-amine

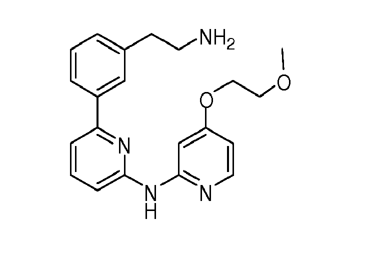

**a) *tert-*Butyl {2-[3-(6-{[4-(2-methoxyethoxy)pyridin-2-yl]amino}pyridin-2-yl)phenyl]ethyl}carbamate**
   Obtained as a oil (87%) from *tert-butyl* {2-[3-(6-aminopyridin-2-yl)phenyl]ethyl}carbamate (preparation 5) and 2-Chloro-4-(2-methoxyethoxy)pyridine (preparation 14) following the experimental procedure as described in preparation 4 followed by purification by flash chromatography.
   LRMS (m/z): 465 (M+1)⁺.
**b) *N*-]6-[3-(2-Aminoethyl)phenyl]pyridin-2-yl}-4-(2-methoxyethoxy)pyridin-2-amine**
   Obtained as a solid (88%) from *tert-*butyl {2-[3-(6-{[4-(2-methoxyethoxy)pyridin-2-yl]amino}pyridin-2-yl)phenyl]ethyl}carbamate (example 27a) following the experimental procedure as described in example 2b.
   LRMS (m/z): 365 (M+1)⁺.
   1 H NMR (400 MHz, METHANOL-*d*₄) δ ppm 3.06 (t, J=7.62 Hz, 2 H) 3.26 (t, *J*=7.52 Hz, 2 H) 3.43 (s, 3 H) 3.73 - 3.83 (m, 2 H) 4.18 - 4.29 (m, 2 H) 6.60 (dd, *J*=5.96, 2.44 Hz, 1 H) 7.23 (d, *J*=8.40 Hz, 1 H) 7.35 (d, J=7.82 Hz, 1 H) 7.42 (d, J=7.62 Hz, 1 H) 7.48 (t, J=7.62 Hz, 1 H) 7.70 - 7.80 (m, 2 H) 7.94 - 7.99 (m, 2 H) 8.04 (d, J=6.06 Hz, 1 H) 8.48 (br. s., 1 H)

### EXAMPLE 28

### N-{6-[3-(2-Aminoethyl)phenyl]pyridin-2-yl}pyrazin-2-amine

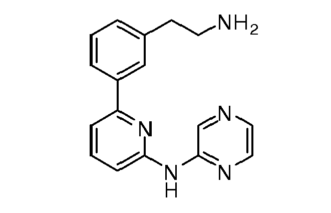

**a) *tert-*Butyl (2-{3-[6-(pyrazin-2-ylamino)pyridin-2-yl]phenyl}ethyl)carbamate**
   Obtained as a solid (58%) from *N*-(6-bromopyridin-2-yl)pyrazin-2-amine (preparation 4) and (3-{2-[(*tert-*butoxycarbonyl)amino]ethyl}phenyl)boronic acid (preparation 3b) following the experimental procedure as described in preparation 1 followed by purification by flash chromatography.
   1 H NMR (400 MHz, CHLOROFORM-*d*) δ ppm 1.43 (s, 9 H) 2.88 (m, 2 H) 3.42 (m, 2 H) 4.78 (br. s., 1 H) 7.23 (d, J=6.80 Hz, 1 H) 7.37 (t, *J*=8.10 Hz, 1 H) 7.54 (d, *J*=8.10 Hz, 1 H) 7.71 (t, J=8.10 Hz, 1 H) 7.81 (s, 1 H) 7.86 (d, *J*=7.60 Hz, 1 H) 7.95 (br. s., 1 H) 8.10 (s, 1 H) 8.17 (s, 1 H) 8.99 (s, 1 H)
**b) *N*-{6-[3-(2-Aminoethyl)phenyl]pyridin-2-yl}pyrazin-2-amine**
   Obtained as a solid (75%) from *tert-*butyl (2-{3-[6-(pyrazin-2-ylamino)pyridin-2-yl]phenyl}ethyl)carbamate (example 28a) following the experimental procedure as described in example 2b.
   1 H NMR (400 MHz, METHANOL-*d*₄) δ ppm 2.82 - 2.90 (m, 2 H) 2.91 - 2.99 (m, 2 H) 7.25-7.31 (m, 1 H) 7.38-7.45 (m, 2 H) 7.46 (d, *J*=1.17 Hz, 1 H) 7.74 (dd, *J*=8.30, 7.52 Hz, 1 H) 7.86 - 7.91 (m, 1 H) 7.93 (br. s., 1 H) 8.03 (d, J=2.74 Hz, 1 H) 8.25 (dd, J=2.64, 1.47 Hz, 1 H) 9.32 (d, J=1.56 Hz, 1 H)

### EXAMPLE 29

### N-{6-[3-(1,2,3,6-Tetrahydropyridin-4-y))pheny)]pyridin-2-y)}pyrazin-2-amine

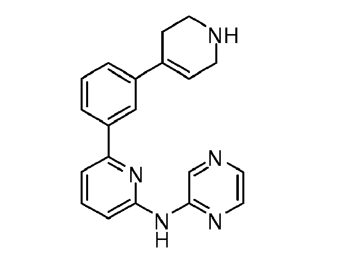

**a) *tert-*Butyl 4-{3-[6-(pyrazin-2-ylamino)pyridin-2-yl]phenyl}-3,6-dihydropyridine-1(*2H*)-Carboxylate**
   Obtained as a solid (37%) from *tert-*butyl 4-[3-(6-aminopyridin-2-yl)phenyl]-3,6-dihydropyridine-1(2*H*)-Carboxylate (example 21 a) and 2-Chloropyrazine following the experimental procedure as described in preparation 4, followed by purification of the crude product by flash chromatography (9:1 hexanes/ethyl acetate to 1:4 hexanes/ethyl acetate).
   LRMS (m/z): 430 (M+1)⁺.
**b) *N*-{6-[3-(1,2,3,6-Tetrahydropyridin-4-yl)phenyl]pyridin-2-yl}pyrazin-2-amine**
   Obtained as a solid (58%) from *tert*-butyl 4-{3-[6-(pyrazin-2-ylamino)pyridin-2-yl]phenyl}-3,6-dihydropyridine-1 (2*H*)-Carboxylate (example 29a) following the experimental procedure as described in example 2b.
   LRMS (m/z): 330 (M+1)⁺.
   1 H NMR (400 MHz, CHLOROFORM-*d*) δ ppm 2.48 - 2.59 (m, 2 H) 3.14 (t, J=5.67 Hz, 2 H) 3.56 (q, *J*=2.87 Hz, 2 H) 6.22 (dt, *J*=3.17, 1.64 Hz, 1 H) 7.39 (d, J=7.62 Hz, 1 H) 7.44 (d, J=5.08 Hz, 2 H) 7.49 (d, J=8.01 Hz, 1 H) 7.62 (br. s., 1 H) 7.72 (t, J=7.91 Hz, 1 H) 7.85 - 7.92 (m, 1 H) 8.04 (s, 1 H) 8.12 (d, J=2.54 Hz, 1 H) 8.19 (dd, *J*=2.64, 1.47 Hz, 1 H) 9.08 (s, 1 H)

### EXAMPLE 30

### N-[6-(3-Piperidin-4-ylphenyl)pyridin-2-yl]pyrazin-2-amine

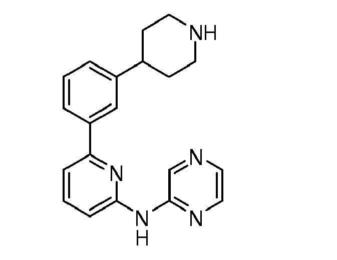

**a) *tert*-Butyl 4-{3-[6-(pyrazin-2-ylamino)pyridin-2-yl]phenyl}piperidine-1-Carboxylate**
   Obtained as a solid (42%) from *tert-*butyl 4-[3-(6-aminopyridin-2-yl)phenyl]piperidine-1-Carboxylate (example 22a) and 2-bromopyrazine following the experimental procedure as described in preparation 12a, followed by purification of the crude product by flash chromatography (99:1 dichloromethane/methanol to 9:1 dichloromethane/methanol).
   LRMS (m/z): 432 (M+1)⁺.
**b) *N-*[6-(3-Piperidin-4-ylphenyl)pyridin-2-yl]pyrazin-2-amine**
   Obtained as a solid (72%) from *tert-*butyl 4-{3-[6-(pyrazin-2-ylamino)pyridin-2-yl]phenyl}piperidine-1-Carboxylate (example 30a) following the experimental procedure as described in example 2b.
   LRMS (m/z): 332 (M+1)⁺.
   1 H NMR (400 MHz, CHLOROFORM-*d*) δ ppm 1.67 - 1.82 (m, 2 H) 1.84 - 1.94 (m, 1H) 2.67-2.74 (m, 2 H) 2.79 (td, *J*=12.11, 1.95 Hz, 2 H) 3.24 (d, *J*=12.11 Hz, 2 H) 7.28 (d, J=7.62 Hz, 1 H) 7.38 (d, *J*=7.62 Hz, 1 H) 7.41 (t, *J*=7.72 Hz, 1 H) 7.47 (d, J=8.21 Hz, 1 H) 7.71 (t, J=7.91 Hz, 1 H) 7.84 (d, J=7.82 Hz, 1 H) 7.91 (t, J=1.66 Hz, 1 H) 8.12 (d, J=2.54 Hz, 1 H) 8.20 (dd, J=2.64, 1.47 Hz, 1 H) 9.07 - 9.17 (m, 1 H) 9.10 (d, *J*=1.56 Hz, 1 H)

### EXAMPLE 31

### 2-]3-[6-(Pyrazin-2-ylamino)pyridin-2-yl]phenyl}acetamide

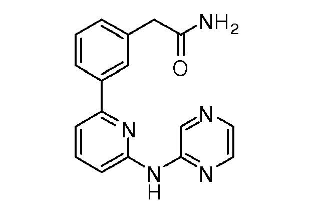

Obtained as a solid (37%) from *N*-(6-bromopyridin-2-yl)pyrazin-2-amine (preparation 4) and 2-[3-(4,4,5,5-tetramethyl-1,3,2-dioxaborolan-2-yl)phenyl]acetamide following the experimental procedure as described in preparation 1, followed by purification of the crude product by flash chromatography (99:1 dichloromethane/methanol to 9:1 dichloromethane/methanol).
LRMS (m/z): 306 (M+1)⁺.
1 H NMR (400 MHz, DMSO-*d*₆) δ ppm 3.46 (s, 2 H) 6.89 (br. s., 1 H) 7.32 (d, J=7.62 Hz, 1 H) 7.38 - 7.49 (m, 2 H) 7.50 (br. s., 1 H) 7.67 (d, *J*=8.40 Hz, 1 H) 7.80 (t, *J*=7.91 Hz, 1 H) 7.90 (d, J=7.62 Hz, 1 H) 7.94 (s, 1 H) 8.09 (d, J=2.54 Hz, 1 H) 8.24 (br. s., 1 H) 9.15(s, 1 H) 10.10 (s, 1 H)

### EXAMPLE 32

### N-[6-(3-Piperazin-1-ylphenyl)pyridin-2-yl]pyrazin-2-amine

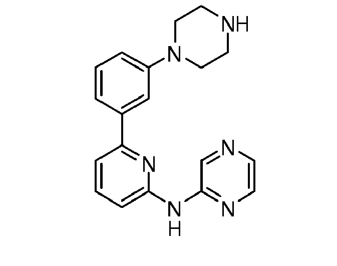

**a) *tert*-Butyl 4-{3-[6-(pyrazin-2-ylamino)pyridin-2-yl]phenyl}piperazine-1-Carboxylate**
   Obtained (40%) from *N*-(6-bromopyridin-2-yl)pyrazin-2-amine (preparation 4) and *tert-*butyl 4-[3-(4,4,5,5-tetramethyl-1,3,2-dioxaborolan-2-yl)phenyl]piperazine-1-Carboxylate (preparation 13) following the experimental procedure as described in preparation 1 using microwave at 100 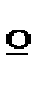 C for 30 minutes, followed by purification by flash chromatography.
   LRMS (m/z): 433 (M+1)⁺.
   1 H NMR (500 MHz, CHLOROFORM-*d*) δ ppm 1.48 (s, 9 H) 3.15 - 3.18 (m, 4 H) 3.55 - 3.58 (m, 4 H) 6.96 (dd, *J*=10.50, 2 Hz, 1 H) 7.33 - 7.37 (m, 2 H) 7.48 (d, J=10.00 Hz, 1 H) 7.53 (d, *J*=10.00 Hz, 1 H) 7.64 (br. s., 1 H) 7.70 (t, J=10.00 Hz, 1 H) 8.08 (d, J=3.00 Hz, 2 H) 8.1 6 - 8.17 (m, 1 H) 8.90 (br. s., 1 H)
**b) *N-*[6-(3-Piperazin-1-ylphenyl)pyridin-2-yl]pyrazin-2-amine**
   Obtained as a solid (96%) from *tert-*butyl 4-{3-[6-(pyrazin-2-ylamino)pyridin-2-yl]phenyl}piperazine-1-Carboxylate (example 32a) following the experimental procedure as described in example 2b.
   LRMS (m/z): 333 (M+1)⁺.
   ¹H NMR (400 MHz, METHANOL-*d*₄) □ ppm 2.92 - 3.09 (m, 4 H) 3.14 - 3.27 (m, 4 H) 7.00 (dd, J=8.11, 2.25 Hz, 1 H) 7.31 (t, J=7.91 Hz, 1 H) 7.39 (dd, J=7.81, 5.86 Hz, 2 H) 7.48 (d, J=7.82 Hz, 1 H) 7.61 - 7.73 (m, 2 H) 8.00 (d, J=2.74 Hz, 1 H) 8.22 (dd, J=2.64, 1.47 Hz, 1 H) 9.28 (d, *J*=1.56 Hz, 1 H)

### EXAMPLE 33

### N²-(2-Aminoethyl)-N⁶-pyrazin-2-yl-2,4'-bipyridine-2',6-diamine

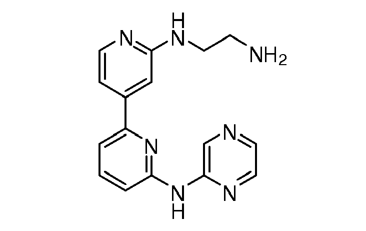

**a) *tert*-Butyl (2-{[6-(pyrazin-2-ylamino)-2,4'-bipyridin-2'-yl]amino}ethyl)carbamate**
   Obtained as a solid (50%) from 2'-fluoro-*N*-pyrazin-2-yl-2,4'-bipyridin-6-amine (preparation 8) and *tert*-butyl (2-aminoethyl)carbamate following the experimental procedure as described in example 10a followed by purification of the crude product by flash chromatography.
   LRMS (m/z): 408 (M+1)⁺.
   1 H NMR (400 MHz, CHLOROFORM-*d*) δ ppm 1.45 (s, 9 H) 3.39 - 3.42 (m, 2 H) 3.51 - 3.56 (m, 2 H) 5.00 (br. s., 1 H) 5.76 (br. s., 1 H) 7.12 (d, J=5.20 Hz, 1 H) 7.17 (s, 1 H) 7.37 - 7.45 (m, 3 H) 7. 73 (t, *J*=8.00 Hz, 1 H) 8.15 - 8.18 (m, 2 H) 8.22 - 8.23 (m, 1 H) 9.35 (br. s., 1 H)
**b) *N²*-(2-Aminoethyl)-*N⁶*-pyrazin-2-yl-2,4'-bipyridine-2',6-diamine**
   Obtained as a solid from *tert-*butyl (2-{[6-(pyrazin-2-ylamino)-2,4'-bipyridin-2'-yl]amino}ethyl)carbamate (example 33a) following the experimental procedure as described in example 2b.
   LRMS (m/z): 308 (M+1)⁺.
   ¹H NMR (400 MHz, DMSO-*d*₆) □ ppm 2.90 (t, J=6.25 Hz, 2 H) 3.42 (q, J=6.12 Hz, 2 H) 7.11 (s, 1 H) 7.13 (dd, J=5.37, 1.47 Hz, 1 H) 7.45 (d, J=7.42 Hz, 1 H) 7.58 (d, *J*=8.21 Hz, 1 H) 7.81 (t, 1 H) 8.10 (d, J=5.47 Hz, 1 H) 8.12 (d, *J*=2.54 Hz, 1 H) 8.26 (dd, J=2.54, 1.56 Hz, 1 H) 9.30 (d, J=1.37 Hz, 1 H)

### EXAMPLE 34

### 2'-(4-Methylpiperazin-1-yl)-N-pyrazin-2-yl-2,4'-bipyridin-6-amine

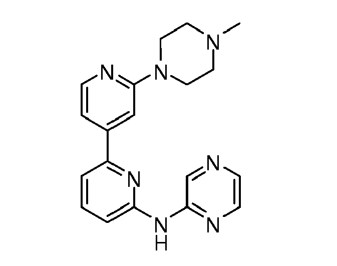

**a) 2'-(4-Methylpiperazin-1-yl)-2,4'-bipyridin-6-amine**
   Obtained as a solid (80%) from 2'-fluoro-2,4'-bipyridin-6-amine (preparation 11) and 1-methylpiperazine following the experimental procedure as described in example 10a, followed by purification of the crude product by flash chromatography.
   LRMS (m/z): 270 (M+1)⁺.
   1 H NMR (500 MHz, CHLOROFORM-*d*) δ ppm 2.35 (s, 3 H) 2.53 - 2.55 (m, 4 H) 3.63 - 3.66 (m, 4 H) 4.62 (br. s., 2 H) 6.51 (d, J=10.50 Hz, 1 H) 7.07 - 7.11 (m, 2 H) 7.28 (s, 1 H) 7.51 (t, J=10.00 Hz, 1 H) 8.24 (d, J=7.00 Hz, 1 H)
**b) 2'-(4-Methylpiperazin-1-yl)-*N*-pyrazin-2-yl-2,4'-bipyridin-6-amine**
   An oven dried resealable Schlenk tube was charged with 2'-(4-methylpiperazin-1-yl)-2,4'-bipyridin-6-amine (example 34a, 0.09 g, 0.33 mmol), 2-Chloropyrazine (0.04 µL, 0.39 mmol), sodium *tert-*butoxide (0.04 g, 0.38 mmol) and dioxane (1 mL). The Schlenk tube was subjected to three cycles of evacuation-backfilling with argon, and tris(dibenzylideneacetone)dipalladium(0) (0.01 g, catalytic amount) and 4,5-bis(diphenylphosphino)-9,9-dimethylxanthene (0.01 g, catalytic amount) were added. After three further cycles of evacuation-backfilling with argon, the Schlenk tube was capped and placed in an oil bath at 95 °C. After 16h, the mixture was cooled and the solvent was removed in vacuo. The residue was purified by flash chromatography to give the title compound (0.09 g, 82%) as a solid.
   LRMS (m/z): 348 (M+1)⁺.
   1 H NMR (500 MHz, CHLOROFORM-*d*) δ ppm 2.35 (s, 3 H) 2.53 - 2.55 (m, 4 H) 3.63 - 3.66 (m, 4 H) 7.16 (dd, J=6.50, 2 Hz, 1 H) 7.34 (s, 1 H) 7.39 (d, J=9.00 Hz, 1 H) 7.61 (d, J=10.00 Hz, 1 H) 7.73 (t, J=10.00 Hz, 1 H) 7.96 (br. s., 1 H) 8.13 (d, J=3.50 Hz, 1 H) 8.19 - 8.21 (m, 1 H) 8.13 (d, J=3.50 Hz, 1 H) 8.98 (d, *J*=2.00 Hz, 1 H)

### EXAMPLE 35

### 2'-Piperazin-1-yl-N-pyrazin-2-yl-2,4'-bipyridin-6-amine

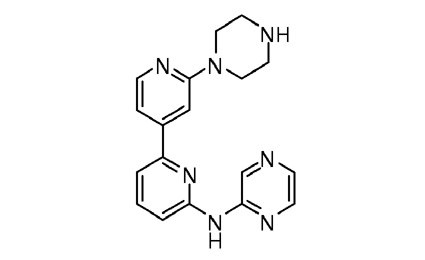

**a) tert-Butyl 4-(6-amino-2,4'-bipyridin-2'-yl)piperazine-1-Carboxylate**
   Obtained as a solid (76%) from 2'-fluoro-2,4'-bipyridin-6-amine (preparation 11) and *tert-*butyl piperazine-1-Carboxylate following the experimental procedure as described in example 10a, followed by purification of the crude product by flash chromatography.
   LRMS (m/z): 356 (M+1)⁺.
**b) *tert-*Butyl 4-[6-(pyrazin-2-ylamino)-2,4'-bipyridin-2'-yl]piperazine-1-Carboxylate** Obtained as a solid (40%) from *tert-*butyl 4-(6-amino-2,4'-bipyridin-2'-yl)piperazine-1-Carboxylate (example 35a) and 2-Chloropyrazine following the experimental procedure as described in example 34b, followed by purification of the crude product by flash chromatography.
   LRMS (m/z): 434 (M+1)⁺.
   1 H NMR (500 MHz, CHLOROFORM-*d*) δ ppm 1.48 (s, 9 H) 3.53 - 3.58 (m, 8 H) 7.16 (d, J=6.50 Hz, 1 H) 7.32 (s, 1 H) 7.37 (d, *J*=9.00 Hz, 1 H) 7.60 (d, *J*=10.00 Hz, 1 H) 7.72 (t, J=10.00 Hz, 1 H) 7.96 (br. s., 1 H) 8.10 - 8.12 (m, 1 H) 8.17 - 8.18 (m, 1 H) 8.26 (d, J=6.50 Hz, 1 H) 8.97 (d, J=2.00 Hz, 1 H)
**c) 2'-Piperazin-1-yl-*N-*pyrazin-2-yl-2,4'-bipyridin-6-amine**
   Obtained as a solid (73%) from *tert-*butyl 4-[6-(pyrazin-2-ylamino)-2,4'-bipyridin-2'-yl]piperazine-1-Carboxylate (example 35b) following the experimental procedure as described in example 2b.
   LRMS (m/z): 334 (M+1)⁺.
   1 H NMR (400 MHz, CHLOROFORM-*d*) δ ppm 2.94 - 2.97 (m, 4 H) 3.53 - 3.56 (m, 4 H) 7.24 (dd, J=6.50, 1.50 Hz, 1 H) 7.42 (s, 1 H) 7.44 (d, J=7.60 Hz, 1 H) 7.51 (d, J=8.00 Hz, 1 H) 7.71 (t, J=7.60 Hz, 1 H) 8.02 (d, *J*=2.40 Hz, 1 H) 8.14 (d, *J*=5.60 Hz, 1 H) 8.21 - 8.22 (m, 1 H) 9.19 (d, J=1.20 Hz, 1 H)

### EXAMPLE 36

### 2'-(4-Acetylpiperazin-1-yl)-N-pyrazin-2-yl-2,4'-bipyridin-6-amine

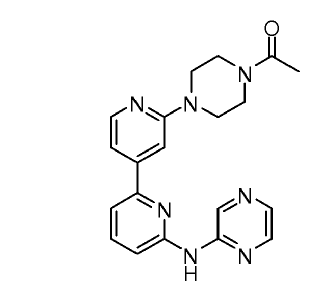

**a) 2'-(4-Acetylpiperazin-1-yl)-2,4'-bipyridin-6-amine**
   Obtained (77%) from 2'-fluoro-2,4'-bipyridin-6-amine (preparation 11) and 1-acetylpiperazine following the experimental procedure as described in example 10a, followed by purification of the crude product by flash chromatography.
   LRMS (m/z): 298 (M+1)⁺.
**b) 2'-(4-Acetylpiperazin-1-yl)-*N*-pyrazin-2-yl-2,4'-bipyridin-6-amine**
   Obtained as a solid (49%) from 2'-(4-acetylpiperazin-1-yl)-2,4'-bipyridin-6-amine (example 36a) and 2-Chloropyrazine following the experimental procedure as described in example 34b, followed by purification of the crude product by flash chromatography.
   LRMS (m/z): 376 (M+1)⁺.
   1 H NMR (400 MHz, METHANOL-*d*₄) δ ppm 2.13 (s, 3 H) 3.48 - 3.73 (m, 8 H) 7.21 (dd, J=5.28, 1.37 Hz, 1 H) 7.36 - 7.49 (m, 3 H) 7.67 (dd, *J*=8.30, 7.52 Hz, 1 H) 8.01 (d, *J*=2.74 Hz, 1 H) 8.12 (d, J=5.28 Hz, 1 H) 8.21 (dd, J=2.74, 1.56 Hz, 1 H) 9.21 (d, J=1.37 Hz, 1 H)

### EXAMPLE 37

### (4r,7r)-4-{[6-(Pyrazin-2-ylamino)-2,4'-bipyridin-2'-yl]amino}adamantan-1-ol

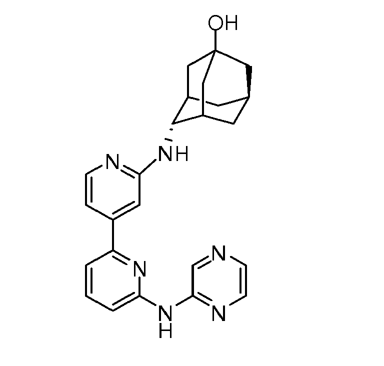

Obtained (12%) from 2'-fluoro-*N*-pyrazin-2-yl-2,4'-bipyridin-6-amine (preparation 8) and (4*r*,7*r*)-4-aminoadamantan-1-ol (sintetized following the procedure described in Tetrahedron letters, 2006, vol. 47, 46, 8063-8067) following the procedure as described in preparation 24a, heating at 140°C for 48 hour and followed by purification by reverse phase chromatography (0% CH₃OH in H₂O to 80% CH₃OH in H₂O).
LRMS (m/z): 415 (M+1)⁺.
1 H NMR (400 MHz, DMSO-*d*₆) δ ppm 1.34 (d, J=12.50 Hz, 3 H) 1.53 - 1.87 (m, 6 H) 1.92 - 2.06 (m, 3 H) 2.10 (br. s., 2 H) 3.98 (br. s., 1 H) 6.55 (d, J=7.42 Hz, 1 H) 7.05 (d, J=5.47 Hz, 1 H) 7.41 (d, J=7.42 Hz, 1 H) 7.63 (d, J=8.21 Hz, 1 H) 7.83 (t, *J*=7.82 Hz, 1 H) 8.07 (d, J=5.47 Hz, 1 H) 8.13 (d, J=2.74 Hz, 1 H) 8.27 (br. s., 2 H) 9.28 (s, 1 H) 10.11 (s, 1 H)

### EXAMPLE 38

### N²-[(1 S,2S,4R)-Bicyclo[2.2.1]hept-2-yl]-N⁶-pyrazin-2-yl-2,4'-bipyridine-2',6-diamine

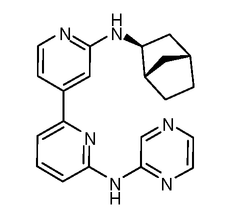

Obtained (47%) from 2'-fluoro-*N*-pyrazin-2-yl-2,4'-bipyridin-6-amine (preparation 8) and (1*S*,2*S*,4*R*)-bicyclo[2.2.1]hept-2-ylamine following the procedure as described in preparation 24a, heating at 140°C for 48 hour, followed by purification by reverse phase chromatography (0% CH₃OH in H₂O to 60% CH₃OH in H₂O).
LRMS (m/z): 359 (M+1)⁺.
1 H NMR (400 MHz, DMSO-*d*₆) □ ppm 1.04 - 1.59 (m, 7 H) 1.65 - 1.81 (m, 1 H) 2.22 (br. s., 2 H) 3.62 (br. s., 1 H) 6.55 (d, *J*=6.64 Hz, 1 H) 6.92 - 7.11 (m, 2 H) 7.43 (d, J=7.42 Hz, 1 H) 7.59 (d, J=8.21 Hz, 1 H) 7.82 (t, J=8.01 Hz, 1 H) 8.08 (d, *J*=5.08 Hz, 1 H) 8.14 (d, *J*=2.34 Hz, 1 H) 8.27 (d, *J*=2.34 Hz, 1 H) 9.33 (s, 1 H) 10.11 (s, 1 H)

### EXAMPLE 39

### (4r,7r)-4-({6-[(4-Methylpyridin-2-yl)amino]-2,4'-bipyridin-2'-yl}amino)adamantan-1-ol

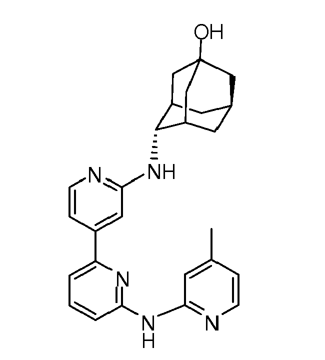

Obtained (32%) from 2'-fluoro-*N*-(4-methylpyridin-2-yl)-2,4'-bipyridin-6-amine
(preparation 7) and (4*r*,7*r*)-4-aminoadamantan-1-ol (sintetized following the procedure described in Tetrahedron letters, 2006, vol. 47, 46, 8063-8067) following the procedure as described in preparation 24a, heating at 140°C for 48 hour and followed by purification by reverse phase chromatography.
LRMS (m/z): 428 (M+1)⁺

### EXAMPLE 40

### 6-[3-(2-Aminoethyl)phenyl]-N(5-methyl-1H-pyrazol-3-yl)pyridin-2-amine

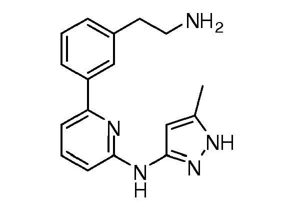

**a) *tert*-Butyl [2-(3-{6-[(1-*tert*-butyl-5-methyl-1*H*-pyrazol-3-yl)amino]pyridin-2-yl}phenyl)ethyl]carbamate**
   Obtained (88%) from 1-*tert-*butyl-5-methyl-1*H-*pyrazol-3-amine and *tert*-butyl {2-[3-(6-bromopyridin-2-yl)phenyl]ethyl}carbamate (preparation 16) following the experimental procedure as described in preparation 4 using dioxane as a solvent, followed by purification of the crude product by flash chromatography (9:1 hexanes/ethyl acetate to 100% ethyl acetate).
   LRMS (m/z): 450 (M+1)⁺
**b) 6-[3-(2-Aminoethyl)phenyl]-*N*(5-methyl-1 *H*-pyrazol-3-yl)pyridin-2-amine**
   *tert-Butyl* [2-(3-{6-[(1-*tert-*butyl-5-methyl-1 *H*-pyrazol-3-yl)amino]pyridin-2-yl}phenyl)ethyl]carbamate (example 40a, 0.08 g, 0.17 mmol) was dissolved in 2,2,2-trifluoroacetic acid (2 mL) and water (0.1 mL), and the mixture was stirred at 90 °C. After 3 hours, satured aqueous sodium carbonate solution was added and the mixture was extracted with dichloromethane. The organic layer was dried (MgSO₄) and the solvent was evaporated. Purification of the residue by reverse phase chromatography (0% CH₃CN in H₂O to 50% CH₃CN in H₂O) gave the title compound (0.02 g, 32%) as a solid.
   LRMS (m/z): 294 (M+1)⁺.
   1 H NMR (400 MHz, METHANOL-*d*₄) δ ppm 2.27 (s, 3 H) 2.98 (t, J=7.33 Hz, 2 H) 3.15 (t, J=7.42 Hz, 2 H) 5.93 (br. s., 1 H) 6.91 (br. s., 1 H) 7.23 (d, J=7.42 Hz, 1 H) 7.30 (d, *J*=7.42 Hz, 1 H) 7.44 (t, J=7.62 Hz, 1 H) 7.63 (t, J=7.82 Hz, 1 H) 7.84 (d, *J*=7.82 Hz, 1 H) 7.87 (br. s., 1 H) 8.56 (s, 1 H)

### EXAMPLE 41

### N-(5-Methyl-1H-pyrazol-3-yl)-6-[3-(1,2,3,6-tetrahydropyridin-4-yl)phenyl]pyridin-2-amine

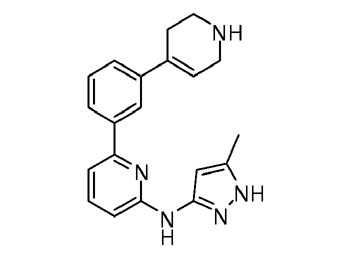

**a) *tert*-Butyl 4-(3-{6-[(1-*tert-*butyl-5-methyl-1*H*-pyrazol-3-yl)amino]pyridin-2-yl}phenyl)-3,6-dihydropyridine-1(2*H*)-Carboxylate**
   Obtained as a solid (39%) from 6-bromo-*N-*(1*-tert-*butyl-5-methyl-1*H-*pyrazol-3-yl)pyridin-2-amine (preparation 17) and *tert*-butyl 4-[3-(4,4,5-trimethyl-1,3,2-dioxaborolan-2-yl)phenyl]-3,6-dihydropyridine-1 (2*H*)-Carboxylate (preparation 9b) following the experimental procedure as described in preparation 1 followed by purification by flash chromatography.
   LRMS (m/z): 488 (M+1)⁺.
   1 H NMR (400 MHz, CHLOROFORM-*d*) δ ppm 1.50 (s, 9 H) 1.64 (s, 9 H) 2.26 (s, 3 H) 2.60 (br. s., 2 H) 3.68 (t, *J*=7.33 Hz, 2 H) 4.08 - 4.14 (m, 2 H) 5.96 (s, 1 H) 6.06 (br. s., 1 H) 6.11 (br. s., 1 H) 6.44 (d, J=8.00 Hz, 1 H) 7.18 (d, J=7.60 Hz, 1 H) 7.40 - 7.43 (m, 2 H) 7.53 (t, *J*=8.00 Hz, 1 H) 7.80 - 7.83 (m, 1 H) 7.99 (s, 1 H)
**b) *N*-(5-Methy)-1*H*-pyrazol-3-y))-6-[3-(1,2,3,6-tetrahydropyridin-4-y))pheny)]pyridin-2-amine**
   Obtained as a solid (80%) from *tert-*butyl 4-(3-{6-[(1-*tert*-butyl-5-methyl-1 *H-*pyrazol-3-yl)amino]pyridin-2-yl}phenyl)-3,6-dihydropyridine-1 (2*H*)-Carboxylate (example 41 a) following the experimental procedure as described in example 40b.
   LRMS (m/z): 332 (M+1)⁺.
   1 H NMR (400 MHz, METHANOL-*d*₄) δ ppm 2.27 (s, 3 H) 2.63 (d, J=1.76 Hz, 2 H) 3.16 (t, J=5.67 Hz, 2 H) 3.57 (d, J=2.34 Hz, 2 H) 6.15 (br. s., 1 H) 6.26 (br. s., 1 H) 6.86 - 6.93 (m, 1 H) 7.24 (d, *J*=7.42 Hz, 1 H) 7.37 - 7.50 (m, 2 H) 7.61 (t, J=7.91 Hz, 1 H) 7.83 (d, *J*=7.62 Hz, 1 H) 8.10 (s, 1 H)

### EXAMPLE 42

### N-(5-Methyl-1H-pyrazol-3-yl)-6-(3-piperidin-4-ylphenyl)pyridin-2-amine

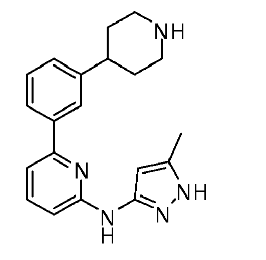

**a) *tert-*Butyl 4-(3-{6-[(1-*tert*-butyl-5-methyl-1*H*-pyrazol-3-yl)amino]pyridin-2-y)}pheny))piperidine-1-Carboxylate**
   Obtained (86%) from *tert*-butyl 4-(3-{6-[(1-*tert*-butyl-5-methyl-1 *H-*pyrazol-3-yl)amino]pyridin-2-yl}phenyl)-3,6-dihydropyridine-1 (2*H*)-Carboxylate (example 41 a) following the experimental procedure as described in example 22a at room temperature for 16 hours.
   LRMS (m/z): 490 (M+1)⁺.
**b) *N*-(5-Methyl-1*H*-pyrazol-3-yl)-6-(3-piperidin-4-yiphenyl)pyridin-2-amine**
   Obtained as a solid (91%) from *tert*-butyl 4-(3-{6-[(1-*tert*-butyl-5-methyl-1*H*-pyrazol-3-yl)amino]pyridin-2-yl}phenyl)piperidine-1-Carboxylate (example 42a) following the experimental procedure as described in example 40b.
   LRMS (m/z): 334 (M+1)⁺.
   1 H NMR (400 MHz, DMSO-*d*₆) δ ppm 1.65 - 1.84 (m, 2 H) 1.92 (d, *J*=11.92 Hz, 2 H) 2.32 (s, 3 H) 2.82 (q, *J*=11.33 Hz, 2 H) 3.10 - 3.29 (m, 3 H) 6.44 (s, 1 H) 7.19 (d, J=8.01 Hz, 1 H) 7.28 - 7.38 (m, 2 H) 7.49 (t, J=7.72 Hz, 1 H) 7.69 (t, J=7.91 Hz, 1 H) 7.92 (d, *J*=7.82 Hz, 1 H) 8.10 (s, 1 H) 9.30 (br. s., 1 H)

### EXAMPLE 43

### N-(5-Methyl-1H-pyrazol-3-yl)-6-(3-piperazin-1-ylphenyl)pyridin-2-amine

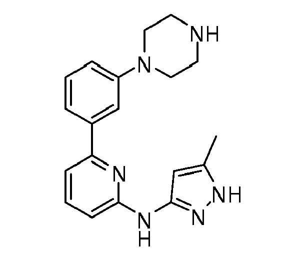

**a) *tert*-Butyl 4-(3-{6-[(1-*tert*-butyl-5-methyl-1*H*-pyrazol-3-yl)amino]pyridin-2-yl}phenyl)piperazine-1-Carboxylate**
   Obtained as a solid (28%) from 6-bromo*-N*-(1-*tert*-butyl-5-methyl-1*H*-pyrazol-3-yl)pyridin-2-amine (preparation 17) *tert*-butyl 4-[3-(4,4,5,5-tetramethyl-1,3,2-dioxaborolan-2-yl)phenyl]piperazine-1-Carboxylate (preparation 13b) following the experimental procedure as described in preparation 1 followed by purification by flash chromatography.
   LRMS (m/z): 491 (M+1)⁺.
   1 H NMR (400 MHz, CHLOROFORM-*d*) δ ppm 1.49 (s, 9 H) 1.64 (s, 9 H) 2.25 (s, 3 H) 3.20 ― 3.23 (m, 4 H) 3.59 ― 3.62 (m, 4 H) 5.96 (s, 1 H) 6.03 (br. s., 1 H) 6.42 (d, J=8.00 Hz, 1 H) 6.97 (dd, J=8.00, 1.60 Hz, 1 H) 7.16 (d, J=7.20 Hz, 1 H) 7.35 (t, J=8.00 Hz, 1 H) 7.43 (d, J=7.60 Hz, 1 H) 7.52 (t, J=8.00 Hz, 1 H) 7.59 (s, 1 H)
**b) *N*-(5-Methyl-1*H*-pyrazol-3-yl)-6-(3-piperazin-1-ylphenyl)pyridin-2-amine**
   Obtained as a solid (84%) from *tert-*butyl 4-(3-{6-[(1-*tert*-butyl-5-methyl-1*H-*pyrazol-3-yl)amino]pyridin-2-yl}phenyl)piperazine-1-Carboxylate (example 43a) following the experimental procedure as described in example 40b.
   LRMS (m/z): 335 (M+1)⁺.
   1 H NMR (400 MHz, METHANOL-*d*₄) δ ppm 2.27 (s, 3 H) 2.94 - 3.07 (m, 4 H) 3.19 - 3.27 (m, 4 H) 6.32 (br. s., 1 H) 6.89 (br. s., 1 H) 7.03 (dd, *J*=8.01, 1.56 Hz, 1 H) 7.22 (d, *J*=7.42 Hz, 1 H) 7.33 (t, J=7.91 Hz, 1 H) 7.43 (d, J=7.62 Hz, 1 H) 7.60 (t, *J*=7.72 Hz, 1 H) 7.68 (br. s., 1 H)

### EXAMPLE 44

### N-(5-Methyl-1H-pyrazol-3-yl)-2'-piperazin-1-yl-2,4'-bipyridin-6-amine

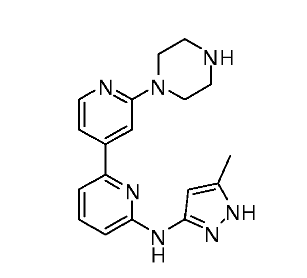

a) **2'-Fluoro-*N*(5-methyl-1*H*-pyrazol-3-yl)-2,4'-bipyridin-6-amine**
   Obtained (20%) from 6-bromo-2'-fluoro-2,4'-bipyridine (preparation 18) and *tert*-butyl 3-amino-5-methyl-1*H*-pyrazole-1-Carboxylate (preparation 19) following the experimental procedure as described in preparation 12a using microwave at 100 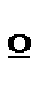 C for 45 minutes, followed by purification by flash chromatography.
   LRMS (m/z): 270 (M+1)⁺.
**b) *tert-*Butyl 4-{6-[(5-methyl-1*H*-pyrazol-3-yl)amino]-2,4'-bipyridin-2'-yl}piperazine-1-Carboxylate**
   Obtained (25%) from 2'-fluoro-*N*-(5-methyl-1*H-*pyrazol-3-yl)-2,4'-bipyridin-6-amine (example 44a) and *tert-*butyl piperazine-1-Carboxylate following the experimental procedure as described in example 10a followed by purification by flash chromatography.
   LRMS (m/z): 436 (M+1)⁺.
**c) *N*-(5-Methyl-1*H*-pyrazol-3-yl)-2'-piperazin-1-yl-2,4'-bipyridin-6-amine**
   Obtained as a solid (67%) from *tert-*butyl 4-{6-[(5-methyl-1*H* pyrazol-3-yl)amino]-2,4'-bipyridin-2'-yl}piperazine-1-Carboxylate (example 44b) following the experimental procedure as described in example 2b.
   LRMS (m/z): 336 (M+1)⁺.
   ¹H NMR (400 MHz, DMSO-*d*₆) □ ppm 2.20 (s, 3 H) 2.82 (br. s., 4 H) 3.47 (d, *J*=5.08 Hz, 4 H) 6.32 (br. s., 1 H) 7.14 (d, *J*=8.21 Hz, 1 H) 7.21 (dd, J=5.18, 1.07 Hz, 1 H) 7.32 (d, *J*=7.42 Hz, 1 H) 7.47 (s, 1 H) 7.60 (t, 1 H) 8.17 (d, *J*=5.28 Hz, 1 H) 9.26 (s, 1 H)

### EXAMPLE 45

### N-(2'-Piperazi n-1-yl-2,4'-bipyridin-6-yl)pyrimidine-4,6-diamine

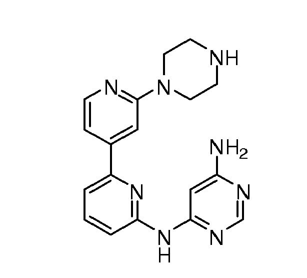

**a) *N*-(6-Chloropyridin-2-yl)pyrimidine-4,6-diamine**
   Obtained as a solid (25%) from 2,6-dichloropyridine and pyrimidine-4,6-diamine following the experimental procedure as described in preparation 4, using dioxane as solvent, followed by purification by flash chromatography (hexanes/ethyl acetate 0% to 100% ethyl acetate).
   LRMS (m/z): 222 (M+1)⁺.
**b) *tert*-Butyl 4-{6-[(6-aminopyrimidin-4-yl)amino]-2,4'-bipyridin-2'-yl}piperazine-1-Carboxylate**
   Obtained (12%) from *N*-(6-Chloropyridin-2-yl)pyrimidine-4,6-diamine (example 45a) and {2-[4-(*tert*-butoxycarbonyl)piperazin-1-yl]pyridin-4-yl}boronic acid (preparation 24b) following the experimental procedure as described in preparation 1, using cesium carbonate as base and dioxane as solvent.
   LRMS (m/z): 449 (M+1)⁺.
**c) *N*-(2'-piperazin-1-yl-2,4'-bipyridin-6-yl)pyrimidine-4,6-diamine**
   Obtained as salt (77%) from *tert-* butyl 4-{6-[(6-aminopyrimidin-4-yl)amino]-2,4'-bipyridin-2'-yl}piperazine-1-Carboxylate (example 45b) following the experimental procedure as described in example 2b.
   LRMS (m/z): 349 (M+1)⁺.
   1 H NMR (400 MHz, DMSO-*d*₆) □ ppm 3.24 (br. s., 4 H) 3.94 (br. s., 4 H) 7.42 (d, J=8.21 Hz, 1 H) 7.51 (d, *J*=5.47 Hz, 1 H) 7.63 (br. s., 1 H) 7.89 (d, J=7.82 Hz, 1 H) 7.99 (t, J=7.82 Hz, 1 H) 8.29 (d, J=5.47 Hz, 1 H) 9.41 (br. s., 2 H)

### EXAMPLE 46

### 3-[3-(2-Aminoethyl)phenyl]-N-[4-(trifluoromethyl)pyridin-2-yl]isoquinolin-1-amine

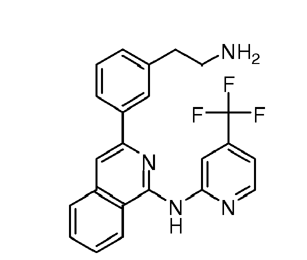

**a) 3-Bromo-*N-*[4-(trifluoromethyl)pyridin-2-yl]isoquinolin-1-amine**
   Obtained (66%) from 3-bromoisoquinolin-l-amine (preparation 20c) and 2-bromo-4-(trifluoromethyl)pyridine following the experimental procedure as described in preparation 4 using dioxane as a solvent, followed by purification of the crude product by flash chromatography (7:3 hexanes/ethyl acetate).
   LRMS (m/z): 368/370 (M+1)⁺.
**b) *tert-*Butyl {2-[3-(1-{[4-(trifluoromethyl)pyridin-2-yl]amino}isoquinolin-3-yl)phenyl]ethyl}carbamate**
   Obtained (43%) from 3-bromo-*N-*[4-(trifluoromethyl)pyridin-2-yl]isoquinolin-1-amine (example 46a) and (3-{2-[(*tert*-butoxycarbonyl)amino]ethyl}phenyl)boronic acid (preparation 3) following the experimental procedure as described in preparation 1, followed by purification of the crude product by flash chromatography.
   LRMS (m/z): 509 (M+1)⁺.
**c) 3-[3-(2-Aminoethyl)phenyl]-***N-***[4-(trifluoromethyl)pyridin-2-yl]isoquinolin-1-amine**
   Obtained as a solid (75%) from *tert*-butyl {2-[3-(1-{[4-(trifluoromethyl)pyridin-2-yl]amino}isoquinolin-3-yl)phenyl]ethyl}carbamate (example 46b) following the experimental procedure as described in example 2b.
   LRMS (m/z): 409 (M+1)⁺.
   1 H NMR (400 MHz, CHLOROFORM-*d*) δ ppm 1.47 (br. s., 2 H) 2.88 (t, J=6.35 Hz, 2 H) 7.17 (d, *J*=4.88 Hz, 1 H) 7.28 (br. s., 1 H) 7.44 (t, J=7.62 Hz, 1 H) 7.57 (t, J=7.42 Hz, 1 H) 7.67 (t, J=7.42 Hz, 1 H) 7.73 (s, 1 H) 7.84 (d, J=8.01 Hz, 1 H) 7.92 - 8.09 (m, 3 H) 8.36 (br. s., 1 H) 8.45 (d, J=5.08 Hz, 1 H) 9.28 (s, 1 H)

### EXAMPLE 47

### 3-[3-(2-Aminoethyl)phenyl]-N-pyrazin-2-ylisoquinolin-1 -amine

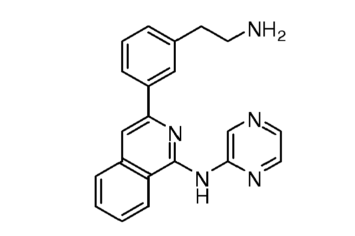

**a) 3-Bromo-*N-*pyrazin-2-ylisoquinolin-1-amine**
   Obtained (74%) from 1,3-dibromoisoquinoline (preparation 20b) and pyrazin-2-amine following the experimental procedure as described in preparation 2 using toluene as solvent, followed by purification of the crude product by flash chromatography (1:1 hexanes/ethyl acetate).
   LRMS (m/z): 301/303 (M+1)⁺.
**b) *tert*-Butyl (2-{3-[1-(pyrazin-2-ylamino)isoquinolin-3-yl]phenyl}ethyl)carbamate**
   Obtained (20%) from 3-bromo-*N-*pyrazin-2-ylisoquinolin-1-amine (example 47a) and (3-{2-[(*tert-\*butoxycarbonyl)amino]ethyl}phenyl)boronic acid (preparation 3) following the experimental procedure as described in preparation 1 using microwave at 110 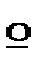 °C for 1 hour, followed by purification by flash chromatography.
   LRMS (m/z): 442 (M+1)⁺.
**c) 3-[3-(2-Aminoethyl)phenyl]**-***N-*pyrazin-2-ylisoquinolin-1-amine**
   Obtained as a solid (85%) from *tert*-butyl (2-{3-[1-(pyrazin-2-ylamino)isoquinolin-3-yl]phenyl}ethyl)carbamate (example 47b) following the experimental procedure as described in example 2b.
   LRMS (m/z): 342 (M+1)⁺.
   1 H NMR (400 MHz, DMSO-*d*₆) δ ppm 2.73 (br. s., 2 H) 3.53 (t, J=6.00 Hz, 2 H) 7.24 (s, 1 H) 7.41 (s, 1 H) 7.60 (s, 1 H) 7.75 (s, 1 H) 7.87 - 8.05 (m, 4 H) 8.27 (br. s., 1 H) 8.40 (s, 1 H) 8.59 (d, J=8.21 Hz, 1 H) 9.67 (br. s., 1 H)

### EXAMPLE 48

### N-(4-Methylpyridin-2-y)-1-[3-(1,2,3,6-tetrahydropyridin-4-y)phenyl]isoquinolin-3-amine

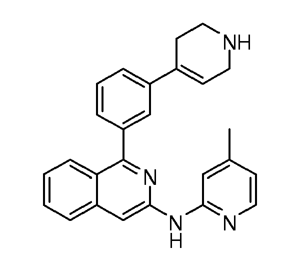

**a) *tert-Butyl* 4-[3-(3-Chloroisoquinolin-1-yl)phenyl]-3,6-dihydropyridine-1(2*H*)-Carboxylate**
   Obtained as a off-white solid (85%) from 1,3-dichloroisoquinoline and *tert*-butyl 4-[3-(4,4,5-trimethyl-1,3,2-dioxaborolan-2-yl)phenyl]-3,6-dihydropyridine-1 (2*H*)-Carboxylate (preparation 9b) following the experimental procedure as described in preparation 1 using dioxane as a solvent, followed by purification by flash chromatography (98:2 hexanes/ethyl acetate to 8:2 hexanes/ethyl acetate).
   LRMS (m/z): 421 (M+1)⁺.
   1 H NMR (300 MHz, CHLOROFORM-*d*) δ ppm 1.49 (s, 9 H) 2.58 (br. s., 2 H) 3.65 (t, J=5.63 Hz, 2 H) 4.09 (d, J=2.75 Hz, 2 H) 6.12 (br. s., 1 H) 7.45 - 7.61 (m, 4 H) 7.67 - 7.76 (m, 3 H) 7.83 (d, J=9.00 Hz, 1 H) 8.08 (dd, J=8.51, 0.82 Hz, 1 H)
**b) tert-Butyl 4-(3-{3-[(4-methylpyridin-2-yl)amino]isoquinolin-1-yl}phenyl)-3,6-dihydropyridine-1 (2*H*)-Carboxylate**
   Obtained (49%) from *tert*-butyl 4-[3-(3-Chloroisoquinolin-1-yl)phenyl]-3,6-dihydropyridine-1(2*H*-Carboxylate (example 48a) and 4-methylpyridin-2-amine following the experimental procedure as described in preparation 4, followed by purification of the crude product by flash chromatography (8:2 hexanes/ethyl acetate to 7:3 hexanes/ethyl acetate).
   LRMS (m/z): 493 (M+1)⁺.
   1 H NMR (300 MHz, CHLOROFORM-*d*) δ ppm 1.48 (s, 9 H) 2.32 (s, 3 H) 2.60 (br. s., 2 H) 3.66 (t, *J*=5.63 Hz, 2 H) 4.09 (br. s., 2 H) 6.13 (br. s., 1 H) 6.69 (d, J=4.94 Hz, 1 H) 6.83 (s, 1 H) 7.21 - 7.33 (m, 1 H) 7.38 (br. s., 1 H) 7.46 - 7.53 (m, 2 H) 7.53 - 7.62 (m, 2 H) 7.70 (s, 1 H) 7.83 (d, J=8.24 Hz, 1 H) 7.92 (d, J=8.51 Hz, 1 H) 8.22 (d, J₌5.22 Hz, 1 H) 8.39 (s, 1 H)
**c) *N*-(4-Methylpyridin-2-yl)-1-[3-(1,2,3,6-tetrahydropyridin-4-yl)phenyl]isoquinolin-3-amine**
   Obtained as a solid (24%) from *tert*-butyl 4-(3-{3-[(4-methylpyridin-2-yl)amino]isoquinolin-1-yl}phenyl)-3,6-dihydropyridine-1(2*H*)-Carboxylate (example 48b) following the experimental procedure as described in example 2b.
   LRMS (m/z): 393 (M+1)⁺.
   1 H NMR (300 MHz, CHLOROFORM-*d*) δ ppm 2.32 (s, 3 H) 2.55 (br. s., 2 H) 3.15 (t, J=5.63 Hz, 2 H) 3.44 - 3.69 (m, 2 H) 6.23 (br. s., 1 H) 6.69 (d, *J*=4.94 Hz, 1 H) 6.84 (s, 1 H) 7.27 - 7.34 (m, 1 H) 7.46 - 7.61 (m, 4 H) 7.71 (s, 1 H) 7.83 (d, *J*=9.06 Hz, 1 H) 7.93 (d, J=8.52 Hz, 1 H) 8.22 (d, J=4.94 Hz, 1 H) 8.39 (s, 1 H)

### EXAMPLE 49

### N-Pyrazin-2-yl-1-[3-(1,2,3,6-tetrahydropyridin-4-yl)phenyl]isoquinolin-3-amine

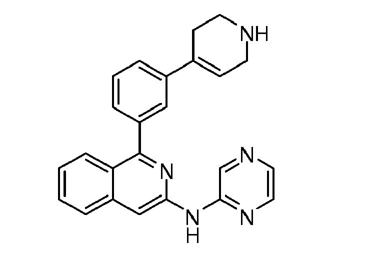

**a) *tert-Butyl* 4-{3-[3-(pyrazin-2-ylamino)isoquinolin-1-yl]phenyl}-3,6-dihydropyridine-1 (2*H*)-Carboxylate**
   Obtained (95%) from *tert-*butyl 4-[3-(3-Chloroisoquinolin-1-yl)phenyl]-3,6-dihydropyridine-1(2*H*)-Carboxylate (example 48a) and pyrazin-2-amine following the experimental procedure as described in preparation 4, followed by purification of the crude product by flash chromatography (100% hexanes to 3:7 hexanes/ethyl acetate).
   LRMS (m/z): 480 (M+1)⁺.
   1 H NMR (300 MHz, CHLOROFORM-*d*) δ ppm 1.49 (s, 9 H) 2.60 (br. s., 2 H) 3.66 (t, J=5.63 Hz, 2 H) 4.10 (br. s., 2 H) 6.13 (br. s., 1 H) 7.36 (t, J=7.14 Hz, 1 H) 7.48 - 7.66 (m, 4 H) 7.70 (s, 1 H) 7.87 (d, *J*=8.24 Hz, 1 H) 7.97 (d, *J*=8.51 Hz, 1 H) 8.08 (d, J=2.75 Hz, 1 H) 8.27 (dd, J=2.75, 1.37 Hz, 1 H) 8.41 (s, 1 H) 8.46 (d, J=1.37 Hz, 1 H)
**b) *N-*Pyrazin-2-yl-1-[3-(1,2,3,6-tetrahydropyridin-4-yl)phenyl]isoquinolin-3-amine**
   Obtained as a solid (82%) from *tert-*butyl 4-{3-[3-(pyrazin-2-ylamino)isoquinolin-1-yl]phenyl}-3,6-dihydropyridine-1(2*H*)-Carboxylate (example 49a) following the experimental procedure as described in example 2b.
   LRMS (m/z): 380 (M+1)⁺.
   1 H NMR (300 MHz, CHLOROFORM-*d*) δ ppm 2.53 (br. s., 2 H) 3.13 (t, J=5.63 Hz, 2 H) 3.55 (d, *J*=2.47 Hz, 2 H) 6.21 (br. s., 1 H) 7.35 (t, *J*=7.69 Hz, 1 H) 7.42 - 7.75 (m, 5 H) 7.86 (d, J=8.24 Hz, 1 H) 7.98 (d, J=8.24 Hz, 1 H) 8.07 (d, J=2.75 Hz, 1 H) 8.26 (br. s., 1 H) 8.39 (s, 1 H) 8.45 (s, 1 H)

### EXAMPLE 50

### N-(5-Methyl-1H-pyrazol-3-yl)-1-[3-(1,2,3,6-tetrahydropyridin-4-yl)phenyl] isoquinolin-3-amine

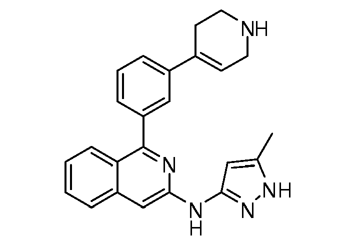

**a) *tert*-Butyl 4-(3-{3-[(1-*tert*-butyl-5-methyl-1*H*-pyrazol-3-yl)amino]isoquinolin-1-yl}phenyl)-3,6-dihydropyridine-1(2*H*)-Carboxylate**
   Obtained (30%) from *tert-butyl* 4-[3-(3-Chloroisoquinolin-1-yl)phenyl]-3,6-dihydropyridine-1(2*H*)-Carboxylate (example 48a) and 1-*tert*-butyl-5-methyl-1*H*-pyrazol-3-amine following the experimental procedure as described in preparation 4, followed by purification of the crude product by flash chromatography (8:2 hexanes/ethyl acetate).
   LRMS (m/z): 538 (M+1)⁺.
   1 H NMR (300 MHz, CHLOROFORM-*d*) δ ppm 1.51 (s, 9 H) 1.68 (s, 9 H) 2.32 (s, 3 H) 2.61 (br. s., 2 H) 3.67 (t, J=5.63 Hz, 2 H) 4.10 (br. s., 2 H) 6.03 (s, 1 H) 6.07 (s, 1 H) 6.13 (br. s., 1 H) 6.70 (s, 1 H) 7.19 - 7.27 (m, 1 H) 7.48 - 7.54 (m, 3 H) 7.55 - 7.62 (m, 2 H) 7.70 (s, 1 H) 7.88 (d, *J*=8.51 Hz, 1 H)
**b) *N*-(5-Methyl-1*H*-pyrazol-3-yl)-1-[3-(1,2,3,6-tetrahydropyridin-4-yl)phenyl]** isoquinolin-3-amine
   Obtained as a solid (27%) from *tert*-butyl 4-(3-{3-[(1-*tert*-butyl-5-methyl-1*H-*pyrazol-3-yl)amino]isoquinolin-1-yl}phenyl)-3,6-dihydropyridine-1(2*H*)-Carboxylate (example 50a) following the experimental procedure as described in example 40b.
   LRMS (m/z): 382 (M+1)⁺.
   1 H NMR (300 MHz, CHLOROFORM-*d*) δ ppm 2.32 (s, 3 H) 2.46 - 2.59 (m, 2 H) 3.14 (t, J=5.63 Hz, 2 H) 3.56 (q, J=2.75 Hz, 2 H) 5.85 (s, 1 H) 6.23 (t, J=3.30 Hz, 1 H) 6.97 (s, 1 H) 7.23 (dd, J=6.45, 5.08 Hz, 1 H) 7.44 - 7.59 (m, 5 H) 7.62 (br. s., 1 H) 7.66 - 7.76 (m, 2 H) 7.90 (d, *J*=7.69 Hz, 1 H)

### EXAMPLE 51

### N-(5-Methyl-1H-pyrazol-3-yl)-1-(3-piperidin-4-ylphenyl)isoquinolin-3-amine

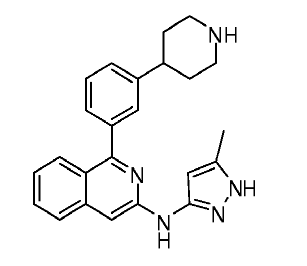

**a) *tert*-Butyl 4-(3-{3-[(1-*tert*-butyl-5-methyl-1*H*-pyrazol-3-yl)amino]isoquinolin-1-y)}phenyl)piperidine-1-Carboxylate**
   Obtained as a solid (67%) from *tert*-butyl 4-(3-{3-[(1-*tert*-butyl-5-methyl-1 *H-*pyrazol-3-yl)amino]isoquinolin-1-yl}phenyl)-3,6-dihydropyridine-1(2*H*)-Carboxylate (example 50a) following the experimental procedure as described in example 22a adding few drops of acetic acid.
   LRMS (m/z): 540 (M+1)⁺.
**b) *N*-(5-Methyl-1 H-pyrazol-3-yl)-1-(3-piperidin-4-ylphenyl)isoquinolin-3-amine**
   Obtained as a solid (29%) from *tert*-butyl 4-(3-{3-[(1-*tert*-butyl-5-methyl-1*H-*pyrazol-3-yl)amino]isoquinolin-1-yl}phenyl)piperidine-1-Carboxylate (example 51 a) following the experimental procedure as described in example 40b.
   LRMS (m/z): 384 (M+1)⁺.
   1 H NMR (300 MHz, CHLOROFORM-*d*) δ ppm 1.63 - 1.85 (m, *J*=12.57, 12.46, 12.46, 3.98 Hz, 2 H) 1.94 (d, J=13.18 Hz, 2 H) 2.29 (s, 3 H) 2.65 - 2.87 (m, 3 H) 3.24 (d, *J*=12.36 Hz, 2 H) 5.84 (s, 1 H) 7.05 (br. s., 1 H) 7.22 (ddd, *J*=8.38, 7.00, 0.82 Hz, 1 H) 7.35 (d, *J*=7.42 Hz, 1 H) 7.41 - 7.57 (m, 4 H) 7.60 (s, 1 H) 7.70 (d, *J*=8.51 Hz, 1 H) 7.90 (d, *J*=8.51 Hz, 1 H)

### EXAMPLE 52

### N-(4-Methylpyridin-2-yl)-1-(2-piperazin-1-ylpyridin-4-yl)isoquinolin-3-amine

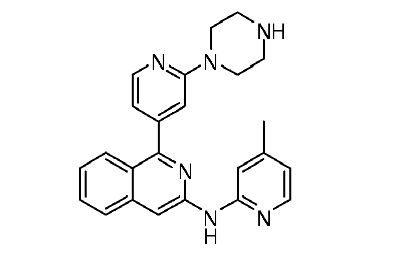

**a) 3-Chloro-1-(2-fluoropyridin-4-yl)isoquinoline**
   Obtained as a bright-white solid (75%) from 1,3-dichloroisoquinoline and (2-fluoropyridin-4-yl)boronic acid following the experimental procedure as described in preparation 1 using dioxane as a solvent, followed by purification by flash chromatography (100% hexanes to 6:4 hexanes/ethyl acetate).
   LRMS (m/z): 259 (M+1)⁺.
   1 H NMR (300 MHz, CHLOROFORM-*d*) δ ppm 7.29 (s, 1 H) 7.53 (d, *J*=4.94 Hz, 1 H) 7.62 (dd, *J*=15.11, 1.10 Hz, 1 H) 7.78 (t, *J*=7.14 Hz, 1 H) 7.81 - 7.93 (m, 2 H) 8.01 (d, *J*=8.79 Hz, 1 H) 8.42 (d, *J*=5.22 Hz, 1 H)
**b) *tert-*Butyl 4-[4-(3-Chloroisoquinolin-1-yl)pyridin-2-yl]piperazine-1-Carboxylate**
   Obtained as a yellow oil (79%) from 3-Chloro-1-(2-fluoropyridin-4-yl)isoquinoline (example 52a) and *tert* butyl piperazine-1-Carboxylate following the experimental procedure as described in example 10a followed by purification of the crude product by flash chromatography (95:5 hexanes/ethyl acetate to 4:6 hexanes/ethyl acetate).
   LRMS (m/z): 425 (M+1)⁺.
   1 H NMR (300 MHz, CHLOROFORM-*d*) δ ppm 1.49 (s, 9 H) 3.59 (d, *J*=8.51 Hz, 8 H) 6.81 - 7.00 (m, 2 H) 7.56 (t, *J*=7.69 Hz, 1 H) 7.67 - 7.88 (m, 3 H) 8.05 (d, *J*=8.24 Hz, 1 H) 8.33 (d, *J*=4.94 Hz, 1 H)
**c) *tert-*Butyl 4-(4-{3-[(4-methylpyridin-2-yl)amino]isoquinolin-1-yl}pyridin-2-yl)piperazine-1 -Carboxylate**
   Obtained as a yellow oil (92%) from *tert*-butyl 4-[4-(3-Chloroisoquinolin-1-yl)pyridin-2-yl]piperazine-1-Carboxylate (example 52b) and 4-methylpyridin-2-amine following the experimental procedure as described in preparation 4, followed by purification of the crude product by flash chromatography (100% hexanes to 8:2 hexanes/propan-2-ol).
   LRMS (m/z): 497 (M+1)⁺.
**d) *N-*(4-Methylpyridin-2-yl)-1-(2-piperazin-1-ylpyridin-4-yl)isoquinolin-3-amine**
   Obtained as a yellow solid (89%) from *tert*-butyl 4-(4-{3-[(4-methylpyridin-2-yl)amino]isoquinolin-1-yl}pyridin-2-yl)piperazine-1-Carboxylate (example 52c) following the experimental procedure as described in example 2b. The residue was disolved in MeOH and passed through an acidic sulphonic SCX column. The compound released from the column with 33% ammonium in ethanol, evaporation of the solvent gave the desired compound.
   LRMS (m/z): 397 (M+1)⁺.
   1 H NMR (300 MHz, CHLOROFORM-*d*) δ ppm 2.32 (s, 3 H) 3.01 (d, *J*=4.39 Hz, 4 H) 3.59 (d, *J*=4.67 Hz, 4 H) 6.70 (d, *J*=4.67 Hz, 1 H) 6.80 (s, 1 H) 6.87 - 6.99 (m, 2 H) 7.30 (t, *J*=7.42 Hz, 1 H) 7.43 (br. s., 1 H) 7.58 (t, *J*=7.28 Hz, 1 H) 7.83 (d, *J*=8.24 Hz, 1 H) 7.91 (d, *J*=8.51 Hz, 1 H) 8.23 (d, *J*=5.22 Hz, 1 H) 8.35 (d, *J*=4.67 Hz, 1 H) 8.45 (s, 1 H)

### EXAMPLE 53

### 1-(2-Piperazin-1-ylpyridin-4-yl)-N-pyrazin-2-ylisoquinolin-3-amine

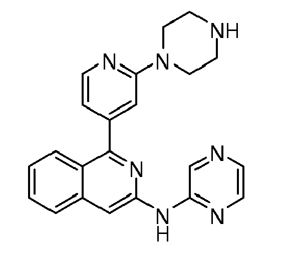

**a) *tert* Butyl 4-{4-[3-(pyrazin-2-ylamino)isoquinolin-1-yl]pyridin-2-yl}piperazine-1-Carboxylate**
   Obtained as a yellow oil (98%) from *tert*-butyl 4-[4-(3-Chloroisoquinolin-1-yl)pyridin-2-yl]piperazine-1-Carboxylate (example 52b) and pyrazin-2-amine following the experimental procedure as described in preparation 4, followed by purification of the crude product by flash chromatography (100% hexanes to 7:3 hexanes/propan-2-ol).
   LRMS (m/z): 484 (M+1)⁺.
**b) 1-(2-Piperazin-1-ylpyridin-4-yl)-*N*-pyrazin-2-ylisoquinolin-3-amine**
   Obtained as a yellow solid (98%) from *tert*-butyl 4-{4-[3-(pyrazin-2-ylamino)isoquinolin-1-yl]pyridin-2-yl}piperazine-1-Carboxylate (example 53a) following the experimental procedure as described in example 52d.
   LRMS (m/z): 384 (M+1)⁺.
   1 H NMR (300 MHz, CHLOROFORM-*d*) δ ppm 3.00 (d, *J*=4.39 Hz, 4 H) 3.58 (d, *J*=4.39 Hz, 4 H) 6.86 - 6.97 (m, 2 H) 7.37 (t, *J*=7.55 Hz, 1 H) 7.63 (t, *J*=7.55 Hz, 1 H) 7.75 (br. s., 1 H) 7.87 (d, *J*=8.24 Hz, 1 H) 7.96 (d, *J*=8.51 Hz, 1 H) 8.09 (d, *J*=2.47 Hz, 1 H) 8.27 (br. s., 1 H) 8.35 (d, *J*=4.67 Hz, 1 H) 8.44 (d, *J*=8.79 Hz, 2 H)

### EXAMPLE 54

### 1-(2-Piperazin-1-ylpyridin-4-yl)-N-pyrimidin-4-ylisoquinolin-3-amine

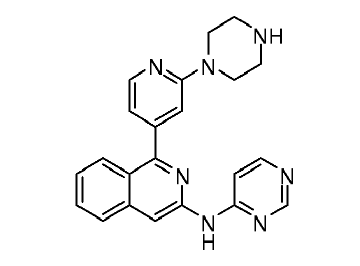

**a) *tert*-Butyl 4-{4-[3-(pyrimidin-4-ylamino)isoquinolin-1-yl]pyridin-2-yl}piperazine-1 -Carboxylate**
   Obtained as a yellow oil (32%) from *tert*-butyl 4-[4-(3-Chloroisoquinolin-1-yl)pyridin-2-yl]piperazine-1-Carboxylate (example 52b) and pyrimidin-4-amine following the experimental procedure as described in preparation 4, followed by purification of the crude product by flash chromatography (100% hexanes to 1:1 hexanes/propan-2-ol).
   LRMS (m/z): 484 (M+1)⁺.
**b) 1-(2-Piperazin-1-ylpyridin-4-yl)-*N*-pyrimidin-4-ylisoquinolin-3-amine**
   Obtained as a yellow solid (79%) from *tert*-butyl 4-{4-[3-(pyrimidin-4-ylamino)isoquinolin-1-yl]pyridin-2-yl}piperazine-1-Carboxylate (example 54a) following the experimental procedure as described in example 52d.
   LRMS (m/z): 384 (M+1)⁺.
   1 H NMR (300 MHz, CHLOROFORM-*d*) δ ppm 3.01 (d, *J*=4.39 Hz, 4 H) 3.59 (d, *J*=4.67 Hz, 4 H) 6.92 (s, 2 H) 7.05 (d, *J*=5.49 Hz, 1 H) 7.27 (s, 1 H) 7.41 (t, *J*=7.55 Hz, 1 H) 7.66 (t, *J*=7.55 Hz, 1 H) 7.79 (br. s., 1 H) 7.90 (d, *J*=8.24 Hz, 1 H) 7.98 (d, *J*=8.51 Hz, 1 H) 8.29 - 8.49 (m, 3 H) 8.89 (s, 1 H)

### EXAMPLE 55

### N-(5-Fluoropyridin-2-yl)-l-(2-piperazin-1-yipyridin-4-yl)isoquinolin-3-amine

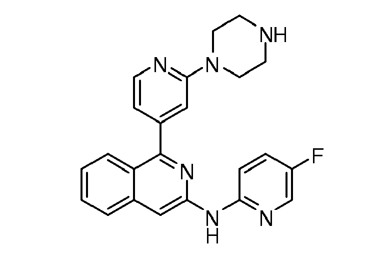

**a) *tert*-Butyl 4-(4-{3-[(5-fluoropyridin-2-yl)amino]isoquinolin-1-yl}pyridin-2-yl)piperazine-1-Carboxylate**
   Obtained as a yellow oil (90%) from *tert*-butyl 4-[4-(3-Chloroisoquinolin-1-yl)pyridin-2-yl]piperazine-1-Carboxylate (example 52b) and 5-fluoropyridin-2-amine following the experimental procedure as described in preparation 4, followed by purification of the crude product by flash chromatography.
   LRMS (m/z): 501 (M+1)⁺.
**b) *N*-(5-Fluoropyridin-2-yl)-l-(2-piperazin-1-ylpyridin-4-yl)isoquinolin-3-amine**
   Obtained as a yellow solid (98%) from *tert*-butyl 4-(4-{3-[(5-fluoropyridin-2-yl)amino]isoquinolin-1-yl}pyridin-2-yl)piperazine-1-Carboxylate (example 55a) following the experimental procedure as described in example 52d.
   LRMS (m/z): 401 (M+1)⁺.
   1 H NMR (300 MHz, CHLOROFORM-*d*) δ ppm 3.02 (d, *J*=4.94 Hz, 4 H) 3.58 (d, *J*=4.94 Hz, 4 H) 6.90 (br. s., 2 H) 7.07 (dd, *J*=9.06, 3.30 Hz, 1 H) 7.23 - 7.41 (m, 2 H) 7.53 (s, 1 H) 7.59 (t, *J*=7.42 Hz, 1 H) 7.82 (d, *J*=8.51 Hz, 1 H) 7.92 (d, *J*=8.52 Hz, 1 H) 8.22 (d, *J=*2.75 Hz, 1 H) 8.28 (s, 1 H) 8.35 (d, *J*=4.94 Hz, 1 H)

### EXAMPLE 56

### 6-1{[1-(2-Piperazin-1-ylpyridin-4-yl)isoquinolin-3-yl]amino}nicotinonitrile

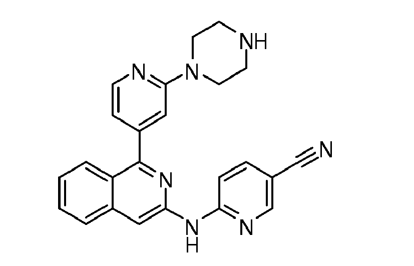

**a) *tert-*Butyl 4-(4-{3-[(5-Cyanopyridin-2-yl)amino]isoquinolin-1-yl}pyridin-2-yl)piperazine-1-Carboxylate**
   Obtained as a yellow oil (70%) from *tert*-butyl 4-[4-(3-Chloroisoquinolin-1-yl)pyridin-2-yl]piperazine-1-Carboxylate (example 52b) and 6-aminonicotinonitrile following the experimental procedure as described in preparation 4, followed by purification of the crude product by flash chromatography.
   LRMS (m/z): 508 (M+1)⁺.
**b) 6-{[1-(2-Piperazin-1-yipyridin-4-yl)isoquinolin-3-yl]aminol}nicotinonitrile**
   Obtained as a yellow solid (93%) from *tert*-butyl 4-(4-{3-[(5-Cyanopyridin-2-yl)amino]isoquinolin-1-yl}pyridin-2-yl)piperazine-1-Carboxylate (example 56a) following the experimental procedure as described in example 52d.
   LRMS (m/z): 408 (M+1)⁺.
   1 H NMR (300 MHz, CHLOROFORM-*d*) δ ppm 3.01 (br. s., 4 H) 3.60 (br. s., 4 H) 6.91 (br. s., 2 H) 7.13 (d, *J*=8.79 Hz, 1 H) 7.41 (t, *J*=7.83 Hz, 1 H) 7.66 (t, *J*=7.42 Hz, 1 H) 7.75 (d, *J*=7.69 Hz, 1 H) 7.89 (d, *J*=7.14 Hz, 2 H) 7.98 (d, *J*=8.52 Hz, 1 H) 8.36 (d, *J*=3.30 Hz, 1 H) 8.40 (br. s., 1 H) 8.64 (br. s., 1 H)

### EXAMPLE 57

### (±)-1-{2-[(3R,5S)-3,5-Dimethylpiperazin-1-yl]pyridin-4-yl}-N-pyrazin-2-ylisoquinolin-3-amine

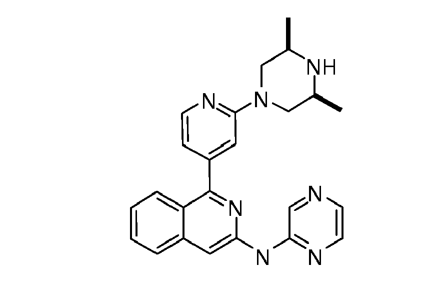

**a) 1-(2-Fluoropyridin-4-yl)-*N*-pyrazin-2-ylisoquinolin-3-amine**
   Obtained as a oil (66%) from 3-Chloro-1-(2-fluoropyridin-4-yl)isoquinoline (example 52a) and pyrazin-2-amine following the experimental procedure as described in preparation 4, followed by purification of the crude product by flash chromatography (100% hexanes to 2:8 hexanes/ethyl acetate).
   LRMS (m/z): 318 (M+1)⁺.
   1 H NMR (300 MHz, CHLOROFORM-*d*) δ ppm 7.28 - 7.32 (m, 1 H) 7.43 (dd, *J*=9.34, 6.32 Hz, 1 H) 7.51 - 7.58 (m, 2 H) 7.67 (t, *J*=7.42 Hz, 1 H) 7.90 (s, 1 H) 7.93 (s, 1 H) 8.13 (d, *J*=2.75 Hz, 1 H) 8.29 (dd, *J*=2.61, 1.51 Hz, 1 H) 8.43 (d, *J*=5.22 Hz, 1 H) 8.49 (s, 1 H) 8.51 (d, *J*=1.37 Hz, 1 H)
**b) (±)-1-{2-[(3R,5S)-3,5-Dimethylpiperazin-1-yl]pyridin-4-yl}-N-pyrazin-2-ylisoquinolin-3-amine**
   Obtained as a solid (47%) from 1-(2-fluoropyridin-4-yl)-N-pyrazin-2-ylisoquinolin-3-amine (example 57a) and (±)-(2*R*,6*S*)-2,6-dimethylpiperazine following the experimental procedure as described in example 10a heating the reaction at 130°C overnight, followed by purification of the crude product by reverse phase chromatography (20% CH₃CN in H₂O to 40% CH₃CN in H₂O).
   LRMS (m/z): 412 (M+1)⁺.
   1 H NMR (300 MHz, CHLOROFORM-*d*) δ ppm 1.40 (s, 3 H) 1.42 (s, 3 H) 3.08 (d, *J*=13.46 Hz, 2 H) 3.20 - 3.38 (m, 2 H) 4.39 (d, *J*=11.54 Hz, 2 H) 6.95 (s, 1 H) 6.98 (d, *J*=4.94 Hz, 1 H) 7.40 (t, *J*=7.69 Hz, 1 H) 7.66 (t, *J*=7.42 Hz, 1 H) 7.78 (br. s., 1 H) 7.85 - 7.98 (m, 2 H) 8.11 (d, *J*=2.47 Hz, 1 H) 8.28 (br. s., 1 H) 8.37 (d, *J*=4.94 Hz, 1 H) 8.43 - 8.54 (m, 3 H)

### EXAMPLE 58

### 1-[2-(2-Methylpiperazin-1-yl)pyridin-4-yl]-N-pyrazin-2-ylisoquinolin-3-amine

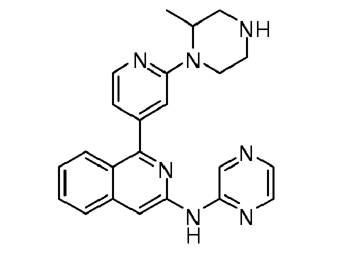

Obtained as a solid (37%) from 1-(2-fluoropyridin-4-yl)-N-pyrazin-2-ylisoquinolin-3-amine (example 57a) and *tert*-butyl 3-methylpiperazine-1-Carboxylate following the experimental procedure as described in example 10a heating the reaction at 130°C for 5 days, followed by purification of the crude product by flash chromatography (100% dichloromethane to 9:1 dichloromethane/methanol).
LRMS (m/z): 398 (M+1)⁺.
1 H NMR (300 MHz, CHLOROFORM-*d*) δ ppm 1.18 (d, *J*=6.32 Hz, 3 H) 2.47 - 2.69 (m, 1 H) 2.98 (dd, *J*=8.24, 6.04 Hz, 3 H) 3.07 - 3.24 (m, 1 H) 4.12 - 4.36 (m, 2 H) 6.87 - 6.94 (m, 2 H) 7.38 (dd, *J*=8.24, 6.87 Hz, 1 H) 7.53 (s, 1 H) 7.64 (t, *J*=7.55 Hz, 1 H) 7.87 (d, *J*=8.24 Hz, 1 H) 7.96 (d, *J*=8.51 Hz, 1 H) 8.10 (d, *J*=2.47 Hz, 1 H) 8.25 - 8.31 (m, 1 H) 8.36 (d, *J*=4.94 Hz, 1 H) 8.44 (d, *J*=1.10 Hz, 1 H) 8.47 (s, 1 H)

### EXAMPLE 59

### 1-{2-[(1 S,4S)-2,5-Diazabicyclo[2.2.1]hept-2-yl]pyridin-4-yl}-N-pyrazin-2-ylisoquinolin-3-amine

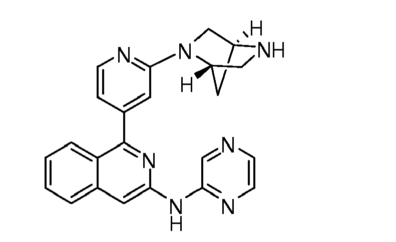

**a) *tert*-Butyl (1*S*,4*S*)-5-{4-[3-(pyrazin-2-y)amino)isoquino)in-1-yl]pyridin-2-yl}-2,5-diazabicyclo[2.2.1]heptane-2-Carboxylate**
   Obtained as a solid (84%) from 1-(2-fluoropyridin-4-yl)-N-pyrazin-2-ylisoquinolin-3-amine (example 57a) and *tert-*butyl (1 *S*,4*S*)-2,5-diazabicyclo[2.2.1 ]heptane-2-Carboxylate following the experimental procedure as described in example 10a, followed by addition of water to the crude. The resultant precipitate was filtered and the solid washed with diethyl ether and dried to give the desired compound.
   LRMS (m/z): 496 (M+1)⁺.
**b) 1-{2-[(1*S*,4*S*)-2,5-Diazabicyclo[2.2.1]hept-2-yl]pyridin-4-yl}-*N*-pyrazin-2-ylisoquinolin-3-amine**
   Obtained as a solid (84%) from *tert*-butyl 5-{4-[3-(pyrazin-2-ylamino)isoquinolin-1-yl]pyridin-2-yl}-2,5-diazabicyclo[2.2.1]heptane-2-Carboxylate (example 59a) following the experimental procedure as described in example 2b.
   LRMS (m/z): 396 (M+1)⁺.
   1 H NMR (300 MHz, CHLOROFORM-*d*) δ ppm 1.77 - 2.02 (m, 2 H) 3.08 - 3.21 (m, 2 H) 3.32 (d, *J*=9.61 Hz, 1 H) 3.64 (d, *J*=9.61 Hz, 1 H) 3.91 (br. s., 1 H) 4.84 (br. s., 1 H) 6.62 (s, 1 H) 6.85 (d, *J*=5.22 Hz, 1 H) 7.37 (t, *J*=7.69 Hz, 1 H) 7.63 (t, *J*=7.55 Hz, 1 H) 7.80 - 7.92 (m, 2 H) 8.00 (d, *J*=8.51 Hz, 1 H) 8.08 (d, *J*=1.37 Hz, 1 H) 8.23 - 8.33 (m, 2 H) 8.45 (d, *J*=4.94 Hz, 2 H)

### EXAMPLE 60

### (±)-1-{2-[(2S,5R)-2,5-Dimethylpiperazin-1-yl]pyridin-4-yl}-N-pyrazin-2-ylisoquinolin-3-amine

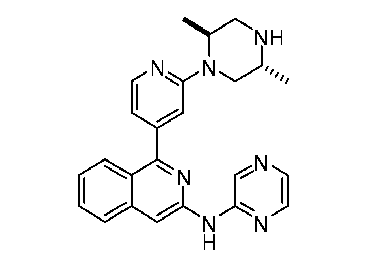

Obtained as a solid (7%) from 1-(2-fluoropyridin-4-yl)-*N*-pyrazin-2-ylisoquinolin-3-amine (example 57a) and (±)-(2*R*,5*S*)-2,5-dimethylpiperazine following the experimental procedure as described in example 10a heating the reaction at 140°C for 2 days, followed by purification of the crude product by reverse phase chromatography (20% CH₃CN in H₂O to 40% CH₃CN in H₂O).
LRMS (m/z): 412 (M+1)⁺.
1 H NMR (300 MHz, CHLOROFORM-*d*) δ ppm 1.62 (s, 3 H) 1.63 (s, 3 H) 2.70 (dt, *J*=12.91, 3.30 Hz, 1 H) 3.20 - 3.54 (m, 3 H) 3.71 (ddd, *J*=12.77, 3.16, 3.02 Hz, 1 H) 4.18 - 4.39 (m, 1 H) 6.86 - 6.97 (m, 2 H) 7.39 (td, *J*=7.69, 1.92 Hz, 1 H) 7.57 (d, *J*=1.37 Hz, 1 H) 7.65 (td, *J*=7.62, 2.06 Hz, 1 H) 7.88 (dd, *J*=8.51, 1.37 Hz, 1 H) 7.99 (ddd, *J*=8.45, 1.24, 1.03 Hz, 1 H) 8.11 (t, *J*=2.88 Hz, 1 H) 8.29 (t, *J*=3.71 Hz, 1 H) 8.37 (td, *J*=3.23, 1.51 Hz, 1 H) 8.46 (t, *J*=2.33 Hz, 2 H)

### EXAMPLE 61

### 1-{4-[3-(Pyrazin-2-ylamino)isoquinolin-1-yl]pyridin-2-yl}azetidin-3-ol

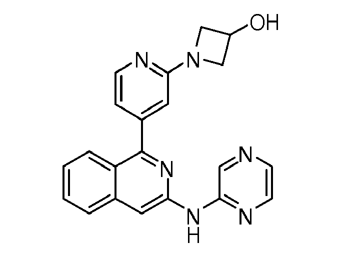

1-(2-Fluoropyridin-4-yl)-*N*-pyrazin-2-ylisoquinolin-3-amine (example 57a, 0.10 g, 0.32 mmol) and potassium carbonate (0.13 g, 0.95 mmol) were added to a stirred suspension of azetidin-3-ol hydrochloride (0.05 g, 0.47 mmol) and ethyl(diisopropyl)amine (0.08 mL, 0.48 mmol) in dimethylsulphoxide (2 mL). The mixture was heated in a sealed tube at 130 °C with stirring. After heating overnight the mixture was cooled and water was added. The resultant precipitate was filtered and the solid washed with diethyl ether. Purification of the precipitate by flash chromatography (95:5 dichloromethane /methanol) gave the title compound (0.07 g, 62%) as a solid.
LRMS (m/z): 371 (M+1)⁺.
1 H NMR (300 MHz, DMSO-*d*₆) δ ppm 3.66 - 3.86 (m, 2 H) 4.22 (t, *J*=7.28 Hz, 2 H) 4.50 - 4.72 (m, 1 H) 5.69 (d, *J*=6.59 Hz, 1 H) 6.62 (s, 1 H) 6.90 (d, *J*=4.94 Hz, 1 H) 7.42 (t, *J*=7.55 Hz, 1 H) 7.69 (t, *J*=7.55 Hz, 1 H) 7.82 - 7.98 (m, 2 H) 8.04 - 8.12 (m, 1 H) 8.25 (d, *J*=4.94 Hz, 1 H) 8.31 (d, *J*=1.37 Hz, 1 H) 8.51 (s, 1 H) 8.71 (s, 1 H) 10.35 (s, 1 H)

### EXAMPLE 62

### 1-{4-[3-(Pyrazin-2-ylamino)isoquinolin-1-yl]pyridin-2-yl}pyrrolidin-3-ol

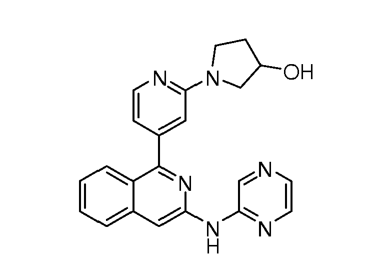

Obtained as a solid (94%) from 1-(2-fluoropyridin-4-yl)-N-pyrazin-2-ylisoquinolin-3-amine (example 57a) and pyrrolidin-3-ol following the experimental procedure as described in example 10a heating the reaction at 130°C for 1 day, followed by purification of the crude product by flash chromatography (95:5 dichloromethane /methanol).
LRMS (m/z): 385 (M+1)⁺.
1 H NMR (300 MHz, DMSO-*d*₆) δ ppm 1.83 - 2.17 (m, 2 H) 3.39 (br. s., 1 H) 3.54 (t, *J*=5.91 Hz, 3 H) 4.42 (br. s., 1 H) 4.99 (d, *J*=3.30 Hz, 1 H) 6.65 (s, 1 H) 6.80 (d, *J*=4.67 Hz, 1 H) 7.41 (t, *J*=7.55 Hz, 1 H) 7.69 (t, *J*=7.55 Hz, 1 H) 7.92 (d, *J*=8.24 Hz, 2 H) 8.06 (d, *J*=2.47 Hz, 1 H) 8.24 (d, *J*=4.94 Hz, 1 H) 8.31 (br. s., 1 H) 8.49 (s, 1 H) 8.72 (s, 1 H) 10.35 (s, 1 H)

### EXAMPLE 63

### 1-{4-[3-(Pyrazin-2-ylamino)isoquinolin-1-yl]pyridin-2-yllpiperidin-4-ol

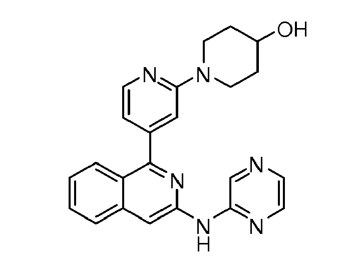

Obtained as a solid (57%) from 1-(2-fluoropyridin-4-yl)-N-pyrazin-2-ylisoquinolin-3-amine (example 57a) and piperidin-4-ol following the experimental procedure as described in example 10a heating the reaction at 130°C for 1 day, followed by purification of the crude product by reverse phase chromatography (0% CH₃CN in H₂O to 40% CH₃CN in H₂O).
LRMS (m/z): 399 (M+1)⁺.
1 H NMR (300 MHz, CHLOROFORM-*d*) δ ppm 1.46 - 1.77 (m, 3 H) 1.99 - 2.09 (m, 1 H) 3.10 - 3.36 (m, 2 H) 3.83 - 4.06 (m, 1 H) 4.09 - 4.26 (m, 2 H) 6.88 (d, *J*=4.94 Hz, 1 H) 6.95 (s, 1 H) 7.38 (t, *J*=7.14 Hz, 1 H) 7.64 (td, *J*=7.55, 0.82 Hz, 1 H) 7.83 - 7.91 (m, 2 H) 7.95 (d, *J*=8.51 Hz, 1 H) 8.05 (br. s., 1 H) 8.10 (d, *J*=2.75 Hz, 1 H) 8.27 (dd, *J*=2.75, 1.37 Hz, 1 H) 8.35 (d, *J*=4.94 Hz, 1 H) 8.45 (s, 1 H) 8.51 (d, *J*=1.37 Hz, 1 H)

### EXAMPLE 64

### 1-{4-[3-(Pyrazin-2-ylamino)isoquinolin-1-yl]pyridin-2-yl}piperazine-2-Carboxylic acid

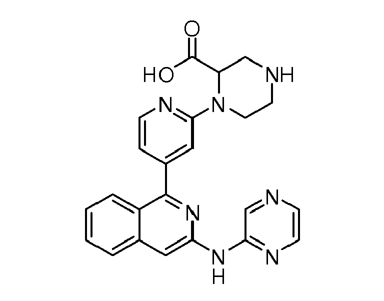

**a) 4-(*tert*-Butoxycarbonyl)-1-{4-[3-(pyrazin-2-ylamino)isoquinolin-1-yl]pyridin-2-yl}piperazine-2-Carboxylic acid**
   Obtained (37%) from 1-(2-fluoropyridin-4-yl)-*N*-pyrazin-2-ylisoquinolin-3-amine (example 57a) and 1-*tert*-butyl 3-methyl piperazine-1,3-dicarboxylate following the experimental procedure as described in example 10a heating the reaction at 130°C for 3 days, followed by addition of water to the crude. Then, 2N aqueous hydrochloric acid was added until the pH was 3-4. The resultant precipitate was filtered and purificated by flash chromatography.
   LRMS (m/z): 528 (M+1)⁺, 526 (M-1)⁻.
**b) 1-{4-[3-(Pyrazin-2-ylamino)isoquinolin-1-yl]pyridin-2-yl}piperazine-2-Carboxylic acid**
   Obtained as a yellow solid (18%) from 4-(*tert*-butoxycarbonyl)-1-{4-[3-(pyrazin-2-ylamino)isoquinolin-1-yl]pyridin-2-yl}piperazine-2-Carboxylic acid (example 64a) following the experimental procedure as described in example 2b, followed by purification of the crude product by reverse phase chromatography (0% CH₃CN in H₂O to 95% CH₃CN in H₂O).
   LRMS (m/z): 428 (M+1)⁺, 426 (M-1)⁻.
   1 H NMR (300 MHz, DMSO-*d*₆) δ ppm 2.57 - 3.31 (m, 6 H) 3.87 - 4.26 (m, 1 H) 6.65 - 7.08 (m, 2 H) 7.33 - 7.50 (m, 1 H) 7.68 (t, *J*=7.69 Hz, 1 H) 7.81 - 7.97 (m, 2 H) 8.06 (br. s., 1 H) 8.18 - 8.37 (m, 2 H) 8.43 - 8.58 (m, 1 H) 8.67 (d, *J*=18.68 Hz, 1 H) 10.35 (s, 1 H)

### EXAMPLE 65

### 4-{4-[3-(Pyrazin-2-ylamino)isoquino)in-1-yl]pyridin-2-y)}piperazin-2-one

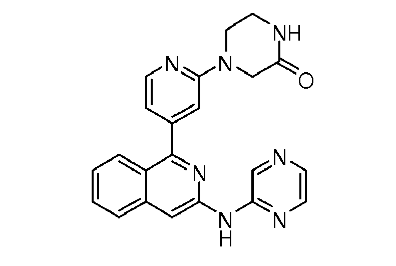

A mixture of 1-(2-fluoropyridin-4-yl)-*N*-pyrazin-2-ylisoquinolin-3-amine (example 57a, 0.03 g, 0.10 mmol), piperazin-2-one (0.02 g, 0.15 mmol) and triethylamine (0.02 mL, 0.15 mmol) in dimethylsulphoxide (2 mL) was heated at 120 °C for 60 minutes in Biotage Initiator Microwave Synthesizer. Then, the mixture was cooled, and dichloromethane and water were added to the mixture. The organic layer was washed with brine, dried (Na₂SO₄) and evaporated. Purification of the crude by flash chromatography (95:5 hexanes/ethyl acetate to 100% ethyl acetate) gave the title compound (0.01 g, 20%) as a solid.
LRMS (m/z): 398 (M+1)⁺.
1 H NMR (400 MHz, CHLOROFORM-*d*) δ ppm 3.53 (ddd, *J*=7.23, 3.03, 2.83 Hz, 2 H) 4.04 (t, *J*=5.28 Hz, 2 H) 4.25 (br. s., 2 H) 6.94 (s, 1 H) 7.03 (d, *J*=5.08 Hz, 1 H) 7.39 (td, *J*=7.67, 1.07 Hz, 1 H) 7.66 (t, *J*=7.52 Hz, 1 H) 7.89 (d, *J*=8.40 Hz, 1 H) 7.95 (d, *J*=8.79 Hz, 1 H) 8.12 (d, *J*=2.74 Hz, 1 H) 8.27 (dd, *J*=2.64, 1.47 Hz, 1 H) 8.39 (d, *J*=5.28 Hz, 1 H) 8.49 - 8.55 (m, 2 H)

### EXAMPLE 66

### 1-{4-[3-(Pyrazin-2-ylamino)isoquinolin-1-yl]pyridin-2-yl}piperazin-2-one

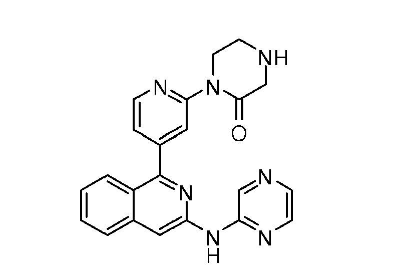

**a) *tert*-Butyl 4-[4-(3-Chloroisoquinolin-1-yl)pyridin-2-yl]-3-oxopiperazine-1-Carboxylate**
   Obtained (54%) from 1,3-dichloroisoquinoline and {2-[4-(*ter*t-butoxycarbonyl)-2-oxopiperazin-1-yl]pyridin-4-yl}boronic acid (preparation 21 b) following the experimental procedure as described in preparation 1 using dioxane as a solvent, followed by purification by flash chromatography (9:1 hexanes/ethyl acetate to 100% ethyl acetate).
   LRMS (m/z): 439 (M+1)⁺.
   1 H NMR (400 MHz, CHLOROFORM-*d*) δ ppm 1.50 (s, 9 H) 3.79 (t, *J*=5.20 Hz, 2 H) 4.19 (t, *J*=4.00 Hz, 2 H) 1.50 (s, 9 H) 4.30 (s, 2 H) 7.50 (dd, *J*=5.20, 1.60 Hz, 1 H) 7.63 (t, *J*=8.00 Hz, 1 H) 7.75 (t, *J*=7.20 Hz, 1 H) 7.79 (s, 1 H) 7.85 (d, *J*=8.40 Hz, 1 H) 8.20 (d, *J*=8.40 Hz, 1 H) 8.29 (s, 1 H) 8.60 (d, *J*=4.80 Hz, 1 H)
**b) *tert*-butyl 3-oxo-4-{4-[3-(pyrazin-2-ylamino)isoquinolin-1-yl]pyridin-2-yl}piperazine-1 -Carboxylate**
   Obtained (30%) from *tert*-butyl 4-[4-(3-Chloroisoquinolin-1-yl)pyridin-2-yl]-3-oxopiperazine-1-Carboxylate (example 66a) and pyrazin-2-amine following the experimental procedure as described in preparation 4, followed by purification of the crude product by flash chromatography (9:1 hexanes/ethyl acetate to 100% ethyl acetate).
   LRMS (m/z): 498 (M+1)⁺.
**c) 1-{4-[3-(Pyrazin-2-ylamino)isoquinolin-1-yl]pyridin-2-yl}piperazin-2-one**
   Obtained as a solid (77%) from *tert*-butyl 3-oxo-4-{4-[3-(pyrazin-2-ylamino)isoquinolin-1-yl]pyridin-2-yl}piperazine-1-Carboxylate (example 66b) following the experimental procedure as described in example 2b.
   LRMS (m/z): 398 (M+1)⁺.
   1 H NMR (400 MHz, CHLOROFORM-*d*) δ ppm 3.28 (d, *J*=5.67 Hz, 2 H) 3.75 (s,
   2 H) 4.10 (d, *J*=5.47 Hz, 2 H) 7.42 (ddd, *J*=8.35, 6.98, 1.07 Hz, 1 H) 7.47 (dd, *J*=4.98, 1.47 Hz, 1 H) 7.57 - 7.68 (m, 2 H) 7.86 (d, *J*=8.40 Hz, 1 H) 8.07 (s, 1 H) 8.10 (d, *J*=2.74 Hz, 2 H) 8.27 (dd, *J*=2.64, 1.47 Hz, 1 H) 8.31 - 8.34 (m, 1 H) 8.41 (s, 1 H) 8.52 (d, *J*=1.37 Hz, 1 H) 8.61 (d, *J*=4.88 Hz, 1 H)

### EXAMPLE 67

### 2'-Piperazin-1-yl-6-(pyrazin-2-ylamino)-2,4'-bipyridine-4-Carbonitrile

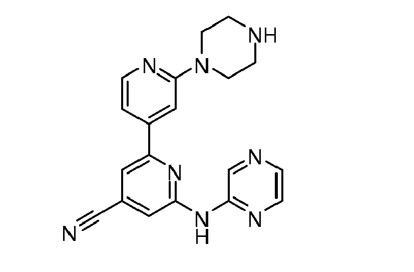

**a) 2-Chloro-6-(pyrazin-2-ylamino)isonicotinonitrile**
   Obtained as a solid (59%) from 2,6-dichloroisonicotinonitrile and pyrazin-2-amine following the experimental procedure as described in preparation 4, using dioxane as solvent.
   LRMS (m/z): 232 (M+1)⁺.
**b) *tert*-butyl 4-[4-Cyano-6-(pyrazin-2-ylamino)-2,4'-bipyridin-2'-yl]piperazine-1-Carboxylate**
   Obtained (58%) from 2-Chloro-6-(pyrazin-2-ylamino)isonicotinonitrile (example 67a) and {2-[4-(*tert*-butoxycarbonyl)piperazin-1-yl]pyridin-4-yl}boronic acid (preparation 24b) following the experimental procedure as described in preparation 1, using cesium carbonate as base and dioxane as solvent, followed by purification by flash chromatography (hexanes/ethyl acetate 30% to 100% ethyl acetate).
   LRMS (m/z): 459 (M+1)⁺.
**c) 2'-piperazin-1-yl-6-(pyrazin-2-ylamino)-2,4'-bipyridine-4-Carbonitrile**
   Obtained as hydrochloride salt (94%) from *tert*-butyl 4-[4-Cyano-6-(pyrazin-2-ylamino)-2,4'-bipyridin-2'-yl]piperazine-1-Carboxylate (example 67b) following the experimental procedure as described in example 2b.
   LRMS (m/z): 359 (M+1)⁺.
   1 H NMR (400 MHz, DMSO-*d*₆) □ ppm 3.28 (br. s., 4 H) 4.05 (d, *J*=4.69 Hz, 4 H) 7.59 (d, *J*=5.86 Hz, 1 H) 7.83 (s, 1 H) 8.20 - 8.33 (m, 3 H) 8.37 (dd, *J*=2.54, 1.37 Hz, 1 H) 9.03 (d, *J*=1.17 Hz, 1 H) 9.65 (br. s., 2 H) 10.78 (s, 1 H).

### EXAMPLE 68

### 6-[3-(2-Aminoethyl)phenyl]-4-phenyl-N-[4-(trifluoromethyl)pyridin-2-yl]pyridin-2-amine

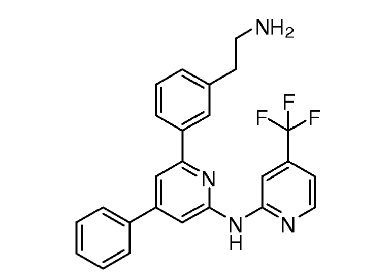

**a) 4-Phenyl-*N*-[4-(trifluoromethyl)pyridin-2-yl]pyridine-2,6-diamine**
   Obtained (37%) from 4-phenylpyridine-2,6-diamine (*Org. Lett.* **2004**, 6, 385) and 1-bromo-3-(trifluoromethyl)benzene following the experimental procedure as described in preparation 4, followed by purification of the crude product by flash chromatography (95:5 hexanes/ethyl acetate to 2:3 hexanes/ethyl acetate).
   LRMS (m/z): 331 (M+1)⁺.
**b) 6-Chloro-4-phenyl-*N*-[4-(trifluoromethyl)pyridin-2-yl]pyridin-2-amine**
   A solution of sodium nitrite (0.02 g, 0.31 mmol) in water (0.2 mL) was added slowly to a stirred suspension of 4-phenyl-*N*-[4-(trifluoromethyl)pyridin-2-yl]pyridine-2,6-diamine (example 68a, 0.05 g, 0.15 mmol) in hydrogen chloride (6N, 2 mL) at -5 °C, and the mixture was stirred at this temperature. After 10 minutes, a solution of copper(I) chloride (0.05 g, 0.46 mmol) in hydrogen chloride (6N, 2 mL) was added to the mixture. The reaction was warmed to room temperature slowly and stirred at this temperature for 1 hour. After heating 1 hour more at 60 °C, the mixture was cooled and 6N aqueous sodium hydroxide was added until the pH was 5-6. The aqueous layer was extracted with dichloromethane, dried (Na₂SO₄) and evaporated. Purification of the crude by flash chromatography (95:5 hexanes/ethyl acetate to 3:2 hexanes/ethyl acetate) gave the title compound (0.03 g, 48%).
   LRMS (m/z): 350/352 (M+1)⁺.
**c) *tert*-Butyl {2-[3-(4-phenyl-6-{[4-(trifluoromethyl)pyridin-2-yl]amino}pyridin-2-yl)phenyl]ethyl}carbamate**
   Obtained (58%) from 6-Chloro-4-phenyl-*N*-[4-(trifluoromethyl)pyridin-2-yl]pyridin-2-amine (example 68b) and (3-{2-[(*tert-*butoxycarbonyl)amino]ethyl}phenyl)boronic acid (preparation 3b) following the experimental procedure as described in preparation 1 followed by purification by flash chromatography.
   LRMS (m/z): 535 (M+1)⁺.
**d) 6-[3-(2-Aminoethyl)phenyl]-4-phenyl-*N*-[4-(trifluoromethyl)pyridin-2-yl]pyridin-2-amine**
   Obtained as a solid (72%) from *tert*-butyl {2-[3-(4-phenyl-6-{[4-(trifluoromethyl)pyridin-2-yl]amino}pyridin-2-yl)phenyl]ethyl}carbamate (example 68c) following the experimental procedure as described in example 2b.
   LRMS (m/z): 435 (M+1)⁺.
   1 H NMR (400 MHz, CHLOROFORM-*d*) δ ppm 2.77 - 2.91 (m, 2 H) 2.92 - 3.22 (m, 2 H) 7.09 (dd, *J*=5.18, 0.88 Hz, 1 H) 7.27 (d, *J*=1.17 Hz, 1 H) 7.30 (d, *J*=7.42 Hz, 1 H) 7.41 - 7.55 (m, 4 H) 7.59 (d, *J*=1.17 Hz, 1 H) 7.65 - 7.73 (m, 2 H) 7.78 (br. s., 1 H) 7.91 (d, *J*=7.82 Hz, 1 H) 7.98 (s, 1 H) 8.42 (d, *J*=5.08 Hz, 1 H) 8.66 (s, 1 H)

### EXAMPLE 69

### N-{4-Pheny)-6-[3-(1,2,3,6-tetrahydropyridin-4-yl)phenyl]pyridin-2-yl}pyrazin-2-amine

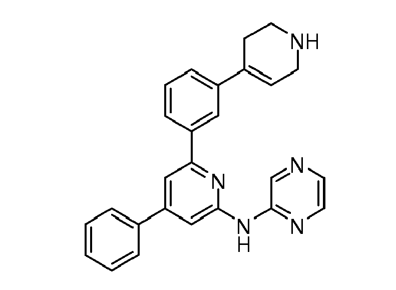

**a) *N*-(6-Bromo-4-phenylpyridin-2-yl)pyrazin-2-amine**
   Obtained (65%) from 2,6-dibromo-4-phenylpyridine (preparation 22d) and pyrazin-2-amine following the experimental procedure as described in preparation 4 followed by purification by flash chromatography (100% hexanes to 2:3 hexanes/ethyl acetate).
   LRMS (m/z): 327/329 (M+1)⁺.
   1 H NMR (500 MHz, CHLOROFORM-*d*) δ ppm 7.33 (d, *J*=1.50 Hz, 1 H) 7.47 - 7.55 (m, 4 H) 7.62 - 7.65 (m, 2 H) 8.00 (d, *J*=2.00 Hz, 1 H) 8.16 (d, *J*=3.00 Hz, 1 H) 8.21 - 8.23 (m, 1 H) 7.74 (d, *J*=2.00 Hz, 1 H)
**b) *tert-*Butyl 4-{3-[4-phenyl-6-(pyrazin-2-ylamino)pyridin-2-yl]phenyl}-3,6-dihydropyridine-1(2*H*)-Carboxylate**
   Obtained (71%) from *N*-(6-bromo-4-phenylpyridin-2-yl)pyrazin-2-amine (example 69a) and *tert-*butyl 4-[3-(4,4,5-trimethyl-1,3,2-dioxaborolan-2-yl)phenyl]-3,6-dihydropyridine-1(2*H*)-Carboxylate (preparation 9b) following the experimental procedure as described in preparation 1 using microwave at 100°C for 1 hour, followed by purification by flash chromatography (100% hexanes to 1:4 hexanes/ethyl acetate).
   LRMS (m/z): 506 (M+1)⁺.
   1 H NMR (500 MHz, CHLOROFORM-*d*) δ ppm 1.51 (s, 9 H) 2.62 (br. s., 2 H) 3.67 - 3.69 (m, 2 H) 4.17 (br. s., 2 H) 6.20 (br. s., 1 H) 7.45 - 7.55 (m, 6 H) 7.59 (d, *J*=1.50 Hz, 1 H) 7.71 - 7.75 (m, 3 H) 7.95 (d, *J*=8.50 Hz, 1 H) 8.06 (br. s., 1 H) 8.15 (d, *J*=3.50 Hz, 1 H) 8.22 - 8.23 (m, 1 H) 9.12 (d, *J*=1.50 Hz, 1 H)
**c) *N*-{4-Phenyl-6-[3-(1,2,3,6-tetrahydropyridin-4-y))phenyl]pyridin-2-yl}pyrazin-2-amine**
   Obtained as a solid (48%) from *tert*-butyl 4-{3-[4-phenyl-6-(pyrazin-2-ylamino)pyridin-2-yl]phenyl}-3,6-dihydropyridine-1 (2*H*)-Carboxylate (example 69b) following the experimental procedure as described in example 2b.
   LRMS (m/z): 406 (M+1)⁺.
   1 H NMR (400 MHz, METHANOL-*d*₄) δ ppm 2.51 - 2.68 (m, 2 H) 3.09 (t, *J*=5.67 Hz, 2 H) 3.50 (d, *J*=2.74 Hz, 2 H) 6.28 (br. s., 1 H) 7.40 - 7.55 (m, 5 H) 7.66 (d, *J*=1.17 Hz, 1 H) 7.71 - 7.81 (m, 4 H) 7.97 (dt, *J*=7.28, 1.44 Hz, 1 H) 8.05 (d, *J*=2.54 Hz, 1 H) 8.19 (br. s., 1 H) 8.27 (dd, *J*=2.74, 1.56 Hz, 1 H) 9.35 (d, *J*=1.56 Hz, 1 H)

### EXAMPLE 70

### N-(5-Methyl-1H-pyrazol-3-yl)-4-phenyl-6-[3-(1,2,3,6-tetrahydropyridin-4-yl)phenyl]pyridin-2-amine

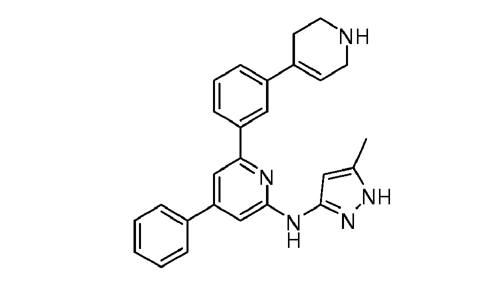

**a) 6-Bromo-N-(1-tert-butyl-5-methyl-1 *H*-pyrazol-3-yl)-4-phenylpyridin-2-amine**
   Obtained (43%) from 2,6-dibromo-4-phenylpyridine (preparation 22d) and *1-tert-*butyl-5-methyl-1 *H* pyrazol-3-amine following the experimental procedure as described in preparation 4 followed by purification by flash chromatography (100% hexanes to 1:1 hexanes/ethyl acetate).
   LRMS (m/z): 385/387 (M+1)⁺.
   1 H NMR (500 MHz, CHLOROFORM-*d*) δ ppm 1.63 (s, 9 H) 2.25 (s, 3 H) 5.98 (s, 1 H) 6.09 (br. s., 1 H) 6.56 (d, *J*=1.50 Hz, 1 H) 7.14 (d, *J*=1.50 Hz, 1 H) 7.42 - 7.47 (m, 5 H)
**b) *tert-Butyl* 4-(3-{6-[(1-*tert*-butyl-5-methyl-1*H*-pyrazol-3-yl)amino]-4-phenylpyridin-2-yl}phenyl)-3,6-dihydropyridine-1 (2*H*)-Carboxylate**
   Obtained (81 %) from 6-bromo-*N*-(1-*tert*-butyl-5-methyl-1*H* pyrazol-3-yl)-4-phenylpyridin-2-amine (example 70a) and *tert*-butyl 4-[3-(4,4,5-trimethyl-1,3,2-dioxaborolan-2-yl)phenyl]-3,6-dihydropyridine-1 (2*H*)-Carboxylate (preparation 9b) following the experimental procedure as described in preparation 1 using microwave at 100°C for 15 minutes, followed by purification by flash chromatography (100% hexanes to 1:4 hexanes/ethyl acetate).
   LRMS (m/z): 564 (M+1)⁺.
   1 H NMR (500 MHz, CHLOROFORM-*d*) δ ppm 1.50 (s, 9 H) 1.67 (s, 9 H) 2.27 (s, 3 H) 2.61 (br. s., 2 H) 3.67 (t, *J*=6.50 Hz, 2 H) 4.11 (br. s., 2 H) 6.00 (s, 1 H) 6.13 (br. s., 1 H) 6.24 (br. s., 1 H) 6.63 (d, *J*=1.50 Hz, 1 H) 7.40 - 7.48 (m, 6 H) 7.55 - 7.59 (m, 2 H) 7.87 - 7.90 (m, 1 H) 8.05 (br. s., 1 H)
**c) *N*-(5-Methyl-1*H*-pyrazol-3-yl)-4-phenyl-6-[3-(1 ,2,3,6-tetrahydropyridin-4-yl)phenyl]pyridin-2-amine**
   Obtained as a solid (18%) from *tert*-butyl 4-(3-{6-[(1-tert-butyl-5-methyl-1*H-*pyrazol-3-yl)amino]-4-phenylpyridin-2-yl}phenyl)-3,6-dihydropyridine-1 (2*H*)-Carboxylate (example 70b) following the experimental procedure as described in example 40b.
   LRMS (m/z): 408 (M+1)⁺.
   1 H NMR (400 MHz, METHANOL-*d*₄) δ ppm 2.28 (s, 3 H) 2.70 (br. s., 2 H) 3.29 ― 3.30 (m, 2 H) 3.62 (br. s., 2 H) 6.20 (br. s., 1 H) 6.26 (br. s., 1 H) 7.15 (br. s., 1 H) 7.43 - 7.50 (m, 6 H) 7.72 (d, *J*=8.40 Hz, 2 H) 7.92 (d, *J*=7.60 Hz, 1 H) 8.16 (br. s., 1 H)

### EXAMPLE 71

### 4-Phenyl-2'-piperazin-1-yl-N-pyrazin-2-yl-2,4'-bipyridin-6-amine

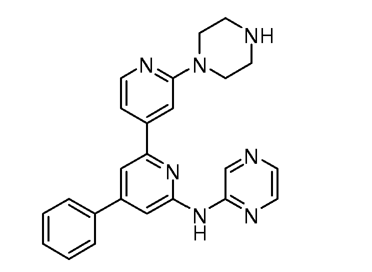

**a) *tert*-Butyl 4-(6-bromo-4-phenyl-2,4'-bipyridin-2'-yl)piperazine-1-Carboxylate** Obtained (18%) from 2,6-dibromo-4-phenylpyridine (preparation 22) and {2-[4-*(tert-*butoxycarbonyl)piperazin-1-yl]pyridin-4-yl}boronic acid (preparation 24) following the experimental procedure as described in preparation 1 followed by purification by flash chromatography (100% hexanes to 100% ethyl acetate).
   LRMS (m/z): 495/497 (M+1)⁺.
**b) *tert-*Butyl 4-[4-phenyl-6-(pyrazin-2-ylamino)-2,4'-bipyridin-2'-yl]piperazine-1-Carboxylate**
   Obtained (33%) from *tert*-butyl 4-(6-bromo-4-phenyl-2,4'-bipyridin-2'-yl)piperazine-1-Carboxylate (example 71 a) and pyrazin-2-amine following the experimental procedure as described in preparation 4 using dioxane as a solvent, followed by reverse phase chromatography (0% CH₃OH in H₂O to 100% CH₃OH in H₂O).
   LRMS (m/z): 510 (M+1)⁺.
**c) 4-Phenyl-2'-piperazin-1-yl-N-pyrazin-2-yl-2,4'-bipyridin-6-amine**
   Obtained as hydrochloride salt (50%) from *tert-*butyl 4-[4-phenyl-6-(pyrazin-2-ylamino)-2,4'-bipyridin-2'-yl]piperazine-1-Carboxylate (example 71b) following the experimental procedure as described in example 40b.
   LRMS (m/z): 410 (M+1)⁺
   1 H NMR (400 MHz, DMSO-*d*₆) δ ppm 3.29 (br. s., 4 H) 4.05 (br. s., 4 H) 7.49 - 7.64 (m, 3 H) 7.70 (d, *J*=5.86 Hz, 1 H) 7.87 (d, *J*=7.03 Hz, 3 H) 8.07 (s, 2 H) 8.18 (d, *J*=2.74 Hz, 1 H) 8.30 (d, *J*=5.86 Hz, 1 H) 8.33 (d, *J*=1.17 Hz, 1 H) 9.25 (s, 1 H) 9.53 (br. s., 1 H) 10.38 (s, 1H)

### EXAMPLE 72

### N-{2-(Pyrazin-2-ylamino)-6-[3-(1,2,3,6-tetrahydropyridin-4-yl)phenyl]pyridin-4-yl}benzamide

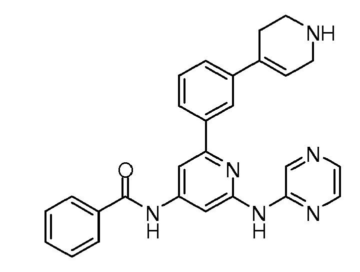

**a) *N*-[2-Bromo-6-(pyrazin-2-ylamino)pyridin-4-yl]benzamide**
   Obtained (35%) from *N*-(2,6-dibromopyridin-4-yl)benzamide (preparation 23) and pyrazin-2-amine following the experimental procedure as described in preparation 4 using microwave at 100°C for 30 minutes, followed by purification by flash chromatography (100% hexanes to 1:2 hexanes/ethyl acetate).
   LRMS (m/z): 370/372 (M+1)⁺.
**b) *tert*-Butyl 4-{3-[4-(benzoylamino)-6-(pyrazin-2-ylamino)pyridin-2-yl]phenyl}-3,6-dihydropyridine-1 (2*H*)-Carboxylate**
   Obtained (68%) from *N*-[2-bromo-6-(pyrazin-2-ylamino)pyridin-4-yl]benzamide (example 72a) and *tert-*butyl 4-[3-(4,4,5-trimethyl-1 ,3,2-dioxaborolan-2-yl)phenyl]-3,6-dihydropyridine-1 (2*H*)-Carboxylate (preparation 9b) following the experimental procedure as described in preparation 1 using microwave at 120°C for 30 minutes, followed by purification by flash chromatography (7:3 hexanes/ethyl acetate to 100% ethyl acetate).
   LRMS (m/z): 549 (M+1)⁺.
**c) *N*-{2-(Pyrazin-2-ylamino)-6-[3-(1,2,3,6-tetrahydropyridin-4-yl)phenyl] pyridin-4-yl}benzamide**
   Obtained as a solid (76%) from *tert*-butyl 4-{3-[4-(benzoylamino)-6-(pyrazin-2-ylamino)pyridin-2-yl]phenyl}-3,6-dihydropyridine-1 (2*H*)-Carboxylate (example 72b) following the experimental procedure as described in example 2b.
   LRMS (m/z): 449 (M+1)⁺.
   1 H NMR (400 MHz, METHANOL-*d*₄) δ ppm 2.60 (ddd, *J*=7.18, 3.22, 2.88 Hz, 2 H) 3.13 (t, *J*=5.86 Hz, 2 H) 3.53 (q, *J*=2.61 Hz, 2 H) 6.25 (dt, *J*=3.22, 1.71 Hz, 1 H) 7.42 (d, *J*=7.62 Hz, 1 H) 7.44 - 7.48 (m, 1 H) 7.50 - 7.55 (m, 2 H) 7.58 - 7.63 (m, 1 H) 7.81 (d, *J*=1.56 Hz, 1 H) 7.89 (dt, *J*=7.52, 1.32 Hz, 1 H) 7.94 - 7.99 (m, 2 H) 8.02 (d, *J*=2.74 Hz, 1 H) 8.04 (d, *J*=1.56 Hz, 1 H) 8.10 (t, *J*=1.56 Hz, 1 H) 8.25 (dd, *J*=2.64, 1.47 Hz, 1 H) 9.26 (d, *J*=1.37 Hz, 1 H)

### EXAMPLE 73

### 3-[({2-(Pyrazin-2-ylamino)-6-[3-(1,2,3,6-tetrahydropyridin-4-yl) phenyl] pyridin-4-yl}amino)carbonyl]benzoic acid

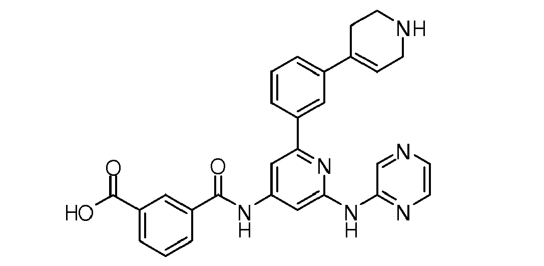

**a) *tert-Butyl* 4-{3-[4-{[3-(tert-butoxycarbonyl)benzoyl]amino}-6-(pyrazin-2-ylamino)pyridin-2-yl]phenyl}-3,6-dihydropyridine-1 (2*H*)-Carboxylate**
   *tert-*Butyl 4-amino-6-(pyrazin-2-ylamino)-3",6"-dihydro-2,4':2',4"-terpyridine-1"(2"*H*)-Carboxylate (preparation 30b, 0.60 g, 1.35 mmol) was added to a stirred solution of 3-(*tert-*butoxycarbonyl)benzoic acid (0.15 g, 0.67 mmol), *N-*[(dimethylamino)(3*H* [1,2,3]triazolo[4,5-b]pyridin-3-yloxy)methylene]-*N*
   methylmethanaminium hexafluorophosphate (0.52 g, 1.37 mmol) and ethyl(diisopropyl)amine (0.30 mL, 1.69 mmol) in N,N'-dimethylformamide (10 mL), and the mixture was stirred at room temperature. After 2 days, water and ethyl acetate were added to the reaction mixture. The organic layer was washed with saturated aqueous sodium bicarbonate solution and brine, dried (MgSO₄) and evaporated in vacuo. The crude product was purified by reverse phase chromatography (0% CH₃OH in H₂O to 100% CH₃OH in H₂O) to give the title compound (0.21 g, 48%).
   LRMS (m/z): 649 (M+1)⁺.
**b) 3-({[6-(Pyrazin-2-ylamino)-1" ",2",3",6"-tetrahydro-2,4':2',4"-terpyridin-4-yl]amino}carbonyl)benzoic acid**
   A solution of *tert*-butyl 4-{3-[4-{[3-(tert-butoxycarbonyl)benzoyl]amino}-6-(pyrazin-2-ylamino)pyridin-2-yl]phenyl}-3,6-dihydropyridine-1(2*H*)-Carboxylate (example 73a, 0.21 g, 0.32 mmol) was dissolved in 4M hydrochloric acid in dioxane (10 mL) and the solution was stirred at room temperature overnight. The solvent was evaporated and the crude was purified by reverse phase chromatography (0% CH₃OH in H₂O to 100% CH₃OH in H₂O to yield 13 mg (8%) of the title compound as a solid.
   LRMS (m/z): 493 (M+1)⁺.
   1 H NMR (400 MHz, CHLOROFORM-*d*) δ ppm 2.60 (br. s., 2 H) 3.68 (t, *J*=5.47 Hz, 2 H) 4.11 (br. s., 3 H) 7.39 - 7.50 (m, 2 H) 7.61 (t, *J*=7.82 Hz, 1 H) 7.87 (br. s., 1 H) 7.91 (d, *J*=6.64 Hz, 1 H) 7.98 - 8.08 (m, 3 H) 8.11 - 8.25 (m, 5 H) 8.29 (br. s., 1 H) 8.50 (s, 1 H) 8.97 (s, 1 H)

### EXAMPLE 74

### (2S)-2-Benzyl-4-oxo-4-{[6-(pyrazin-2-ylamino)-1" ",2",3",6"-tetrahydro-2,4':2',4"-terpyridin-4-yl]aminol}butanoic acid

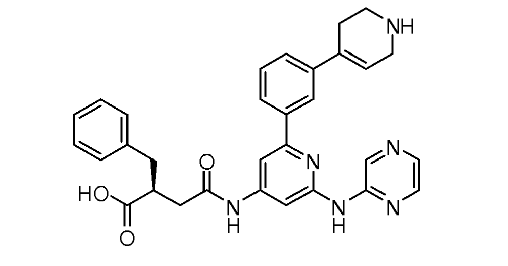

**a) *tert*-Butyl 4-{3-[4-{[(3*S*)-3-benzyl-4-methoxy-4-oxobutanoyl]amino}-6-(pyrazin-2-ylamino)pyridin-2-yl]phenyl}-3,6-dihydropyridine-1(2*H*)-Carboxylate**
   Obtained (34%) from *tert*-butyl 4-amino-6-(pyrazin-2-ylamino)-3",6"-dihydro-2,4':2',4"-terpyridine-1"(2"*H*)-Carboxylate (preparation 30b) and (3*S*)-3-benzyl-4-methoxy-4-oxobutanoic acid following the experimental procedure as described in example 73a followed by purification by reverse phase chromatography (0% CH₃OH in H₂O to 100% CH₃OH in H₂O).
   LRMS (m/z): 649 (M+1)⁺.
   1 H NMR (400 MHz, CHLOROFORM-*d*) □ ppm 0.01 (s, 1 H) 1.53 (s, 9 H) 2.43 - 2.59 (m, 3 H) 2.78 (dd, *J*=15.63, 9.77 Hz, 1 H) 2.88 (dd, *J*=13.68, 8.21 Hz, 1 H) 3.13 (dd, *J*=13.68, 5.86 Hz, 1 H) 3.30 - 3.41 (m, 1 H) 3.66 (t, *J*=5.67 Hz, 2 H) 3.73 (s, 3 H) 4.09 (br. s., 2 H) 6.06 (br. s., 1 H) 7.15 - 7.22 (m, 2 H) 7.29 - 7.35 (m, 3 H) 7.36 - 7.44 (m, 2 H) 7.69 (br. s., 1 H) 7.75 (s, 1 H) 7.81 (d, *J*=7.03 Hz, 1 H) 7.93 (s, 1 H) 8.08 (d, *J*=2.34 Hz, 2 H) 8.14 (d, *J*=1.56 Hz, 1 H) 8.84 (s, 1 H)
**b) (2*S*)-2-Benzyl-4-{[2-[3-[1-(*tert*-butoxycarbonyl)-1,2,3,6-tetrahydropyridin-4-yl]phenyl}-6-(pyrazin-2-ylamino)pyridin-4-yl]amino}-4-oxobutanoic acid**
   Lithium hydroxide (5 mg, 0.12 mmol) was added to a stirred solution of *tert-*butyl 4-{3-[4-{[(3*S*)-3-benzyl-4-methoxy-4-oxobutanoyl]amino}-6-(pyrazin-2-ylamino)pyridin-2-yl]phenyl}-3,6-dihydropyridine-1 (2*H*)-Carboxylate (example 74a, 0.05 g, 0.08 mmol) in tetrahydrofuran (3 mL) and water (1 mL), and the mixture was stirred at room temperature. After 1 hour, the solvent was evaporated to give a crude which was used in the next reaction stept without any further treatment.
   LRMS (m/z): 635 (M+1)⁺.
**c) (2*S*)-2-Benzyl-4-oxo-4-{[6-(pyrazin-2-ylamino)-1",2",3",6"-tetrahydro-2,4':2',4"-terpyridin-4-yl]amino}butanoic acid**
   Obtained as a salt (30%) from (2*S*)-2-benzyl-4-{[2-{3-[1-(*tert*-butoxycarbonyl)-1,2,3,6-tetrahydropyridin-4-yl]phenyl}-6-(pyrazin-2-ylamino)pyridin-4-yl]amino}-4-oxobutanoic acid (example 74b) following the experimental procedure as described in example 73b stirring at room temperature for 30 min, followed by purification by reverse phase chromatography (0% CH₃OH in H₂O to 100% CH₃OH in H₂O).
   LRMS (m/z): 535 (M+1)⁺.

### EXAMPLE 75

### N-[2'-Piperazin-1-yl-6-(pyrazin-2-ylamino)-2,4'-bipyridin-4-yl] benzamide

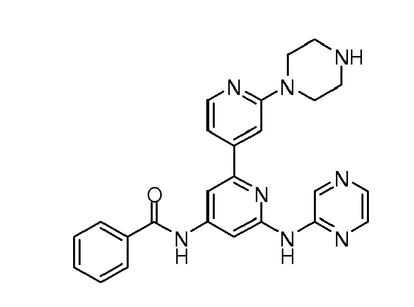

**a) *N*-[2'-Fluoro-6-(pyrazin-2-ylamino)-2,4'-bipyridin-4-yl]benzamide**
   Obtained (61 %) from *N*-[2-bromo-6-(pyrazin-2-ylamino)pyridin-4-yl]benzamide
   (example 72a) and (2-fluoropyridin-4-yl)boronic acid (preparation 24) following the experimental procedure as described in preparation 1, followed by purification by flash chromatography.
   LRMS (m/z): 387 (M+1)⁺.
**b) *tert*-Butyl 4-[4-(benzoylamino)-6-(pyrazin-2-ylamino)-2,4'-bipyridin-2'-yl]piperazine-1-Carboxylate**
   Obtained as a solid (73%) from *N*-[2'-fluoro-6-(pyrazin-2-ylamino)-2,4'-bipyridin-4-yl]benzamide (example 75a) and *tert*-butyl piperazine-1-Carboxylate following the experimental procedure as described in example 10a, followed by purification by flash chromatography.
   LRMS (m/z): 553 (M+1)⁺.
**c) *N*-[2'-Piperazin-1-yl-6-(pyrazin-2-ylamino)-2,4' -bipyridin-4-yl]benzamide**
   Obtained as a solid (21%) from *tert*-butyl 4-[4-(benzoylamino)-6-(pyrazin-2-ylamino)-2,4'-bipyridin-2'-yl]piperazine-1-Carboxylate (example 75b) following the experimental procedure as described in example 2b.
   LRMS (m/z): 453 (M+1)⁺.
   1 H NMR (500 MHz, METHANOL-*d*₄) δ ppm 2.87 (d, *J*=5.08 Hz, 4 H) 3.47 (d, *J*=5.08 Hz, 4 H) 7.16 (d, *J*=6.50 Hz, 1 H) 7.36 (s, 1 H) 7.44 (t, *J*=8.50 Hz, 2 H) 7.51 (d, *J*=9.00 Hz, 1 H) 7.75 (d, *J*=1.50 Hz, 1 H) 7.86 (s, 1 H) 7.88 (s, 1 H) 7.94 (d, *J*=3.5 Hz, 1 H) 8.06 - 8.08 (m, 2 H) 8.14 - 8.16 (m, 1 H) 9.08 (d, *J*=1.50 Hz, 1 H)

### EXAMPLE 76

### N-[2'-Piperazin-1-yl-6-(pyrazin-2-ylamino)-2,4'-bipyridin-4-yl]-3-(trifluoromethyl) benzamide

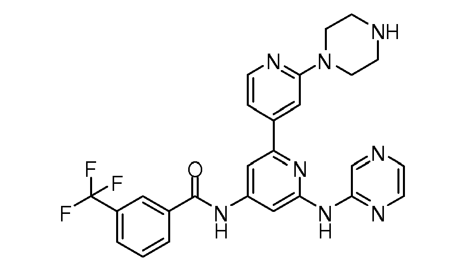

**a) *tert*-Butyl 4-(6-(pyrazin-2-ylamino)-4-{[3-(trifluoromethyl)benzoyl]amino}-2,4'-bipyridin-2'-yl)piperazine-1-Carboxylate**
   3-(Trifluoromethyl)benzoyl chloride (0.03 mL, 0.18 mmol) was added to a stirred solution of *tert*-butyl 4-[4-amino-6-(pyrazin-2-ylamino)-2,4'-bipyridin-2'-yl]piperazine-1-Carboxylate (preparation 26, 0.04 g, 0.09 mmol) and triethylamine (0.03 mL, 0.18 mmol) in tetrahydrofuran (0.5 mL), and the mixture was stirred at room temperature. After 3 hours, water and ethyl acetate were added to the reaction mixture. The organic layer was washed with brine, and dried (MgSO₄) and evaporated. Purification of the crude by flash chromatography (7:3 hexanes/ethyl acetate to 100% ethyl acetate) gave the title compound (0.03 g, 53%).
   LRMS (m/z): 621 (M+1)⁺.
   1 H NMR (500 MHz, ACETONE) δ ppm 1.48 (s, 9 H) 3.55 - 3.58 (m, 4 H) 3.64 - 3.67 (m, 4 H) 7.25 (dd, *J*=6.50, 2.00 Hz, 1 H) 7.48 (s, 1 H) 7.83 (t, *J*=10.00 Hz, 1 H) 7.98 - 8.00 (m, 2 H) 8.14 (dd, *J*=6.50, 1.50 Hz, 1 H) 8.23 - 8.24 (m, 1 H) 8.27 - 8.29 (m, 2 H) 8.33 - 8.35 (m, 2 H) 9.20 (s, 1 H) 9.34 (d, *J*=2.00 Hz, 1 H) 10.20 (s, 1 H)
**b) *N-*[2'-Piperazin-1-yl-6-(pyrazin-2-ylamino)-2,4'-bipyridin-4-yl]-3-(trifluoromethyl) benzamide**
   Obtained as a solid (76%) from *tert-*butyl 4-(6-(pyrazin-2-ylamino)-4-{[3-(trifluoromethyl)benzoyl]aminol-2,4'-bipyridin-2'-yl)piperazine-1-Carboxylate (example 76a) following the experimental procedure as described in example 2b.
   LRMS (m/z): 521 (M+1)⁺.
   1 H NMR (400 MHz, DMSO-d*₆*) δ ppm 2.82 (t, *J*=5.08 Hz, 4 H) 3.49 (t, *J*=5.08 Hz, 4 H) 7.14 (d, *J*=4.69 Hz, 1 H) 7.34 (s, 1 H) 7.81 (t, *J*=7.82 Hz, 1 H) 7.89 (d, *J*=1.37
   Hz, 1 H) 8.01 (d, *J*=7.62 Hz, 1 H) 8.11 (d, *J*=2.74 Hz, 1 H) 8.22 - 8.35 (m, 5 H) 9.19 (d, *J*=1.37 Hz, 1 H) 10.17 (s, 1 H) 10.85 (s, 1 H)

### EXAMPLE 77

### N-[2'-Piperazin-1-yl-6-(pyrazin-2-ylamino)-2,4' -bipyridin-4-yl]-1,3-benzodioxole-5-Carboxamide

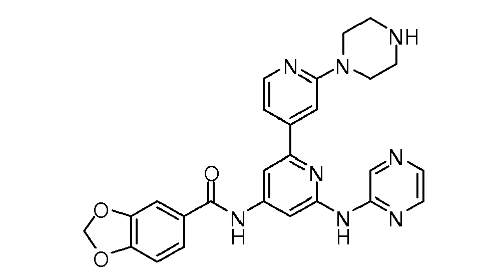

**a) *tert*-Butyl 4-[4-[(1,3-benzodioxol-5-ylcarbonyl)amino]-6-(pyrazin-2-ylamino)-2,4'-bipyridin-2'-yl]piperazine-1-Carboxylate**
   Obtained (15%) from *tert*-butyl 4-[4-amino-6-(pyrazin-2-ylamino)-2,4'-bipyridin-2'-yl]piperazine-1-Carboxylate (preparation 26) and 1,3-benzodioxole-5-Carbonyl chloride following the experimental procedure as described in example 76a, followed by purification by flash chromatography.
   LRMS (m/z): 597 (M+1)⁺.
   1 H NMR (500 MHz, ACETONE) δ ppm 1.48 (s, 9 H) 3.54 - 3.59 (m, 4 H) 3.64 - 3.67 (m, 4 H) 6.14 (s, 2 H) 7.00 (d, *J*=10.00 Hz, 1 H) 7.25 (dd, *J*=6.00, 1.50 Hz, 1 H) 7.48 (s, 1 H) 7.53 (d, *J*=2.00 Hz, 1 H) 7.67 (dd, *J*=8.50, 2.00 Hz, 1 H) 7.98 (d, *J*=2.00 Hz, 1 H) 8.13 (d, *J*=3.00 Hz, 1 H) 8.22 - 8.23 (m, 1 H) 8.27 - 8.29 (m, 2 H) 9.14 (s, 1 H) 9.38 (d, *J*=2.00 Hz, 1 H) 9.79 (s, 1 H)
**b) *N*-[2'-Piperazin-1-yl-6-(pyrazin-2-ylamino)-2,4'-bipyridin-4-yl]-1,3-benzodioxole-5-Carboxamide**
   Obtained as a solid (12%) from *tert*-butyl 4-[4-[(1,3-benzodioxol-5-ylcarbonyl)amino]-6-(pyrazin-2-ylamino)-2,4'-bipyridin-2'-yl]piperazine-1 -Carboxylate (example 77a) following the experimental procedure as described in example 2b.
   LRMS (m/z): 497 (M+1)⁺.
   1 H NMR (500 MHz, DMSO-*d*ₛ) δ ppm 2.85 (t, *J*=6.00 Hz, 4 H) 3.51 (t, *J*=6.00 Hz, 4 H) 6.14 (s, 2 H) 7.08 (d, *J*=10.00 Hz, 1 H) 7.14 (dd, *J*=6.50, 1.50 Hz, 1 H) 7.33 (s, 1 H) 7.55 (d, *J*=2.00 Hz, 1 H) 7.63 (dd, *J*=10.50, 2.00 Hz, 1 H) 7.88 (d, *J*=2.00 Hz, 1 H) 8.10 (d, *J*=3.00 Hz, 1 H) 8.23 - 8.26 (m, 3 H) 9.20 (d, *J*=2.00 Hz, 1 H) 10.10 (s, 1 H) 10.44 (s, 1 H)

### EXAMPLE 78

### 2-(3-Methoxyphenyl)-N-[2'-piperazin-1-yl-6-(pyrazin-2-ylamino)-2,4'-bipyridin-4-yl]acetamide

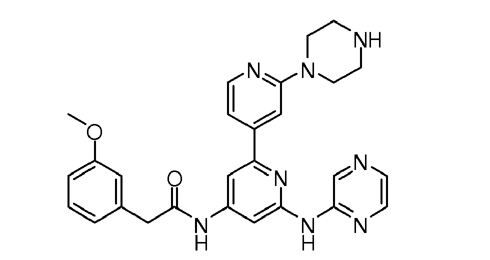

a) ***tert*-Butyl 4-[4-{[(3-methoxyphenyl)acetyl]amino}-6-(pyrazin-2-ylamino)-2,4'-bipyridin-2'-yl]piperazine-1-Carboxylate**
   Obtained (30%) from *tert*-butyl 4-[4-amino-6-(pyrazin-2-ylamino)-2,4'-bipyridin-2'-yl]piperazine-1-Carboxylate (preparation 26) and (3-methoxyphenyl)acetyl chloride following the experimental procedure as described in example 76a, followed by purification by flash chromatography.
   LRMS (m/z): 597 (M+1)⁺.
   1 H NMR (500 MHz, ACETONE) δ ppm 1.47 (s, 9 H) 3.54 - 3.56 (m, 4 H) 3.61 - 3.63 (m, 4 H) 3.76 (s, 2 H) 3.79 (s, 3 H) 6.85 (dd, *J*=10.00, 3.50 Hz, 1 H) 6.96 - 6.99 (m, 2 H) 7.20 (dd, *J*=6.50, 1.50 Hz, 1 H) 7.25 (t, *J*=9.50 Hz, 1 H) 7.42 (s, 1 H) 7.86 (d, *J*=2.00 Hz, 1 H) 8.05 (s, 1 H) 8.11 (d, *J*=3.00 Hz, 1 H) 8.20 - 8.21 (m, 1 H) 8.25 (d, *J*=6.50 Hz, 1 H) 9.10 (s, 1 H) 9.29 (d, *J*=1.50 Hz, 1 H) 9.77 (s, 1 H)
**b) 2-(3-Methoxyphenyl)-*N*-[2'-piperazin-1-yl-6-(pyrazin-2-ylamino)-2,4 '-bipyridin-4-yl]acetamide**
   Obtained as a solid (92%) from *tert*-butyl 4-[4-{[(3-methoxyphenyl)acetyl]amino}-6-(pyrazin-2-ylamino)-2,4'-bipyridin-2'-yl]piperazine-1-Carboxylate (example 78a) following the experimental procedure as described in example 2b.
   LRMS (m/z): 497 (M+1)⁺.
   1 H NMR (400 MHz, DMSO-d*₆*) δ ppm 2.80 (t, *J*=4.88 Hz, 4 H) 3.45 (t, *J*=5.28 Hz, 4 H) 3.66 (s, 2 H) 3.73 (s, 3 H) 6.79 - 6.84 (m, 1 H) 6.87 - 6.93 (m, 2 H) 7.08 (dd, *J*=5.28, 1.17 Hz, 1 H) 7.23 (t, *J*=8.11 Hz, 1 H) 7.27 (s, 1 H) 7.74 (d, *J*=1.56 Hz, 1 H) 7.98 (d, *J*=1.37 Hz, 1 H) 8.08 (d, *J*=2.74 Hz, 1 H) 8.20 (d, *J*=5.28 Hz, 1 H) 8.23 (dd, *J*=2.74, 1.56 Hz, 1 H) 9.13 (d, *J*=1.56 Hz, 1 H) 10.07 (s, 1 H) 10.59 (s, 1 H)

### EXAMPLE 79

### 4-({[2'-Piperazin-1-yl-6-(pyrazin-2-ylamino)-2,4'-bipyridin-4-yl]amino}carbonyl) benzoic acid

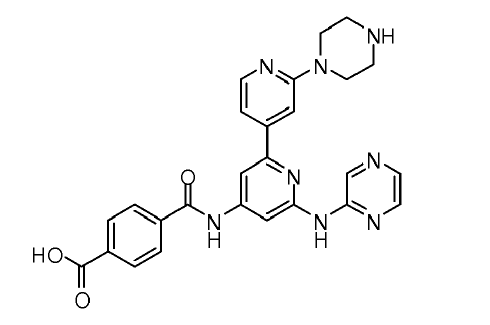

**a) *tert*-Butyl 4-[4-{[4-(methoxycarbonyl)benzoyl]amino}-6-(pyrazin-2-ylamino)-2,4'-bipyridin-2'-yl]piperazine-1-Carboxylate**
   Obtained (29%) from *tert*-butyl 4-[4-amino-6-(pyrazin-2-ylamino)-2,4'-bipyridin-2'-yl]piperazine-1-Carboxylate (preparation 26) and methyl 4-(chlorocarbonyl)benzoate following the experimental procedure as described in example 76a, followed by purification by flash chromatography.
   LRMS (m/z): 611 (M+1)⁺.
**b) 4-({[2'-[4-(*tert*-Butoxycarbonyl)piperazin-1-yl]-6-(pyrazin-2-ylamino)-2,4'-bipyridin-4-yl]amino}carbonyl)benzoic acid**
   Obtained (75%) from *tert*-butyl 4-[4-{[4-(methoxycarbonyl)benzoyl]amino}-6-(pyrazin-2-ylamino)-2,4'-bipyridin-2'-yl]piperazine-1-Carboxylate (example 79a) following the experimental procedure as described in example 74b, followed by purification by reverse phase chromatography (50% CH₃CN in H₂O to 100% CH₃CN).
   LRMS (m/z): 597 (M+1)⁺.
   1 H NMR (500 MHz, ACETONE) δ ppm 1.48 (s, 9 H) 3.56 - 3.58 (m, 4 H) 3.65 - 3.68 (m, 4 H) 7.26 (d, *J*=6.50 Hz, 1 H) 7.50 (s, 1 H) 8.04 (d, *J*=1.00 Hz, 1 H) 8.11 - 8.18 (m, 5 H) 8.24 (s, 1 H) 8.28 (d, *J*=6.50, 1 H) 8.33 (s, 1 H) 9.37 (s, 1 H)
**c) 4-({[2'-Piperazin-1-yl-6-(pyrazin-2-ylamino)-2,4'-bipyridin-4-yl]aminol}arbonyl)benzoic acid**
   Obtained as trifluoroacetate salt (98%) from 4-({[2'-[4-(*tert* butoxycarbonyl)piperazin-1-yl]-6-(pyrazin-2-ylamino)-2,4'-bipyridin-4-yl]amino}carbonyl)benzoic acid (example 79b) following the experimental procedure as described in example 2b, followed by the solvent evaporation.
   LRMS (m/z): 497 (M+1)⁺.
   1 H NMR (400 MHz, DMSO-*d*₆) δ ppm 3.18 - 3.28 (m, 4 H) 3.80 (t, *J*=5.08 Hz, 4 H) 7.27 (dd, *J*=5.28, 0.98 Hz, 1 H) 7.45 (s, 1 H) 7.97 (d, *J*=1.56 Hz, 1 H) 8.09 (br. s., 4 H) 8.11 (d, *J*=2.54 Hz, 1 H) 8.23 (d, *J*=1.37 Hz, 1 H) 8.27 (dd, *J*=2.64, 1.47 Hz, 1 H) 8.30 (d, *J*=5.28 Hz, 1 H) 8.97 (br. s., 2 H) 9.21 (d, *J*=1.37 Hz, 1 H) 10.20 (s, 1 H) 10.88 (s, 1 H)

### EXAMPLE 80

### 4-Fluoro-N-[2'-piperazin-1-yl-6-(pyrazin-2-ylamino)-2,4'-bipyridin-4-yl]benzamide

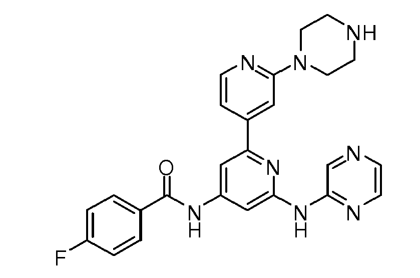

a) ***tert*-Butyl 4-[4-[(4-fluorobenzoyl)amino]-6-(pyrazin-2-ylamino)-2,4'-bipyridin-2'-yl]piperazine-1-Carboxylate**
   Obtained (24%) from *tert*-butyl 4-[4-amino-6-(pyrazin-2-ylamino)-2,4'-bipyridin-2'-yl]piperazine-1-Carboxylate (preparation 26) and 4-fluorobenzoyl chloride following the experimental procedure as described in example 76a, followed by purification by flash chromatography.
   LRMS (m/z): 571 (M+1)⁺.
   1 H NMR (500 MHz, ACETONE) δ ppm 1.46 (s, 9 H) 3.55 - 3.57 (m, 4 H) 3.64 - 3.66 (m, 4 H) 7.25 (dd, *J*=6.50, 1.50 Hz, 1 H) 7.30 - 7.34 (m, 2 H) 7.49 (s, 1 H) 8.00 (d, *J*=2.00 Hz, 1 H) 8.12 - 8.15 (m, 3 H) 8.23 - 8.24 (m, 1 H) 8.27 - 8.28 (m, 2 H) 9.18 (s, 1 H) 9.37 (d, *J*=1.50 Hz, 1 H) 10.02 (s, 1 H)
**b) 4-Fluoro-N-[2'-piperazin-1-yl-6-(pyrazin-2-ylamino)-2,4'-bipyridin-4-**yl]benzamide
   Obtained as a solid (72%) from *tert*-butyl 4-[4-[(4-fluorobenzoyl)amino]-6-(pyrazin-2-ylamino)-2,4'-bipyridin-2'-yl]piperazine-1-Carboxylate (example 80a) following the experimental procedure as described in example 2b.
   LRMS (m/z): 471 (M+1)⁺.
   1 H NMR (400 MHz, DMSO-*d*₆) δ ppm 2.82 (t, *J*=5.08 Hz, 4 H) 3.48 (t, *J*=5.08 Hz, 4 H) 7.13 (dd, *J*=5.18, 1.07 Hz, 1 H) 7.33 (s, 1 H) 7.40 (t, *J*=8.79 Hz, 2 H) 7.89 (d, *J*=1.37Hz, 1 H) 8.05 - 8.12 (m, 3 H) 8.22 - 8.27 (m, 3 H) 9.19 (d, *J*=1.37 Hz, 1 H) 10.13 (s, 1 H) 10.65 (s, 1 H)

### EXAMPLE 81

### 4-Methoxy-N-[2'-piperazin-1-yl-6-(pyrazin-2-ylamino)-2,4'-bipyridi n-4-yl]benzamide

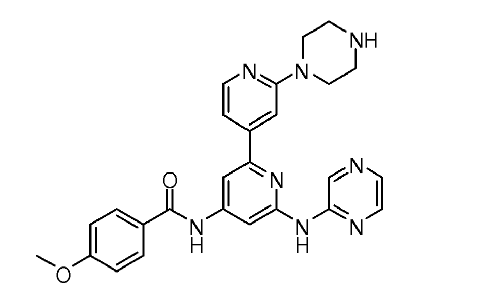

a) ***tert*-Butyl 4-[4-[(4-methoxybenzoyl)amino]-6-(pyrazin-2-ylamino)-2,4'-bipyridin-2'-yl]piperazine-1-Carboxylate**
   Obtained (16%) from *tert*-butyl 4-[4-amino-6-(pyrazin-2-ylamino)-2,4'-bipyridin-2'-yl]piperazine-1-Carboxylate (preparation 26) and 4-methoxybenzoyl chloride following the experimental procedure as described in example 76a, followed by purification by flash chromatography.
   LRMS (m/z): 583 (M+1)⁺.
   1 H NMR (500 MHz, ACETONE) δ ppm 1.48 (s, 9 H) 3.55 - 3.57 (m, 4 H) 3.63 - 3.66 (m, 4 H) 3.90 (s, 3 H) 7.07 (dd, *J*=8.50, 2.50 Hz, 2 H) 7.25 (dd, *J*=6.50, 1.50 Hz, 1 H) 7.48 (s, 1 H) 8.00 (d, *J*=2.00 Hz, 1 H) 8.04 (dd, *J*=8.50, 2.50 Hz, 2 H) 8.12 (d, *J*=1.50 Hz, 1 H) 8.22 - 8.23 (m, 1 H) 8.26 - 8.27 (m, 2 H) 9.15 (s, 1 H) 9.38 (d, *J*=1.00 Hz, 1 H) 9.83 (s, 1 H)
**b) 4-Methoxy-N-[2'-piperazin-1-yl-6-(pyrazin-2-ylamino)-2,4'-bipyridin-4-yl]benzamide**
   Obtained as a solid (59%) from *tert*-butyl 4-[4-[(4-methoxybenzoyl)amino]-6-(pyrazin-2-ylamino)-2,4'-bipyridin-2'-yl]piperazine-1-Carboxylate (example 81 a) following the experimental procedure as described in example 2b.
   LRMS (m/z): 483 (M+1)⁺.
   1 H NMR (400 MHz, DMSO-*d*₆) δ ppm 2.82 (t, *J*=4.88 Hz, 4 H) 3.48 (t, *J*=5.08 Hz, 4 H) 3.84 (s, 3 H) 7.05 - 7.11 (m, 2 H) 7.14 (dd, *J*=5.28, 0.98 Hz, 1 H) 7.33 (s, 1 H) 7.90 (d, *J*=1.56 Hz, 1 H) 7.98 - 8.04 (m, 2 H) 8.10 (d, *J*=2.74 Hz, 1 H) 8.21 - 8.28 (m, 3 H) 9.20 (d, *J*=1.37 Hz, 1 H) 10.10 (s, 1 H) 10.47 (s, 1 H)

### EXAMPLE 82

### 3-Phenyl-N-[2'-piperazin-1-yl-6-(pyrazin-2-ylamino)-2,4'-bipyridin-4-yl]propanamide

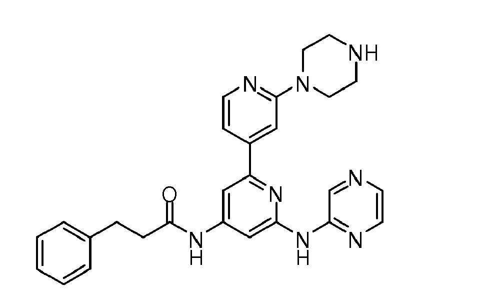

a) ***tert*-Butyl 4-[4-[(3-phenylpropanoyl)amino]-6-(pyrazin-2-ylamino)-2,4'-bipyridin-**2'-yl]piperazine-1-Carboxylate
   Obtained (21%) from *tert*-butyl 4-[4-amino-6-(pyrazin-2-ylamino)-2,4'-bipyridin-2'-yl]piperazine-1-Carboxylate (preparation 26) and 3-phenylpropanoyl chloride following the experimental procedure as described in example 76a, followed by purification by flash chromatography.
   LRMS (m/z): 581 (M+1)⁺.
   1 H NMR (500 MHz, METHANOL-*d*₄) δ ppm 1.49 (s, 9 H) 2.72 (t, *J*=9.50 Hz, 2 H) 3.01 (t, *J*=9.50 Hz, 2 H) 3.58 (s, 8 H) 7.17 - 7.27 (m, 8 H) 7.44 (s, 1 H) 7.70 (d, *J*=1.50 Hz, 1 H) 7.93 (d, *J*=2.00 Hz, 1 H) 8.03 (d, *J*=3.50 Hz, 1 H) 8.17 (d, *J*=7.00 Hz, 1 H) 8.23 - 8.24 (m, 1 H) 9.13 (d, *J*=1.50 Hz, 1 H)
**b) 3-Phenyl-*N*-[2'-piperazin-1-yl-6-(pyrazi-n-2-ylamino)-2,4'-bipyridin-4-yl]propanamide**
   Obtained as a solid (91%) from *tert-*butyl 4-[4-[(3-phenylpropanoyl)amino]-6-(pyrazin-2-ylamino)-2,4'-bipyridin-2'-yl]piperazine-1-Carboxylate (example 82a) following the experimental procedure as described in example 2b.
   LRMS (m/z): 481 (M+1)⁺.
   ¹H NMR (400 MHz, DMSO-*d₆*) δ ppm 2.70 (t, *J*=7.72 Hz, 2 H) 2.81 (t, 4 H) 2.92 (t, J=7.72 Hz, 2 H) 3.46 (t, 4 H) 7.08 (dd, J=5.28, 1.17 Hz, 1 H) 7.20 - 7.36 (m, 5 H) 7.72 (d, *J=1.56* Hz, 1 H) 7.97 (d, *J=1.56* Hz, 1 H) 8.09 (d, *J=2.73* Hz, 1 H) 8.23 (dd, *J=*9.87*,* 4.59 Hz, 2 H) 9.15 (d, *J*=1.56 Hz, 1 H) 10.07 (s, 1 H) 10.38 (s, 1 H)

### EXAMPLE 83

### 1,3-Dimethyl-N-[2'-piperazin-1-yl-6-(pyrazin-2-ylamino)-2,4'-bipyridin-4-yl]-1H-pyrazole-5-Carboxamide

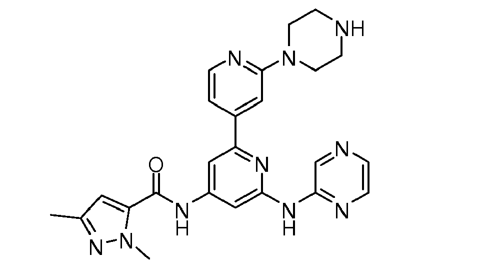

**a) *tert*-Butyl 4-[4-{[(1,3-dimethyl-1*H*-pyrazol-5-yl)carbonyl]amino}-6-(pyrazin-2-ylamino)-2,4'-bipyridin-2'-yl]piperazine-1-Carboxylate**
   Obtained (90%) from *tert-*butyl 4-[4-amino-6-(pyrazin-2-ylamino)-2,4'-bipyridin-2'-yl]piperazine-1-Carboxylate (preparation 26) and 1,3-dimethyl-1 *H*-pyrazole-5-Carbonyl chloride following the experimental procedure as described in example 76a, followed by purification by flash chromatography.
   LRMS (m/z): 571 (M+1)⁺.
   1 H NMR (500 MHz, ACETONE) δ ppm 1.48 (s, 9 H) 2.21 (s, 3 H) 3.55 ― 3.57 (m, 4 H) 3.64 ― 3.66 (m, 4 H) 4.10 (s, 3 H) 6.81 (s, 1 H) 7.23 (dd, *J*=6.50, 1.50 Hz, 1 H) 7.47 (s, 1 H) 7.92 (d, *J*=2.00 Hz, 1 H) 8.13 (d, *J*=3.50 Hz, 1 H) 8.21 ― 8.28 (m, 3 H) 9.18 (s, 1 H) 9.38 (s, 1 H) 9.74 (s, 1 H)
**b) 1,3-Dimethyl-*N*-[2'-piperazin-1-yl-6-(pyrazin-2-ylamino)-2,4'-bipyridin-4-yl]-1*H-*pyrazole-5-Carboxamide**
   Obtained as a solid (64%) from *tert-*butyl 4-[4-{[(1,3-dimethyl-1 H-pyrazol-5-yl)carbonyl]amino}-6-(pyrazin-2-ylamino)-2,4'-bipyridin-2'-yl]piperazine-1-Carboxylate (example 83a) following the experimental procedure as described in example 2b.
   LRMS (m/z): 471 (M+1)⁺.
   ¹H NMR (400 MHz, METHANOL-*d*₄) δ ppm 2.27 (s, 3 H) 2.99 (t, 3 H) 3.58 (t, 3 H) 4.09 (s, 3 H) 6.79 (s, 1 H) 7.23 (d, *J*=4.49 Hz, 1 H) 7.44 (s, 1 H) 7.79 (s, 1 H) 8.03 (d, *J*=2.74 Hz, 1 H) 8.08 (s, 1 H) 8.17 (d, *J*=5.47 Hz, 1 H) 8.24 (dd, *J*=2.64, 1.47 Hz, 1 H) 9.15 (d, *J*=1.37 Hz, 1 H)

### EXAMPLE 84

### 2-(2,4-Difluorophenyl)-N-[2'-piperazin-1-yl-6-(pyrazin-2-ylamino)-2,4'-bipyridin-4-yl]acetamide

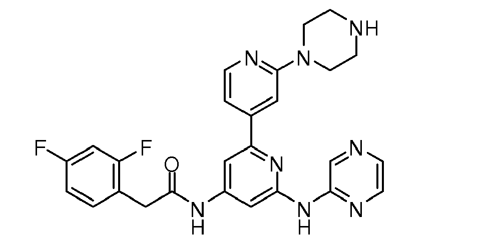

**a) *tert-*Butyl 4-[4-{[(2,4-difluorophenyl)acetyl]amino}-6-(pyrazin-2-ylamino)-2,4'-bipyridin-2'-yl]piperazine-1-Carboxylate**
   Obtained (55%) from *tert-*butyl 4-[4-amino-6-(pyrazin-2-ylamino)-2,4'-bipyridin-2'-yl]piperazine-1-Carboxylate (preparation 26) and (2,4-difluorophenyl)acetic acid following the experimental procedure as described in example 73a, followed by purification by flash chromatography (4:1 hexanes/ethyl acetate to 100% ethyl acetate).
   LRMS (m/z): 603 (M+1)⁺.
   1 H NMR (500 MHz, METHANOL-*d*₄) δ ppm 1.50 (s, 9 H) 3.59 (s, 8 H) 3.82 (s, 2 H) 6.95 ― 7.01 (m, 2 H) 7.26 (dd, *J=*6.50, 1.50 Hz, 1 H) 7.38 ― 7.46 (m, 2 H) 7.76 (d, *J*=2.00 Hz, 1 H) 7.99 (d, *J*=2.00 Hz, 1 H) 8.05 (d, *J*=3.50 Hz, 1 H) 8.20 (d, *J*=6.50 Hz, 1 H) 8.25 ― 8.26 (m, 1 H) 9.13 (d, *J*=2.00 Hz, 1 H)
**b) 2-(2,4-Difluorophenyl)-*N*-[2'-piperazin-1-yl-6-(pyrazin-2-ylamino)-2,4'-bipyridin-4-yl]acetamide**
   Obtained as a solid (80%) from *tert-*butyl 4-[4-1{[(2,4-difluorophenyl)acetyl]amino"}-6-(pyrazin-2-ylamino)-2,4'-bipyridin-2'-yl]piperazine-1-Carboxylate (example 84a) following the experimental procedure as described in example 2b.
   LRMS (m/z): 503 (M+1)⁺.
   ¹H NMR (400 MHz, DMSO-*d*₆) δ ppm 2.82 (t, 4 H) 3.47 (t, 4 H) 3.80 (s, 2 H) 7.03 - 7.08 (m, 1 H) 7.09 (dd, *J=*5.08*,* 1.17 Hz, 2 H) 7.22 (td, *J*=9.77, 2.54 Hz, 1 H) 7.28 (s, 1 H) 7.45 (td, *J=*8.65*,* 6.74 Hz, 1 H) 7.76 (d, *J=*1.56 Hz, 1 H) 7.98 (d, *J*=1.37 Hz, 1 H) 8.09 (d, *J*=2.74 Hz, 1 H) 8.21 (d, *J*=5.28 Hz, 1 H) 8.24 (dd, *J*=2.74, 1.56 Hz, 1 H) 9.13 (d, *J*=1.37 Hz, 1 H) 10.09 (s, 1 H) 10.68 (s, 1 H)

### EXAMPLE 85

### N-[2'-Piperazin-1-yl-6-(pyrazin-2-ylamino)-2,4'-bipyridin-4-yl]nicotinamide

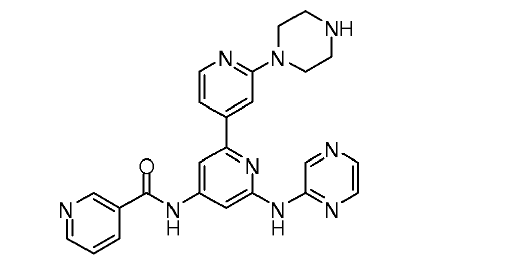

**a) *tert-*Butyl 4-{6-(pyrazi n-2-ylamino)-4-[(pyridin-3-ylcarbonyl)amino]-2,4'-bipyridin-2'-yl}piperazine-1-Carboxylate**
   Obtained (30%) from *tert-*butyl 4-[4-amino-6-(pyrazin-2-ylamino)-2,4'-bipyridin-2'-yl]piperazine-1-Carboxylate (preparation 26) and nicotinic acid following the experimental procedure as described in example 73a, followed by purification by flash chromatography.
   LRMS (m/z): 554 (M+1)⁺.
   1 H NMR (400 MHz, DMSO-*d*₆) δ ppm 1.44 (s, 9 H) 3.46 ― 3.49 (m, 4 H) 3.58 ― 3.60 (m, 4 H) 7.20 (dd, *J=*6.50*,* 1.00 Hz, 1 H) 7.39 (s, 1 H) 7.60 ― 7.63 (m, 1 H) 7.92 (d, *J*=2.00 Hz, 1 H) 8.13 (d, *J*=3.50 Hz, 1 H) 8.26 ― 8.29 (m, 3 H) 8.34 ― 8.37 (m, 1 H) 8.81 (dd, *J=*6.00*,* 2.00 Hz, 1 H) 9.16 (d, *J*=2.00 Hz, 1 H) 9.20 (s, 1 H) 10.19 (s, 1 H) 10.88 (br. s, 1H)
**b) *N*-[2'-Piperazin-1-yl-6-(pyrazi n-2-ylamino)-2,4'-bipyridin-4-yl]nicotinamide**
   Obtained as a solid (62%) from *tert-*butyl 4-{6-(pyrazin-2-ylamino)-4-[(pyridin-3-ylcarbonyl)amino]-2,4'-bipyridin-2'-yl}piperazine-1-Carboxylate (example 85a) following the experimental procedure as described in example 2b.
   LRMS (m/z): 454 (M+1)⁺.
   ¹H NMR (400 MHz, DMSO-*d*₆) δ ppm 2.83 (t, 4 H) 3.49 (t, 4 H) 7.14 (dd, *J*=5.28, 1.17 Hz, 1 H) 7.33 (s, 1 H) 7.60 (ddd, *J*=8.01, 4.88, 0.78 Hz, 1 H) 7.90 (d, *J=*1.56 Hz, 1 H) 8.11 (d, *J*=2.54 Hz, 1 H) 8.21 - 8.29 (m, 3 H) 8.30 - 8.36 (m, 1 H) 8.79 (dd, *J*=4.79, 1.66 Hz, 1 H) 9.14 (d, *J*=2.15 Hz, 1 H) 9.19 (d, *J*=1.56 Hz, 1 H) 10.16 (s, 1 H) 10.84 (br. s., 1 H)

### EXAMPLE 86

### N-[2'-Piperazin-1-yl-6-(pyrazin-2-ylamino)-2,4'-bipyridin-4-yl]quinoline-6-Carboxamide

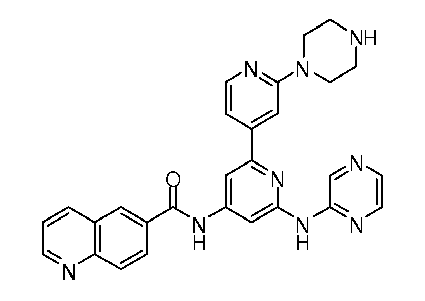

**a) *tert*-Butyl 4-{6-(pyrazin-2-ylamino)-4-[(quinolin-6-ylcarbonyl)amino]-2,4'-bipyridin-2'-yl}piperazine-1-Carboxylate**
   Obtained (26%) from *tert-*butyl 4-[4-amino-6-(pyrazin-2-ylamino)-2,4'-bipyridin-2'-yl]piperazine-1-Carboxylate (preparation 26) and quinoline-6-Carboxylic acid following the experimental procedure as described in example 73a, followed by purification by flash chromatography.
   LRMS (m/z): 604 (M+1)⁺.
   1 H NMR (400 MHz, DMSO-*d*₆) δ ppm 1.44 (s, 9 H) 3.46 - 3.49 (m, 4 H) 3.59 - 3.61 (m, 4 H) 7.22 (dd, *J*=6.50, 1.00 Hz, 1 H) 7.40 (s, 1 H) 7.66 ― 7.69 (m, 1 H) 7.97 (s, 1 H) 8.13 ― 8.19 (m, 2 H) 8.28 ― 8.33 (m, 4 H) 8.58 (d, *J*=10.00 Hz, 1 H) 8.72 (s, 1 H) 9.04 ― 9.05 (m, 1 H) 9.22 (s, 1 H) 10.20 (s, 1 H) 10.96 (br. s, 1 H)
**b) *N*-[2'-Piperazin-1-yl-6-(pyrazin-2-ylamino)-2,4'-bipyridin-4-yl]quinoline-6-Carboxamide**
   Obtained as a solid (63%) from *tert-*butyl 4-{6-(pyrazin-2-ylamino)-4-[(quinolin-6-ylcarbonyl)amino]-2,4'-bipyridin-2'-yl}piperazine-1-Carboxylate (example 86a) following the experimental procedure as described in example 2b.
   LRMS (m/z): 504 (M+1)⁺.
   ¹H NMR (400 MHz, DMSO-*d*₆) δ ppm 2.84 (t, 4 H) 3.50 (t, 4 H) 7.16 (dd, *J*=5.28*,* 0.98 Hz, 1 H) 7.35 (s, 1 H) 7.65 (dd, *J*=8.30, 4.20 Hz, 1 H) 7.95 (d, *J*=1.56 Hz, 1 H) 8.11 (d, *J*=2.54 Hz, 1 H) 8.16 (d, *J*=8.79 Hz, 1 H) 8.25 (d, *J*=5.28 Hz, 1 H) 8.26 - 8.34 (m, 3 H) 8.56 (dd, *J*=8.60, 0.98 Hz, 1 H) 8.70 (d, *J*=1.95 Hz, 1 H) 9.03 (dd, *J*=4.20, 1.66 Hz, 1 H) 9.20 (d, *J*=1.56 Hz, 1 H) 10.17 (s, 1 H) 10.91 (s, 1 H)

### EXAMPLE 87

### Methyl 3-({[2'-piperazin-1-yl-6-(pyrazin-2-ylamino)-2,4'-bipyridin-4-yl]amino} carbonyl)benzoate

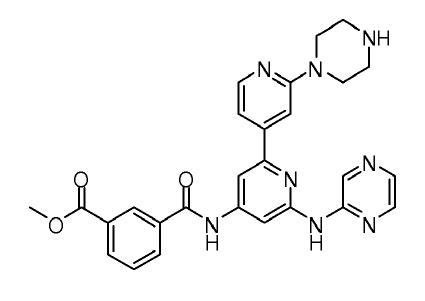

**a) *tert*-Butyl 4-[4-{[3-(methoxycarbonyl)benzoyl]amino}-6-(pyrazin-2-ylamino)-2,4'-bipyridin-2'-yl]piperazine-1-Carboxylate**
   Obtained (49%) from *tert-*butyl 4-[4-amino-6-(pyrazin-2-ylamino)-2,4'-bipyridin-2'-yl]piperazine-1-Carboxylate (preparation 26) and 3-(methoxycarbonyl)benzoic acid following the experimental procedure as described in example 73a, followed by purification by flash chromatography.
   LRMS(m/z):611 (M+1)⁺.
   1 H NMR (400 MHz, DMSO-*d*₆) δ ppm 1.44 (s, 9 H) 3.46 - 3.49 (m, 4 H) 3.56 - 3.60 (m, 4 H) 3.93 (s, 3 H) 7.20 (dd, *J*=5.20, 1.20 Hz, 1 H) 7.39 (s, 1 H) 7.74 (t, *J*=7.60 Hz, 1 H) 7.93 (d, *J*=1.20 Hz, 1 H) 8.13 (d, *J*=2.80 Hz, 1 H) 8.20 ― 8.22 (m, 1 H) 8.27 ― 8.29 (m, 4 H) 8.58 (t, *J*=1.60 Hz, 1 H) 9.21 (s, 1 H) 10.18 (s, 1 H) 10.88 (br. s, 1 H)
**b) Methyl 3-({[2'-piperazin-1-yl-6-(pyrazin-2-ylamino)-2,4'-bipyridin-4-yl]amino} carbonyl)benzoate**
   Obtained as a solid (72%) from *tert*-butyl 4-[4-{[3-(methoxycarbonyl)benzoyl]amino}-6-(pyrazin-2-ylamino)-2,4'-bipyridin-2'-yl]piperazine-1-Carboxylate (example 87a) following the experimental procedure as described in example 2b.
   LRMS (m/z): 511 (M+1)⁺
   1 H NMR (400 MHz, DMSO-*d*₆) δ ppm 2.85 (t, *J*=4.88 Hz, 4 H) 3.50 (t, *J*=5.08 Hz, 4 H) 3.91 (s, 3 H) 7.15 (dd, *J*=5.18, 1.07 Hz, 1 H) 7.34 (s, 1 H) 7.72 (t, *J*=7.82 Hz, 1 H) 7.91 (d, *J*=1.37 Hz, 1 H) 8.11 (d, *J*=2.74 Hz, 1 H) 8.19 (dt, *J*=7.82, 1.37 Hz, 1 H) 8.24 (d, *J*=5.28 Hz, 1 H) 8.25 - 8.29 (m, 3 H) 8.56 (t, *J*=1.56 Hz, 1 H) 9.20 (d, *J*=1.56 Hz, 1 H) 10.15 (s, 1 H) 10.86 (s, 1 H)

### EXAMPLE 88

### 3-({[2'-Piperazin-1-yl-6-(pyrazin-2-ylamino)-2,4'-bipyridin-4-yl]amino}carbonyl) benzoic acid

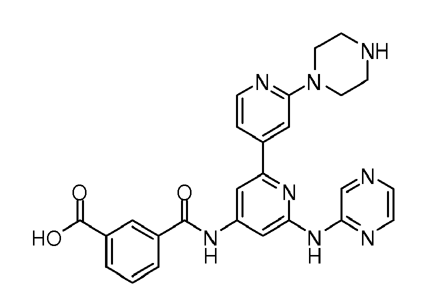

**a) 3-({[2'-[4-(tert-Butoxycarbonyl)piperazin-1-yl]-6-(pyrazin-2-ylamino)-2,4'-bipyridin-4-yl]aminolcarbonyl)benzoic acid**
   Obtained (62%) from *tert-*butyl 4-[4-{[3-(methoxycarbonyl)benzoyl]aminol-6-(pyrazin-2-ylamino)-2,4'-bipyridin-2'-yl]piperazine-1-Carboxylate (example 87a) following the experimental procedure as described in example 74b, followed by purification by reverse phase chromatography.
   LRMS (m/z): 597 (M+1)⁺.
   1 H NMR (400 MHz, DMSO-*d*₆) δ ppm 1.43 (s, 9 H) 3.47 - 3.49 (m, 4 H) 3.58 - 3.60 (m, 4 H) 7.21 (dd, *J*=5.60, 1.20 Hz, 1 H) 7.39 (s, 1 H) 7.68 (t, *J*=7.60 Hz, 1 H) 7.94 (d, *J*=1.60 Hz, 1 H) 8.13 (d, *J*=2.80 Hz, 1 H) 8.17 ― 8.22 (m, 3 H) 8.27 ― 8.30 (m, 3 H) 8.58 (s, 1 H) 9.22 (s, 1 H) 10.17 (s, 1 H) 10.87 (s, 1 H)
**b) 3-({[2'-Piperazin-1-yl-6-(pyrazin-2-ylamino)-2,4'-bipyridin-4-yl]amino}carbonyl) benzoic acid**
   Obtained as solid (86%) from 3-({[2'-[4-(*tert*-butoxycarbonyl)piperazin-1-yl]-6-(pyrazin-2-ylamino)-2,4'-bipyridin-4-yl]aminolcarbonyl)benzoic acid (example 88a) following the experimental procedure as described in example 2b.
   LRMS (m/z): 497 (M+1)⁺.
   ¹H NMR (400 MHz, DMSO-*d*₆) δ ppm 3.30 (t, 4 H) 4.04 (t, 4 H) 7.45 (d, *J*=6.25 Hz, 1 H) 7.63 - 7.82 (m, 2 H) 8.24 - 8.44 (m, 3 H) 8.60 (s, 1 H) 9.16 (s, 1 H) 9.63 (br. s., 2 H) 10.50(br.s., 1 H) 11.08(s, 1 H)

### EXAMPLE 89

### N-[2'-Piperazin-1-yl-6-(pyrazin-2-ylamino)-2,4'-bipyridin-4-yl]-2-pyridin-3-ylacetamide

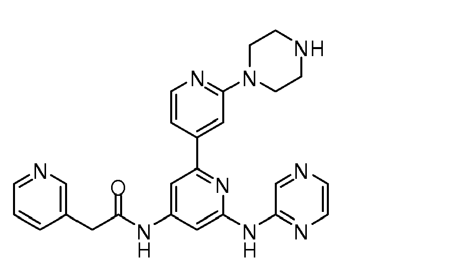

**a) *tert-*Butyl 4-{6-(pyrazin-2-ylamino)-4-[(pyridin-3-ylacetyl)amino]-2,4'-bipyridin-2'-yl}piperazine-1-Carboxylate**
   Obtained (43%) from *tert-*butyl 4-[4-amino-6-(pyrazin-2-ylamino)-2,4'-bipyridin-2'-yl]piperazine-1-Carboxylate (preparation 26) and pyridin-3-ylacetic acid following the experimental procedure as described in example 73a, followed by purification by reverse phase chromatography.
   LRMS (m/z): 568 (M+1)⁺.
   1 H NMR (400 MHz, DMSO-*d*₆) δ ppm 1.43 (s, 9 H) 3.45 - 3.47 (m, 4 H) 3.55 - 3.58 (m, 4 H) 7.14 (dd, *J*=5.20, 1.20 Hz, 1 H) 7.34 (s, 1 H) 8.36 ― 8.39 (m, 1 H) 7.75 ― 7.78 (m, 2 H) 8.00 (d, *J*=1.60 Hz, 1 H) 8.11 (d, *J*=2.40 Hz, 1 H) 8.24 ― 8.26 (m, 2 H) 8.49 (dd, *J*=4.40, 1.20 Hz, 1 H) 8.54 (t, *J*=1.60 Hz, 1 H) 9.14 (s, 1 H) 10.12 (s, 1 H) 10.73 (br. s, 1 H)
**b) *N*-[2'-Piperazin-1-yl-6-(pyrazin-2-ylamino)-2,4'-bipyridin-4-yl]-2-pyridin-3-ylacetamide**
   Obtained as a solid (79%) from *tert-*butyl 4-{6-(pyrazin-2-ylamino)-4-[(pyridin-3-ylacetyl)amino]-2,4'-bipyridin-2'-yl}piperazine-1-Carboxylate (example 89a) following the experimental procedure as described in example 2b.
   LRMS (m/z): 468 (M+1)⁺.
   ¹H NMR (400 MHz, DMSO-*d*₆) □ ppm 2.81 (t, 4 H) 3.46 (d, *J*=5.08 Hz, 4 H) 3.78 (s, 2 H) 7.08 (dd, *J*=5.18, 1.07 Hz, 2 H) 7.28 (s, 1 H) 7.36 (ddd, *J*=7.82, 4.79, 0.68 Hz, 1 H) 7.70 - 7.78 (m, 2 H) 7.99 (d, *J*=1.56 Hz, 1 H) 8.09 (d, *J*=2.74 Hz, 1 H) 8.20 (d, *J*=5.28 Hz, 1 H) 8.23 (dd, *J*=2.64, 1.47 Hz, 1 H) 8.46 (dd, *J*=4.79, 1.66 Hz, 1 H) 8.53 (d, *J*=2.15 Hz, 1 H) 9.13 (d, *J*=1.37 Hz, 1 H) 10.09 (s, 1 H) 10.69 (s, 1 H)

### EXAMPLE 90

### Methyl 4-(2-oxo-2-{[2'-piperazin-1-yl-6-(pyrazin-2-ylamino)-2,4'-bipyridin-4-yl]amino}ethyl)benzoate

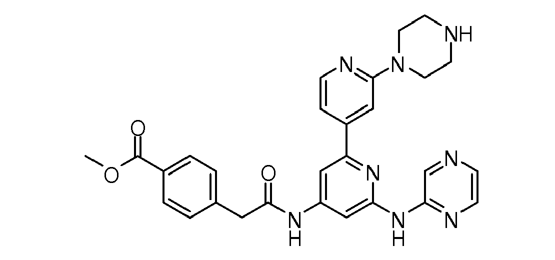

**a) *tert-*Butyl 4-[4-({[4-(methoxycarbonyl)phenyl]acetyl}amino)-6-(pyrazin-2-ylamino)-2,4'-bipyridin-2'-yl]piperazine-1-Carboxylate**
   Obtained (32%) from *tert-*butyl 4-[4-amino-6-(pyrazin-2-ylamino)-2,4'-bipyridin-2'-yl]piperazine-1-Carboxylate (preparation 26) and 2-(4-(methoxycarbonyl)phenyl)acetic acid following the experimental procedure as described in example 73a.
   LRMS (m/z): 625 (M+1)⁺.
   1 H NMR (400 MHz, CD₃OD) δ ppm 1.48 (s, 9 H) 3.54 (s, 8 H) 3.79 (s, 2 H) 3.88 (s, 3 H) 7.17 (dd, *J*=5.20, 1.20 Hz, 1 H) 7.36 (s, 1 H) 7.45 (s, 1 H) 7.47 (s, 1 H) 7.68 (d, *J*=1.60 Hz, 1 H) 7.92 (d, *J*=1.60 Hz, 1 H) 7.96 (d, *J*=1.60 Hz, 1 H) 7.98 (d, *J*=1.60 Hz, 1 H) 8.00 (d, *J*=2.80 Hz, 1 H) 8.13 (d, *J*=5.20 Hz, 1 H) 8.20 (m, 1 H) 9.08 (d, *J*=1.60 Hz, 1 H)
**b) Methyl 4-(2-oxo-2-{[2'-piperazin-1-yl-6-(pyrazin-2-ylamino)-2,4'-bipyridin-4-yl]amino}ethyl)benzoate**
   Obtained (99%) from *tert*-butyl 4-[4-({[4-(methoxycarbonyl)phenyl]acetyl}amino)-6-(pyrazin-2-ylamino)-2,4'-bipyridin-2'-yl]piperazine-1-Carboxylate (example 90a) following the experimental procedure as described in example 2b.
   LRMS (m/z): 525 (M+1)⁺.
   1 H NMR (400 MHz, DMSO-*d*₆) δ ppm 2.80 (t, *J*=5.08 Hz, 4 H) 3.45 (t, *J*=5.28 Hz, 4 H) 3.80 - 3.82 (m, 5 H) 7.06 (dd, *J*=5.28, 1.17 Hz, 1 H) 7.26 (s, 1 H) 7.46 (d, *J*=8.60 Hz, 2 H) 7.73 (d, *J*=1.56 Hz, 1 H) 7.91 (d, *J*=8.60 Hz, 2 H) 7.97 (d, *J*=1.56 Hz, 1 H) 8.07 (d, *J*=2.74 Hz, 1 H) 8.19 (d, *J*=5.28 Hz, 1 H) 8.21 (dd, *J*=2.64, 1.47 Hz, 1 H) 9.11 (d, *J*=1.37 Hz, 1 H) 10.07 (s, 1 H) 10.67 (s, 1 H)

### EXAMPLE 91

### 4-(2-Oxo-2-1{[2'-piperazin-1-yl-6-(pyrazin-2-ylamino)-2,4'-bipyridin-4-yl]amino}ethyl)benzoic acid

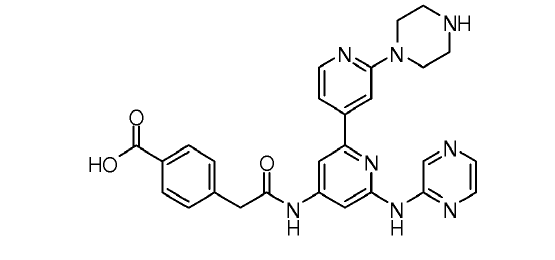

**a) 4-(2-{[2'-[4-(*tert*-Butoxycarbonyl)piperazin-1-yl]-6-(pyrazin-2-ylamino)-2,4 '-bipyridin-4-yl]amino}-2-oxoethyl)benzoic acid**
   Obtained (50%) from *tert-*butyl 4-[4-({[4-(methoxycarbonyl)phenyl]acetyl}amino)-6-(pyrazin-2-ylamino)-2,4'-bipyridin-2'-yl]piperazine-1-Carboxylate (example 90a) following the experimental procedure as described in example 74b.
   LRMS (m/z): 611 (M+1)⁺
   1 H NMR (400 MHz, CD₃OD) δ ppm 1.48 (s, 9 H) 3.54 (s, 8 H) 3.79 (s, 2 H) 7.17 (dd, *J*=5.20, 1.20 Hz, 1 H) 7.36 (s, 1 H) 7.45 (s, 1 H) 7.47 (s, 1 H) 7.68 (d, *J*=1.60Hz, 1 H) 7.92 (d, *J*=1.60 Hz, 1 H) 7.96 (d, *J*=1.60 Hz, 1 H) 7.98 (d, *J*=1.60 Hz, 1 H) 8.00 (d, *J*=2.80 Hz, 1 H) 8.13 (d, *J*=5.20 Hz, 1 H) 8.20 (m, 1 H) 9.08 (d, *J*=1.60 Hz, 1 H) 10.80 (s, 1 H)
**b) 4-(2-Oxo-2-{[2'-piperazin-1-yl-6-(pyrazin-2-ylamino)-2,4'-bipyridin-4-yl]amino}ethyl)benzoic acid**
   Obtained as a trifluoroacetate salt (100%) from 4-(2-{[2'-[4-(*tert-*butoxycarbonyl)piperazin-1-yl]-6-(pyrazin-2-ylamino)-2,4'-bipyridin-4-yl]amino}-2-oxoethyl)benzoic acid (example 91 b) following the experimental procedure as described in example 2b, without the treatment with sodium carbonate.
   LRMS (m/z): 511 (M+1)⁺
   1 H NMR (400 MHz, DMSO-*d*₆) δ ppm 3.25 (br. s., 4 H) 3.76 - 3.88 (m, 6 H) 7.26 (d, *J*=5.28 Hz, 1 H) 7.43 (s, 1 H) 7.48 (d, *J*=8.40 Hz, 2 H) 7.86 (s, 1 H) 7.90 - 7.98 (m, 3 H) 8.12 (d, *J*=2.74 Hz, 1 H) 8.25 - 8.34 (m, 2 H) 8.86 (br. s., 2 H) 9.17 (s, 1 H) 10.17 (s, 1 H) 10.79 (s, 1 H)

### EXAMPLE 92

### N-[2'-Piperazin-1-yl-6-(pyrazin-2-ylamino)-2,4'-bipyridin-4-yl]piperidine-4-Carboxamide

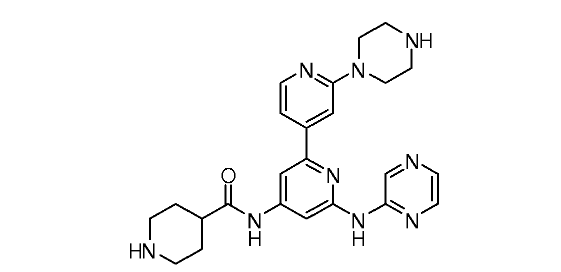

**a) *tert-*Butyl 4-[4-({[1-(tert-butoxycarbonyl)piperidin-4-yl]carbonyl}amino)-6-(pyrazin-2-ylamino)-2,4'-bipyridin-2'-yl]piperazine-1-Carboxylate**
   Obtained as a solid (31%) from *tert-*butyl 4-[4-amino-6-(pyrazin-2-ylamino)-2,4'-bipyridin-2'-yl]piperazine-1-Carboxylate (preparation 26) and *1-*(tert-butoxycarbonyl)piperidine-4-Carboxylic acid following the experimental procedure as described in example 73a.
   LRMS (m/z): 660 (M+1)⁺
   1 H NMR (400 MHz, DMSO-*d*₆) δ ppm 1.40 (s, 9 H) 1.42 (s, 9H) 1.75-1.85 (m, 2 H) 2.46-2.50 (m, 4 H) 3.15 (m, 1 H) 3.40-3-50 (m, 4 H) 3.50-3.60 (m, 4 H) 3.95-4-05 (m, 2 H) 7.12 (dd, *J*=5.60, 1.20 Hz, 1 H) 7.32 (s, 1 H) 7.77 (d, *J*=1.60 Hz, 1 H) 7.98 (d, *J*=1.20 Hz, 1 H) 8.09 (d, *J*=2.40 Hz, 1 H) 8.23-8-25 (m, 2 H) 9.14 (d, *J*=1.20 Hz, 1 H) 10.08 (s, 1 H) 10.39 (s, 1 H)
**b) N-[2'-Piperazin-1-yl-6-(pyrazin-2-ylamino)-2,4'-bipyridin-4-yl]piperidine-4-Carboxamide**
   2,2,2-Trifluoroacetic acid (1 mL) was added to a stirred solution *tert-*butyl 4*-*[4-({[1-(*tert-*butoxycarbonyl)piperidin-4-yl]carbonyl}amino)-6-(pyrazin-2-ylamino)-2,4'-bipyridin-2'-yl]piperazine-1-Carboxylate (example 92a, 46 mg, 0.069 mmol) in dichloromethane (4 mL) and the mixture was stirred at room temperature. After 15 minutes, the solvent was evaporated and methanol was added. Dowex® resin was added, it was stirred at room temperature for 1 hour and the resin was filtered, washed with methanol, dichloromethane and again with methanol. 7N ammonia in methanol was added and the solution was stirred at room temperature for 30 minutes. The resulting solid was filtered and dried to give the title compound (0.025 g, 79%) as a solid.
   LRMS (m/z): 460 (M+1)⁺.
   1 H NMR (400 MHz, DMSO-*d*₆) δ ppm 1.44 - 1.58 (m, 2 H) 1.65 - 1.79 (m, 2 H) 2.36 - 2.55 (m, 5 H) 2.74 - 2.82 (m, 4 H) 2.94 - 3.05 (m, 2 H) 3.36 - 3.48 (m, 4 H) 7.06 (dd, *J*=5.28, 1.37 Hz, 1 H) 7.26 (s, 1 H) 7.74 (d, *J*=1.37 Hz, 1 H) 7.97 (d, *J*=1.37 Hz, 1 H) 8.07 (d, *J*=2.74 Hz, 1 H) 8.19 (d, *J*=5.47 Hz, 1 H) 8.22 (dd, *J*=2.64, 1.47 Hz, 1 H) 9.13 (d, *J*=1.37 Hz, 1 H) 10.03 (s, 1 H) 10.28 (s, 1 H)

### EXAMPLE 93

### 1-Acetyl-N-[2'-piperazin-1-yl-6-(pyrazin-2-ylamino)-2,4'-bipyridin-4-yl]piperidine-4-Carboxamide

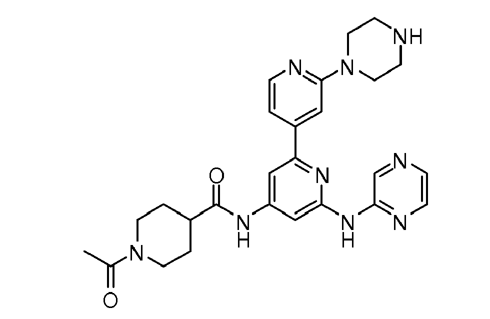

**a) *tert-*Butyl 4-[4-{[(1-acetylpiperidin-4-yl)carbonyl]amino}-6-(pyrazin-2-ylamino)-2,4'-bipyridin-2'-yl]piperazine-1-Carboxylate**
   Obtained as a solid (34%) from *tert-*butyl 4-[4-amino-6-(pyrazin-2-ylamino)-2,4'-bipyridin-2'-yl]piperazine-1-Carboxylate (preparation 26) and 1-acetylpiperidine-4-Carboxylic acid following the experimental procedure as described in example 73a.
   LRMS (m/z): 602 (M+1)⁺.
   1 H NMR (400 MHz, DMSO-*d*ₛ) δ ppm 1.41 (s, 9 H) 1.56-1.60 (m, 1 H) 1.81-1.88 (m, 2 H) 2.00 (s, 3 H) 2.55-2.67 (m, 2 H) 3.03-3.09 (m, 2 H) 3.43-3.46 (m, 4 H) 3.54-3.57 (m, 4 H) 3.87 (d, *J*=13.60 Hz, 2 H) 4.40 (d, *J*=13.20 Hz, 2 H) 7.20 (dd, *J*=5.20, 1.20 , 1 H) 7.33 (s, 1 H) 7.77 (d, *J*=1.20 Hz, 1 H) 8.09 (d, *J*=2.80 Hz, 1 H) 8.23-8.24 (m. 2H) 9.15 (d, *J*=1.20 Hz, 1 H) 10.08 (s, 1 H) 10.45 (s, 1 H)
**b) 1-Acetyl-*N*-[2'-piperazin-1-yl-6-(pyrazin-2-ylamino)-2,4'-bipyridin-4-yl]piperidine-4-Carboxamide**
   Obtained as a solid (81%) from *tert-*butyl 4-[4-{[(1-acetylpiperidin-4-yl)carbonyl]amino}-6-(pyrazin-2-ylamino)-2,4'-bipyridin-2'-yl]piperazine-1 -Carboxylate (example 93a) following the experimental procedure as described in example 92b.
   LRMS (m/z): 502 (M+1)⁺.
   1 H NMR (400 MHz, DMSO-*d*₆) δ ppm 1.02 (t, *J*=11.43 Hz, 2 H) 1.19 (s, 3 H) 1.63 - 1.69 (m, 4 H) 1.96 - 2.08 (m, 4 H) 2.25 (t, *J*=11.72 Hz, 1 H) 2.67 (d, *J*=5.08 Hz, 4 H) 2.98 - 3.14 (m, 1 H) 3.50 - 3.68 (m, 1 H) 6.28 (dd, *J*=5.18, 1.27 Hz, 1 H) 6.48 (s, 1 H) 6.95 (d, *J*=1.56 Hz, 1 H) 7.18 (d, *J*=1.56 Hz, 1 H) 7.28 (d, *J*=2.74 Hz, 1 H) 7.40 (d, *J*=5.47 Hz, 1 H) 7.43 (dd, *J*=2.64, 1.47 Hz, 1 H) 8.34 (d, *J*=1.56 Hz, 1 H) 9.26 (s, 1 H) 9.57 (s, 1 H)

### EXAMPLE 94

### 4-Oxo-4-{[2'-piperazin-1-yl-6-(pyrazin-2-ylamino)-2,4'-bipyridin-2,4'-ylamino) butanoic acid

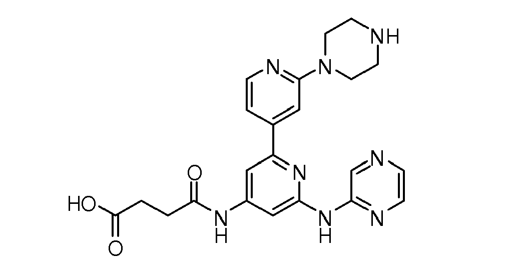

**a) 4-{[2'-[4-(*tert*-Butoxycarbonyl)piperazin-1-yl]-6-(pyrazin-2-ylamino)-2,4'-bipyridin-4-yl]aminol-4-oxobutanoic acid**
   A mixture of *tert-*butyl 4-[4-amino-6-(pyrazin-2-ylamino)-2,4'-bipyridin-2'-yl]piperazine-1-Carboxylate (preparation 26, 50 mg, 0.11 mmol) and succinic anhydride (37 mg, 0.37 mmol) in acetone (1 mL) was heated at 60ºC for 6 days. The mixture was cooled to room temperature, the solid was filtered, washed with ethyl acetate and dried to yield the title compound (40 mg, 66%) as a solid.
   LRMS (m/z): 549 (M+1)⁺.
   1 H NMR (400 MHz, DMSO-*d*₆) δ ppm 1.49 (s, 9 H) 2.70-2.72 (m, 4 H) 3.58 (s, 8 H) 7.25 (d, *J=5.60* Hz, 1 H) 7.43 (s, 1 H) 7.71 (d, *J*=1.20 Hz, 1 H) 8.03 (d, *J*=1.60 Hz, 1 H) 8.17 (d, *J*=5.20 Hz, 1 H) 8.24-8.25 (m, 1 H) 9.14 (d, *J*=1.20 Hz, 1 H) 10.51 (s, 1 H)
**b) 4-Oxo-4-{[2'-piperazin-1-yl-6-(pyrazin-2-ylamino)-2,4'-bipyridin-4-yl]amino} butanoic acid**
   Obtained (12%) from 4-{[2'-[4-(*tert-*butoxycarbonyl)piperazin-1-yl]-6-(pyrazin-2-ylamino)-2,4'-bipyridin-4-yl]aminol-4-oxobutanoic acid (example 94a) following the experimental procedure as described in example 2b.
   LRMS (m/z): 449 (M+1)⁺.
   1 H NMR (400 MHz, DMSO-*d*₆) δ ppm 2.52 - 2.57 (m, 2 H) 2.60 - 2.69 (m, 2 H) 2.84 - 2.95 (m, 4 H) 3.48 - 3.62 (m, 6 H) 7.12 (dd, *J*=5.18, 1.27 Hz, 1 H) 7.32 (s, 1 H) 7.76 (d, *J*=1.56 Hz, 1 H) 7.97 (d, *J*=1.56 Hz, 1 H) 8.10 (d, *J*=2.54 Hz, 1 H) 8.19 - 8.33 (m, 2 H) 9.17 (d, *J*=1.37 Hz, 1 H) 10.08 (s, 1 H) 10.51 (s, 1 H)

### EXAMPLE 95

### N-[2'-Piperazin-1-yl-6-(pyrazin-2-ylamino)-2,4'-bipyridin-4-yl]pyrimidine-5-Carboxamide

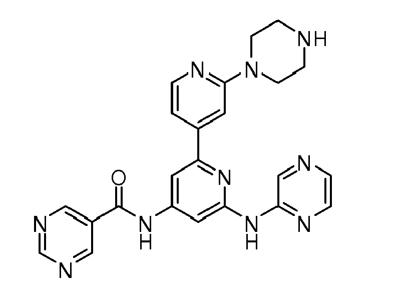

**a) *tert-*Butyl 4-{6-(pyrazin-2-ylamino)-4-[(pyrimidino-5-ylcarbonyl)amino]-2,4'-bipyridin-2'-yl}piperazine-1-Carboxylate**
   Obtained as a solid (28%) from *tert-*butyl 4-[4-amino-6-(pyrazin-2-ylamino)-2,4'-bipyridin-2'-yl]piperazine-1-Carboxylate (preparation 26) and pyrimidine-5-Carboxylic acid following the experimental procedure as described in example 73a.
   LRMS (m/z): 555 (M+1)⁺.
   1 H NMR (400 MHz, DMSO-*d*₆) δ ppm 1.41 (s, 9 H) 3.40-3.45 (m, 4 H) 3.55-3.60 (m, 4 H) 7.17 (d, *J*=5.60 Hz, 1 H) 7.37 (s, 1 H) 7.90 (s, 1 H) 8.11 (d, *J*=2.40 Hz, 1 H) 8.22 (d, *J*=1.20 Hz, 1 H) 8.26 (dd, *J*=2.80, 1.60 Hz, 2 H) 9.17 (d, *J*=1.20 Hz, 1 H) 9.30 (s, 2 H) 9.38 (s, 1 H) 10.21 (s, 1 H) 11.05 (b.r. s, 1 H)
**b) *N*-[2'-Piperazin-1-yl-6-(pyrazin-2-ylamino)-2,4'-bipyridin-4-yl]pyrimidine-5-Carboxamide**
   Obtained as a solid (40%) from *tert-*butyl 4-{6-(pyrazin-2-ylamino)-4-[(pyrimidin-5-ylcarbonyl)amino]-2,4'-bipyridin-2'-yl}piperazine-1-Carboxylate (example 95a) following the experimental procedure as described in example 92b.
   LRMS (m/z): 455 (M+1)⁺.
   1 H NMR (400 MHz, DMSO-*d*₆ □ ppm 2.89 (d, *J*=4.88 Hz, 4 H) 3.53 (d, *J*=5.08 Hz, 4 H) 7.14 (dd, *J*=5.28, 1.17 Hz, 1 H) 7.33 (s, 1 H) 7.88 (d, *J*=1.56 Hz, 1 H) 8.09 (d, *J*=2.54 Hz, 1 H) 8.18 - 8.29 (m, 4 H) 9.15 (d, *J*=1.37 Hz, 1 H) 9.28 (s, 2 H) 9.36 (s, 1 H) 10.18 (s, 1 H) 11.03 (br. s., 1 H)

### EXAMPLE 96

### 5-({[2'-Piperazin-1-yl-6-(pyrazin-2-ylamino)-2,4'-bipyridin-4-yl]amino}carbonyl) pyridine-2-Carboxylic acid

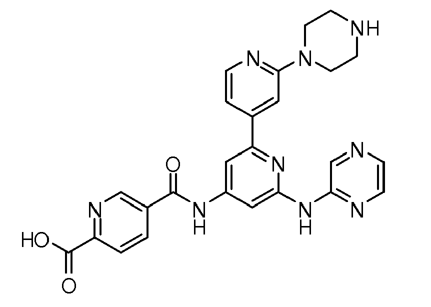

**a) *tert*-Butyl 4-[4-({[6-(Methoxycarbonyl)pyridin-3-yl]carbonyl}amino)-6-(pyrazin-2-ylamino)-2,4'-bipyridin-2'-yl]piperazine-1-Carboxylate**
   Obtained (56%) from *tert-*butyl 4-[4-amino-6-(pyrazin-2-ylamino)-2,4'-bipyridin-2'-yl]piperazine-1-Carboxylate (preparation 26) and 6-(methoxycarbonyl)nicotinic acid following the experimental procedure as described in example 73a.
   LRMS (m/z): 612 (M+1)⁺.
   1 H NMR (400 MHz, DMSO-*d*₆) δ ppm 1.42 (s, 9 H) 3.44-3.47 (m, 4 H) 3.56-3.58 (m, 4 H) 3.92 (s, 3 H) 7.18 (dd, *J=*5.20, 0.80 Hz, 1 H) 7.37 (s, 1 H) 7.92 (d, *J*=1.60 Hz, 1 H) 8.11 (d, *J*=2.80 Hz, 1 H) 8.21 (d, *J*=8.20 Hz, 1 H) 8.25-8.27 (m, 3 H) 8.52 (dd, *J*=8.40, 2.20 Hz, 1 H) 9.18 (d, *J*=1.60 Hz, 1 H) 9.23 (d, *J*=0.80 Hz, 1 H) 10.19 (s, 1 H)
**b) 5-({[2'-[4-(*tert*-Butoxycarbonyl)piperazin-1-yl]-6-(pyrazin-2-ylamino)-2,4'-bipyridin-4-yl]amino}carbonyl)pyridine-2-Carboxylic acid**
   Obtained (33%) from *tert-*butyl 4-[4-({[6-(methoxycarbonyl)pyridin-3-yl]carbonyl}amino)-6-(pyrazin-2-ylamino)-2,4'-bipyridin-2'-yl]piperazine-1-Carboxylate (example 96a) following the experimental procedure as described in example 74b.
   LRMS (m/z): 598 (M+1)⁺.
   1 H NMR (400 MHz, DMSO-*d*₆) δ ppm 1.42 (s, 9 H) 3.45-3.47 (m, 4 H) 3.56-3.57 (m, 4 H) 7.18 (dd, *J*=5.20, 0.80 Hz, 1 H) 7.37 (s, 1 H) 7.90 (d, *J*=1.20 Hz, 1 H) 8.11 (d, *J*=2.40 Hz, 1 H) 8.19 (d, *J*=4.00 Hz, 1 H) 8.24-8.27 (m, 2 H) 8.49 (dd, *J*=8.00, 2.40 Hz, 1 H) 9.19 (dd, *J*=12.00, 1.40 Hz, 1 H) 10.19 (s, 1 H) 10.99 (s, 1 H)
**c) 5-({[2'-Piperazin-1-yl-6-(pyrazin-2-ylamino)-2,4'-bipyridin-4-yl]amino}carbonyl) pyridine-2-Carboxylic acid**
   Obtained as a solid (100%) from 5-({[2'-[4-(*tert*-butoxycarbonyl)piperazin-1-yl]-6-(pyrazin-2-ylamino)-2,4'-bipyridin-4-yl]amino}carbonyl)pyridine-2-Carboxylic acid (example 96b) following the expermental procedure as described in example 2b.
   LRMS (m/z): 498 (M+1)⁺.
   1 H NMR (400 MHz, DMSO-*d*₆) □ ppm 3.14 (br. s., 4 H) 3.73 (br. s., 4 H) 7.26 (d, *J*=5.28 Hz, 1 H) 7.38 (br. s., 1 H) 7.96 (s, 1 H) 8.04 - 8.16 (m, 2 H) 8.21 - 8.33 (m, 3 H) 8.40 (d, *J*=8.60 Hz, 1 H) 9.15 (br. s., 1 H) 9.21 (s, 1 H) 10.18 (s, 1 H)

### EXAMPLE 97

### 5-({[2'-Piperazin-1-yl-6-(pyrazin-2-ylamino)-2,4'-bipyridin-4-yl]amino}carbonyl) nicotinic acid

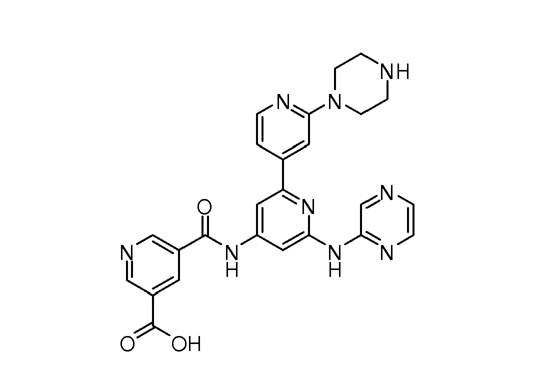

**a) *tert*-Butyl 4-[4-({[5-(Methoxycarbonyl)pyridin-3-yl]carbonyl}amino)-6-(pyrazin-2-ylamino)-2,4'-bipyridin-2'-yl]piperazine-1-Carboxylate**
   Obtained (51%) from *tert-*butyl 4-[4-amino-6-(pyrazin-2-ylamino)-2,4'-bipyridin-2'-yl]piperazine-1-Carboxylate (preparation 26) and 5-(methoxycarbonyl)nicotinic acid following the experimental procedure as described in example 76a.
   LRMS (m/z): 612 (M+1)⁺.
   1 H NMR (400 MHz, DMSO-*d*₆) δ ppm 1.42 (s, 9 H) 3.44-3.47 (m, 4 H) 3.56-3.59 (m, 4 H) 3.94 (s, 3 H) 7.18 (dd, *J*=5.00, 1.00 Hz, 1 H) 7.37 (s, 1 H) 8.12 (d, *J*=2.80 Hz, 1 H) 8.25-8.27 (m, 3 H) 8.82 (t, *J*=2.00 Hz, 1 H) 9.18 (d, *J*=1.60 Hz, 1 H) 9.26 (d, *J*=1.60 Hz, 1 H) 9.35 (d, *J*=2.40 Hz, 1 H) 10.19 (s, 1 H) 11.02 (s, 1 H)
**b) 5-({[2'-[4-(*tert*-Butoxycarbonyl)piperazin-1-yl)]-6-(pyrazin-2-ylamino)-2,4'-bipyridin-4-yl]aminolcarbonyl)nicotinic acid**
   Obtained (73%) from *tert-*butyl 4-[4-({[5-(methoxycarbonyl)pyridin-3-yl]carbonyl}amino)-6-(pyrazin-2-ylamino)-2,4'-bipyridin-2'-yl]piperazine-1-Carboxylate (example 97a) following the experimental procedure as described in example 74b.
   LRMS (m/z): 598 (M+1)⁺.
   1 H NMR (400 MHz, DMSO-*d*₆) δ ppm 1.42 (s, 9 H) 3.45-3.50 (m, 4 H) 3.58-3.62 (m, 4 H) 7.18 (dd, *J*=5.20, 0.80 Hz, 1 H) 7.37 (s, 1 H) 7.91 (d, *J*=1.60 Hz, 1 H) 8.11 (d, *J*=2.40 Hz, 1 H) 8.26-8.27 (m, 3 H) 8.82 (t, *J=* 2.20 Hz, 1 H) 9.18 (d, *J*=1.60 Hz, 1 H) 9.24 (d, *J*=2.00 Hz, 1 H) 9.32 (d, *J*=2.40 Hz, 1 H) 10.19 (s, 1 H) 11.00 (s, 1 H)
**c) 5-({[2'-Piperazin-1-yl-6-(pyrazin-2-ylamino)-2,4'-bipyridin-4-yl]amino}carbonyl) nicotinic acid**
   Obtained as a solid (100%) from 5-({[2'-[4-(*tert-*Butoxycarbonyl)piperazin-1-yl]-6-(pyrazin-2-ylamino)-2,4'-bipyridin-4-yl]amino}carbonyl)nicotinic acid (example 97b) following the experimentla procedure as described in example 2b.
   LRMS (m/z): 498 (M+1)⁺.
   1 H NMR (400 MHz, DMSO-*d*₆) δ ppm 3.26 (br. s., 4 H) 3.82 (d, *J*=5.08 Hz, 4 H) 7.30 (dd, *J*=5.37, 1.07 Hz, 1 H) 7.47 (s, 1 H) 7.98 (d, *J*=1.37 Hz, 1 H) 8.14 (d, *J*=2.54 Hz, 1 H) 8.22 (d, *J*=1.56 Hz, 1 H) 8.30 (dd, *J*=2.54, 1.56 Hz, 1 H) 8.33 (d, *J*=5.28 Hz, 1 H) 8.84 (t, *J*=2.15 Hz, 2 H) 8.86 (br. s., 1 H) 9.22 (d, *J*=1.56 Hz, 1 H) 9.27 (d, *J*=1.95 Hz, 1 H) 9.35 (d, *J*=2.15 Hz, 1 H) 10.24 (s, 1 H) 11.08 (s, 1 H)

### EXAMPLE 98

### (2S)-2-Benzyl-4-oxo-4-{[2'-piperazin-1-yl-6-(pyrazin-2-ylamino)-2,4'-bipyridin-4-yl]amino}butanoic acid

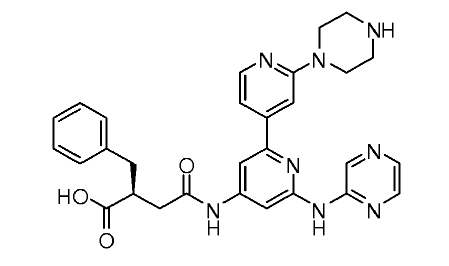

**a) *tert*-Butyl 4-[4-{[(3S)-3-benzyl-4-methoxy-4-oxobutanoyl]amino}-6-(pyrazin-2-ylamino)-2,4'-bipyridin-2'-yl]piperazine-1-Carboxylate**
   Obtained (36%) from *tert-*butyl 4-[4-amino-6-(pyrazin-2-ylamino)-2,4'-bipyridin-2'-yl]piperazine-1-Carboxylate (preparation 26) and (3S)-3-benzyl-4-methoxy-4-oxobutanoic acid following the experimental procedure as described in example 73a, followed by purification by reverse phase chromatography
   LRMS (m/z): 653 (M+1)⁺.
**b) (2S)-2-Benzyl-4-{[2'-[4-(*tert*-butoxycarbonyl)piperazin-1-yl]-6-(pyrazin-2-ylamino)-2,4'-bipyridin-4-yl]amino}-4-oxobutanoic acid**
   Obtained (89%) from *tert*-butyl 4-[4-{[(3S)-3-benzyl-4-methoxy-4-oxobutanoyl]amino}-6-(pyrazin-2-ylamino)-2,4'-bipyridin-2'-yl]piperazine-1-Carboxylate (example 98a) following the experimental procedure as described in example 74b. The product was used in next reaction without purification.
   LRMS (m/z): 639 (M+1)⁺.
**c) (2S)-2-Benzyl-4-oxo-4-{[2'-piperazin-1-yl-6-(pyrazin-2-ylamino)-2,4'-bipyridin-4-yl]amino}butanoic acid**
   Obtained as salt (13%) from *tert-*butyl 4-[4-({[trans-4-(methoxycarbonyl) cyclohexyl]carbonyl} amino)-6-(pyrazin-2-ylamino)-2,4'-bipyridin-2'-yl]piperazine-1-Carboxylate (example 98b) following the experimental procedure described in example 2b followed by purification by reverse phase chromatography.
   LRMS (m/z): 539 (M+1)⁺.
   1 H NMR (600 MHz, DMSO-*d*₆) δ ppm 2.41 (dd, *J*=16.14, 4.40 Hz, 1 H) 2.58 - 2.71 (m, 1 H) 2.80 (dd, *J*=13.50, 7.63 Hz, 1 H) 2.84 - 2.90 (m, 4 H) 2.93 - 2.99 (m, 1 H) 3.03 - 3.09 (m, 1 H) 3.48 - 3.54 (m, 4 H) 7.08 (dd, *J*=5.28, 1.17 Hz, 1 H) 7.21 (d, *J*=7.04 Hz, 3 H) 7.26 - 7.31 (m, 3 H) 7.70 (d, *J*=1.17 Hz, 1 H) 7.91 (d, *J*=1.76 Hz, 1 H) 8.08 (d, *J*=2.35 Hz, 1 H) 8.21 (d, *J*=5.28 Hz, 1 H) 8.23 (dd, *J*=2.64, 1.47 Hz, 1 H) 9.16 (s, 1 H) 10.05 (s, 1 H) 10.52 (br.s., 1H)

### EXAMPLE 99

### trans-4-({[2'-Piperazin-1-yl-6-(pyrazin-2-ylamino)-2,4'-bipyridin-4-yl]amino} carbonyl)cyclohexanecarboxylic acid

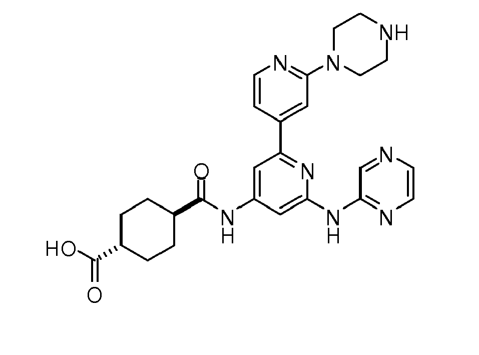

**a) *tert*-Butyl 4-[4-({[*trans*-4-(methoxycarbonyl)cyclohexyl]carbonyl]amino)-6-(pyrazin-2-ylamino)-2,4'-bipyridin-2'-yl]piperazine-1-Carboxylate**
   Obtained (44%) from *tert-*butyl 4-[4-amino-6-(pyrazin-2-ylamino)-2,4'-bipyridin-2'-yl]piperazine-1-Carboxylate (preparation 26) and *trans*-4-(methoxycarbonyl)cyclohexanecarboxylic acid following the experimantal procedure as described in example 73a followed by purification by reverse phase chromatography (0% CHₐOH in H₂O to 100% CHₐOH in H₂O).
   LRMS (m/z): 617 (M+1)⁺.
   1 H NMR (400 MHz, DMSO-*d*₆) □ ppm 1.34-1.38 (m, 2 H) 1.42 (s, 9 H) 1.45-1.49 (m, 2 H) 1.89-2.00 (m, 4 H) 2.33-2.40 (m, 2 H) 3.44-3.46 (m, 4 H) 3.54-3.57 (m, 4 H) 3.59 (s, 3 H) 7.12 (dd, *J*=5.20, 0.80 Hz, 1 H) 7.32 (s, 1 H) 7.76 (d, *J*=1.60 Hz, 1 H) 7.99 (d, *J*=1.20 Hz, 1 H) 8.09 (d, *J*=2.80 Hz, 1 H) 8.23-8.25 (m, 2 H) 9.14 (d, *J*=1.20 Hz, 1 H) 10.07 (s, 1 H) 10.32 (s, 1 H)
**b) *trans*-4-({[2'-Piperazin-1-yl-6-(pyrazin-2-ylamino)-2,4'-bipyridin-4-yl]amino} carbonyl)cyclohexanecarboxylic acid**
   Obtained as ammonium salt (43%) from *tert-*butyl 4-[4-({[trans-4-(methoxycarbonyl) cyclohexyl]carbonyl} amino)-6-(pyrazin-2-ylamino)-2,4'-bipyridin-2'-yl]piperazine-1-Carboxylate (example 99a) following the experimental procedure described in example 74b followed by the experimental procedure described in example 92b.
   LRMS (m/z): 503 (M+1)⁺.
   ¹H NMR (400 MHz, DMSO-*d*₆) □ ppm 1.21 - 1.54 (m, 4 H) 1.80 - 2.00 (m, 4 H) 2.01 - 2.16 (m, *J*=11.58, 11.58, 3.13, 2.83 Hz, 1 H) 2.27 - 2.42 (m, *J*=11.53, 11.53, 3.13, 2.93 Hz, 1 H) 2.80 (t, 4 H) 3.46 (t, 4 H) 7.09 (dd, *J*=5.28, 0.98 Hz, 1 H) 7.29 (s, 1 H) 7.78 (d, *J*=1.56 Hz, 1 H) 8.02 (d, *J*=1.37 Hz, 1 H) 8.08 (d, *J*=2.54 Hz, 1 H) 8.20 (d, *J*=5.08 Hz, 1 H) 8.24 (dd, *J*=2.64, 1.47 Hz, 1 H) 9.15 (d, *J*=1.37 Hz, 1 H) 10.04 (s, 1 H) 10.42 (s, 1 H)

### EXAMPLE 100

### (3S)-3-Benzyl-4-oxo-4-{[2'-piperazin-1-yl-6-(pyrazin-2-ylamino)-2,4'-bipyridin-4-yl]amino}butanoic acid

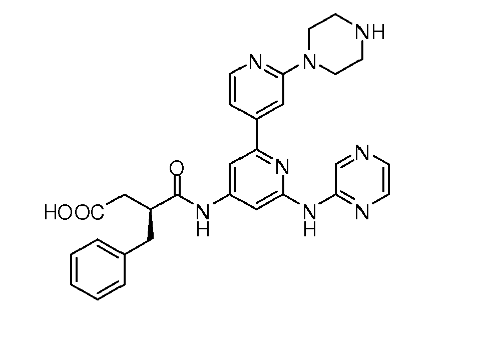

Obtained as byproduct (7%) of example 98c.
LRMS (m/z): 539 (M+1)⁺.
1 H NMR (600 MHz, DMSO-*d*₆) δ ppm 2.17 (dd, *J*=17.02, 4.11 Hz, 1 H) 2.52 - 2.62 (m, 1 H) 2.65 (dd, *J*=13.50, 8.22 Hz, 1 H) 2.79 - 2.88 (m, 4 H) 2.97 (dd, *J*=13.21, 6.16 Hz, 1 H) 3.07 - 3.18 (m, 1 H) 3.44 - 3.55 (m, 4 H) 7.09 (d, *J*=5.28 Hz, 1 H) 7.18 (t, *J*=7.04 Hz, 1 H) 7.22 - 7.25 (m, 2 H) 7.26 - 7.31 (m, 2 H) 7.73 (s, 1 H) 7.98 (br. s., 1 H) 8.09 (d, *J*=2.35 Hz, 1 H) 8.22 (d, *J*=5.28 Hz, 1 H) 8.25 (d, *J*=1.76 Hz, 1 H) 8.35 (br. s., 2 H) 9.16 (s, 1 H) 10.10 (s, 1 H) 10.95 (br. s., 1 H) 10.94 (br. s., 1 H)

### EXAMPLE 101

### N-[2'-Piperazin-1-yl-6-(pyrazin-2-ylamino)-2,4'-bipyridin-4-yl]acetamide

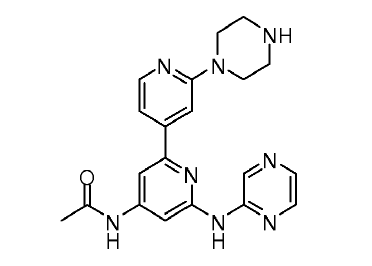

**a) *tert*-Butyl 4-[4-(acetylamino)-6-(pyrazin-2-ylamino)-2,4'-bipyridin-2'-yl]piperazine-1-Carboxylate**
   Obained (17%) from *tert-*butyl 4-[4-amino-6-(pyrazin-2-ylamino)-2,4'-bipyridin-2'-yl]piperazine-1-Carboxylate (preparation 26) and acetyl chloride following the experimental procedure as described in example 76a followed by purification by flash chromatography (hexanes/ethyl acetate 50:50 to 100% ethyl acetate).
   LRMS (m/z): 491 (M+1)⁺.
   1 H NMR (400 MHz, DMSO-*d*₆) □ ppm 1.42 (s, 9 H) 2.10 (s, 3 H) 3.45-3.46 (m, 4 H) 3.54-3.56 (m, 4 H) 7.11 (d, *J*=5.20 Hz, 1 H) 7.32 (s, 1 H) 7.71 (s, 1 H) 7.95 (s, 1 H) 8.09-8.10 (m, 1 H) 8.23-8.25 (m, 1 H) 9.17 (s, 1 H) 10.08 (s, 1 H) 10.39 (s, 1 H)
**b) *N*-[2'-Piperazin-1-yl-6-(pyrazin-2-ylamino)-2,4'-bipyridin-4-yl]acetamide**

   Obtained as a formiate salt (7%) from *tert-*butyl 4-[4-(acetylamino)-6-(pyrazin-2-ylamino)-2,4'-bipyridin-2'-yl]piperazine-1-Carboxylate (example 101 a) following the experimental procedure as described in example 92b following by purification by reverse phase chromatography chromatography (0% CH₃OH in H₂O to 100% CH₃OH in H₂O with ammonium formiate).
   LRMS (m/z): 391 (M+1)⁺.
   ¹H NMR (400 MHz, DMSO-*d*₆) □ ppm 2.10 (s, 3 H) 2.93 (t, 4 H) 3.56 (t, 4 H) 7.11 (dd, *J*=5.18, 1.07 Hz, 1 H) 7.32 (s, 1 H) 7.72 (d, *J*=1.37 Hz, 1 H) 7.94 (d, *J*=1.37 Hz, 1 H) 8.09 (d, *J*=2.74 Hz, 1 H) 8.21 - 8.26 (m, 3 H) 9.17 (d, *J*=1.37 Hz, 1 H) 10.08 (s, 1 H) 10.42 (S, 1 H)

### EXAMPLE 102

### N-(2'-Piperazin-1-yl-6-{[4-(trifluoromethyl)pyridin-2-yl]amino}-2,4'-bipyridin-4-yl)nicotinamide

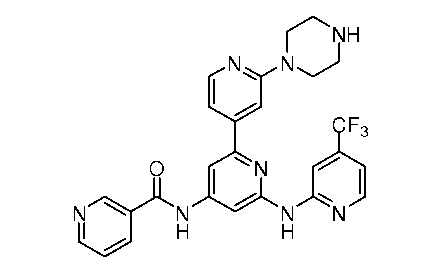

**a) *tert-*Butyl 4-(4-[(pyridin-3-ylcarbonyl)amino]-6-{[4-(trifluoromethyl) pyridin-2-yl]amino}-2,4'-bipyridin-2'-yl)piperazine-1-Carboxylate**
   Obtained (55%) from *tert*-butyl 4-(4-amino-6-1[4-(trifluoromethyl)pyridin-2-yl]aminol-2,4'-bipyridin-2'-yl)piperazine-1-Carboxylate (preparation 27c) and nicotinic acid following the experimental procedure as described in example 73a stirring at room temperature for 24h followed by purification by reverse phase chromatography (0% CHₐOH in H₂O to 100% CHₐOH in H₂O).
   LRMS (m/z): 621 (M+1)⁺.
   1 H NMR (400 MHz, DMSO-*d*₆) δ ppm 1.44 (s, 9 H) 3.48-3-55 (m, 4 H) 3.56-3.75 (m, 4 H) 7.17 (d, *J*=5.20 Hz, 1 H) 7.23 (d, *J*=5.20 Hz, 1 H) 7.36 (s, 1 H) 7.61 (dd, *J*=7.60, 5.20 Hz, 1 H) 7.91 (d, *J*=1.20 Hz, 1 H) 8.09 (s, 1 H) 8.27 (d, *J*=5.20 Hz, 1 H) 8.35 (dt, *J*=8.00, 2.00 Hz, 1 H) 8.52 (d, *J*=5.20 Hz, 1 H) 8.55 (s, 1 H) 8.81 (dd, *J*=4.80, 1.60 Hz, 1 H) 9.16 (d, *J*=2.00 Hz, 1 H) 10.37 (s, 1 H) 10.89 (s, 1 H)
**b) N-(2'-Piperazin-1-yl-6-{[4-(trifluoromethyl)pyridin-2-yl]amino}-2,4'-bipyridin-4-yl)nicotinamide**
   Obtained (50%) from *tert-*butyl 4-(4-[(pyridin-3-ylcarbonyl)amino]-6-{[4-(trifluoromethyl)pyridin-2-yl]amino}-2,4'-bipyridin-2'-yl)piperazine-1-Carboxylate (example 102a) following the experimental procedure as described in example 92b.
   LRMS (m/z): 521 (M+1)⁺.
   ¹H NMR (400 MHz, DMSO-*d*₆) □ ppm 2.84 (t, 4 H) 3.48 (t, 4 H) 7.12 (dd, *J*=5.28, 0.78 Hz, 1 H) 7.21 (dd, *J*=5.18, 0.88 Hz, 1 H) 7.31 (s, 1 H) 7.60 (dd, *J*=7.91, 4.79 Hz, 1 H) 7.88 (d, *J*=1.56 Hz, 1 H) 8.08 (d, *J*=1.37 Hz, 1 H) 8.23 (d, *J*=5.28 Hz, 1 H) 8.33 (dt, *J*=7.96, 1.98 Hz, 1 H) 8.50 (d, *J*=5.08 Hz, 1 H) 8.54 (s, 1 H) 8.79 (dd, *J*=4.69, 1.56 Hz, 1 H) 9.14 (d, *J*=2.15 Hz, 1 H) 10.35 (s, 1 H) 10.84 (s, 1 H)

### EXAMPLE 103

### 4-{[(2'-Piperazin-1-y)-6-{[4-(trifluoromethyl)pyridin-2-yl]amino}-2,4'-bipyridin-4-yl)amino]carbonyl}benzoic acid

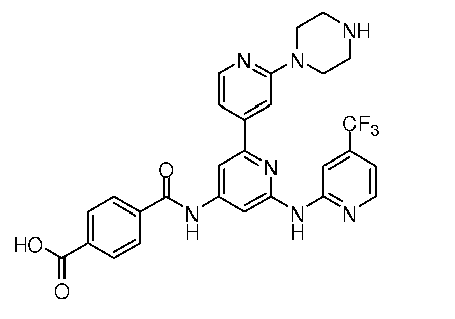

**a) Methyl 4-[(2,6-dibromopyridin-4-yl)amino]benzoate**
   Obtained (69%) from 2,6-dibromopyridin-4-amine and methyl 4-(chlorocarbonyl)benzoate following the experimental procedure as described in example 76a stirring at room temperature for 48h followed by purification by flash chromatography (100% hexanes to 100% ethyl acetate).
   LRMS (m/z): 413/415/417 (M+1)⁺
   1 H NMR (400 MHz, DMSO-*d*₆) □ ppm 3.90 (s, 3 H) 8.06-8.08 (m, 4 H) 8.13-8.14 (m, 2 H) 11.03 (s, 1 H)
**b) Methyl 4-[(2-bromo-6-{[4-(trifluoromethyl)pyridin-2-yl]amino}pyridin-4-yl)amino]benzoate**
   Obtained (38%) from methyl 4-[(2,6-dibromopyridin-4-yl)amino]benzoate (example 103a) and 4-(trifluoromethyl)pyridin-2-amine following the experimental procedure as described in preparation 27a.
   LRMS (m/z): 495/497 (M+1)⁺.
   1 H NMR (400 MHz, DMSO-*d*₆) □ ppm 3.89 (s, 3 H) 7.21 (d, *J*=5.60 Hz, 1 H) 7.67 (s, 1 H) 7.89 (s, 1 H) 8.06-8.11 (m, 4 H) 8.31-8.32 (m, 1 H) 8.49 (d, *J*=4.80 Hz, 1 H) 10.47 (s, 1 H) 10.90 (s, 1 H)
**c) *tert*-Butyl 4-(4-{[4-(methoxycarbonyl)phenyl]amino}-6-{[4-(trifluoromethyl) pyridin-2-yl]amino}-2,4'-bipyridin-2'-yl)piperazine-1-Carboxylate**
   Obtained (42%) from methyl 4-[(2-bromo-6-{[4-(trifluoromethyl)pyridin-2-yl]aminolpyridin-4-yl)amino]benzoate (example 103b) and {2-[4-(*tert* butoxycarbonyl)piperazin-1-yl]pyridin-4-yl}boronic acid (preparation 24) following the experimental procedure as described in preparation 1, followed by purification by flash chromatography (100% hexanes to 100% ethyl acetate).
   LRMS (m/z): 678 (M+1)⁺.
   1 H NMR (400 MHz, DMSO-*d*₆) □ ppm 1.43 (s, 9 H) 3.46-3.48 (m, 4 H) 3.55-3.56 (m, 4 H) 3.92 (s, 3 H) 7.17 (d, *J*=6.00 Hz, 1 H) 7.23 (d, *J*=6.40 Hz, 1 H) 7.36 (s, 1 H) 7.90 (s, 1 H) 8.11 (s, 1 H) 8.11-8.13 (m, 5 H) 8.26 (d, *J*=5.20 Hz, 1 H) 8.51 (d, *J*=4.80 Hz, 1 H) 8.56 (s, 1 H) 10.36 (s, 1 H) 10.84 (s, 1 H)
**d) 4-{[(2'-Piperazin-1-yl-6-{[4-(trifluoromethyl)pyridin-2-yl]amino}-2,4'-bipyridin-4-yl)amino]carbonyl}benzoic acid**
   Obtained (28%) from *tert*-butyl 4-(4-{[4-(methoxycarbonyl)phenyl]amino}-6-{[4-(trifluoromethyl) pyridin-2-yl]amino}-2,4'-bipyridin-2'-yl)piperazine-1-Carboxylate (example 103c) following the experimental procedure described in example 74b followed by the experimental procedure described in example 92b.
   LRMS (m/z): 564 (M+1)⁺.
   ¹H NMR (400 MHz, DMSO-*d*₆) □ ppm 2.90 (br. s., 4 H) 3.54 (t, 4 H) 7.15 (dd, *J*=5.28, 0.78 Hz, 1 H) 7.20 (dd, *J*=5.18, 1.07 Hz, 1 H) 7.33 (s, 1 H) 7.92 (d, *J*=1.37 Hz, 1 H) 7.95 - 8.05 (m, 4 H) 8.14 (d, *J*=1.37 Hz, 1 H) 8.23 (d, *J*=5.08 Hz, 1 H) 8.49 (d, *J*=5.28 Hz, 1 H) 8.57 (s, 1 H) 10.32 (s, 1 H) 10.77 (s, 1 H)

### EXAMPLE 104

### 5-Oxo-3-phenyl-5-(2'-(piperazin-1-yl)-6-(pyrazin-2-ylamino)-2,4'-bipyridin-4-ylamino)pentanoic acid

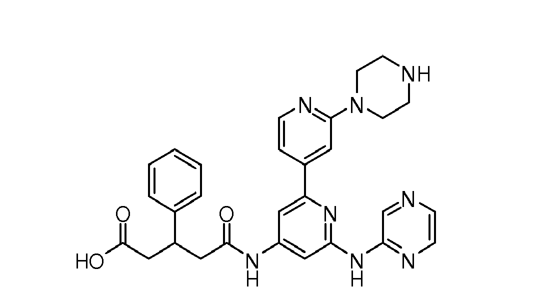

**a) 5-{[2'-[4-(*tert*-Butoxycarbonyl)piperazin-1-yl]-6-(pyrazin-2-ylamino)-2,4'-bipyridin-4-yl]amino}-5-oxo-3-phenylpentanoic acid**
   4-Phenyldihydro-2*H*-pyran-2,6(3*H*)-dione (0.13 g, 0.67 mmol) and *N*,*N*-dimethylpyridin-4-amine (10 mg, 0.08 mmol) were added to a solution of *tert*-butyl 4-[4-amino-6-(pyrazin-2-ylamino)-2,4'-bipyridin-2'-yl]piperazine-1-Carboxylate (preparation 26, 0.07 g, 0.16 mmol) in pyridine (1 mL) and mixture stirred at 115 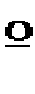 C for 48 hour. The crude was concentrated in vacuo and the resulting oil was purificated by reverse phase chromatography to give the title compound (27 mg, 26%).
   LRMS (m/z): 639 (M+1)⁺.
**b) 5-Oxo-3-phenyl-5-(2'-(piperazin-1-yl)-6-(pyrazin-2-ylamino)-2,4'-bipyridin-4-ylamino)pentanoic acid**
   Obtained from 5-{[2'-[4-(*tert*-butoxycarbonyl)piperazin-1-yl]-6-(pyrazin-2-ylamino)-2,4'-bipyridin-4-yl]amino}-5-oxo-3-phenylpentanoic acid (example 104a) following the experimental procedure as described in example 2b.
   LRMS (m/z): 539 (M+1)⁺.

### EXAMPLE 105

### (3R)-3-(Cyclohexylmethyl)-4-oxo-4-{[2'-piperazin-1-yl-6-(pyrazin-2-ylamino)-2,4'-bipyridin-4-yl]amino}butanoic acid

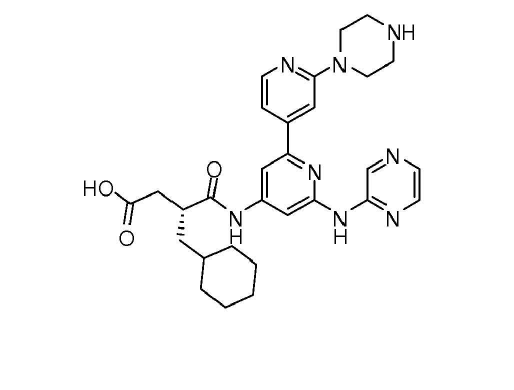

**a) *tert*-Butyl 4-[4-{[(2R)-4-tert-butoxy-2-(cyclohexylmethyl)-4-oxobutanoyl]amino}-6-(pyrazin-2-ylamino)-2,4'-bipyridin-2'-yl]piperazine-1-Carboxylate**
   Obtained as a oil (6%) from *tert*-butyl 4-[4-amino-6-(pyrazin-2-ylamino)-2,4'-bipyridin-2'-yl]piperazine-1-Carboxylate (preparation 26) and (2R)-4-*tert*-butoxy-2-(cyclohexylmethyl)-4-oxobutanoic acid following the experimental procedure as described in example 73a, using azabenzotriazol-1-yl)-1,1,3,3-tetramethyl uronium hexafluorophosphate as coupling agent, followed by purification by flash chromatography (hexanes/ethyl acetate 20% to 60% ethyl acetate).
   LRMS (m/z): 701 (M+1)⁺.
**b) (3*R*)-3-(Cyclohexylmethyl)-4-oxo-4-{[2'-piperazin-1-yl-6-(pyrazin-2-ylamino)-2,4'-bipyridin-4-yl]amino}butanoic acid**
   Obtained as hydrochloride salt (77%) from *tert*-butyl 4-[4-{[(2R)-4-tert-butoxy-2-(cyclohexylmethyl)-4-oxobutanoyl]amino}-6-(pyrazin-2-ylamino)-2,4'-bipyridin-2'-yl]piperazine-1-Carboxylate (example 105a) following the experimental procedure as described in example 73b.
   LRMS (m/z): 545 (M+1)⁺.
   1 H NMR (400 MHz, DMSO-*d*₆) □ ppm 0.79 - 0.96 (m, 2 H) 1.10 - 1.38 (m, 5 H) 1.46 - 1.72 (m, 4 H) 1.83 (d, *J*=13.68 Hz, 1 H) 2.29 - 2.45 (m, 2 H) 2.54 - 2.64 (m, 1 H) 2.93 - 3.05 (m, 1 H) 3.26 (s, 4 H) 3.91 (s, 4 H) 7.31 (d, *J*=5.86 Hz, 1 H) 7.54 (s, 1 H) 7.93 (s, 1 H) 8.03 (s, 1 H) 8.14 (d, *J*=2.34 Hz, 1 H) 8.24 - 8.34 (m, 2 H) 9.15 (s, 1 H) 9.27 (s, 2 H) 10.29 (s, 1 H) 10.61 (s, 1 H)

### EXAMPLE 106

### 2-({[2'-Piperazin-1-yl-6-(pyrazin-2-ylamino)-2,4'-bipyridin-4-yl]amino}carbonyl)cyclohexanecarboxylic acid

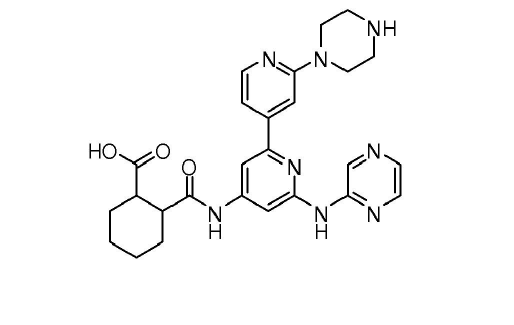

**a) 2-({[2'-[4-(*tert*-Butoxycarbonyl)piperazin-1-yl]-6-(pyrazin-2-ylamino)-2,4'-bipyridin-4-yl]amino}carbonyl)cyclohexanecarboxylic acid**
   Hexahydro-2-benzofuran-1,3-dione (0.25 g, 1.6 mmol), *N*,*N*-dimethylpyridin-4-amine (15 mg, 0.12 mmol) and pyridine (0.17 mL, 2.1 mmol) were added to a solution of *tert-*butyl 4-[4-amino-6-(pyrazin-2-ylamino)-2,4'-bipyridin-2'-yl]piperazine-1-Carboxylate (preparation 26, 0.15 g, 0.33 mmol) in dimethylformamide (5 mL) and mixture stirred at 100 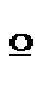 C for 48 hour. The crude was poured onto water, 2N sodium hydroxide was added and the product was extracted with ethyl acetate (99%).
   LRMS (m/z): 603 (M+1)⁺.
**b) 2-({[2'-Piperazin-1-yl-6-(pyrazin-2-ylamino)-2,4'-bipyridin-4-yl]amino}carbonyl)cyclohexanecarboxylic acid**
   Obtained as hydrochloride salt (45%) from 2-({[2'-[4-(*tert*-butoxycarbonyl)piperazin-1-yl]-6-(pyrazin-2-ylamino)-2,4'-bipyridin-4-yl]amino}carbonyl)cyclohexanecarboxylic acid (example 106a) following the experimental procedure as described in example 73b.
   LRMS (m/z): 503 (M+1)⁺
   1 H NMR (400 MHz, DMSO-*d*₆) □ ppm 1.28 - 1.50 (m, 3 H) 1.59 - 1.69 (m, 1 H) 1.70 - 1.84 (m, 2 H) 1.97 - 2.20 (m, 2 H) 2.66 - 2.76 (m, 1 H) 3.04 (q, *J*=4.30 Hz, 1 H) 3.28 (s, 4 H) 4.02 (d, *J*=5.47 Hz, 4 H) 7.36 (d, *J*=5.86 Hz, 1 H) 7.66 (s, 1 H) 7.94 (s, 1 H) 8.08 (s, 1 H) 8.17 (d, *J*=2.74 Hz, 1 H) 8.28 (d, *J*=5.86 Hz, 1 H) 8.30 - 8.33 (m, 1 H) 9.14 (s, 1 H) 9.59 (s, 2 H) 10.42 - 10.53 (m, 1 H) 10.57 (s, 1 H)

### EXAMPLE 107

### 2-({[2'-Piperazin-1-yl-6-(pyrazin-2-ylamino)-2,4'-bipyridin-4-yl]amino}carbonyl)benzoic acid

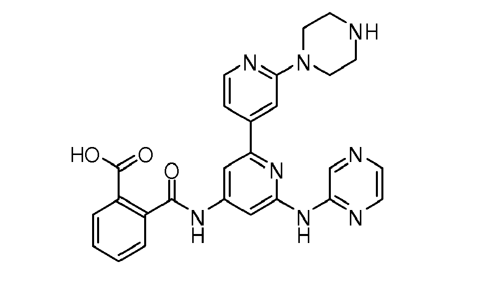

**a) 2-({[2'-[4-(*tert*-Butoxycarbonyl)piperazin-1-yl]-6-(pyrazin-2-ylamino)-2,4'-bipyridin-4-yl]amino}carbonyl)benzoic acid**
   Obtained as a solid from *tert*-butyl 4-[4-amino-6-(pyrazin-2-ylamino)-2,4'-bipyridin-2'-yl]piperazine-1-Carboxylate (preparation 26) and 2-benzofuran-1,3-dione following the experimental procedure as described in example 106a, followed by purification by reverse phase chromatography (0% CH₃OH in H₂O to 100% CH₃OH in H₂O)-
   LRMS (m/z): 597 (M+1)⁺.
**b) 2-({[2'-Piperazin-1-yl-6-(pyrazin-2-ylamino)-2,4'-bipyridin-4-yl]amino}carbonyl)benzoic acid**
   Obtained as hydrochloride salt (59%) from 2-({[2'-[4-(*tert-*butoxycarbonyl)piperazin-1-yl]-6-(pyrazin-2-ylamino)-2,4'-bipyridin-4-yl]amino}carbonyl)benzoic acid (example 107a) following the experimental procedure as described in example 73b.
   LRMS (m/z): 497 (M+1)⁺.
   1 H NMR (400 MHz, DMSO-*d*₆) □ ppm 3.27 (s, 4 H) 3.96 (s, 4 H) 7.34 (d, *J*=5.08 Hz, 1 H) 7.56 - 7.66 (m, 3 H) 7.68 - 7.74 (m, 1 H) 7.91 - 7.96 (m, 2 H) 8.15 (d, *J*=2.74 Hz, 1 H) 8.17 (s, 1 H) 8.26 - 8.32 (m, 2 H) 9.17 (d, *J*=1.17 Hz, 1 H) 9.50 (s, 2 H) 10.39 (s, 1 H) 10.96 (s, 1 H)

### EXAMPLE 108

### (3R)-3-Methyl-4-oxo-4-{[2'-piperazin-1-yl-6-(pyrazin-2-ylamino)-2,4'-bipyridin-4-yl]amino}butanoic acid

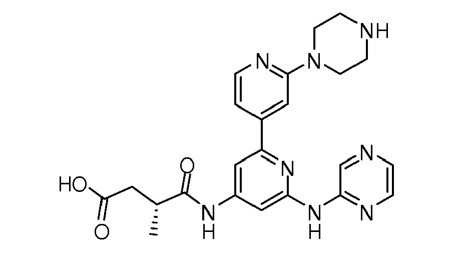

**a) *tert*-Butyl 4-[4-{[(2*R*)-4-methoxy-2-methyl-4-oxobutanoyl]amino}-6-(pyrazin-2-ylamino)-2,4'-bipyridin-2'-yl]piperazine-1-Carboxylate**
   Obtained as a oil (9%) from *tert*-butyl 4-[4-amino-6-(pyrazin-2-ylamino)-2,4'-bipyridin-2'-yl]piperazine-1-Carboxylate (preparation 26) and (2R)-4-methoxy-2-methyl-4-oxobutanoic acid following the experimental procedure as described in example 105a heating at 70 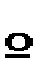 C, followed by purification by flash chromatography (hexanes/ethyl acetate 20% to 80% ethyl acetate).
   LRMS (m/z): 577 (M+1)⁺.
**b) (3*R*)-4-{[2'-[4-(*tert*-Butoxycarbonyl)piperazin-1-yl]-6-(pyrazin-2-ylamino)-2,4'-bipyridin-4-yl]amino}-3-methyl-4-oxobutanoic acid**
   2N Sodium hydroxide (0.02 mL, 0.32 mmol) was added to a suspension of *tert*-butyl 4-[4-{[(2*R*)-4-methoxy-2-methyl-4-oxobutanoyl]amino}-6-(pyrazin-2-ylamino)-2,4'-bipyridin-2'-yl]piperazine-1-Carboxylate (example 108a, 0.02 g, 0.03 mmol) in ethanol (2 mL) and mixture was stirred at room temperature overnight. Then solvent was removed to yield the title compound (94%) as a sodium salt.
   LRMS (m/z): 563 (M+1)⁺.
**c) (3*R*)-3-Methy)-4-oxo-4-{[2'-piperazin-1-yl-6-(pyrazin-2-ylamino)-2,4'-bipyridin-4-yl]amino}butanoic acid**
   Obtained as hydrochloride salt (77%) from (3*R*)-4-{[2'-[4-(*tert*-butoxycarbonyl)piperazin-1-yl]-6-(pyrazin-2-ylamino)-2,4'-bipyridin-4-yl]amino}-3-methyl-4-oxobutanoic acid (example 108b) following the experimental procedure as described in example 73b.
   LRMS (m/z): 463 (M+1)⁺.

### EXAMPLE 109

### (3S)-4-Phenyl-3-{[3-({[2'-piperazin-1-yl-6-(pyrazin-2-ylamino)-2,4'-bipyridin-4-yl]amino}carbonyl)benzoyl]amino}butanoic acid

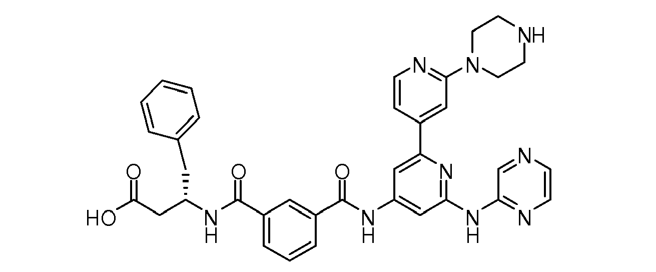

**a) *tert*-Butyl 4-[4-{[3-(*tert*-butoxycarbonyl)benzoyl]amino}-6-(pyrazin-2-ylamino)-2,4'-bipyridin-2'-yl]piperazine-1-Carboxylate**
   Obtained as a oil (69%) from *tert*-butyl 4-[4-amino-6-(pyrazin-2-ylamino)-2,4'-bipyridin-2'-yl]piperazine-1-Carboxylate (preparation 26) and 3-(*tert-*butoxycarbonyl)benzoic acid following the experimental procedure as described in example 105a heating at 60 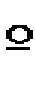 C, followed by purification by flash chromatography (hexanes/ethyl acetate 0% to 80% ethyl acetate).
   LRMS (m/z): 653 (M+1)⁺.
**b) 3-({[2'-[4-(*tert*-Butoxycarbonyl)piperazin-1-yl]-6-(pyrazin-2-ylamino)-2,4'-bipyridin-4-yl]amino}carbonyl)benzoic acid**
   8N Sodium hydroxide (1.2 mL, 9.60 mmol) was added to a suspension of *tert-*butyl 4-[4-{[3-(*tert*-butoxycarbonyl)benzoyl]amino}-6-(pyrazin-2-ylamino)-2,4'-bipyridin-2'-yl]piperazine-1-Carboxylate (example 109a, 0.26 mg, 0.40 mmol) in methanol (10 mL) and the mixture was stirred at rt overnight. The solvent was removed, the crude was neutralized with acetic acid, resuspended in water and product extracted with ethyl acetate. Organic layer was washed with water and brine, dried (MgSO₄), filtered and concentrated to yield the title compound (0.08 g, 30%) as a a solid.
   LRMS (m/z): 597 (M+1)⁺.
**c) (*tert*-Butyl 4-[4-{[3-({[(1*S*)-1-benzyl-3-*tert*-butoxy-3-oxopropyl]amino}carbonyl)-benzoyl]amino}-6-(pyrazin-2-ylamino)-2,4'-bipyridin-2'-yl]piperazine-1-Carboxylate**
   Obtained as a oil (66%) from 3-({[2'-[4-(*tert*-butoxycarbonyl)piperazin-1-yl]-6-(pyrazin-2-ylamino)-2,4'-bipyridin-4-yl]amino}carbonyl)benzoic acid (preparation 109b) and *tert-*butyl (3*S*)-3-amino-4-phenylbutanoate following the experimental procedure as described in example 105a, followed by purification by flash chromatography (hexanes/ethyl acetate 0% to 100% ethyl acetate).
   LRMS (m/z): 814 (M+1⁺).
**d) (3*S*)-4-Phenyl-3-{[3-({[2'-piperazin-1-yl-6-(pyrazin-2-ylamino)-2,4'-bipyridin-4-yl]amino}carbonyl)benzoyl]amino}butanoic acid**
   Obtained as hydrochloride salt (83%) from (*tert*-butyl 4-[4-{[3-({[(1*S*)-1-benzyl-3-*tert-*butoxy-3-oxopropyl]amino}carbonyl)benzoyl]amino}-6-(pyrazin-2-ylamino)-2,4'-bipyridin-2'-yl]piperazine-1-Carboxylate (example 109c) following the experimental procedure as described in example 73b.
   LRMS (m/z): 658 (M+1)⁺.
   1 H NMR (400 MHz, DMSO-*d*₆) δ ppm 2.53 - 2.72 (m, 2 H) 2.74 - 3.02 (m, 2 H) 3.94 (br. s., 4 H) 4.53 (dd, *J*=12.70, 6.06 Hz, 1 H) 7.16 (dd, *J*=7.82, 3.91 Hz, 1 H) 7.20 - 7.32 (m, 3 H) 7.37 (d, *J*=5.08 Hz, 1 H) 7.55 - 7.74 (m, 2 H) 7.99 (d, *J*=7.82 Hz, 1 H) 8.06 - 8.41 (m, 5 H) 8.54 (br. s., 1 H) 8.72 (d, *J*=8.21 Hz, 1 H) 9.17 (s, 1 H) 9.37 (br. s., 2 H) 10.39 (br. s., 1 H) 11.03 (br. s., 1 H)

### EXAMPLE 110

### 3,3-Dimethyl-5-oxo-5-1[2'-piperazin-1-yl-6-(pyrazin-2-ylamino)-2,4'-bipyridin-4-yl]amino}pentanoic acid

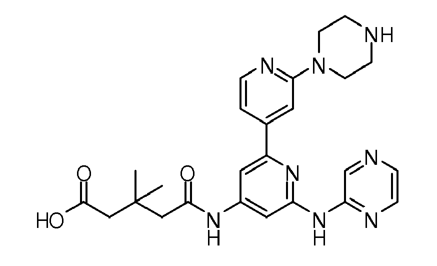

**a) 5-{[2'-[4-(*tert*-Butoxycarbonyl)piperazin-1-yl]-6-(pyrazin-2-ylamino)-2,4'-**bipyridin-4-yl]amino}-3,3-dimethyl-5-oxopentanoic acid
   Obtained (94%) as a oil from *tert-*butyl 4-[4-amino-6-(pyrazin-2-ylamino)-2,4'-bipyridin-2'-yl]piperazine-1-Carboxylate (preparation 26) and 4,4-dimethyldihydro-2*H*-pyran-2,6(3*H*)-dione following the experimental procedure as described in example 106a.
   LRMS (m/z): 591 (M+1)⁺.
**b) 3,3-Dimethyl-5-oxo-5-1[2'-piperazin-1-yl-6-(pyrazin-2-ylamino)-2,4'-bipyridin-4-yl]amino}pentanoic acid**
   Obtained as hydrochloride salt (41 %) from 5-{[2'-[4-(*tert*-butoxycarbonyl)piperazin-1-yl]-6-(pyrazin-2-ylamino)-2,4'-bipyridin-4-yl]amino}-3,3-dimethyl-5-oxopentanoic acid (example 110a) following the experimental procedure as described in example 73b.
   LRMS (m/z): 491 (M+1)⁺.
   1 H NMR (400 MHz, DMSO-*d*₆) δ ppm 1.12 (s, 6 H) 2.37 (s, 2 H) 3.28 (br. s., 4 H) 3.99 (br. s., 4 H) 7.35 (d, *J*=5.86 Hz, 1 H) 7.64 (br. s., 1 H) 7.92 (s, 1 H) 8.06 (s, 1 H) 8.16 (br. s., 1 H) 8.23 - 8.37 (m, 1 H) 9.15 (br. s., 1 H) 9.52 (br. s., 2 H) 10.43 (br. s., 1 H) 10.64 (br. s., 1 H)

### EXAMPLE 111

### 2-Methyl-3-phenyl-N-[2'-piperazin-1-yl-6-(pyrazin-2-ylamino)-2,4'-bipyridin-4-yl]propanamide

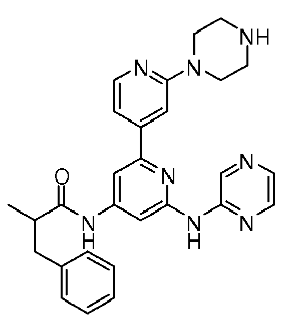

**a) tert-Butyl 4-[4-[(2-methyl-3-phenylpropanoyl)amino]-6-(pyrazin-2-ylamino)-2,4'-bipyridin-2'-yl]piperazine-1-Carboxylate**
   Obtained as a oil (36%) from tert-butyl 4-[4-amino-6-(pyrazin-2-ylamino)-2,4'-bipyridin-2'-yl]piperazine-1-Carboxylate (preparation 26) and 2-methyl-3-phenylpropanoic acid following the experimental procedure as described in example 105a, followed by purification by flash chromatography (hexanes/ethyl acetate 20% to 100% ethyl acetate).
   LRMS (m/z): 595 (M+1)⁺.
**b) 2-Methyl-3-phenyl-N-[2'-piperazin-1-yl-6-(pyrazin-2-ylamino)-2,4'-bipyridin-4-yl]propanamide**
   Obtained as a solid (14%) from tert-butyl 4-[4-[(2-methyl-3-phenylpropanoyl)amino]-6-(pyrazin-2-ylamino)-2,4'-bipyridin-2'-yl]piperazine-1-Carboxylate (example 110a) following the experimental procedure as described in example 73b.
   LRMS (m/z): 495 (M+1)⁺.
   1 H NMR (400 MHz, DMSO-d₆) δ ppm 1.12 (d, *J=6.64* Hz, 3 H) 2.64 (dd, J=13.68, 7.42 Hz, 1 H) 2.82 - 3.08 (m, 2 H) 3.13 - 3.34 (m, 4 H) 3.85 (t, 4 H) 7.21 - 7.34 (m, 4 H) 7.46 (s, 1 H) 7.84 (d, *J=1.56* Hz, 1 H) 7.97 (d, *J=1.56* Hz, 1 H) 8.13 (d, *J=2.74* Hz, 1 H) 8.26 - 8.33 (m, 2 H) 9.07 (br. s., 2 H) 9.16 (d, *J=1.56* Hz, 1 H) 10.20 (br. s., 1 H)

### EXAMPLE 112

### 5-Methyl-3-({[2'-piperazin-1-yl-6-(pyrazin-2-ylamino)-2,4'-bipyridin-4-yl]amino}carbonyl)hexanoic acid

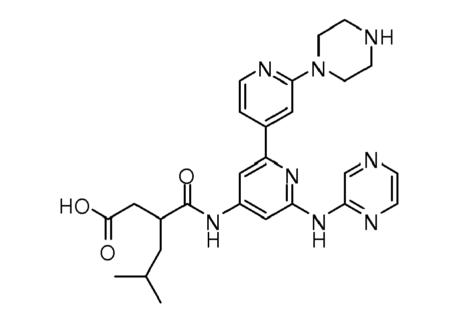

**a) 3-({[2'-[4-(*tert*-Butoxycarbonyl)piperazin-1-yl]-6-(pyrazin-2-ylamino)-2,4'-bipyridin-4-yl]amino}carbonyl)-5-methylhexanoic acid**
   Obtained (12%) from tert-butyl 4-[4-amino-6-(pyrazin-2-ylamino)-2,4'-bipyridin-2'-yl]piperazine-1-Carboxylate (preparation 26) and 2-(2-tert-butoxy-2-oxoethyl)-4-methylpentanoic acid following the experimental procedure as described in example 73a, followed by purification by flash chromatography (hexanes/ethyl acetate 0% to 80% ethyl acetate).
   LRMS (m/z): 605 (M+1)⁺.
**b) 5-Methyl-3-({[2'-piperazin-1-yl-6-(pyrazin-2-ylamino)-2,4'-bipyridin-4-yl]amino}carbonyl)hexanoic acid**
   Obtained as salt (35%) from 3-({[2'-[4-(tert-butoxycarbonyl)piperazin-1-yl]-6-(pyrazin-2-ylamino)-2,4'-bipyridin-4-yl]amino}carbonyl)-5-methylhexanoic acid (example 112a) following the experimental procedure described in example 73b.
   LRMS (m/z): 505 (M+1)⁺.
   1 H NMR (400 MHz, DMSO-d₆) δ ppm 0.88 (br. s., 3 H) 0.93 (br. s., 3 H) 1.16 - 1.39 (m, 2 H) 1.47 - 1.65 (m, 2 H) 2.96 (br. s., 1 H) 3.15 - 3.36 (m, 4 H) 3.57 (br. s., 1 H) 3.82 - 4.06 (m, 4 H) 7.34 (br. s., 1 H) 7.59 (br. s., 1 H) 7.97 (br. s., 1 H) 8.04 - 8.22 (m, 2 H) 8.22 - 8.39 (m, 2 H) 9.13 (br. s., 1 H) 9.53 (br. s., 2 H) 10.37 (br. s., 1 H) 10.71 (br. s., 1 H)

### EXAMPLE 113

### (3R)-4-Oxo-3-phenyl-4-{[2'-piperazin-1-yl-6-(pyrazin-2-ylamino)-2,4'-bipyridin-4-yl]amino}butanoic acid

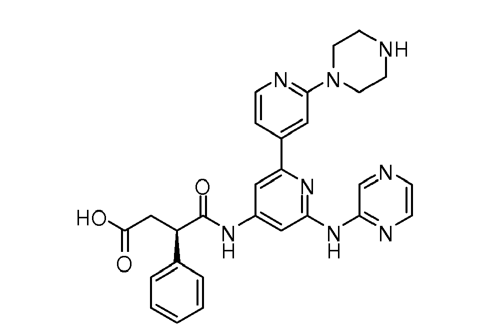

**a) *tert*-Butyl 4-[4-{[(2*R*)-4-(benzyloxy)-4-oxo-2-phenylbutanoyl]amino}-6-(pyrazin-2-ylamino)-2,4'-bipyridin-2'-yl]piperazine-1-Carboxylate**
   Obtained (73%) from tert-butyl 4-[4-amino-6-(pyrazin-2-ylamino)-2,4'-bipyridin-2'-yl]piperazine-1-Carboxylate (preparation 26) and (2R)-4-(benzyloxy)-4-oxo-2-phenylbutanoic acid following the experimental procedure as described in example 73a, followed by purification by flash chromatography (hexanes/ethyl acetate 0% to 80% ethyl acetate).
   LRMS (m/z): 715 (M+1)⁺.
**b) Benzyl (3R)-4-oxo-3-phenyl-4-{[2'-piperazin-1 -yl-6-(pyrazin-2-ylamino)-2,4'-bipyridin-4-yl]amino}butanoate**
   Obtained as salt (64%) from *tert-butyl* 4-[4-{[(2R)-4-(benzyloxy)-4-oxo-2-phenylbutanoyl]amino}-6-(pyrazin-2-ylamino)-2,4'-bipyridin-2'-yl]piperazine-1-Carboxylate (example 113a) following the experimental procedure described in example 73b.
   LRMS (m/z): 615 (M+1)⁺.
**c) (3*R*)-4-Oxo-3-phenyl-4-{[2'-piperazin-1-yl-6-(pyrazin-2-ylamino)-2,4'-bipyridin-4-yl]amino}butanoic acid**
   To a solution of benzyl (3R)-4-oxo-3-phenyl-4-{[2'-piperazin-1-yl-6-(pyrazin-2-ylamino)-2,4'-bipyridin-4-yl]amino}butanoate (example 113b, 0.23 g, 0.35 mmol) in tetrahydrofurane (50 mL) and methanol (10 mL) was added palladium on charcoal (10%, 0.04 g). The reaction mixture was hydrogenated at 40 psi for 24 hours. The catalyst was filtered through Celite@ and the solvent removed under reduced pressure giving a crude, which was purified by reverse phase. The product obtained was dissolved in 4M hydrochloric acid in dioxane (2 mL) and the solution was stirred at room temperature overnight. The solvent was evaporated to give the title compound (8 mg, 4%) as a solid.
   LRMS (m/z): 525 (M+1)⁺.
   1 H NMR (400 MHz, DMSO-*d*₆) δ ppm 2.58 - 2.73 (m, 2 H) 3.01 - 3.21 (m, 4 H) 3.74 - 4.03 (m, 4 H) 4.15 - 4.30 (m, 1 H) 7.22 - 7.48 (m, 5 H) 7.54 (br. s., 1 H) 7.89 (br. s., 1 H) 8.02 (br. s., 1 H) 8.14 (br. s., 1 H) 8.28 (d, *J=8.99* Hz, 2 H) 9.12 (br. s., 1 H) 9.27 (br. s., 2 H) 10.26 (br. s., 1 H) 10.84 (br. s., 1 H)

### EXAMPLE 114

### (3R)-3-Benzyl-4-oxo-4-{[2'-piperazin-1-yl-6-(pyrazin-2-ylamino)-2,4'-bipyridin-4-yl]amino}butanoic acid

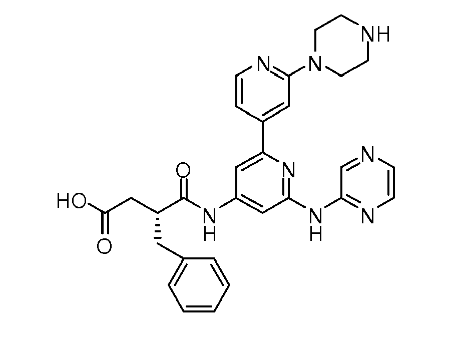

**a) *tert*-Butyl 4-[4-{[(2*R*)-2-benzyl-4-tert-butoxy-4-oxobutanoyl]amino}-6-(pyrazin-2-ylamino)-2,4'-bipyridin-2'-yl]piperazine-1-Carboxylate**
   Obtained (27%) from tert-butyl 4-[4-amino-6-(pyrazin-2-ylamino)-2,4'-bipyridin-2'-yl]piperazine-1-Carboxylate (preparation 26) and (2R)-2-benzyl-4-tert-butoxy-4-oxobutanoic acid following the experimental procedure as described in example 73a, using 1-methylpyrrolidin-2-one as a solvent and heating at 100°C for 48 hours, followed by purification by reverse phase chromatography (0% ACN/CH₃OH 1:1 in H₂O to 100% ACN/CH₃OH 1:1 in H₂O).
   LRMS (m/z): 695 (M+1)⁺.
**b) (3*R*)-3-Benzyl-4-oxo-4-1[2'-piperazin-1-yl-6-(pyrazin-2-ylamino)-2,4'-bipyridin-4-yl]amino}butanoic acid**
   Obtained as hydrochloride salt (98%) from tert-butyl 4-[4-{[(2R)-2-benzyl-4-tert-butoxy-4-oxobutanoyl]amino}-6-(pyrazin-2-ylamino)-2,4'-bipyridin-2'-yl]piperazine-1-Carboxylate (example 114a) following the experimental procedure as described in example 73b.
   LRMS (m/z): 539 (M+1)⁺.
   1 H NMR (400 MHz, DMSO-*d*₆) δ ppm 2.23 (dd, *J=16.80,* 4.30 Hz, 1 H) 2.65 (dd, *J=17.00,* 9.96 Hz, 2 H) 3.02 (dd, J=13.68, 6.25 Hz, 1 H) 3.22 (dd, *J=8.21,* 4.30 Hz, 1 H) 3.29 (br. s., 4 H) 4.00 (br. s., 4 H) 7.21 (td, *J=6.06,* 2.74 Hz, 1 H) 7.27 - 7.34 (m, 4 H) 7.37 (d, *J=5.47* Hz, 1 H) 7.66 (s, 1 H) 7.95 (d, *J*=1.17 Hz, 1 H) 8.07 (d, *J=1.17* Hz, 1 H) 8.17 (d, *J=2.74* Hz, 1 H) 8.28 (d, *J=6.25* Hz, 1 H) 8.32 (dd, *J=2.54,* 1.37 Hz, 1 H) 9.12 (d, *J=1.17* Hz, 1 H) 9.51 (br. s., 2 H) 10.46 (br. s., 1 H)

### EXAMPLE 115

### N-[2'-[(1S,4S)-2,5-Diazabicyclo[2.2.1 ]hept-2-yl]-6-(pyrazin-2-ylamino)-2,4'-bipyridin-4-yl]nicotinamide

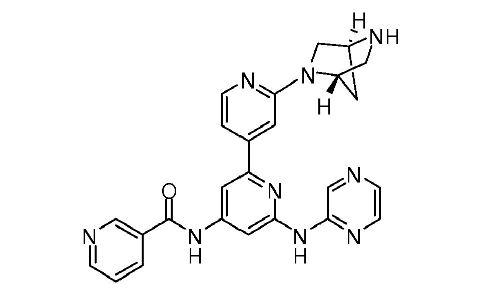

**a) *tert-Butyl* 5-{6-(pyrazin-2-ylamino)-4-[(pyridin-3-ylcarbonyl)amino]-2,4'-bipyridin-2'-yl}-2,5-diazabicyclo[2.2.1]heptane-2-Carboxylate**
   Obtained (53%) from tert-butyl 5-(4-amino-6-1[4-(trifluoromethyl)pyridin-2-yl]aminol-2,4'-bipyridin-2'-yl)-2,5-diazabicyclo[2.2.1]heptane-2-Carboxylate (preparation 28) and nicotinic acid following the experimental procedure as described in example 73a.
   LRMS (m/z): 566 (M+1)⁺.
   1 H NMR (400 MHz, DMSO-d₆) δ ppm 1.34 (s, 4 H) 1.40 (s, 5 H) 1.91 (s, 3 H) 1.95-1.97 (m, 1 H) 3.24.3.27 (m, 1 H) 3.61 (d, *J=8.00* Hz, 1 H) 4.50 (d, *J=8.00* Hz, 1 H) 4.47-4.53 (m, 1 H) 4.86 (s, 1 H) 7.07 (s, 1 H) 7.12 (d, *J=4.8* Hz, 1 H) 7.59-7.63 (m, 1 H) 7.89 (s, 1 H) 8.13 (d, *J=2.80* Hz, 1 H) 8.19 (s, 1 H) 8.21-8.24 (m, 2 H) 8.28 (s, 1 H) 8.33-8.36 (m, 2 H) 8.81 (dd, *J=4.80,* 1.60 Hz, 1 H) 9.15 (d, *J=1.60* Hz, 1 H) 9.32 (s, 1 H) 10.18 (s, 1 H) 10.87 (s, 1 H)
**b) *N-*[2'-[(1*S*,4*S*)-2,5-Diazabicyclo[2.2.1]hept-2-yl]-6-(pyrazin-2-ylamino)-2,4'-bipyridin-4-yl]nicotinamide**
   Obtained (62%) from tert-butyl 5-{6-(pyrazin-2-ylamino)-4-[(pyridin-3-ylcarbonyl)amino]-2,4'-bipyridin-2'-yl}-2,5-diazabicyclo[2.2.1 ]heptane-2-Carboxylate (example 115a) following the experimental procedure as described in example 2b.
   LRMS (m/z): 466 (M+1)⁺.
   ¹H NMR (400 MHz, DMSO-*d*₆) δ ppm 1.84 (d, *J=1*0.16 Hz, 1 H) 1.99 (d, *J=9.77* Hz, 1 H) 3.04 (d, *J*=10.16 Hz, 1 H) 3.15 (d, 1 H) 3.44 (d, *J=10.16* Hz, 1 H) 3.60 (dd, *J=9.87,* 1.86 Hz, 1 H) 4.14 (br. s., 2 H) 4.83 (s, 1 H) 7.05 (s, 1 H) 7.12 (dd, *J=5.28,* 0.98 Hz, 1 H) 7.59 (dd, *J=7.82,* 4.49 Hz, 1 H) 7.92 (d, *J=1.37* Hz, 1 H) 8.12 (d, *J=2.54* Hz, 1 H) 8.19 (d, *J=1.37* Hz, 1 H) 8.21 (d, *J=5.28* Hz, 1 H) 8.34 (dt, *J=8.01,* 1.95, 1.76 Hz, 1 H) 8.79 (dd, *J=4.88,* 1.56 Hz, 1 H) 9.14 (d, *J=1.95* Hz, 1 H) 9.27 (d, *J=1.37* Hz, 1 H) 10.18 (s, 1 H) 10.91 (s, 1 H)

### EXAMPLE 116

### 4-({[2'-[(1S,4S)-2,5-Diazabicyc)o[2.2.1]hept-2-y)]-6-(pyrazin-2-y)amino)-2,4'-bipyridin-4-yl]amino}carbonyl)benzoic acid

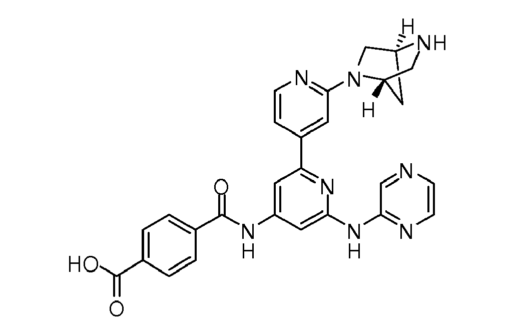

**a) *tert*-Butyl 5-[4-{[4-(methoxycarbonyl)benzoyl]amino}-6-(pyrazin-2-ylamino)-2,4'-bipyridin-2'-yi]-2,5-diazabicyclo[2.2.1]heptane-2-Carboxylate**
   Obtained (33%) from tert-butyl 4-[4-amino-6-(pyrazin-2-ylamino)-2,4'-bipyridin-2'-yl]piperazine-1-Carboxylate (preparation 26) and methyl 4-(chlorocarbonyl)benzoate following the experimental procedure as described in example 76a.
   LRMS (m/z): 623 (M+1)⁺.
**b) 4-({[2'-[5-(*tert*-Butoxycarbonyl)-2,5-diazabicyclo[2.2.1]hept-2-yl]-6-(pyrazin-2-ylamino)-2,4'-bipyridin-4-yl]amino}carbonyl)benzoic acid**
   Obtained (63%) from *tert-butyl* 5-[4-{[4-(methoxycarbonyl)benzoyl]aminol-6-(pyrazin-2-ylamino)-2,4'-bipyridin-2'-yl]-2,5-diazabicyclo[2.2.1]heptane-2-Carboxylate (example 116a) following the experimental procedure as described in example 74b.
   LRMS (m/z): 609 (M+1)⁺.
**c) 4-({[2'-[(1*S*,4*S*)-2,5-Diazabicyclo[2.2.1]hept-2-yl]-6-(pyrazin-2-y)amino)-2,4'-bipyridin-4-yl]amino}carbonyl)benzoic acid**
   Obtained (100%) from 4-({[2'-[5-(tert-butoxycarbonyl)-2,5-diazabicyclo[2.2.1]hept-2-yl]-6-(pyrazin-2-ylamino)-2,4'-bipyridin-4-yl]aminol carbonyl) benzoic acid (example 116b) following the experimental procedure as described in example 2b.
   LRMS (m/z): 509 (M+1)⁺.
   1H NMR (400 MHz, DMSO-d₆) δ ppm 1.97 (d, *J=10.36* Hz, 1 H) 2.20 (d, *J=10.36* Hz, 1 H) 3.20 - 3.80 (m, 6 H) 4.55 (br. s., 1 H) 4.98 (s, 1 H) 7.12 - 7.17 (m, 1 H) 7.23 (d, *J=5.28* Hz, 1 H) 7.98 (s, 1 H) 8.11 (s, 2 H) 8.14 (d, *J=2.54* Hz, 1 H) 8.20 (d, *J=1.17* Hz, 1 H) 8.25 - 8.32 (m, 2 H) 8.63 (br. s., 1 H) 9.18 (br. s., 1 H) 9.31 (s, 1 H) 10.22 (s, 1 H) 10.90 (s, 1 H)

### EXAMPLE 117

### N-(2'-[(1S,4S)-2,5-Diazabicyclo[2.2.1]hept-2-yl]-6-{[4-(trifluoromethyl)pyridin-2-yl]amino}-2,4'-bipyridin-4-yl)nicotinamide

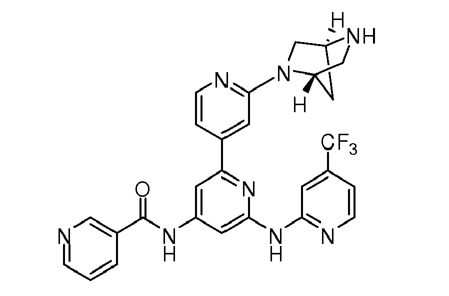

**a) *tert-*Butyl (1*S*,4*S*)-5-(4-[(pyridin-3-ylcarbonyl)amino]-6-1[4-(trifluoromethyl)pyridin-2-yl]amino}-2,4'-bipyridin-2'-yl)-2,5-diazabicyclo[2.2.1]heptane-2-Carboxylate**
   Obtained (92%) from tert-butyl (1S,4S)-5-(4-amino-6-{[4-(trifluoromethyl)pyridin-2-yl]amino}-2,4'-bipyridin-2'-yl)-2,5-diazabicyclo[2.2.1]heptane-2-Carboxylate (preparation 28) and nicotinic acid following the experimental procedure as described in example 73a followed by purification by reverse phase chromatography (0% CH₃OH in H₂O to 100% CH₃OH in H₂O).
   LRMS (m/z): 633 (M+1)⁺.
   1 H NMR (400 MHz, DMSO-d₆) δ ppm 1.35 (s, 4 H) 1.40 (s, 5 H) 1.93 (d, *J=8.80* Hz, 3H) 3.22-3.25 (m, 2 H) 3.58 (t, *J=8.00* Hz, 1 H) 4.50 (d, *J=17.20* Hz, 1 H) 4.88 (s, 1 H) 7.00 (s, 1 H) 7.10 (d, *J*=5.60 Hz, 1 H) 7.23 (s, 1 H) 7.61 (dd, *J=8.00,* 4.80 Hz, 1 H) 7.87 (s, 1 H) 8.11 (d, *J=12.40* Hz, 1 H) 8.20 (d, *J=5.20* Hz, 1 H) 8.35 (dt, *J=8.00,* 2.00 Hz, 1 H) 8.51-8.56 (m, 2 H) 8.81 (dd, *J=4.80,* 1.60 Hz, 1 H) 9.16 (d, *J=2.00* Hz, 1 H) 10.36 (s, 1 H) 10.86 (s, 1 H)
**b) *N*-(2'-[(1*S*,4*S*)-2,5-Diazabicyclo[2.2.1]hept-2-yl]-6-1[4-(trifluoromethyl)pyridin-2-yl]amino}-2,4'-bipyridin-4-yl)nicotinamide**
   Obtained (37%) from *tert-butyl* (1 S,4S)-5-(4-[(pyridin-3-ylcarbonyl)amino]-6-{[4-(trifluoromethyl)pyridin-2-yl]amino}-2,4'-bipyridin-2'-yl)-2,5-diazabicyclo[2.2.1]heptane-2-Carboxylate (example 117a) following the experimental procedure as described in example 91b.
   LRMS (m/z): 533 (M+1)⁺.
   1 H NMR (400 MHz, DMSO-d₆) δ ppm 2.86 (d, *J=5.08* Hz, 4 H) 3.50 (d, *J=5.28* Hz, 4 H) 7.14 (d, *J=5.28* Hz, 1 H) 7.23 (d, *J=5.08* Hz, 1 H) 7.33 (s, 1 H) 7.62 (dd, *J=7.91,* 4.79 Hz, 1 H) 7.90 (d, *J=1.56* Hz, 1 H) 8.10 (d, *J=1.37* Hz, 1 H) 8.25 (d, *J=5.28* Hz, 1 H) 8.32 - 8.39 (m, 1 H) 8.52 (d, *J=5.08* Hz, 1 H) 8.56 (s, 1 H) 8.81 (dd, *J=4.69,* 1.56 Hz, 1 H) 9.16 (d, *J=2.15* Hz, 1 H) 10.37 (s, 1 H) 10.86 (s, 1 H)

### EXAMPLE 118

### 4-{[(2'-[(1S,4S)-2,5-Diazabicyclo[2.2.1]hept-2-yl]-6-{[4-(trifluoromethyl)pyridin-2-yl]amino}-2,4'-bipyridin-4-yl)amino]carbonyl}benzoic acid

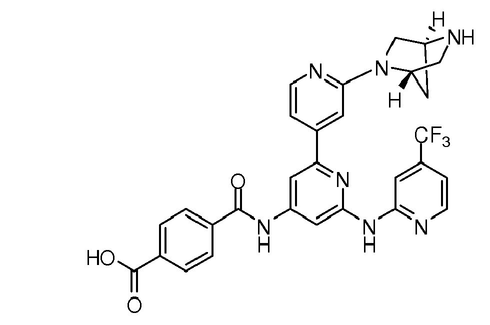

**a) *tert-*Butyl (1*S*,4*S*)-5-(4-{[4-(methoxycarbonyl)benzoyl]amino}-6-{[4-(trifluoromethyl)pyridin-2-yl]amino}-2,4'-bipyridin-2'-yl)-2,5-diazabicyclo[2.2.1 ]heptane-2-Carboxylate**
   Obtained (60%) from *tert-butyl* 5-(4-amino-6-{[4-(trifluoromethyl)pyridin-2-yl]amino}-2,4'-bipyridin-2'-yl)-2,5-diazabicyclo[2.2.1]heptane-2-Carboxylate (preparation 28) and methyl 4-(chlorocarbonyl)benzoate following the experimental procedure as described in example 76a followed by purification by reverse phase chromatography.
   LRMS (m/z): 690 (M+1)⁺.
   1 H NMR (500 MHz, DMSO-d₆) □ ppm 1.35 (s, 4 H) 1.40 (s, 5 H) 1.94 (d, *J*=11.00 Hz, 3H) 3.22-3.25 (m, 2 H) 3.58 (t, *J=8.00* Hz, 1 H) 3.91 (s, 3 H) 4.50 (d, *J*=22.00 Hz, 1 H) 4.88 (s, 1 H) 7.00 (s, 1 H) 7.10 (d, *J=7.00* Hz, 1 H) 7.22 (s, 1 H) 7.87 (s, 1 H) 8.13 (m, 5 H) 8.20 (d, *J=6.50* Hz, 1 H) 8.51-8.56 (m, 2 H) 10.35 (s, 1 H) 10.84 (s, 1 H)
**b) 4-{[(2'-[(1*S*,4*S*)-5-(tert-Butoxycarbonyl)-2,5-diazabicyclo[2.2.1]hept-2-yl]-6-{[4-(trifluoromethyl)pyridin-2-yl]amino}-2,4'-bipyridin-4-yl)amino]carbonyl}benzoic acid**
   Obtained from *tert-butyl* (1 S,4S)-5-(4-{[4-(methoxycarbonyl)benzoyl]amino}-6-{[4-(trifluoromethyl)pyridin-2-yl]amino}-2,4'-bipyridin-2'-yl)-2,5-diazabicyclo[2.2.1 ]heptane-2-Carboxylate (preparation 118a) following the experimental procedure as described in example 74b.
   LRMS (m/z): 676 (M+1)⁺.
**c) 4-{[(2'-[(1*S*,4*S*)-2,5-Diazabicyclo[2.2.1]hept-2-yl]-6-{[4-(trifluoromethyl)pyridin-2-yl]amino}-2,4'-bipyridin-4-yl)amino]carbonyl}benzoic acid**
   Obtained (82%) from 4-{[(2'-[(1 *S*,4*S*)-5-(tert-butoxycarbonyl)-2,5-diazabicyclo[2.2.1 ]hept-2-yl]-6-{[4-(trifluoromethyl)pyridin-2-yl]amino}-2,4'-bipyridin-4-yl)amino]carbonyllbenzoic acid (example 118b) following the experimental procedure as described in example 2b.
   LRMS (m/z): 576 (M+1)⁺.
   1 H NMR (400 MHz, DMSO-d₆) δ ppm 1.88 - 1.98 (m, 1 H) 2.04 - 2.16 (m, 1 H) 3.10 - 3.35 (m, 2 H) 4.37 (s, 1 H) 4.94 (s, 1 H) 6.95 - 7.02 (m, 1 H) 7.15 - 7.24 (m, 2 H) 7.90 (s, 1 H) 7.94 - 8.02 (m, 4 H) 8.11 (d, *J=1.17* Hz, 1 H) 8.20 (d, *J=4.00* Hz, 1 H) 8.25 (s, 1 H) 8.49 (d, *J=4.00* Hz, 1 H) 8.52 (s, 1 H) 10.31 (s, 1 H) 10.75 (s, 1 H)

### EXAMPLE 119

### N-[2'-Piperazin-1-yl-6-(pyrazin-2-ylamino)-2,4'-bipyridin-4-yl]benzenesulfonamide

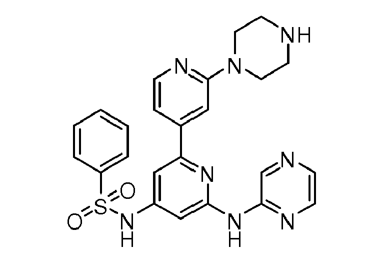

**a) *tert*-Butyl 4-[4-[(phenylsulfonyl)amino]-6-(pyrazin-2-ylamino)-2,4'-bipyridin-2'-yl]piperazine-1-Carboxylate**
   Benzenesulfonyl chloride (0.04 mL, 0.33 mmol) was added to a stirred solution of *tert-*butyl 4-[4-amino-6-(pyrazin-2-ylamino)-2,4'-bipyridin-2'-yl]piperazine-1-Carboxylate (preparation 26, 0.10 g, 0.22 mmol) and pyridine (0.05 mL, 0.65 mmol) in tetrahydrofurane (1 mL), and the mixture was stirred at room temperature. After 16 hours, saturated aqueous sodium carbonate solution and ethyl acetate were added to the reaction mixture. The organic layer was washed with brine, and dried (MgSO₄) and evaporated. Purification of the crude by reverse phase chromatography (0% CH₃OH in H₂O to 100% CH₃OH in H₂O) gave the title compound (0.01 g, 9%).
   LRMS (m/z): 589 (M+1)⁺.
   1 H NMR (500 MHz, DMSO-d₆) δ ppm 1.43 (s, 9 H) 3.44 - 3.46 (m, 4 H) 3.54 - 3.56 (m, 4 H) 7.04 (dd, *J=7.00,* 1.00 Hz, 1 H) 7.11 (d, *J=2.00* Hz, 1 H) 7.24 (s, 1 H) 7.59 - 7.65 (m, 4 H) 7.97 - 7.99 (m, 2 H) 8.11 (d, *J=3.00* Hz, 1 H) 8.23 (d, *J=7.00* Hz, 1 H) 8.27 - 8.28 (m, 1 H) 9.07 (d, *J=1.50* Hz, 1 H) 10.08 (s, 1 H)
**b) *N*-[2'-Piperazin-1-yl-6-(pyrazin-2-ylamino)-2,4'-bipyridin-4- yl]benzene sulfonamide**
   Obtained as a solid (92%) from tert-butyl 4-[4-[(phenylsulfonyl)amino]-6-(pyrazin-2-ylamino)-2,4'-bipyridin-2'-yl]piperazine-1-Carboxylate (example 119a) following the experimental procedure as described in example 2b.
   LRMS (m/z): 489 (M+1)⁺.
   1 H NMR (500 MHz, DMSO-d₆) δ ppm 3.06 (t, *J=6.50* Hz, 4 H) 3.63 (t, *J=6.50* Hz, 4 H) 7.02 (d, *J=2.00* Hz, 1 H) 7.09 (d, *J=6.50* Hz, 1 H) 7.24 (s, 1 H) 7.29 (d, *J=1.50*
   Hz, 1 H) 7.46 - 7.51 (m, 3 H) 7.88 - 7.92 (m, 2 H) 8.04 (d, *J*=3.00 Hz, 1 H) 8.20 - 8.23 (m, 2 H) 9.16 (d, *J=1.50* Hz, 1 H) 9.76 (br. s, 1 H)

### EXAMPLE 120

### N-[2'-Piperazin-1-yl-6-(pyrazin-2-ylamino)-2,4'-bipyridin-4-yl]-3-(trifluoromethyl) benzenesulfonamide

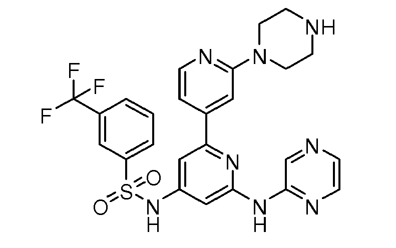

**a) *tert*-Butyl 4-[6-(pyrazin-2-ylamino)-4-({[3-(trifluoromethyl)phenyl] sulfonyl} amino)-2,4'-bipyridin-2'-yl]piperazine-1-Carboxylate**
   Obtained (15%) from tert-butyl 4-[4-amino-6-(pyrazin-2-ylamino)-2,4'-bipyridin-2'-yl]piperazine-1-Carboxylate (preparation 26) and 3-(trifluoromethyl)benzenesulfonyl chloride following the experimental procedure as described in example 119a, followed by purification by reverse phase chromatography (0% CH₃OH in H₂O to 100% CH₃OH in H₂O).
   LRMS (m/z): 657 (M+1)⁺.
   1 H NMR (500 MHz, DMSO-d₆) δ ppm 1.43 (s, 9 H) 3.44 - 3.46 (m, 4 H) 3.54 - 3.56 (m, 4 H) 7.06 (dd, *J=6.50,* 1.50 Hz, 1 H) 7.10 (d, *J=2.00* Hz, 1 H) 7.26 (s, 1 H) 7.68 (br. s, 1 H) 7.86 (t, *J=10.00* Hz, 1 H) 8.04 (d, *J=10.00* Hz, 1 H) 8.11 - 8.13 (m, 1 H) 8.22 - 8.27 (m, 4 H) 9.05 (s, 1 H) 10.09 (br. s, 1 H)
**b) *N*-[2'-Piperazin-1-yl-6-(pyrazin-2-ylamino)-2,4'-bipyridin-4-yl]-3-(trifluoromethyl)benzenesulfonamide**
   Obtained as a solid (78%) from *tert-butyl* 4-[6-(pyrazin-2-ylamino)-4-({[3-(trifluoromethyl)phenyl]sulfonyl}amino)-2,4'-bipyridin-2'-yl]piperazine-1-Carboxylate (example 120a) following the experimental procedure as described in example 2b.
   LRMS (m/z): 557 (M+1)⁺.
   1 H NMR (500 MHz, DMSO-d₆) δ ppm 3.18 (t, *J=7.00* Hz, 4 H) 3.74 (t, *J=6.00* Hz, 4 H) 6.99 (d, *J=2.50* Hz, 1 H) 7.15 - 7.19 (m, 2 H) 7.31 (s, 1 H) 7.68 (d, *J=9.50* Hz, 1 H) 7.80 (d, *J*=10.00 Hz, 1 H) 8.02 (d, *J=3.00* Hz, 1 H) 8.09 (s, 1 H) 8.13 (d, *J*=10.00 Hz, 1 H) 8.18 - 8.21 (m, 2 H) 9.20 (d, *J=1.50* Hz, 1 H) 9.61 (br. s, 1 H)

### EXAMPLE 121

### N-[2'-Piperazin-1-yl-6-(pyrazin-2-ylamino)-2,4'-bipyridin-4-yl]-3-(trifluoromethoxy) benzenesulfonamide

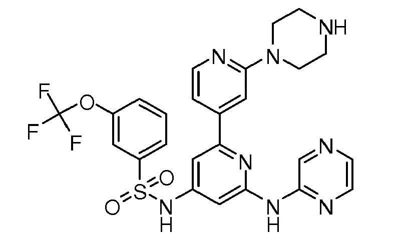

**a) *tert*-Butyl 4-[6-(pyrazin-2-ylamino)-4-({[3-(trifluoromethoxy)phenyl]sulfonyl} amino)-2,4'-bipyridin-2'-yl]piperazine-1-Carboxylate**
   Obtained (19%) from tert-butyl 4-[4-amino-6-(pyrazin-2-ylamino)-2,4'-bipyridin-2'-yl]piperazine-1-Carboxylate (preparation 26) and 3-(trifluoromethoxy)benzenesulfonyl chloride following the experimental procedure as described in example 119a, followed by purification by reverse phase chromatography (20% CH₃OH in H₂O to 100% CH₃OH in H₂O).
   LRMS (m/z): 673 (M+1)⁺.
   1 H NMR (500 MHz, DMSO-d₆) δ ppm 1.41 (s, 9 H) 3.43 - 3.45 (m, 4 H) 3.52 - 3.55 (m, 4 H) 7.04 (dd, *J=6.50,* 1.00 Hz, 1 H) 7.08 (d, *J=2.00* Hz, 1 H) 7.23 (s, 1 H) 7.64 - 7.67 (m, 2 H) 7.75 (t, *J=10.00* Hz, 1 H) 7.89 (s, 1 H) 7.96 - 7.98 (m, 1 H) 8.10 - 8.12 (m, 1 H) 8.21 (d, *J=6.5* Hz, 1 H) 8.24 - 8.26 (m, 1 H) 9.06 (s, 1 H) 10.08 (br. s, 1 H) (1 H not observed)
**b) *N*-[2'-Piperazin-1-yl-6-(pyrazin-2-ylamino)-2,4'-bipyridin-4-yl]-3-(trifluoromethoxy)benzenesulfonamide**
   Obtained as a solid (92%) from *tert-butyl* 4-[6-(pyrazin-2-ylamino)-4-({[3-(trifluoromethoxy)phenyl]sulfonyl}amino)-2,4'-bipyridin-2'-yl]piperazine-1-Carboxylate (example 121 a) following the experimental procedure as described in example 2b.
   LRMS (m/z): 573 (M+1)⁺.
   1 H NMR (500 MHz, DMSO-d₆) δ ppm 3.17 (t, *J=7.00* Hz, 4 H) 3.72 (t, *J=6.00* Hz, 4 H) 6.97 (d, *J=2.00* Hz, 1 H) 7.15 - 7.17 (m, 2 H) 7.30 (s, 1 H) 7.42 (d, *J=10.00* Hz, 1 H) 7.57 (t, *J=9.50* Hz, 1 H) 7.73 (s, 1 H) 7.86 (d, *J=10.00* Hz, 1 H) 8.02 (d, *J=3.50* Hz, 1 H) 8.18 - 8.21 (m, 2 H) 9.22 (d, *J=1.50* Hz, 1 H) 9.61 (br. s, 1 H) (2H not observed).

### EXAMPLE 122

### (3S)-4-Phenyl-3-[({[2'-piperazin-1-yl-6-(pyrazin-2-ylamino)-2,4'-bipyridin-4-yl]amino}carbonyl)amino]butanoic acid

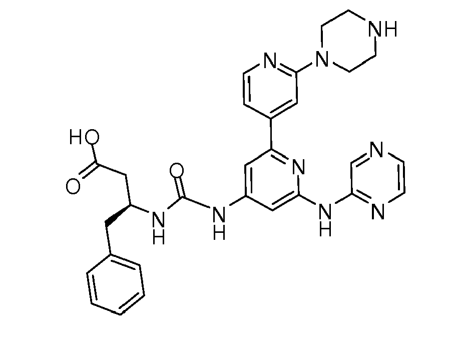

**a) *tert*-Butyl 4-[4-isocyanato-6-(pyrazin-2-ylamino)-2,4'-bipyridin-2'-yl]piperazine-1-Carboxylate**
   To a solution of *tert-butyl* 4-[4-amino-6-(pyrazin-2-ylamino)-2,4'-bipyridin-2'-yl]piperazine-1-Carboxylate (preparation 26, 0.4 g, 0.89 mmol) in dichloromethane (3 mL) / tetrahydrofurane (3 mL) at 0°C triphosgene (0.1 g, 0.35 mmol) in dichloromethane (3 mL) was added and then trielthylamine (0.3 mL, 2.15 mmol). Reaction was stirred at rt for 3 hour and product used in next reaction without further work-up or purification.
   LRMS (m/z): 507(M+33, methyl carbamate observed)⁺.
**b) *tert*-Butyl 4-[4-[({[(1*S*)-1-benzyl-3-tert-butoxy-3-oxopropyl]amino}carbonyl)amino]-6-(pyrazin-2-ylamino)-2,4'-bipyridin-2'-yl]piperazine-1-Carboxylate**
   To a solution of *tert-butyl* 4-[4-isocyanato-6-(pyrazin-2-ylamino)-2,4'-bipyridin-2'-yl]piperazine-1-Carboxylate (example 122a, 234 mg, 0.44 mmol) in tetrahydrofurane (2 mL) *tert*-butyl (3S)-3-amino-4-phenylbutanoate (123 mg, 0.52 mmol) in tetrahydrofurane (2 mL) and triethylamine (0.12 mL, 0.87 mmol) were added and mixture stirred at rt for 1 hour. Solvent was removed and crude purified by reverse phase chromatography (0% CH₃OH in H₂O to 100% CH₃OH in H₂O) to yield the title compound (14%) as a solid.
   LRMS (m/z): 710 (M+1)⁺.
**c) (3S)-4-Phenyl-3-[({[2'-piperazin-1-yl-6-(pyrazin-2-ylamino)-2,4'-bipyridin-4-yl]amino}carbonyl)amino]butanoic acid**
   Obtained as hydrochloride salt (91%) from tert-butyl 4-[4-[({[(1S)-1-benzyl-3-tert-butoxy-3-oxopropyl]amino}carbonyl)amino]-6-(pyrazin-2-ylamino)-2,4'-bipyridin-2'-yl]piperazine-1-Carboxylate (example 122b) following the experimental procedure as described in example 73b.
   LRMS (m/z): 554 (M+1)⁺.

### EXAMPLE 123

### N-[2'-Piperazin-1-yl-6-(pyrazin-2-ylamino)-2,4'-bipyridin-4-yl]-3,4-dihydroisoquinoline-2(1 H)-Carboxamide

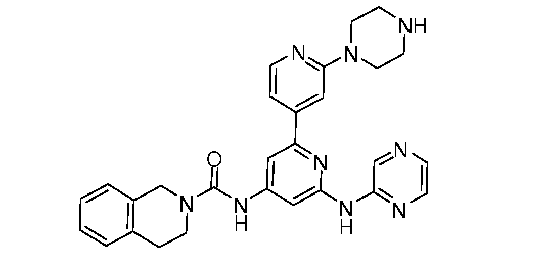

**a) *tert*-Butyl 4-[4-[(3,4-dihydroisoquinolin-2(1H)-ylcarbonyl)amino]-6-(pyrazin-2-ylamino)-2,4'-bipyridin-2'-yl]piperazine-1-Carboxylate**
   Obtained from *tert-butyl* 4-[4-isocyanato-6-(pyrazin-2-ylamino)-2,4'-bipyridin-2'-yl]piperazine-1-Carboxylate (example 122a) and 1,2,3,4-tetrahydroisoquinoline following the experimental procedure described in example 122b.
   LRMS (m/z): 565 (M+1)⁺.
**b) *N*-[2'-Piperazin-1-yl-6-(pyrazin-2-ylamino)-2,4'-bipyridin-4-yl]-3,4-dihydroisoquinoline-2(1*H*)-Carboxamide**
   Obtained as a solid (63%) from tert-butyl 4-[4-[(3,4-dihydroisoquinolin-2(1 H)-ylcarbonyl)amino]-6-(pyrazin-2-ylamino)-2,4'-bipyridin-2'-yl]piperazine-1-Carboxylate (example 123a) following the experimental procedure as described in example 73b, followed by purification by reverse phase chromatography (0% CH₃OH in H₂O to 60% CH₃OH in H₂O).
   LRMS (m/z): 508 (M+1)⁺.
   1 H NMR (400 MHz, DMSO-d₆) δ ppm 2.81 - 3.00 (m, 6 H) 3.54 (d, *J=4.30* Hz, 4 H) 3.76 (t, *J*=5.86 Hz, 4 H) 4.69 (s, 2 H) 7.14 (dd, *J=5.28,* 0.98 Hz, 1 H) 7.19 - 7.24 (m, 2 H) 7.33 (s, 1 H) 7.70 (d, *J=1.56* Hz, 1 H) 7.93 (d, *J=1.56* Hz, 1 H) 8.09 (d, *J=2.74* Hz, 1 H) 8.22 (br. s., 1 H) 8.23 (br. s., 1 H) 8.25 (dd, *J=3.13,* 1.95 Hz, 1 H) 9.13 (s, 1 H) 9.20 (d, *J=1.17* Hz, 1 H) 9.98 (s, 1 H)

### EXAMPLE 124

### 4-(Benzyloxy)-N-[2'-piperazin-1-yl-6-(pyrazin-2-ylamino)-2,4'-bipyridin-4-yl]piperidine-1-Carboxamide

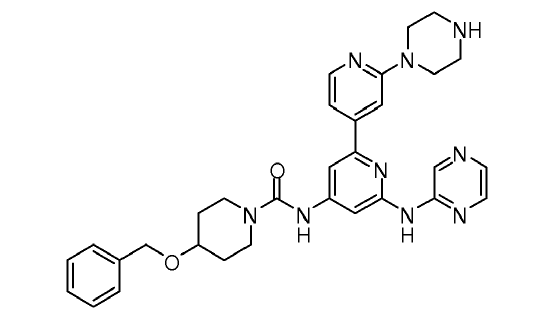

**a) *tert-*Butyl 4-[4-({[4-(benzyloxy)piperidin-1-yl]carbonyl}amino)-6-(pyrazin-2-ylamino)-2,4'-bipyridin-2'-yl]piperazine-1-Carboxylate**
   Obtained from *tert-butyl* 4-[4-isocyanato-6-(pyrazin-2-ylamino)-2,4'-bipyridin-2'-yl]piperazine-1-Carboxylate (example 122a) and 4-(benzyloxy)piperidine following the experimental procedure described in example 122b.
   LRMS (m/z): 623 (M+1)⁺.
**b) 4-(Benzyloxy)-*N*-[2'-piperazin-1-yl-6-(pyrazin-2-ylamino)-2,4'-bipyridin-4-yl]piperidine-1-Carboxamide**
   Obtained as a solid (63%) from tert-butyl 4-[4-({[4-(benzyloxy)piperidin-1-yl]carbonyl}amino)-6-(pyrazin-2-ylamino)-2,4'-bipyridin-2'-yl]piperazine-1-Carboxylate (example 124a) following the experimental procedure as described in example 73b..
   LRMS (m/z): 566 (M+1)⁺.
   1 H NMR (400 MHz, DMSO-d₆) □ ppm 3.26 (br. s., 8 H) 3.79 - 3.90 (m, 8 H) 4.09 - 4.17 (m, 2 H) 4.56 (s, 2 H) 7.24 - 7.33 (m, 2 H) 7.36 (d, *J=4.30* Hz, 3 H) 7.56 (br. s., 1 H) 7.64 - 7.77 (m, 1 H) 7.91 (s, 1 H) 7.98 (s, 1 H) 8.20 (d, *J=2.34* Hz, 1 H) 8.27 - 8.37 (m, 2 H) 9.05 (br. s., 1 H) 9.40 (br. s., 2 H) 9.52 (br. s., 1 H)

### EXAMPLE 125

### N⁶-(4-Methylpyridin-2-yl)-2'-piperazin-1-yl-2,4'-bipyridine-4,6-diamine

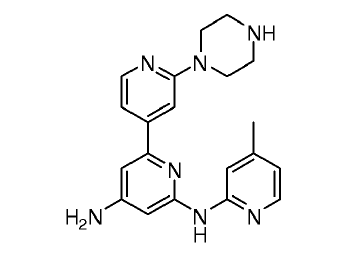

**a) *tert*-Butyl 4-{6-[(4-methylpyridin-2-yl)amino]-4-nitro-2,4'-bipyridin-2'-y)}piperazine-1-Carboxylate**
   Obtained (52%) from 6-Chloro-N-(4-methylpyridin-2-yl)-4-nitropyridin-2-amine (preparation 29) and and {2-[4-(tert-butoxycarbonyl)piperazin-1-yl]pyridin-4-yl}boronic acid (preparation 24) following the experimental procedure described in preparation 1 using a misture of toluene/ethanol 2:1 as solvent followed by purification by flash chromatography (hexane/ethyl acetate 50:50).
   LRMS (m/z): 492 (M+1)⁺.
**b) *tert*-Butyl 4-{4-amino-6-[(4-methylpyridin-2-yl)amino]-2,4'-bipyridin-2'-yl}piperazine-1-Carboxylate**
   Obtained (62%) from tert-butyl 4-{6-[(4-methylpyridin-2-yl)amino]-4-nitro-2,4'-bipyridin-2'-yl}piperazine-1-Carboxylate (example 125a) following the experimental procedure as described in preparation 26c.
   LRMS (m/z): 462 (M+1)⁺.
   1 H NMR (400 MHz, METHANOL-d₄) δ ppm 1.49 (s, 9 H) 2.34 (s, 3 H) 3.57 (s, 8 H) 6.73-6.78 (m, 3 H) 7.19 (d, *J=5.20* Hz, 1 H) 7.42 (s, 1 H) 7.56 (s, 1 H) 8.02 (d, *J=5.20* Hz, 1 H) 8.16 (d, *J=5.60* Hz, 1 H)
**c) *N*⁶-(4-Methylpyridin-2-yl)-2'-piperazin-1-yl-2,4'-bipyridine-4,6-diamine**
   Obtained (70%) from tert-butyl 4-{4-amino-6-[(4-methylpyridin-2-yl)amino]-2,4'-bipyridin-2'-yl}piperazine-1-Carboxylate (example 125b) following the experimental procedure as described in example 91 b.
   LRMS (m/z): 362 (M+1)⁺.
   ¹H NMR (400 MHz, DMSO-d₆) δ ppm 2.25 (s, 3 H) 2.84 (t, 4 H) 3.47 (t, 4 H) 5.97 (s, 2 H) 6.66 (dd, *J=5.18,* 0.68 Hz, 1 H) 6.69 (d, *J=1.76* Hz, 1 H) 6.76 (d, *J=1.56* Hz, 1 H) 7.06 (dd, *J=5.28,* 0.98 Hz, 1 H) 7.34 (s, 1 H) 7.78 (s, 1 H) 8.15 (d, *J=5.28* Hz, 1 H) 9.16 (s, 1 H)

### EXAMPLE 126

### 2'-[(1S,4S)-2,5-Diazabicyclo[2.2.1]hept-2-y)]-N⁶-(4-methylpyridin-2-yl)-2,4'-bipyridine-4,6-diamine

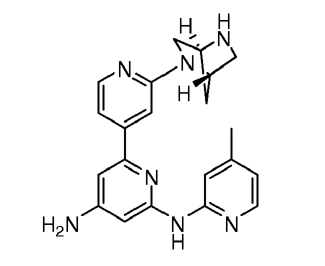

**a) *tert*-Butyl (1 S,4S)-5-{6-[(4-methylpyridin-2-yl)amino]-4-nitro-2,4'-bipyridin-2'-yl}-2,5-diazabicyclo[2.2.1]heptane-2-Carboxylate**
   Obtained (44%) from 6-Chloro-N(4-methylpyridin-2-yl)-4-nitropyridin-2-amine (preparation 29) and *tert-butyl* (1S,4S)-5-[4-(4,4,5,5-tetramethyl-1,3,2-dioxaborolan-2-yl)pyridin-2-yl]-2,5-diazabicyclo[2.2.1]heptane-2-Carboxylate (preparation 25) following the experimental procedure as described in preparation 1, followed by purification by flash chromatography (hexanes/ethyl acetate 1:1).
   LRMS (m/z): 504 (M+1)⁺.
   1 H NMR (400 MHz, METHANOL-d₄) δ ppm 1.41 (s, 4 H) 1.48 (s, 5 H) 2.04 (s, 2 H) 2.36 (s, 3 H) 3.39 (d, *J*=10.00 Hz, 1 H) 3.41-3.47 (m, 2 H) 3.67 (d, *J=9.60* Hz, 1 H) 4.62 (s, 1 H) 4.91 (s, 1 H) 6.83 67 (d, *J=4.80* Hz, 1 H) 7.21 (d, *J=10.40* Hz, 1 H) 7.27 (d, *J*=5.60 Hz, 1 H) 7.38 (s, 1 H) 8.00 (s, 1 H) 8.15 (d, *J=5.20* Hz, 2 H) 8.63 (s, 1 H)
**b) *tert*-Butyl (1*S*,4*S*)-5-{4-amino-6-[(4-methylpyridin-2-yl)amino]-2,4'-bipyridin-2'-yl}-2,5-diazabicyclo[2.2.1]heptane-2-Carboxylate**
   Obtained (51%) from tert-butyl (1S,4S)-5-{6-[(4-methylpyridin-2-yl)amino]-4-nitro-2,4'-bipyridin-2'-yl}-2,5-diazabicyclo[2.2.1]heptane-2-Carboxylate (example 126a) following the experimental procedure described in preparation 26c.
   LRMS (m/z): 474 (M+1)⁺.
   1 H NMR (400 MHz, METHANOL-d₄) δ ppm 1.41 (s, 4 H) 1.47 (s, 5 H) 2.03 (s, 2 H) 2.34 (s, 3 H) 3.39 (d, *J*=10.40 Hz, 1 H) 3.43-3.45 (m, 2 H) 3.66 (d, *J*=10.40 Hz, 1 H) 4.60 (s, 1 H) 4.86 (s, 1 H) 6.74 (d, *J*=5.20 Hz, 1 H) 6.77 (s, 1 H) 7.08-7.12 (m, 2 H) 7.52 (s, 1 H) 8.03 (d, *J*=5.20 Hz, 1 H) 8.09 (d, *J*=5.20 Hz, 1 H)
**c) 2'-[(1*S*,4*S*)-2,5-Diazabicyclo[2.2.1]hept-2-yl]-N⁶-(4-methylpyridin-2-yl)-2,4'-bipyridine-4,6-diamine**
   Obtained (57%) from tert-butyl (1 S,4S)-5-{4-amino-6-[(4-methylpyridin-2-yl)amino]-2,4'-bipyridin-2'-yl}-2,5-diazabicyclo[2.2.1]heptane-2-Carboxylate (example 126b) following the experimental procedure described in example 91 b.
   LRMS (m/z): 374 (M+1)⁺.
   ¹H NMR (400 MHz, DMSO-d₆) δ ppm 1.67 (d, *J*=8.99 Hz, 1 H) 1.79 (d, *J*=8.99 Hz, 1 H) 2.26 (s, 3 H) 2.52 (s, 1 H) 2.83 (d, 1 H) 2.88 - 2.94 (m, 1 H) 3.15 (s, 2 H) 3.23 (d, *J*=8.99 Hz, 1 H) 3.51 (dd, *J*=8.99*,* 1.95 Hz, 1 H) 3.70 (br. s., 1 H) 4.69 (s, 1 H) 5.97 (s, 2 H) 6.65 (dd, *J*=5.18*,* 0.88 Hz, 2 H) 6.77 (d, *J*=1.76 Hz, 1 H) 6.95 (s, 1 H) 6.98 (dd, *J*=5.37*,* 1.27 Hz, 1 H) 7.74 (s, 1 H) 8.03 (d, *J*=5.08 Hz, 1 H) 8.08 (d, *J*=5.28 Hz, 1 H) 9.14 (s, 1 H)

### EXAMPLE 127

### 2'-Piperazin-1-yl-N⁶-pyrazin-2-yl-2,4'-bipyridine-4,6-diamine

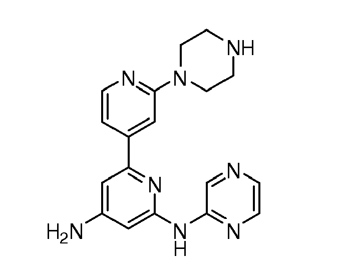

Obtained (74%) from tert-butyl 4-[4-amino-6-(pyrazin-2-ylamino)-2,4'-bipyridin-2'-yl]piperazine-1-Carboxylate (preparation 26) following the experimental procedure as described in example 91 b.
LRMS (m/z): 349 (M+1)⁺.
¹H NMR (400 MHz, DMSO-d₆) δ ppm 2.80 (t, 4 H) 3.44 (t, 4 H) 6.10 (s, 2 H) 6.74 (d, *J*=1.56 Hz, 1 H) 6.86 (d, *J*=1.76 Hz, 1 H) 7.06 (dd, *J*=5.18*,* 1.07 Hz, 1 H) 7.27 (s, 1 H) 8.01 (d, *J*=2.74 Hz, 1 H) 8.13 - 8.19 (m, 2 H) 9.14 (d, *J*=1.37 Hz, 1 H) 9.64 (s, 1 H)

### EXAMPLE 128

### N⁴-Methyl-2'-(piperazin-1-yl)-N⁶-(pyrazin-2-yl)-2,4'-bipyridine-4,6-diamine

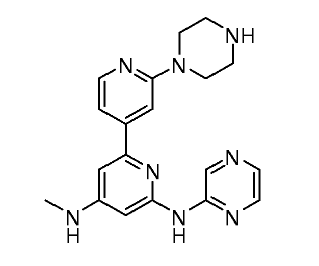

**a) *tert*-Butyl 4-[4-(methylamino)-6-(pyrazin-2-ylamino)-2,4'-bipyridin-2'-yl]piperazine-1-Carboxylate**
   To a solution of *tert-butyl* 4-[4-amino-6-(pyrazin-2-ylamino)-2,4'-bipyridin-2'-yl]piperazine-1-Carboxylate (preparation 26, 393 mg, 0.87 mmol) in methanol (12 mL), acetic acid (0.10 mL, 1.75 mmol) and formaldehyde (0.13 mL, 1.75 mmol) were added and reaction stirred at rt. After 5 hours, sodium cyanoborohydride (279 mg, 4.38 mmol) was added and mixture stirred overnight. Solvent was removed and saturated sodium carbonate solution and ethyl acetate were added to the crude. The organic layer was washed with brine, dried (MgSO₄) and evaporated. The crude was purified by flash chromatography (dichloromethane/methanol 0% to 6% methanol) to yield the title compound (0.22 g, 54%).
   LRMS (m/z): 463 (M+1)⁺.
**b) *N*⁴-Methyl-2'-(piperazin-1-yl)-*N*⁶-(pyrazin-2-yl)-2,4'-bipyridine-4,6-diamine**
   Obtained as a solid (70%) from tert-butyl 4-[4-(methylamino)-6-(pyrazin-2-ylamino)-2,4'-bipyridin-2'-yl]piperazine-1-Carboxylate (example 128a) following the experimental procedure as described in example 2b, followed by purification by reverse phase chromatography.
   LRMS (m/z): 363 (M+1)⁺.
   ¹H NMR (400 MHz, DMSO-d₆) δ ppm 2.77 (s, 3 H) 2.81 (t, *J*=5.20 Hz, 4 H) 3.47 (t, *J*=4.80 Hz, 4 H) 6.63 (d, *J*=4.8 Hz, 1 H) 6.75 (d, *J*=1.60 Hz, 1 H) 6.82 (s, 1 H) 7.42 (d, *J*=5.20 Hz, 1 H) 7.33 (s, 1 H) 8.04 (d, *J*=2.80 Hz, 1 H) 8.17 - 8.21 (m, 2 H) 9.23 (s, 1 H) 9.65 (s, 1 H)

### EXAMPLE 129

### N⁴, N⁴-Dimethyl-2'-piperazin-1-yl-N⁶-pyrazin-2-yl-2,4'-bipyridine-4,6-diamine

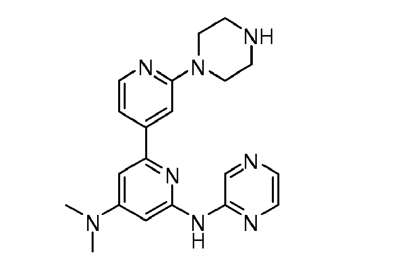

**a) *N*⁴, *N*⁴-Dimethyl-2'-piperazin-1-yl-*N*⁶-pyrazin-2-yl-2,4'-bipyridine-4,6-diamine**
   To a solution of *tert-butyl* 4-[4-amino-6-(pyrazin-2-ylamino)-2,4'-bipyridin-2'-yl]piperazine-1-Carboxylate (preparation 26, 50 mg, 0.11 mmol) in methanol (2 mL), acetic acid (13 µL, 0.22 mmol) and formaldehyde (45 µL, 0.55 mmol) were added and reaction stirred at rt. After 5 hours, sodium cyanoborohydride (35 mg, 0.55 mmol) was added and mixture stirred overnight. Solvent was removed and saturated sodium carbonate solution and dichloromethane were added to the crude. The organic layer was washed with brine, dried (MgSO₄) and evaporated. The crude was purified by flash chromatography (dichloromethane/methanol 0% to 10% methanol) to yield the title compound (0.20 g, 38%).
   LRMS (m/z): 477 (M+1)⁺.
**b) *N*⁴, *N*⁴-Dimethyl-2'-piperazin-1-yl-*N*⁶-pyrazin-2-yl-2,4'-bipyridine-4,6-diamine**
   Obtained as a solid (60%) from N⁴, N⁴-dimethyl-2'-piperazin-1-yl-W-pyrazin-2-yl-2,4'-bipyridine-4,6-diamine (example 129a) following the experimental procedure as described in example 92b.
   LRMS (m/z): 377 (M+1)⁺.

### EXAMPLE 130

### 1-(2'-(Piperazin-1-yl)-6-(pyrazin-2-ylamino)-2,4'-bipyridin-4-yl)piperidin-4-ol

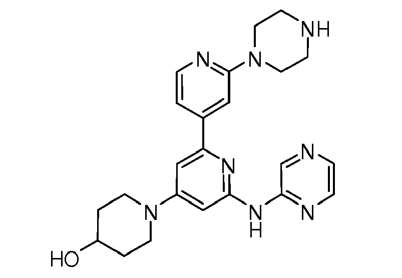

**a) 1-(2,6-Dichloropyridin-4-yl)piperidin-4-ol**
   Obtained (38%) from 2,4,6-trichloropyridine and piperidin-4-ol following the procedure
   as described in preparation 24a.
   LRMS (m/z): 248 (M+1)⁺.
**b) 1-[2-Chloro-6-(pyrazin-2-ylamino)pyridin-4-yl]piperidin-4-ol**
   Obtained (60%) from 1-(2,6-dichloropyridin-4-yl)piperidin-4-ol (example 130a) and pyrazin-2-amine following the experimental procedure as described in preparation 4, using dioxane as solvent. LRMS (m/z): 306 (M+1)⁺.
**c) *tert-*Butyl 4-[4-(4-hydroxypiperidin-1-yl)-6-(pyrazin-2-ylamino)-2,4'-bipyridin-2'-yl]piperazine-1-Carboxylate**
   Obtained (11%) from 1-[2-Chloro-6-(pyrazin-2-ylamino)pyridin-4-yl]piperidin-4-ol (example 130b) and {2-[4-(tert-butoxycarbonyl)piperazin-1-yl]pyridin-4-yl}boronic acid (preparation 24b) following the experimental procedure as described in preparation 1, using palladium tetrakis(triphenylphosphine), potassium carbonate as base and tetrahydrofurane/water as solvent.
   LRMS (m/z): 533 (M+1)⁺.
**d) 1-(2'-(Piperazin-1-yl)-6-(pyrazin-2-ylamino)-2,4'-bipyridin-4-yl)piperidin-4-ol**
   Obtained (28%) from tert-butyl 4-[4-(4-hydroxypiperidin-1-yl)-6-(pyrazin-2-ylamino)-2,4'-bipyridin-2'-yl]piperazine-1-Carboxylate (example 130c) following the experimental procedure described in example 91 b.
   LRMS (m/z): 433 (M+1)⁺.
   1 H NMR (250 MHz, DMSO-*d*₆) δ ppm 1.21 (q, 2 H) 1.89 (d, 2 H) 2.84 (t, 4 H) 3.13 (t, 2 H) 3.52 (t, 4 H) 3.78 (m, 3 H) 4.78 (d, 1 H) 7.08 (d, 2 H) 7.25 (d, 1 H) 7.39 (s, 1 H) 8.15-8.25 (m, 2 H) 9.27 (s, 1 H) 9.68 (br. s., 1 H).

### EXAMPLE 131

### 1-[2'-Piperazin-1-yl-6-(pyrazin-2-ylamino)-2,4'-bipyridin-4-yl]piperidine-4-Carboxylic acid

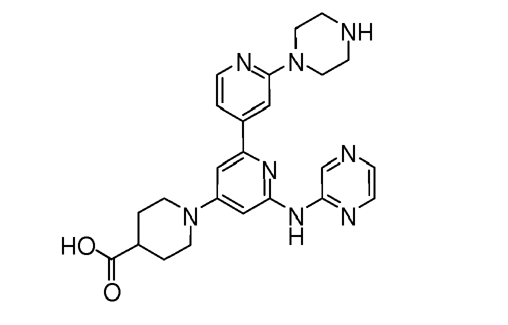

**a) Methyl 1-(2,6-dichloropyridin-4-yl)piperidine-4-Carboxylate**
   Obtained (23%) from 2,4,6-trichloropyridine methyl piperidine-4-Carboxylate following the procedure as described in preparation 24a.
   LRMS (m/z): 290 (M+1)⁺.
**b) *tert-*Butyl 4-{6-Chloro-4-[4-(methoxycarbonyl)piperidin-1-yl]-2,4'-bipyridin-2'-yl}piperazine-1-Carboxylate**
   Obtained (17%) from methyl 1-(2,6-dichloropyridin-4-yl)piperidine-4-Carboxylate (example 131a) and {2-[4-(tert-butoxycarbonyl)piperazin-1-yl]pyridin-4-yl}boronic acid (preparation 24b) following the experimental procedure as described in preparation 1, using palladium tetrakis(triphenylphosphine), potassium carbonate as base and tetrahydrofurane/water as solvent.
   LRMS (m/z): 517 (M+1)⁺.
**c) *tert*-Butyl 4-[4-[4-(methoxycarbonyl)piperidin-1-yl]-6-(pyrazin-2-ylamino)-2,4'-bipyridin-2'-yl]piperazine-1-Carboxylate**
   Obtained (50%) from tert-butyl 4-{6-Chloro-4-[4-(methoxycarbonyl)piperidin-1-yl]-2,4'-bipyridin-2'-yl}piperazine-1-Carboxylate (example 131b) and pyrazin-2-amine following the experimental procedure as described in preparation 4, using dioxane as solvent.
   LRMS (m/z): 575 (M+1)⁺.
**d) 1-[2'-Piperazin-1-yl-6-(pyrazin-2-ylamino)-2,4'-bipyridin-4-yl]piperidine-4-Carboxylic acid**
   Obtained as a trifluoroacetate salt from tert-butyl 4-[4-[4-(methoxycarbonyl)piperidin-1-yl]-6-(pyrazin-2-ylamino)-2,4'-bipyridin-2'-yl]piperazine-1-Carboxylate (example 131c), by hydrolysis of the methyl ester with lithium hydroxide in methanol, and then following the experimental procedure described in example 2b.
   LRMS (m/z): 461 (M+1)⁺.
   1 H NMR (250 MHz, DMSO-d₆) δ ppm 1.58 (q, 2 H) 1.91 (d, 2 H) 2.95-3.15 (m, 6 H) 3.70 (t, 4 H) 3.92 (d, 2 H) 7.06 (d, 2 H) 7.18 (d, 1 H) 7.25 (s, 1 H) 8.05 (d, 1 H) 8.22 (d, 2 H) 9.31 (s, 1 H) 9.65 (br. s., 1 H)

### EXAMPLE 132

### N⁴-Butyl-2'-piperazin-1-yl-N⁶-pyrazin-2-yl-2,4'-bipyridine-4,6-diamine

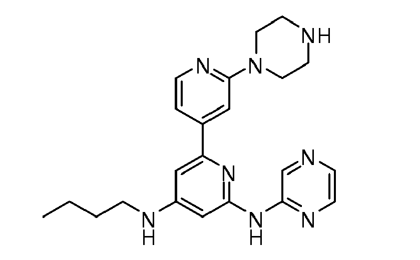

**a) *tert*-Butyl 4-[4-(butylamino)-6-(pyrazin-2-ylamino)-2,4'-bipyridin-2'-yl]piperazine-1-Carboxylate**
   To a solution of *tert-butyl* 4-[4-amino-6-(pyrazin-2-ylamino)-2,4'-bipyridin-2'-yl]piperazine-1-Carboxylate (preparation 26, 100 mg, 0.22 mmol) in methanol (8 mL), acetic acid (0.064 mL, 1.12 mmol) and butyraldehyde (0.030 mL, 0.33 mmol) were added and reaction stirred at room temperature overnight. Then sodium cyanoborohydride (9 mg, 0.14 mmol) was added and mixture stirred for 6 hour more. Solvent was removed and crude was purified by flash chromatography (hexanes/ethyl acetate 0% to 80% ethyl acetate) to yield the title compound (51 %) as a oil.
   LRMS (m/z): 505 (M+1)⁺.
**b) *N*⁴-Butyl-2'-piperazin-1-yl-*N*⁶-pyrazin-2-yl-2,4'-bipyridine-4,6-diamine**
   Obtained as hydrochloride salt (92%) from tert-butyl 4-[4-(butylamino)-6-(pyrazin-2-ylamino)-2,4'-bipyridin-2'-yl]piperazine-1-Carboxylate (example 132a) following the experimental procedure as described in example 73b.
   LRMS (m/z): 405 (M+1)⁺.
   1 H NMR (400 MHz, DMSO-*d*₆) δ ppm 0.94 (t, *J*=7.23 Hz, 3 H) 1.29 - 1.51 (m, 2 H) 1.51 - 1.83 (m, 2 H) 2.95 - 3.42 (m, 6 H) 3.96 (br. s., 4 H) 7.46 (br. s., 1 H) 8.11 - 8.48 (m, 3 H) 8.48 - 8.81 (m, 2 H) 9.55 (br. s., 2 H)

### EXAMPLE 133

### trans-4-{[2'-Piperazin-1-yl-6-(pyrazin-2-ylamino)-2,4'-bipyridin-4-yl]aminolcyclohexanecarboxylic acid

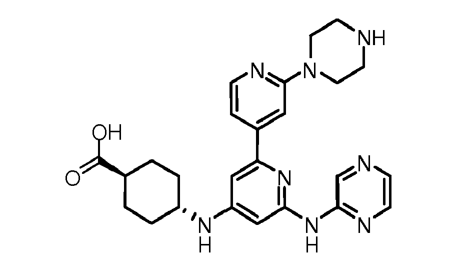

**a) Methyl *trans*-4-[(2,6-dichloropyridin-4-yl)amino]cyclohexanecarboxylate**
   A mixture of 2,4,6-trichloropyridine (2.22 g, 12.20 mmol), methyl *trans-4-*aminocyclohexanecarboxylate hydrochloride (2.6 g, 13.42 mmol) and cesium carbonate (6.74 g, 48.8 mmol) in DMF (30 mL) was stirred in a sealed tube at 150 °C. After 5.5 hours, the crude was poured on water (30 mL) and EtOAc (30 mL). The aqueous phase was extracted with EtOAc (3x20 mL). The combined organic phases were dried (Na₂SO₄), filtered and concentrated. Purification of the residue by flash chromatography (hexanes/ethyl acetate 15% to 30% ethyl acetate) gave the title compound (1.02 g, 28%) as a yellow solid.
   LRMS (m/z): 303/305 (M+1)⁺.
   1 H NMR (250 MHz, CHLOROFORM-*d*) δ ppm 1.24 (m, 4H), 1.6 (m, 2H), 2.11 (m, 2H), 2.31 (m, 1 H), 3.26 (m, 1 H), 3.69 (s, 3H), 4.32 (d, *J* = 7.3 Hz, 1 H), 6.34 (s, 2H)
**b) *tert*-Butyl 4-(6-Chloro-4-{[*trans*-4-(methoxycarbonyl)cyclohexyl]amino}-2,4'-bipyridin-2'-yl)piperazine-1-Carboxylate**
   A mixture of methyl *trans*-4-[(2,6-dichloropyridin-4-yl)amino]cyclohexanecarboxylate (example 133a, 0.25 g, 0.824 mmol), {2-[4-(*tert*-butoxycarbonyl)piperazin-1-yl]pyridin-4-yl}boronic acid (preparation 24b, 0.66 g, 0.989 mmol), potassium carbonate (0.227 g, 1.64 mmol) and tetrakis(triphenylphosphine)palladium(0) (0.047 g, 0.041 mmol) in THF/H₂O (11 mL, 10:1) was stirred in a sealed tube at 100 °C for 2 days. The residue was filtered through Celite, washed with MeOH, and concentrated. Purification of the residue by flash chromatography (hexanes/ethyl acetate 20% to 50% ethyl acetate) gave the title compound (0.12 g, 14%) as yellow oil.
   LRMS (m/z): 530/532 (M+1)⁺.
**c) *tert-*Butyl 4-[4-][*trans*-4-(methoxycarbonyl)cyclohexyl]amino}-6-(pyrazin-2-ylamino)-2,4'-bipyridin-2'-yl]piperazine-1-Carboxylate**
   Obtained (15%) from *tert-butyl* 4-(6-Chloro-4-{[trans-4-(methoxycarbonyl)cyclohexyl]amino}-2,4'-bipyridin-2'-yl)piperazine-1-Carboxylate (example 133b) and pyrazin-2-amine following the experimental procedure as described in preparation 4 using dioxane as a solvent, followed by purification of the crude product by flash chromatography (98:2 dichloromethane /methanol to 95:5 dichloromethane /methanol).
   LRMS (m/z): 589 (M+1)⁺.
**d) *trans*-4-{[2'-[4-(*tert*-Butoxycarbonyl)piperazin-1-yl]-6-(pyrazin-2-ylamino)-2,4'-bipyridin-4-yl]amino}cyclohexanecarboxylic acid**
   Obtained from tert-butyl 4-[4-{[trans-4-(methoxycarbonyl)cyclohexyl]amino}-6-(pyrazin-2-ylamino)-2,4'-bipyridin-2'-yl]piperazine-1-Carboxylate (example 133c) following the experimental procedure as described in example 74b.
   LRMS (m/z): 575 (M+1)⁺.
**e) *trans*-4-{[2'-Piperazin-1-yl-6-(pyrazin-2-ylamino)-2,4'-bipyridin-4-yl]amino}cyclohexanecarboxylic acid**
   Obtained as a solid (84%) from trans-4-1[2'-[4-(tert-butoxycarbonyl)piperazin-1-yl]-6-(pyrazin-2-ylamino)-2,4'-bipyridin-4-yl]amino}cyclohexanecarboxylic acid (example 133d) following the experimental procedure as described in example 2b.
   LRMS (m/z): 475 (M+1)⁺.
   1 H NMR (400 MHz, DMSO-d₆) δ ppm 1.26 (m, 3H), 1.52 (m, 2H), 2.02 (m, 4H), 2.19 (m, 1 H), 2.83 (m, 4H), 3.48 (m, 4H), 6.25 (d, J = 7.6 Hz, 1 H), 6.79 (d, J = 1.8 Hz, 1 H), 6.81 (d, J = 1.8 Hz, 1 H), 7.11 (dd, J = 5.3 Hz, J = 1.2 Hz, 1 H), 7.29 (s, 1 H), 8.03 (d, J = 2.3 Hz, 1 H), 8.18 (m, 2H), 9.24 (d, *J* = 1.8 Hz, 1 H), 9.32 (s, 1 H)

### EXAMPLE 134

### 4-{[2'-Piperazin-1-yl-6-(pyrazin-2-ylamino)-2,4'-bipyridin-4-yl]amino}cyclohexanol

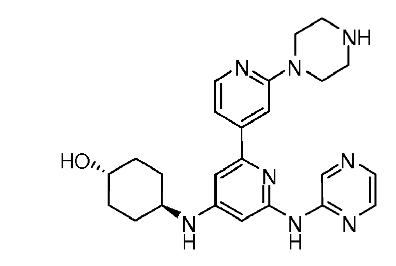

**a) *trans*-4-[(2,6-Dichloropyridin-4-yl)amino]cyclohexanol**
   Obtained (25%) from 2,4,6-trichloropyridine and trans-4-aminocyclohexanol hydrochloride following the experimental procedure as described in example 133a.
   LRMS (m/z): 261/263 (M+1)⁺.
   1 H NMR (250 MHz, CHLOROFORM-d) δ ppm 1.67-1.32 (m, 4H), 2.07 (m, 4H), 3.27 (m, 1 H), 3.68 (m, 1H), 4.31 (d, *J* = 6.8 Hz, 1 H), 6.34 (s, 2H)
**b) *tert-*Butyl 4-{6-Chloro-4-[(trans-4-hydroxycyclohexyl)amino]-2,4'-bipyridin-2'-yl}piperazine-1-Carboxylate**
   Obtained (20%) from trans-4-[(2,6-dichloropyridin-4-yl)amino]cyclohexanol (example 134a) and {2-[4-(tert-butoxycarbonyl)piperazin-1-yl]pyridin-4-yl}boronic acid (preparation 24b) following the experimental procedure as described in example 133b.
   LRMS (m/z): 488/2490 (M+1)⁺.
**c) *tert*-Butyl 4-[4-[(*trans*-4-hydroxycyclohexyl)amino]-6-(pyrazin-2-ylamino)-2,4'-bipyridin-2'-yl]piperazine-1-Carboxylate**
   Obtained (50%) from tert-butyl 4-{6-Chloro-4-[(trans-4-hydroxycyclohexyl)amino]-2,4'-bipyridin-2'-yl}piperazine-1-Carboxylate (example 134b) and pyrazin-2-amine following the experimental procedure as described in preparation 4 using dioxane as a solvent, followed by purification of the crude product by flash chromatography (99:1 dichloromethane /methanol to 90:10 dichloromethane /methanol).
   LRMS (m/z): 547 (M+1)⁺.
**d) 4-{[2'-Piperazin-1-yl-6-(pyrazin-2-ylami no)-2,4'-bipyridin-4-yl]amino}cyclohexanol**
   Obtained as a solid (37%) from tert-butyl 4-[4-[(trans-4-hydroxycyclohexyl)amino]-6-(pyrazin-2-ylamino)-2,4'-bipyridin-2'-yl]piperazine-1 -Carboxylate (example 134c) following the experimental procedure as described in example 2b, followed by purification of the crude product by flash chromatography (dichloromethane /methanol/ aqueous ammonia).
   LRMS (m/z): 447 (M+1)⁺.
   1 H NMR (400 MHz, DMSO-d₆) δ ppm 1.27 (m, 4H), 1.90 (m, 4H), 2.60 (m,1 H), 2.95 (m, 1 H), 3.62-3.20 (m, 8H), 6.50 (d, J = 7.4 Hz, 1 H), 6.78 (m, 2H), 7.14 (d, J = 4.7 Hz, 1 H), 7.34 (s, 1 H), 8.04 (sa, 1 H), 8.20 (m, 2H), 9.29 (s, 1 H), 9.63 (s, 1 H)

### EXAMPLE 135

### N⁴ -(3-Phenylpropyl)-2'-piperazin-1-yl-N⁶-pyrazin-2-yl-2,4'-bipyridine-4,6-diamine

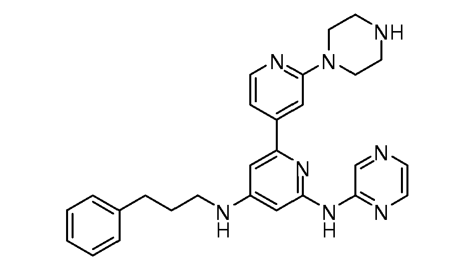

**a) *tert*-Butyl 4-[4-[(3-phenylpropyl)amino]-6-(pyrazin-2-ylamino)-2,4'-bipyridin-2'-yl]piperazine-1-Carboxylate**
   Obtained (62%) from tert-butyl 4-[4-amino-6-(pyrazin-2-ylamino)-2,4'-bipyridin-2'-yl]piperazine-1-Carboxylate (preparation 26) and 3-phenylpropanal following the procedure as described in example 126a.
   LRMS (m/z): 567 (M+1 )⁺.
**b) *N*⁴-(3-Phenylpropyl)-2'-piperazin-1-yl-*N*⁶-pyrazin-2-yl-2,4'-bipyridine-4,6-diamine**
   Obtained as hydrochloride salt (99%) from tert-butyl 4-[4-[(3-phenylpropyl)amino]-6-(pyrazin-2-ylamino)-2,4'-bipyridin-2'-yl]piperazine-1-Carboxylate (example 135a) following the experimental procedure as described in example 73b.
   LRMS (m/z): 467 (M+1)⁺.
   1 H NMR (400 MHz, DMSO-d₆) δ ppm 1.93 (t, 2 H) 2.73 (t, J=7.62 Hz, 2 H) 3.22 (br. s., 4 H) 3.27 - 3.47 (m, 2 H) 3.96 (br. s., 4 H) 7.16 - 7.23 (m, 1 H) 7.24 - 7.34 (m, 5 H) 7.48 (br. s., 1 H) 8.33 (d, J=2.34 Hz, 1 H) 8.36 - 8.40 (m, 2 H) 8.58 (d, J=0.78 Hz, 1 H) 8.78 (br. s., 1 H) 9.57 (br. s., 2 H)

### EXAMPLE 136

### 3-Benzyl-4-{[2'-piperazin-1-yl-6-(pyrazin-2-ylamino)-2,4'-bipyridin-4-yl]amino}butanoic acid

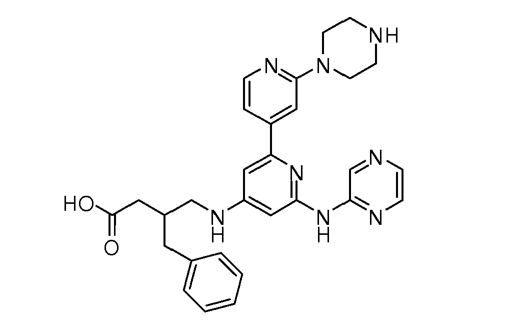

**a) *tert*-Butyl 4-[4-[(2-benzyl-4-methoxy-4-oxobutyl)amino]-6-(pyrazin-2-ylamino)-2,4'-bipyridin-2'-yl]piperazine-1-Carboxylate**
   Obtained (21%) from tert-butyl 4-[4-amino-6-(pyrazin-2-ylamino)-2,4'-bipyridin-2'-yl]piperazine-1-Carboxylate (preparation 26) and methyl 3-benzyl-4-oxobutanoate following the procedure as described in example 132a.
   LRMS (m/z): 639 (M+1)⁺.
**b) 3-Benzyl-4-{[2'-[4-(*tert*-butoxycarbonyl)piperazin-1-yl]-6-(pyrazin-2-ylamino)-2,4'-bipyridin-4-yl]amino}butanoic acid**
   Obtained (36%) tert-butyl 4-[4-[(2-benzyl-4-methoxy-4-oxobutyl)amino]-6-(pyrazin-2-ylamino)-2,4'-bipyridin-2'-yl]piperazine-1-Carboxylate (example 136a) following the experimental procedure as described in example 108b.
   LRMS (m/z): 625 (M+1)⁺.
**c) 3-Benzyl-4-{[2'-piperazin-1-yl-6-(pyrazin-2-ylamino)-2,4'-bipyridin-4-yl]aminolbutanoic acid**
   Obtained as hydrochloride salt (47%) from 3-benzyl-4-{[2'-[4-(*tert* butoxycarbonyl)piperazin-1-yl]-6-(pyrazin-2-ylamino)-2,4'-bipyridin-4-yl]amino}butanoic acid (example 136b) following the experimental procedure as described in example 73b.
   LRMS (m/z): 525 (M+1)⁺.
   1 H NMR (400 MHz, DMSO-d₆) δpm 2.26 - 2.35 (m, 3 H) 2.54 - 2.69 (m, 1 H) 2.81 (dd, J=13.87, 5.67 Hz, 1 H) 3.19 - 3.26 (m, 6 H) 3.88 - 3.93 (m, 4 H) 7.05 (s, 1 H) 7.17 - 7.25 (m, 5 H) 7.28 - 7.33 (m, 3 H) 7.40 (s, 1 H) 7.66 - 7.74 (m, 1 H) 8.31 - 8.43 (m, 3 H) 8.58 (s, 1 H) 9.24 (s, 2 H)

### EXAMPLE 137

### 4-{[2'-Piperazin-1-yl-6-(pyrazin-2-ylamino)-2,4'-bipyridin-4-yl]amino}butanoic acid

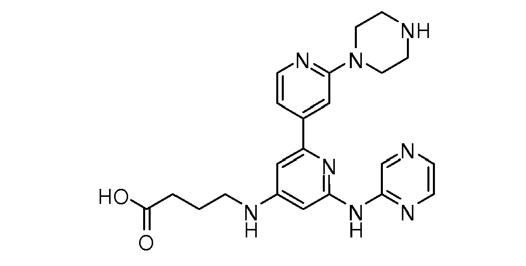

**a) *tert*-Butyl 4-[4-[(4-methoxy-4-oxobutyl)amino]-6-(pyrazin-2-ylamino)-2,4'-bipyridin-2'-yl]piperazine-1-Carboxylate**
   Obtained (46%) from tert-butyl 4-[4-amino-6-(pyrazin-2-ylamino)-2,4'-bipyridin-2'-yl]piperazine-1-Carboxylate (preparation 26) and methyl 4-oxobutanoate following the procedure as described in example 132a.
   LRMS (m/z): 549 (M+1)⁺.
**b) 4-{[2'-[4-(*tert*-Butoxycarbonyl)piperazin-1-yl]-6-(pyrazin-2-ylamino)-2,4'-bipyridin-4-yl]aminolbutanoic acid**
   Obtained (76%) tert-butyl 4-[4-[(4-methoxy-4-oxobutyl)amino]-6-(pyrazin-2-ylamino)-2,4'-bipyridin-2'-yl]piperazine-1-Carboxylate (example 137a) following the experimental procedure as described in example 108b.
   LRMS (m/z): 535 (M+1)⁺.
**c) 4-{[2'-Piperazin-1-y)-6-(pyrazin-2-y)amino)-2,4'-bipyridin-4-y)]amino}butanoic acid**
   Obtained as hydrochloride salt (41 %) from 4-1[2'-[4-(tert-butoxycarbonyl)piperazin-1-yl]-6-(pyrazin-2-ylamino)-2,4'-bipyridin-4-yl]amino}butanoic acid (example 137b) following the experimental procedure as described in example 73b.
   LRMS (m/z): 425 (M+1)⁺.
   1 H NMR (400 MHz, DMSO-d₆) δpm 1.80 (quin, J=7.03 Hz, 2 H) 2.32 (t, J=7.23 Hz, 2 H) 2.81 (t, 4 H) 3.14 (br. s., 2 H) 3.47 (t, 4 H) 6.74 (br. s., 1 H) 6.80 (d, J=1.56 Hz, 1 H) 6.83 (s, 1 H) 7.15 (d, J=5.47 Hz, 1 H) 7.33 (s, 1 H) 8.03 (d, J=2.34 Hz, 1 H) 8.18 (d, J=5.08 Hz, 1 H) 8.20 (dd, J=2.74, 1.56 Hz, 1 H) 9.23 (s, 1 H) 9.63 (s, 1 H)

### EXAMPLE 138

### 5'-(Piperazin-1-yl)-M-(pyrazin-2-yl)-2,3'-bipyridine-4,6-diamine

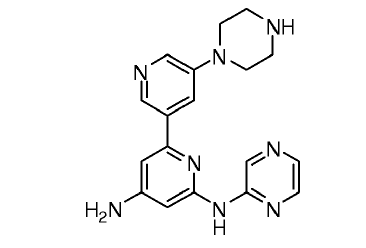

**a) *tert*-Butyl 4-(4-nitro-6-(pyrazin-2-ylamino)-2,3'-bipyridin-5'-yl)piperazine-1-Carboxylate**
   Obtained (86%) from N-(6-Chloro-4-nitropyridin-2-yl)pyrazin-2-amine (preparation 26a) and *tert*-butyl 4-(5-(4,4,5,5-tetramethyl-1,3,2-dioxaborolan-2-yl)pyridin-3-yl)piperazine-1-Carboxylate (preparation 31 b) following the experimental procedure as described in preparation 1, using cesium carbonate as base and dioxane as solvent.
   LRMS (m/z): 479 (M+1)⁺.
**b) *tert*-Butyl 4-(4-amino-6-(pyrazin-2-ylamino)-2,3'-bipyridin-5'-yl)piperazine-1-Carboxylate**
   Obtained as a solid (62%) from tert-butyl 4-(4-nitro-6-(pyrazin-2-ylamino)-2,3'-bipyridin-5'-yl)piperazine-1-Carboxylate (example 138a) following the experimental procedure as described in preparation 22c, followed by purification by reverse phase chromatography (0% CH₃OH in H₂O to 100% CH₃OH in H₂O)
   LRMS (m/z): 449 (M+1)⁺.
**c) 5'-(Piperazin-1-yl)-*N*⁶-(pyrazin-2-yl)-2,3'-bipyridine-4,6-diamine**
   Obtained as a solid (34%) from tert-butyl 4-(4-amino-6-(pyrazin-2-ylamino)-2,3'-bipyridin-5'-yl)piperazine-1-Carboxylate (example 138b) following the experimental procedure as described in example 73b.
   LRMS (m/z): 349 (M+1)⁺.
   1 H NMR (400 MHz, DMSO-d₆) δ ppm 3.27 (br. s., 4 H) 3.64 (t, 4 H) 6.89 (d, J=1.95 Hz, 1 H) 6.93 (d, J=1.56 Hz, 1 H) 8.31 (d, J=0.78 Hz, 1 H) 8.35 (d, J=2.74 Hz, 1 H) 8.49 (br. s., 1 H) 8.61 (d, J=2.74 Hz, 2 H) 9.25 (br. s., 2 H)

### EXAMPLE 139

### 3-]3-[4-Amino-6-(pyrazin-2-ylamino)pyridin-2-yl]phenyl}propanoic acid

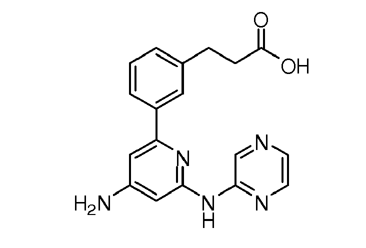

**a) *tert*-Butyl 3-{3-[4-nitro-6-(pyrazin-2-ylamino)pyridin-2-yl]phenyl}propanoate** Obtained (54%) from N-(6-Chloro-4-nitropyridin-2-yl)pyrazin-2-amine (preparation 26a) and *tert*-butyl 3-[3-(4,4,5,5-tetramethyl-1,3,2-dioxaborolan-2-yl)phenyl]propanoate (preparation 33) following the experimental procedure as described in preparation 1 using dioxane as reaction solvent.
   LRMS (m/z): 422 (M+1 )⁺.
**b) *tert*-Butyl 3-]3-[4-amino-6-(pyrazin-2-ylamino)pyridin-2-yl]phenyl}propanoate**
   To a solution of *tert-butyl* 3-13-[4-nitro-6-(pyrazin-2-ylamino)pyridin-2-yl]phenyllpropanoate (example 139a, 0.10 g, 0.24 mmol) in methanol (15 mL) was added palladium on charcoal (0.05 mg, 0.04 mmol). The reaction mixture was hydrogenated at 50 psi for 48 hours. The catalyst was filtered through Celite® and the solvent removed under reduced pressure giving a crude, which was purified by reverse phase to give the title compound (21 mg, 29%) as a oil.
   LRMS (m/z): 392 (M+1)⁺.
**c) 3-{3-[4-Amino-6-(pyrazin-2-ylamino)pyridin-2-yl]phenyl}propanoic acid**
   Obtained (96%) from *tert*-butyl 3-13-[4-amino-6-(pyrazin-2-ylamino)pyridin-2-yl]phenyllpropanoate (example 139b) following the experimental procedure as described in example 73b.
   LRMS (m/z): 336 (M+1)⁺.
   1 H NMR (400 MHz, DMSO-d₆) δpm 2.66 (t, J=7.42 Hz, 2 H) 2.96 (t, J=7.23 Hz,2H)6.72(s,1 H) 6.87(s,1 1 H) 7.48 - 7.62 (m, 2 H) 7.66 (d, J₌7.42 Hz, 1 H) 7.71 (s, 1 H) 8.34 (d, J=1.95 Hz, 1 H) 8.41 (br. s., 1 H) 8.60 (s, 1 H) 11.98 (s, 1 H)

### EXAMPLE 140

### 3-]3-[4-Amino-6-(pyrazin-2-ylamino)pyridin-2-yl]phenyl}propanamide

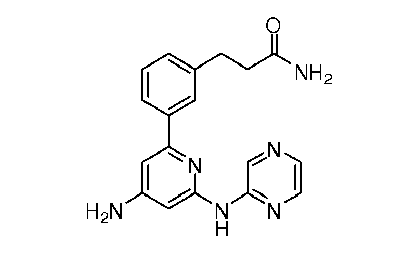

**a) 3-{3-[4-Nitro-6-(pyrazin-2-ylamino)pyridin-2-yl]phenyl}propanoic acid**
   Obtained (97%) from *tert-butyl* 3-{3-[4-nitro-6-(pyrazin-2-ylamino)pyridin-2-yl]phenyllpropanoate (example 139a) following the experimental procedure as described in example 73b.
   LRMS (m/z): 366 (M+1)⁺.
**b) 3-(3-[4-Nitro-6-(pyrazin-2-ylamino)pyridin-2-yl]phenyl}propanamide**
   1-(3-Dimethyllaminopropyl)-3-ethylcarbodiimide hydrochloride (0.29 g, 1.54 mmol),
   1H-benzo[d][1,2,3]triazol-1-ol hydrate (0.10 g, 0.70 mmol) and 32% aqueous ammonium hydroxide solution (1.04 mL, 8.45 mmol) were sequentially added to a stirred solution of 3-{3-[4-nitro-6-(pyrazin-2-ylamino)pyridin-2-yl]phenyl}propanoic acid (example 140a, 0.51 g, 1.40 mmol) in 1-methylpyrrolidin-2-one (12 mL) at room temperature. After stirring overnight, water and ethyl acetate were added to the mixture. The organic layer was washed with 8N aqueous sodium hydroxide solution, brine, dried (MgSO₄) and evaporated. Purification of the residue by reverse phase chromatography (0% ACN/CH₃OH 1:1 in H₂O to 100% ACN/CH₃OH 1:1 in H₂O gave the title compound (110 mg, 21 %) as a yellow solid.
   LRMS (m/z): 365 (M+1)⁺.
**c) 3-(3-[4-Amino-6-(pyrazin-2-ylamino)pyridin-2-yl]phenyl}propanamide**
   Obtained as a hydrochloride salt (37%) from 3-13-[4-nitro-6-(pyrazin-2-ylamino)pyridin-2-yl]phenyllpropanamide (example 140b) following the experimental procedure as described in preparation 26c heating at 80°C for 3 days, followed by addition of 4M hydrochloric acid in dioxane. The solution was stirred at room temperature for 30 minutes. The resulting solid was filtered and dried to give the title compound.
   LRMS (m/z): 335 (M+1)⁺.
   1 H NMR (400 MHz, DMSO-d₆) δ ppm 2.66 (t, J=7.42 Hz, 2 H) 2.96 (t, J=7.23 Hz,2H)6.72(s,1 H)6.87(s,1 1 H) 7.47 - 7.61 (m, 2 H) 7.66 (d, *J=7.42* Hz, 1 H) 7.71 (s, 1 H) 7.78 (br. s., 1 H) 8.06 (br. s., 1 H) 8.34 (d, J=1.95 Hz, 1 H) 8.41 (s, 1 H) 8.60 (s, 1 H) 11.98 (s, 1 H) 14.02 (s, 1 H)

### EXAMPLE 141

### (3S)-4-Phenyl-3-(2'-(piperazin-1-yl)-6-(pyrazin-2-ylamino)-2,4'-bipyridine-4-Carboxamido)butanoic acid

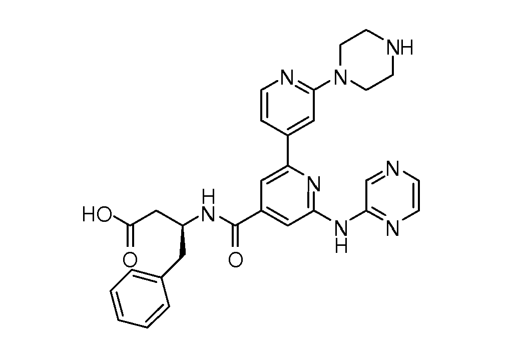

**a) *tert*-Butyl 2'-(4-(tert-butoxycarbonyl)piperazin-1-yl)-6-(pyrazin-2-ylamino)-2,4'-bipyridine-4-Carboxylate**
   Obtained (64%) from tert-butyl 2-Chloro-6-(pyrazin-2-ylamino)isonicotinate (preparation 32b) and {2-[4-( tert-butoxycarbonyl)piperazin-1-yl]pyridin-4-yl}boronic acid (preparation 24b) following the experimental procedure as described in preparation 1, using cesium carbonate as base and dioxane as solvent, followed by purification by flash chromatography (hexanes/ethyl acetate 0% to 40% ethyl acetate).
   LRMS (m/z): 479 (M+1)⁺.
**b) 2'-(4-(*tert*-Butoxycarbonyl)piperazin-1-yl)-6-(pyrazin-2-ylamino)-2,4'-bipyridine-4-Carboxylic acid**
   Obtained (87%) from *tert*-butyl 2'-(4-(tert-butoxycarbonyl)piperazin-1-yl)-6-(pyrazin-2-ylamino)-2,4'-bipyridine-4-Carboxylate (example 141 a) following the experimental procedure as described in example 109b.
   LRMS (m/z): 478 (M+1)⁺.
**c) *tert*-Butyl 4-[4-({[(1*S*)-1-benzyl-3-*tert*-butoxy-3-oxopropyl]amino}carbonyl)-6-(pyrazin-2-ylamino)-2,4'-bipyridin-2'-yl]piperazine-1-Carboxylate**
   Obtained as a solid (97%) from 2'-(4-(tert-butoxycarbonyl)piperazin-1-yl)-6-(pyrazin-2-ylamino)-2,4'-bipyridine-4-Carboxylic acid (example 141 b) and (S)-tert-butyl 3-amino-4-phenylbutanoate following the experimental procedure as described in example 105a.
   LRMS (m/z): 695 (M+1)⁺.
**d) (3S)-4-Phenyl-3-(2'-(piperazin-1-yl)-6-(pyrazin-2-ylamino)-2,4'-bipyridine-4-Carboxamido)butanoic acid**
   Obtained as a solid (84%) from *tert-*butyl 4-[4-({[(1*S*)-1-benzyl-3-*tert*-butoxy-3-oxopropyl]amino}carbonyl)-6-(pyrazin-2-ylamino)-2,4'-bipyridin-2'-yl]piperazine-1-Carboxylate (example 141c) following the experimental procedure as described in example 73b.
   LRMS (m/z): 539 (M+1)⁺.
   1 H NMR (400 MHz, DMSO-*d₆*) δ ppm 2.53 - 2.59 (m, 1 H) 2.60 - 2.71 (m, 1 H) 2.79 - 2.94 (m, 1 H) 2.96 - 3.06 (m, 1 H) 3.28 (br. s., 4 H) 3.57 (br. s., 4 H) 4.45 - 4.65 (m, 1 H) 7.18 (br. s., 1 H) 7.29 (br. s., 3 H) 7.56 (d, *J*=5.47 Hz, 1 H) 7.91 (br. s., 1 H) 8.07 (s, 1 H) 8.18 (d, *J*=1.17 Hz, 1 H) 8.27 - 8.36 (m, 1 H) 9.08 (d, *J*=7.42 Hz, 1 H) 9.17 (s, 1H) 9.33 (br.s., 1H) 10.43 (s, 1H)

### EXAMPLE 142

### 2'-Piperazin-1-yl-6-(pyrazin-2-ylamino)-N-pyridin-3-yl-2,4'-bipyridine-4-Carboxamide

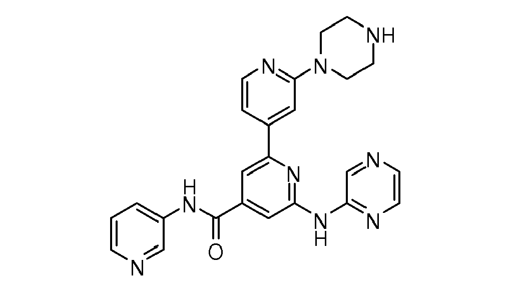

**a) *tert-*Butyl 4-{6-(pyrazin-2-ylamino)-4-[(pyridin-3-ylamino)carbonyl]-2,4'-bipyridin-2'-yl}piperazine-1-Carboxylate**
   Obtained as a solid (87%) from 2'-(4-(*tert*-butoxycarbonyl)piperazin-1-yl)-6-(pyrazin-2-ylamino)-2,4'-bipyridine-4-Carboxylic acid (example 141b) and pyridin-3-amine following the experimental procedure as described in example 105a.
   LRMS (m/z): 695 (M+1)⁺.
**b) 2'-Piperazin-1-yl-6-(pyrazin-2-ylamino)-*N*-pyridin-3-yl-2,4'-bipyridine-4-Carboxamide**
   Obtained as hydrochloride salt (90%) from *tert-*butyl 4-{6-(pyrazin-2-ylamino)-4-[(pyridin-3-ylamino)carbonyl]-2,4'-bipyridin-2'-yl}piperazine-1-Carboxylate (example 142a) following the experimental procedure as described in example 73b.
   LRMS (m/z): 454 (M+1)⁺.
   1 H NMR (400 MHz, DMSO-*d*₆) δ ppm 3.28 (br. s., 4 H) 4.11 (br. s., 4 H) 7.68 (d, *J*=3.91 Hz, 1 H) 8.03 (d, *J*=4.69 Hz, 1 H) 8.20 (br. s., 2 H) 8.28 - 8.43 (m, 2 H) 8.69 (d, *J*=3.91 Hz, 1 H) 8.85 (br. s., 1 H) 9.11 (d, *J*=7.82 Hz, 1 H) 9.18 (br. s., 1 H) 9.41 (br. s., 2 H) 9.59 (br. s., 1 H)

### EXAMPLE 143

### N-{[2'-Piperazin-1-yl-6-(pyrazin-2-ylamino)-2,4'-bipyridin-4-yl]carbonyl}-L-phenylalanine

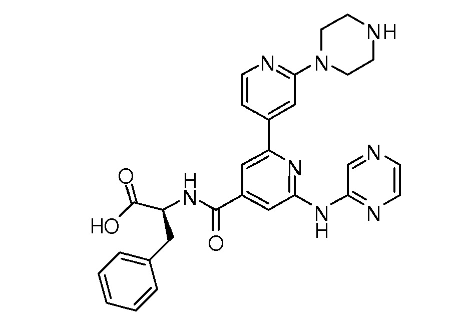

**a) Ethyl *N*-(2,6-dichloroisonicotinoyl)-L-phenylalaninate**
   Obtained as a solid (80%) from 2,6-dichloroisonicotinic acid and ethyl L-phenylalaninate following the experimental procedure as described in example 105a.
   LRMS (m/z): 368 (M+1)⁺.
**b) Ethyl *N-*[2-Chloro-6-(pyrazin-2-ylamino)isonicotinoyl]-L-phenylalaninate**
   Obtained as a solid (75%) from ethyl *N*-(2,6-dichloroisonicotinoyl)-L-phenylalaninate (example 143a) and pyrazin-2-amine following the experimental procedure as described in preparation 4, using cesium carbonate as base and dioxane as solvent.
   LRMS (m/z): 426 (M+1)⁺.
**c) Ethyl *N*-{[2'-[4-(*tert*-butoxycarbonyl)piperazin-1-yl]-6-(pyrazin-2-ylamino)-2,4'-bipyridin-4-yl]carbonyl}-L-phenylalaninate**
   Obtained (21 %) from ethyl N-[2-Chloro-6-(pyrazin-2-ylamino)isonicotinoyl]-L-phenylalaninate (example 143b) and {2-[4-(*tert*-butoxycarbonyl)piperazin-1-yl]pyridin-4-yl}boronic acid (preparation 24b) following the experimental procedure as described in preparation 1, using cesium carbonate as base and dioxane as solvent, followed by purification by flash chromatography (hexanes/ethyl acetate 0% to 80% ethyl acetate).
   LRMS (m/z): 653 (M+1)⁺.
**d) *N*-{[2'-[4-(*tert*-Butoxycarbonyl)piperazin-1-yl]-6-(pyrazin-2-ylamino)-2,4'-bipyridin-4-yl]carbonyl}-L-phenylalanine**
   Obtained (72%) from ethyl *N*-{[2'-[4-(*tert*-butoxycarbonyl)piperazin-1-yl]-6-(pyrazin-2-ylamino)-2,4'-bipyridin-4-yl]carbonyl}-L-phenylalaninate (example 143c) following the experimental procedure as described in example 108b, them the crude was acidified with acetic acid and extracted with ethyl acetate.
   LRMS (m/z): 625 (M+1)⁺.
**e) *N*-{[2'-Piperazin-1-yl-6-(pyrazin-2-ylamino)-2,4'-bipyridin-4-yl]carbonyl}-L-phenylalanine**
   Obtained as hydrochloride salt (100%) from N-{[2'-[4-(*tert*-butoxycarbonyl)piperazin-1-yl]-6-(pyrazin-2-ylamino)-2,4'-bipyridin-4-yl]carbonyl}-L-phenylalanine (example 143d) following the experimental procedure as described in example 73b.
   LRMS (m/z): 525 (M+1)⁺.
   1 H NMR (400 MHz, DMSO-*d*₆) δ ppm 3.18 - 3.24 (m, 2 H) 3.29 (s, 4 H) 4.06 (s, 4 H) 4.62 - 4.72 (m, 1 H) 7.19 (t, *J*=7.23 Hz, 1 H) 7.28 (t, *J*=7.42 Hz, 2 H) 7.39 (d, *J*=7.03 Hz, 2 H) 7.59 (d, *J*=5.86 Hz, 1 H) 7.95 (s, 1 H) 8.11 (s, 1 H) 8.18 (d, *J*=2.34 Hz, 2 H) 8.26 - 8.35 (m, 2 H) 9.15 (s, 1 H) 9.37 (d, *J*=7.81 Hz, 1 H) 9.46 (s, 2 H) 10.47 (s, 1 H)

### EXAMPLE 144

### (3S)-3-Benzyl-4-oxo-4-({[2'-piperazin-1-yl-6-(pyrazin-2-ylamino)-2,4'-bipyridin-4-yl]methyl}amino)butanoic acid

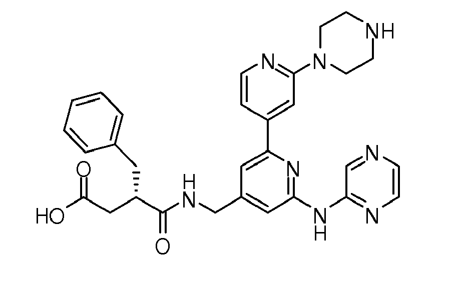

**a) 2-Chloro-6-(pyrazin-2-ylamino)isonicotinonitrile**
   Obtained (56%) from 2,6-dichloroisonicotinonitrile and pyrazin-2-amine following the experimental procedure as described in preparation 4 using dioxane as a solvent.
   LRMS (m/z): 232(M+1)⁺.
**b) *tert*-Butyl 4-[4-Cyano-6-(pyrazin-2-ylamino)-2,4'-bipyridin-2'-yl]piperazine-1-Carboxylate**
   Obtained (58%) from 2-Chloro-6-(pyrazin-2-ylamino)isonicotinonitrile (example 144a) and {2-[4-(*tert-*butoxycarbonyl)piperazin-1-yl]pyridin-4-yl}boronic acid (preparation 24) following the experimental procedure as described in preparation 1, using cesium carbonate as base and dioxane as solvent, followed by purification by flash chromatography (hexanes/ethyl acetate 30% to 100% ethyl acetate).
   LRMS (m/z): 459 (M+1)⁺.
**c) *tert*-Butyl 4-[4-(aminomethyl)-6-(pyrazin-2-ylamino)-2,4'-bipyridin-2'-yl]piperazine-1-Carboxylate**
   *tert*-Butyl 4-[4-Cyano-6-(pyrazin-2-ylamino)-2,4'-bipyridin-2'-yl]piperazine-1-Carboxylate (example 144b, 481 mg, 1.05 mmol) in tetrahydrofuran (6 mL) was added dropwise to a stirred suspension of lithium aluminium hydride (79 mg, 2.08 mmol) in tetrahydrofuran (4 mL). The mixture was stirred overnight at room temperature, then n H₂O (79 µL), n NaOH 2N and 3n H₂O were added dropwise to the reaction mixture. The mixture was stirred 10 minutes at room temperature, filtered through Celite® and the solvent was removed in vacuo. The residue was dissolved in ethyl acetate and water. The organic layer was separated, washed with brine, dried (MgSO₄) and evaporated to give the title compound (359 mg, 67%) as brown oil.
   LRMS (m/z): 463 (M+1)⁺.
**d) (3*S*)-3-Benzyl-4-({[2'-[4-(*tert*-butoxycarbonyl)piperazin-1-yl]-6-(pyrazin-2-ylamino)-2,4'-bipyridin-4-yl]methyl}amino)-4-oxobutanoic acid**
   Obtained (67%) from *tert*-butyl 4-[4-(aminomethyl)-6-(pyrazin-2-ylamino)-2,4'-bipyridin-2'-yl]piperazine-1-Carboxylate (example 144c) and (2*S*)-2-benzyl-4-*tert*-butoxy-4-oxobutanoic acid following the experimental procedure as described in example 73a using triethylamine instead ethyl(diisopropyl)amine, followed by purification by reverse phase chromatography (0% CH₃OH in H₂O to 100% CH₃OH in H₂O).
   LRMS (m/z): 653 (M+1)⁺.
**e) (3*S*)-3-Benzyl-4-oxo-4-({[2'-piperazin-1-yl-6-(pyrazin-2-ylamino)-2,4'-bipyridin-4-yl]methyl}amino)butanoic acid**
   Obtained as hydrochloride salt (85%) from (3*S*)-3-benzyl-4-({[2'-[4-(*tert-*butoxycarbonyl)piperazin-1-yl]-6-(pyrazin-2-ylamino)-2,4'-bipyridin-4-yl]methyl}amino)-4-oxobutanoic acid (example 144d) following the experimental procedure as described in example 73b.
   LRMS (m/z): 553 (M+1)⁺.
   1 H NMR (400 MHz, DMSO-*d*₆) δ ppm 2.18 (dd, *J*=16.61, 3.32 Hz, 1 H) 2.55 - 2.71 (m, 2 H) 2.93 - 3.10 (m, 2 H) 3.29 (br. s., 4 H) 4.06 (br. s., 4 H) 4.24 - 4.45 (m, 2 H) 7.13 - 7.19 (m, 1 H) 7.24 (d, *J*=2.34 Hz, 4 H) 7.59 (d, *J*=3.13 Hz, 1 H) 7.68 (d, *J*=13.68 Hz, 1 H) 7.80 - 7.85 (m, 1 H) 8.14 (d, *J*=2.74 Hz, 1 H) 8.26 (dd, *J*=2.54, 1.37 Hz, 1 H) 8.28 (d, *J*=6.25 Hz, 1 H) 8.68 - 8.83 (m, 1 H) 9.16 (s, 1 H) 9.59 (br. s., 2 H) 10.26-10.38 (m, 1 H)

### EXAMPLE 145

### 3-(4-{[2'-Piperazin-1-yl-6-(pyrazin-2-ylamino)-2,4'-bipyridin-4-yl]methoxy}phenyl)propanoic acid

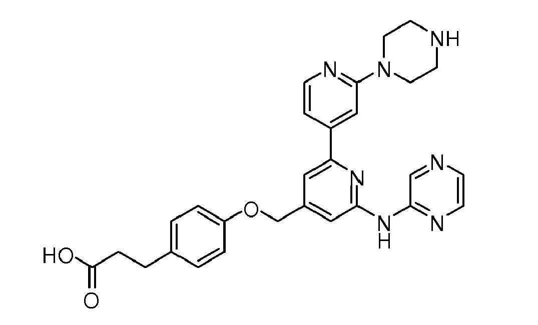

**a) Methyl 2,6-dichloroisonicotinate**
   Sulfuric acid (3 mL, 56.28 mmol) was added dropwise to a stirred solution of 2,6-dichloroisonicotinic acid (6.60 g, 34.38 mmol) in methanol (100 mL), the reaction mixture was heated under reflux. After 16 hours, the reaction mixture was slow cooled, and the solvent was removed in vacuo. Ethyl acetate and satured aqueous sodium carbonate solution were added to the crude. The organic layer was washed with brine, dried (Na₂SO₄), concentrated in vacuo and the resulting oil was used without further purification.
   LRMS (m/z): 207 (M+1)⁺.
**b) Methyl 2-Chloro-6-(pyrazin-2-ylamino)isonicotinate**
   Obtained (16%) from methyl 2,6-dichloroisonicotinate (example 145a) and pyrazin-2-amine following the experimental procedure as described in preparation 4 using dioxane as a solvent, followed by purification of the crude product by flash chromatography (hexanes/ethyl acetate 0% to 100% ethyl acetate).
   LRMS (m/z): 265 (M+1)⁺.
**c) Methyl 2'-[4-(*tert*-butoxycarbonyl)piperazin-1-yl]-6-(pyrazin-2-ylamino)-2,4'-bipyridine-4-Carboxylate**
   Obtained (42%) from methyl 2-Chloro-6-(pyrazin-2-ylamino)isonicotinate (example 145b) and {2-[4-(*tert*-butoxycarbonyl)piperazin-1-yl]pyridin-4-yl}boronic acid (preparation 24b) following the experimental procedure as described in preparation 1.
   LRMS (m/z): 492 (M+1)⁺.
**d) *tert-*Butyl 4-[4-(hydroxymethyl)-6-(pyrazin-2-ylamino)-2,4'-bipyridin-2'-yl]piperazine-1-Carboxylate**
   Lithium aluminium hydride (156 mg, 4.11 mmol) was added dropwise to a stirred suspension of methyl 2'-[4-(*tert*-butoxycarbonyl)piperazin-1-yl]-6-(pyrazin-2-ylamino)-2,4'-bipyridine-4-Carboxylate (example 145c, 660 mg, 1.34 mmol) in tetrahydrofuran (10 mL). The mixture was stirred overnight at room temperature, then H₂O (5 mL) was added dropwise to the reaction mixture. The mixture was stirred 10 minutes at room temperature and the solvent was removed in vacuo. The residue was dissolved in ethyl acetate and water. The organic layer was separated, washed with brine, dried (MgSO₄) and evaporated to give the title compound (210 mg, 307%) as a brown foam.
   LRMS (m/z): 464 (M+1)⁺.
**e) *tert*-Butyl 4-[4-{[4-(3-ethoxy-3-oxopropyl)phenoxy]methyl}-6-(pyrazin-2-ylamino)-2,4'-bipyridin-2'-yl]piperazine-1-Carboxylate**
   Ethyl 3-(4-hydroxyphenyl)propanoate (0.08 g, 0.43 mmol), diisopropyl (*E*)-diazene-1,2-dicarboxylate (0.18 g, 0.87 mmol), triphenylphosphine (0.13 g, 0.48 mmol) and N-ethyl-*N*-isopropylpropan-2-amine (91µL, 0.52 mmol) were added to a stirred solution of *tert-*butyl 4-[4-(hydroxymethyl)-6-(pyrazin-2-ylamino)-2,4'-bipyridin-2'-yl]piperazine-1-Carboxylate (example 145d, 0.10 g, 0.22 mmol) in tetrahydrofuran (5 mL) and the mixture was warmed to 80ºC. After 2 hours, the reaction mixture was cooled, and the solvent was removed in vacuo. The residue was dissolved in ethyl acetate and water. The organic layer was separated, washed with brine, dried (MgSO₄) and evaporated. Purification of the residue by reverse phase chromatography (0% CH₃OH in H₂O to 100% CH₃OH in H₂O) gave the title compound (0.02 g, 15%) as a off-white solid.
   LRMS (m/z): 640 (M+1)⁺.
**f) 3-(4-{[2'-[4-(*tert*-Butoxycarbonyl)piperazin-1-yl]-6-(pyrazin-2-ylamino)-2,4'-bipyridin-4-yl]methoxy}phenyl)propanoic acid**
   Obtained (76%) from *tert*-butyl 4-[4-{[4-(3-ethoxy-3-oxopropyl)phenoxy]methyl}-6-(pyrazin-2-ylamino)-2,4'-bipyridin-2'-yl]piperazine-1-Carboxylate (example 145e) following the experimental procedure as described in example 74b.
   LRMS (m/z): 612 (M+1)⁺.
**g) 3-(4-{[2'-Piperazin-1-yl-6-(pyrazin-2-ylamino)-2,4'-bipyridin-4-yl]methoxy}phenyl)propanoic acid**
   Obtained as salt (41%) from 3-(4-{[2'-[4-(*tert*-Butoxycarbonyl)piperazin-1-yl]-6-(pyrazin-2-ylamino)-2,4'-bipyridin-4-yl]methoxy}phenyl)propanoic acid (example 145f) following the experimental procedure as described in example 73b.
   LRMS (m/z): 512 (M+1)⁺.

### EXAMPLE 146

### 4-Methoxy-2'-piperazin-1-yl-N-pyrazin-2-yl-2,4'-bipyridin-6-amine

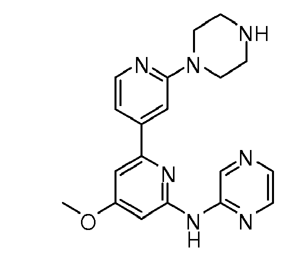

**a) *tert*-Butyl 4-(6-Chloro-4-methoxy-2,4'-bipyridin-2'-yl)piperazine-1-Carboxylate**
   Obtained (32%) from 2,6-dichloro-4-methoxypyridine and {2-[4-(*tert-*butoxycarbonyl)piperazin-1-yl]pyridin-4-yl}boronic acid (preparation 24b) following the experimental procedure as described in preparation 1, followed by purification of the crude product by flash chromatography (hexanes/ethyl acetate 0% to 35% ethyl acetate).
   LRMS (m/z): 405 (M+1)⁺.
**b) *tert-*Butyl 4-[4-methoxy-6-(pyrazin-2-ylamino)-2,4'-bipyridin-2'-yl]piperazine-1-Carboxylate**
   Obtained (4%) from *tert*-butyl 4-(6-Chloro-4-methoxy-2,4'-bipyridin-2'-yl)piperazine-1-Carboxylate (example 146a) and pyrazin-2-amine following the experimental procedure as described in preparation 4 using dioxane as a solvent, followed by purification of the crude product by reverse phase chromatography (0% CH₃OH in H₂O to 100% CH₃OH in H₂O).
   LRMS (m/z): 464 (M+1)⁺.
**c) 4-Methoxy-2'-piperazin-1-yl-*N*-pyrazin-2-yl-2,4'-bipyridin-6-amine**
   Obtained as hydrochloride salt (98%) from *tert*-butyl 4-[4-methoxy-6-(pyrazin-2-ylamino)-2,4'-bipyridin-2'-yl]piperazine-1-Carboxylate (example 146b) following the experimental procedure as described in example 73b.
   LRMS (m/z): 364 (M+1)⁺.
   1 H NMR (400 MHz, DMSO-*d*₆) δ ppm 3.29 (br. s., 4 H) 3.95 (s, 3 H) 4.05 (br. s., 4 H) 7.46 (d, *J*=17.19 Hz, 2 H) 7.60 (d, *J*=5.08 Hz, 1 H) 7.80 (s, 1 H) 8.16 (s, 1 H) 8.26 (d, *J*=6.25 Hz, 1 H) 8.31 (s, 1 H) 9.14 (s, 1 H) 9.60 (s, 1 H) 10.38 (br. s., 1 H)

### BIOLOGICAL TESTING

### Syk Enzyme assay- Radioactive filtration kinase assay

Recombinant human full-length Syk was supplied by Millipore. The activity of Syk kinase was assessed using a radioactive filtration kinase assay.
10 µl of substrate poly-GT (100 µg/ml final) and 4 µl of Syk (1nM) final) in 50 mM Tris-HCl (pH 7.5), 0.1 mM EGTA, 4 mM Mg(CH₃COO)₂, 0.1 mM Na₃VO₄, 0.1% (v/v) β-mercaptoethanol, 0.133 mg/ml BSA were added to 1.5 µl of various concentrations of compound or DMSO vehicle (5% final). The reaction was initiated by the addition of 14.5 µl of 0.36 µCi [γ-³³P] ATP (10 mCi/ml, PerkinElmer). This was prepared in a half-area NBS plate (Corning) and allowed to stand at room temperature for 40 min.
15 µl of reaction mixture was transferred to a filter plate (Millipore) previously pre-wetted with a solution of 75 mM phosphoric acid. The reaction was terminated with the addition of 200 µl phosphoric acid. The filter plate was washed three times under vacuum on a vacuum manifold (Millipore cat# MSVMHTS00) at 8" Hg vacuum level with 200 µl phosphoric acid per wash.
30 µl Optiphase™ Supermix (PerkinElmer) was added to each test well and allowed to incubate for at least 1 h before counting. Radioactivity counting was performed in a Wallac MicroBeta^{®}, and the tyrosine phosphorylation activity by Syk was calculated.

| **Example** | **Syk IC₅₀ (nM)** | **LAD₂IC₅₀(nM)** |
|---|---|---|
| **14** | 14 | 70 |
| **20** | 8 | 186 |
| **22** | 1,18 | 368 |
| **23** | 0,56 | 331 |
| **26** | 1,8 | 206 |
| **29** | 20 | 183 |
| **52** | 18 | 434 |
| **76** | 7 | 310 |
| **77** | 9 | 230 |
| **79** | 8 | 140 |
| **80** | 6 | 290 |
| **81** | 12 | 430 |
| **82** | 8 | 280 |
| **83** | 7 | 170 |
| **85** | 6 | 123 |
| **87** | 6 | 330 |
| **88** | 6 | 81 |
| **89** | 4 | 350 |
| **95** | 9 | 320 |
| **96** | 10 | 200 |
| **98** | 16 | 82 |
| **101** | 8 | 117 |
| **109** | 13 | 100 |
| **127** | 4 | 118 |
| **128** | 4,1 | 190 |
| **130** | 8,2 | 400 |
| **138** | 2,5 | 150 |

As it can be seen form the table, compounds of the present invention are potent inhibitor of Syk kinases. Preferred compounds of the invention posses an IC₅₀ value for the inhibition of Syk kinase of less than 1 µM, preferably less than 100 nM, more preferably less than 20 nM, being most preferable less than 10 nM.

Compounds described herein have shown activity inhibiting LAD2 degranulation with an IC₅₀ value of <10 µM, preferably less than 1 µM and more preferably less than 100 nM.

### In vitro JAK kinase Assays

Compounds were screened for their ability to inhibit JAK1, JAK2 and JAK3 using the assays as indicated below.

The catalytic domains of human JAK1 (aa 850-1154), JAK2 (aa 826-1132), JAK3 (aa 795-1124) and Tyk2 (aa 871-1187) were expressed as N-terminal GST-fusion proteins using a baculovirus expression system and were purchased from Carna Bioscences. The enzymatic activity was assayed using as substrate a biotinylated peptide, poly (GT)-Biotin (CisBio). The peptide concentration in the reactions was 60 nM for JAK1, 20 nM for JAK2, 140 nM for JAK3 and 50 nM for Tyk2. The degree of phosphorylation was detected by TR-FRET (time-resolved fluorescence energy transfer).

IC₅₀s of compounds were measured for each kinase in a reaction mixture containing the enzyme, ATP and the peptide in 8 mM MOPS (pH 7.0), 10 mM MgCl₂, 0.05% β-mercaptoethanol, 0.45 mg/mL BSA. The ATP concentration in the reactions was 3 µM for JAK1, 0.2 µM for JAK2, 0.6 µM for JAK3 and 1.8 µM for Tyk2. The enzymatic reactions took place for 30 minutes at room temperature. Then, the reactions were stopped with 20 µL of quench detection buffer (50 mM HEPES, 0.5 M KF, EDTA 0.25 M, 0.1% (w/v) BSA, pH 7.5) containing 0.115 µg/mL of anti-phosphoTyr (PT66)-Cryptate (CisBio) and a variable concentration of SA-XL665 (CisBio) to keep the SA-B ratio constant. Incubate for 3 h and read on Victor 2V spectrofluorometer (PerkinElmer) set to read fluorescence resonance energy transfer.

Some of the acronyms used above have the following meaning:
aa: aminoacids
GST: glutathione-S-transferase
MOPS: 3-(N-morpholino)propane sulfonic acid
BSA: bovine serum albumin
ATP: adenosine tri-phosphate
EDTA: ethylenediaminetetraacetic acid
HEPES: 4-(2-hydroxyethyl)-1-piperazineethanesulfonic acid

Table 2 depicts IC₅₀ values for certain exemplary compounds described in the invention. In Table 2, "A" represents an IC₅₀ value of less than 0.1 µM (100 nM), "B" represents an IC₅₀ value in the range of 0.1 µM (100 nM) to 1 µM, and C represents an IC₅₀ value higher than 1 µM.

**Table 2**

| **Example** | **JAK1 IC₅₀(nM)** | **JAK2 IC₅₀(nM)** | **JAK3 IC₅₀(nM)** |
|---|---|---|---|
| **78** | C | A | B |
| **79** | - | A | B |
| **80** | C | A | B |
| **85** | C | A | B |
| **88** | - | A | B |
| **100** | C | B | B |

As it can be seen form the table, compounds of the present invention are potent inhibitor of JAK1, JAK2 and JAK3 kinases.

### LAD2 cells assay

LAD2 is a human mast cell line depending on stem cell factor for its survival, maturation and differentiation. This mast cell line was established by the NIH from bone marrow aspirates from a patient with mast cell sarcoma. LAD2 cells resemble CD34+ - derived human mast cells as they have granules containing tryptase, histamine and β-hexosaminidase. They also express c-kit and FcεRI (Kirshenbaum, et al., Leukemia Research 27: 677-682, 2003).

### Sensitization of LAD2 cells

Cells were sensitized (0.2x10⁶ cells/ml) in its normal growth factor (Complete StemPro-34 SFM, Gibco 10639-011 containing StemPro-34 nutrient supplement (1/40); glutamine (2 mM) and supplemented with 100 ng/ml SCF) by adding 100 ng/ml of biotin-labeled IgE (USBiological) overnight (16-18 h, 37º, 5% CO₂) in a humidified atmosphere (90%).

### Compound preparation

Compounds were prepared from a 10⁻²M stock in 100% DMSO to give seven successive 1/3 dilutions in DMSO. From each concentration, 2 further 1/10 dilutions were prepared in release buffer (137 mM NaCl, 2.70 mM KCI, 0.4 mM NaH₂PO₄, 5.6 mM (D)-glucose, 1.8 mM CaCl₂, 1.3 mM MgSO₄, 0.025% BSA). Finally, 5 µl from each diluted concentration were transferred into the assay plate (flat 96-well Nunc). The final compound concentrations tested ranged from 1x10⁻⁵M to 4.12x10⁻⁹M with a final proportion of DMSO in the assay of 0.1 %. Control wells were treated with 0.1 % DMSO.

### LAD2 cells activation

Sensitized LAD2 cells were centrifuged (300 g, 3 min, R.T.) and washed 2 times with release buffer. Cells were resuspended in release buffer and adjusted to a density of 0.2x10⁶/ml. 45 µl of the adjusted cells were transfered to the assay plate (∼10,000cells/well) containg 5 µl of the final compound concentrations. LAD2 cells were incubated with the compound for 30 min (37ºC, 5% CO₂ 90% of humidity) and then activated with 125 ng/ml of streptavidin (Pierce 21125) for 30 min (37ºC, 5% CO₂, 90% of humidity). After the activation, cells were centrifuged at 1200 g, 10 min, 12ºC and supernatats were removed and transfered to another well for determination of released β-hexosaminidase. Empty wells were filled with 50 µl of release buffer for determination of intracellular β-hexosaminidase. Cells were lysed with 2 cycles of freeze-thaw.

### β-hexosaminidase assay

Cells were incubated for 90 minutes, 37ºC with 1 mM β-hexosaminidase substrate (p-nitrophenyl N-acetyl-D-glucosamide, in citric buffer, pH 4.5). Reaction was stopped with 0.4 M glycine solution (pH 10.0) and the plate is read immediately at 405 nm.

### PHARMACEUTICAL COMPOSITIONS

### Administration routes

The pharmaceutical formulations may conveniently be presented in unit dosage form and may be prepared by any of the methods well known in the art of pharmacy. All methods include the step of bringing the active ingredient(s) into association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil- in-water liquid emulsion or a water-in-oil liquid emulsion. The active ingredient may also be presented as a bolus, electuary or paste.

A syrup formulation will generally consist of a suspension or solution of the compound or salt in a liquid carrier for example, ethanol, peanut oil, olive oil, glycerine or water with flavouring or colouring agent.

Where the composition is in the form of a tablet, any pharmaceutical carrier routinely used for preparing solid formulations may be used. Examples of such carriers include magnesium stearate, talc, gelatine, acacia, stearic acid, starch, lactose and sucrose.

A tablet may be made by compression or moulding, optionally with one or more accessory ingredients. Compressed tablets may be prepared by compressing in a suitable machine the active ingredient in a free-flowing form such as a powder or granules, optionally mixed with a binder, lubricant, inert diluent, lubricating, surface active or dispersing agent.

Moulded tablets may be made by moulding in a suitable machine a mixture of the powdered compound moistened with an inert liquid diluent. The tablets may optionally be coated or scored and may be formulated so as to provide slow or controlled release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered, for example aqueous gums, celluloses, silicates or oils, and are incorporated in a soft gelatine capsule.

Dry powder compositions for topical delivery to the lung by inhalation may, for example, be presented in capsules and cartridges of for example gelatine or blisters of for example laminated aluminium foil, for use in an inhaler or insufflator. Formulations generally contain a powder mix for inhalation of the compound of the invention and a suitable powder base (carrier substance) such as lactose or starch. Use of lactose is preferred.

Each capsule or cartridge may generally contain between 2 µg and 150 µg of each therapeutically active ingredient. Alternatively, the active ingredient (s) may be presented without excipients.

Packaging of the formulation may be suitable for unit dose or multi-dose delivery. In the case of multi-dose delivery, the formulation can be pre-metered or metered in use. Dry powder inhalers are thus classified into two groups: (a) single dose, (b) multi dose devices.

For inhalers of the first type, single doses have been weighed by the manufacturer into small containers, which are mostly hard gelatine capsules. A capsule has to be taken from a separate box or container and inserted into a receptacle area of the inhaler. Next, the capsule has to be opened or perforated with pins or cutting blades in order to allow part of the inspiratory air stream to pass through the capsule for powder entrainment or to discharge the powder from the capsule through these perforations by means of centrifugal force during inhalation. After inhalation, the emptied capsule has to be removed from the inhaler again. Mostly, disassembling of the inhaler is necessary for inserting and removing the capsule, which is an operation that can be difficult and burdensome for some patients.

Other drawbacks related to the use of hard gelatine capsules for inhalation powders are (a) poor protection against moisture uptake from the ambient air, (b) problems with opening or perforation after the capsules have been exposed previously to extreme relative humidity, which causes fragmentation or indenture, and (c) possible inhalation of capsule fragments. Moreover, for a number of capsule inhalers, incomplete expulsion has been reported (Ex. Nielsen et al, 1997).

Some capsule inhalers have a magazine from which individual capsules can be transferred to a receiving chamber, in which perforation and emptying takes place, as described in WO92/03175. Other capsule inhalers have revolving magazines with capsule chambers that can be brought in line with the air conduit for dose discharge (Ex. WO91/02558 and GB2242134). They comprise the type of multiple unit dose inhalers together with blister inhalers, which have a limited number of unit doses in supply on a disk or on a strip.

Blister inhalers provide better moisture protection of the medicament than capsule inhalers. Access to the powder is obtained by perforating the cover as well as the blister foil, or by peeling off the cover foil. When a blister strip is used instead of a disk, the number of doses can be increased, but it is inconvenient for the patient to replace an empty strip. Therefore, such devices are often disposable with the incorporated dose system, including the technique used to transport the strip and open the blister pockets.

Multi-dose inhalers do not contain pre-measured quantities of the powder formulation. They consist of a relatively large container and a dose measuring principle that has to be operated by the patient. The container bears multiple doses that are isolated individually from the bulk of powder by volumetric displacement. Various dose measuring principles exist, including rotatable membranes (Ex. EP0069715) or disks (Ex. GB2041763; EP0424790; DE4239402 and EP0674533), rotatable cylinders (Ex. EP0166294; GB2165159 and WO92/09322) and rotatable frustums (Ex. WO92/00771), all having cavities which have to be filled with powder from the container. Other multi dose devices have measuring slides (Ex. US5201308 and WO97/00703) or measuring plungers with a local or circumferential recess to displace a certain volume of powder from the container to a delivery chamber or an air conduit (Ex. EP0505321, WO92/04068 and WO92/04928), or measuring slides such as the Genuair® (formerly known as Novolizer SD2FL), which is described in the following patent applications: WO97/000703, WO03/000325 and WO03/061742.

Reproducible dose measuring is one of the major concerns for multi dose inhaler devices.

The powder formulation has to exhibit good and stable flow properties, because filling of the dose measuring cups or cavities is mostly under the influence of the force of gravity.

For reloaded single dose and multiple unit dose inhalers, the dose measuring accuracy and reproducibility can be guaranteed by the manufacturer. Multi dose inhalers on the other hand, can contain a much higher number of doses, whereas the number of handlings to prime a dose is generally lower.

Because the inspiratory air stream in multi-dose devices is often straight across the dose measuring cavity, and because the massive and rigid dose measuring systems of multi dose inhalers can not be agitated by this inspiratory air stream, the powder mass is simply entrained from the cavity and little de-agglomeration is obtained during discharge.

Consequently, separate disintegration means are necessary. However in practice, they are not always part of the inhaler design. Because of the high number of doses in multi-dose devices, powder adhesion onto the inner walls of the air conduits and the de-agglomeration means must be minimized and/or regular cleaning of these parts must be possible, without affecting the residual doses in the device. Some multi dose inhalers have disposable drug containers that can be replaced after the prescribed number of doses has been taken (e. g. WO 97/000703). For such semi-permanent multi dose inhalers with disposable drug containers, the requirements to prevent drug accumulation are even more strict.

Apart from applications through dry powder inhalers the compositions of the invention can be administered in aerosols which operate via propellant gases or by means of so-Called atomisers, via which solutions of pharmacologically-active substances can be sprayed under high pressure so that a mist of inhalable particles results. The advantage of these atomisers is that the use of propellant gases can be completely dispensed with.

Such atomisers are described, for example, in PCT Patent Application No. W0 91/14468 and International Patent Application No. WO 97/12687, reference here is being made to the contents thereof.

Spray compositions for topical delivery to the lung by inhalation may for example be formulated as aqueous solutions or suspensions or as aerosols delivered from pressurised packs, such as a metered dose inhaler, with the use of a suitable liquefied propellant. Aerosol compositions suitable for inhalation can be either a suspension or a solution and generally contain the active ingredient (s) and a suitable propellant such as a fluorocarbon or hydrogen-Containing chlorofluorocarbon or mixtures thereof, particularly hydrofluoroalkanes, e. g. dichlorodifluoromethane, trichlorofluoromethane, dichlorotetra-fluoroethane, especially 1,1, 1, 2-tetrafluoroethane, 1,1, 1,2, 3,3, 3-heptafluoro-n-propane or a mixture thereof. Carbon dioxide or other suitable gas may also be used as propellant.

The aerosol composition may be excipient free or may optionally contain additional formulation excipients well known in the art such as surfactants, for example, oleic acid or lecithin and cosolvents, for example, ethanol. Pressurised formulations will generally be retained in a canister (for example, an aluminium canister) closed with a valve (for example, a metering valve) and fitted into an actuator provided with a mouthpiece.

Medicaments for administration by inhalation desirably have a controlled particle size.

The optimum particle size for inhalation into the bronchial system is usually 1-10 µm, preferably 2-5 µm. Particles having a size above 20 µm are generally too large when inhaled to reach the small airways. To achieve these particle sizes, the particles of the active ingredient as produced may be size reduced by conventional means, for example, by micronisation. The desired fraction may be separated out by air classification or sieving. Preferably, the particles will be crystalline.

Achieving high dose reproducibility with micronised powders is difficult because of their poor flowability and extreme agglomeration tendency. To improve the efficiency of dry powder compositions, the particles should be large while in the inhaler, but small when discharged into the respiratory tract. Thus, an excipient such as lactose or glucose is generally employed. The particle size of the excipient will usually be much greater than the inhaled medicament within the present invention. When the excipient is lactose it will typically be present as milled lactose, preferably crystalline alpha lactose monohydrate.

Pressurized aerosol compositions will generally be filled into canisters fitted with a valve, especially a metering valve. Canisters may optionally be coated with a plastics material e. g. a fluorocarbon polymer as described in WO96/32150. Canisters will be fitted into an actuator adapted for buccal delivery.

Typical compositions for nasal delivery include those mentioned above for inhalation and further include non-pressurized compositions in the form of a solution or suspension in an inert vehicle such as water optionally in combination with conventional excipients such as buffers, anti-microbials, tonicity modifying agents and viscosity modifying agents which may be administered by nasal pump.

Typical dermal and transdermal formulations comprise a conventional aqueous or non-aqueous vehicle, for example a cream, ointment, lotion or paste or are in the form of a medicated plaster, patch or membrane.

Preferably the composition is in unit dosage form, for example a tablet, capsule or metered aerosol dose, so that the patient may administer a single dose.

Each dosage unit contains suitably from 0.5 µg to 500 µg, and preferably from 5 µg to 100 µg of a compound according to the invention.

The amount of each active which is required to achieve a therapeutic effect will, of course, vary with the particular active, the route of administration, the subject under treatment, and the particular disorder or disease being treated.

The active ingredients may be administered from 1 to 6 times a day, sufficient to exhibit the desired activity. Preferably, the active ingredients are administered once or twice a day.

### Combination products

A pharmaceutical composition according to the invention comprises a compound of formula (I) and a therapeutically effective amount of one or more additional therapeutic agents selected from the group consisting of a β2-adrenergic agonist, a corticosteroid, an anti-Cholinergic agent, an anti-allergic agent, an anti-inflammatory agent, an immunosuppressant, a PDE4 inhibitor, a topically acting p38 inhibitor, an IKK2 inhibitor, an A_{2A} agonist, a Pl3Kδγinhibitor and an anti-infective agent, for simultaneous, separate or sequential use in the treatment of the human or animal body.

Examples of suitable β2-agonists that can be combined with the compounds of the present invention are: terbutaline sulphate, eformoterol fumarate, formoterol fumarate, bambuterol, ibuterol, isoprenaline hydrochloride, dopexamine, metaprotenerol, tulobuterol, procaterol hydrochloride, sibenadet hydrochloride, mabuterol hydrochloride, albuterol sulphate, salbutamol sulphate, salmefamol, salmeterol xinafoate, carmoterol hydrochloride, (R)-albuterol hydrochloride, Levalbuterol hydrochloride; Levosalbutamol hydrochloride; (-)-Salbutamol hydrochloride, (R,R)-Formoterol tartrate; Arformoterol tartrate, sulfonterol, Bedoradrine sulphate, Indacaterol, Trantinterol hydrochloride, Milveterol hydrochloride, Olodaterol, fenoterol hydrobromide, rimoterol hydrobromide, riproterol hydrochloride, Vilanterol broxaterol, pirbuterol hydrochloride, bitolterol mesylate, clenbuterol hydrochloride, AZD-3199, GSK-159802; GSK-597901, GSK-678007, GSK-961081; 4-[2-[3-(1H-Benzimidazol-1-yl)-1,1-dimethylpropylamino]-1-hydroxyethyl]-2-(4-methoxybenzylamino)phenol, 1-[2H-5-hydroxy-3-oxo-4H-1,4-benzoxazin-8-yl]-2-[3-(4-N, N-dimethylaminophenyl)-2-methyl-2-propylamino]ethanol, 1-[2H-5-hydroxy-3-oxo-4H-1,4-benzoxazin-8-yl]-2-[3-(4-domethoxyphenyl)-2-methyl-2-propylamino]ethanol, 1-[2H-5-hydroxy-3-oxo-4H-1,4-benzoxazin-8-yl]-2-[3-(4-n-butyloxyhenyl)-2-methyl-2-propylamino]ethanol, KUL-1248, HOKU-81, SM-110444, RP-58802B, LAS 100977 and compounds described in international patent applications Nos. WO2007/124898, W02006/122788A1, WO2008/046598 and W02008095720..

The following β2-agonists are of special interest for the combination with the compounds of formula (I): arformoterol, bambuterol, bitolterol, broxaterol, carbuterol, clenbuterol, dopexamine, fenoterol, formoterol, hexoprenaline, ibuterol, isoprenaline, levosalbutamol, mabuterol, meluadrine, nolomirole, orciprenaline, pirbuterol, procaterol, (R,R)-formoterol, reproterol, ritodrine, rimoterol, salbutamol, salmeterol, sibenadet, sulfonterol, terbutaline, tulobuterol, GSK-597901, LAS100977, milveterol, carmoterol and indacaterol, optionally in the form of their racemates, their enantiomers, their diastereomers, and mixtures thereof, and optionally their pharmacologically-Compatible acid addition salts.

Still most preferred are the following β2-agonists: formoterol, salmeterol, GSK-597901, LAS100977, milveterol and indacaterol, optionally in the form of their racemates, their enantiomers, their diastereomers and mixtures thereof, and optionally their pharmacologically-Compatible acid addition salts. Still more preferred are LAS100977, salmeterol and formoterol.

Examples of suitable corticosteroids and glucocorticoids that can be combined with compounds of the present invention are prednisolone, methylprednisolone, dexamethasone, dexamethasone cipecilate, naflocort, deflazacort, halopredone acetate, budesonide, beclomethasone dipropionate, hydrocortisone, triamcinolone acetonide, fluocinolone acetonide, fluocinonide, clocortolone pivalate, methylprednisolone aceponate, dexamethasone palmitoate, tipredane, hydrocortisone aceponate, prednicarbate, alclometasone dipropionate, halometasone, methylprednisolone suleptanate, mometasone furoate, rimexolone, prednisolone farnesylate, ciclesonide, butixocort propionate, RS-85095, CGP-13774, GW-250495, deltacortisone, NO-Prednisolone, etiprednol dicloacetate, QAE-397, 7beta-OH-EPIA, RPR-106541, deprodone propionate, fluticasone propionate, fluticasone furoate, halobetasol propionate, loteprednol etabonate, betamethasone butyrate propionate, flunisolide, prednisone, dexamethasone sodium phosphate, triamcinolone, betamethasone 17-valerate, betamethasone, betamethasone dipropionate, 21-Chloro-11 beta-hydroxy-17alpha-[2-(methylsulfanyl)acetoxy]-4-pregnene-3,20-dione, Desisobutyrylciclesonide, hydrocortisone acetate, hydrocortisone sodium succinate, prednisolone sodium phosphate and hydrocortisone probutate, Prednisolone sodium metasulfobenzoate and clobetasol propionate.

Examples of suitable M3 antagonists (anticholinergics) that can be combined with the compounds of the present invention are tiotropium salts, oxitropium salts, flutropium salts, ipratropium salts, glycopyrronium salts, trospium salts, zamifenacin, revatropate, espatropate, darotropium bromide, CI-923, NPC-14695, BEA-2108, 3-[2-Hydroxy-2,2-bis(2-thienyl)acetoxy]-1-(3-phenoxypropyl)-1-azoniabicyclo[2.2.2]octane salts (in particular aclidinium salts, more preferably aclidinium bromide), 1-(2-Phenylethyl)-3-(9H-xanthen-9-ylcarbonyloxy)-1-azoniabicyclo[2.2.2]octane salts, 2-oxo-1,2,3,4-tetrahydroquinazoline-3-Carboxylic acid endo-8-methyl-8-azabicyclo[3.2.1]oct-3-yl ester salts (DAU-5884), 3-(4-Benzylpiperazin-1-yl)-1-Cyclobutyl-1-hydroxy-1-phenylpropan-2-one (NPC-14695), N-[1-(6-Aminopyridin-2-ylmethyl)piperidin-4-yl]-2(R)-[3,3-difluoro-1 (R)-Cyclopentyl]-2-hydroxy-2-phenylacetamide (J-104135), 2(R)-Cyclopentyl-2-hydroxy-N-[1-[4(S)-methylhexyl]piperidin-4-yl]-2-phenylacetamide (J-1 06366), 2(R)-Cyclopentyl-2-hydroxy-N-[1-(4-methyl-3-pentenyl)-4-piperidinyl]-2-phenylacetamide (J-104129), 1-[4-(2-Aminoethyl)piperidin-1-yl]-2(R)-[3,3-difluorocyclopent-1 (R)-yl]-2-hydroxy-2-phenylethan-1-one (Banyu-280634), N-[N-[2-[N-[1-(Cyclohexylmethyl)piperidin-3(R)-ylmethyl]carbamoyl]ethyl]carbamoylmethyl]-3,3,3-triphenylpropionamide (Banyu CPTP), 2(R)-Cyclopentyl-2-hydroxy-2-phenylacetic acid 4-(3-azabicyclo[3.1.0]hex-3-yl)-2-butynyl ester (Ranbaxy 364057), 3(R)-[4,4-Bis(4-fluorophenyl)-2-oxoimidazolidin-1-yl]-1-methyl-1-[2-oxo-2-(3-thienyl)ethyl]pyrrolidinium iodide, N-[1-(3-Hydroxybenzyl)-1-methylpiperidinium-3(S)-yl]-N-[N-[4-(isopropoxycarbonyl)phenyl]carbamoyl]-L-tyrosinamide trifluoroacetate, UCB-101333, Merck's OrM3, 7-endo-(2-hydroxy-2,2-diphenylacetoxy)-9,9-dimethyl-3-oxa-9-azoniatricyclo[3.3.1.0(2,4)]nonane salts, 3(R)-[4,4-Bis(4-fluorophenyl)-2-oxoimidazolidin-1-yl]-1-methyl-1-(2-phenylethyl)pyrrolidinium iodide, trans-4-[2-[Hydroxy-2,2-(dithien-2-yl)acetoxy]-1-methyl-1-(2-phenoxyethyl)piperidinium bromide from Novartis (412682), 7-(2,2-diphenylpropionyloxy)-7,9,9-trimethyl-3-oxa-9-azoniatricyclo[3.3.1.0*2,4*]nonane salts, 7-hydroxy-7,9,9-trimethyl-3-oxa-9-azoniatricyclo[3.3.1.0*2,4*]nonane 9-methyl-9H-fluorene-9-Carboxylic acid ester salts, all of them optionally in the form of their racemates, their enantiomers, their diastereomers and mixtures thereof, and optionally in the form of their pharmacologically-Compatible acid addition salts. Among the salts chlorides, bromides, iodides and methanesulphonates are preferred.

Examples of suitable anti-allergic agents that can be combined with the compounds of the present invention are anti-histamines (e.g. Methapyrilene, Mequitazine, Azelastine hydrochloride, Acrivastine, Emedastine difumarate, Emedastine fumarate, Loratadine, Cyproheptadine hydrochloride, Diphenhydramine hydrochloride, Doxepin hydrochloride, Promethazine hydrochloride, Levocabastine hydrochloride, Desloratadine, Cinnarizine, Setastine hydrochloride, Mizolastine, Ebastine, Cetirizine hydrochloride, Epinastine hydrochloride, Olopatadine hydrochloride, Bepotastine besilate,Triprolidine hydrochloride, Rupatadine fumarate, Fexofenadine hydrochloride, Levocetirizine dihydrochloride, Ketotifen, Azatadine maleate, Dimethindene maleate, Clemastine fumarate, Alcaftadine, Bilastine, Vapitadine hydrochloride, AZD-1744, GSK-1004723D, GSK-835726, SUN-1334H), CRTH2 antagonists (e.g. Ramatroban, AMG-009, OC-000459), or mast cell stabilizers (e.g. nedocromil, pemirolast potassium, chromoglycate sodium, suplatast tosilate).

Examples for suitable anti-inflammatory agents that can be combined with the compounds of the present invention are non-steroidal anti-inflammatory drugs (NSAID), leukotriene antagonists (e.g. Ibudilast, Pranlukast hydrate, Zafirlukast, Montelukast, Tipelukast), FLAP inhibitors, lipoxygenase inhibitors, cyclooxygenase inhibitors (e.g. diclofenac, ibuprofen, celecoxib); elastase inhibitors, PDE inhibitors (e.g. theophylline) or another Syk kinase inhibitor.

Examples for suitable immunosupressants that can be combined with the compounds of the present invention are picremolimus, tacromilus, cyclosporine A, leflunomide, methotrexate, anti-TNF agents and compounds described in International patent applications Nos. PCT/EP2008/006573 and W02008/077639.

Examples of suitable PDE4 inhibitors that can be combined with compounds of the present invention are benafentrine dimaleate, etazolate, denbufylline, rolipram, cipamfylline, zardaverine, arofylline, filaminast, tipelukast, tofimilast, piclamilast, tolafentrine, mesopram, drotaverine hydrochloride, lirimilast, roflumilast, cilomilast, oglemilast, apremilast, tetomilast, filaminast, (R)-(+)-4-[2-(3-Cyclopentyloxy-4-methoxyphenyl)-2-phenylethyl]pyridine (CDP-840), N-(3,5-Dichloro-4-pyridinyl)-2-[1-(4-fluorobenzyl)-5-hydroxy-1 H-indol-3-yl]-2-oxoacetamide (GSK-842470), 9-(2-Fluorobenzyl)-N6-methyl-2-(trifluoromethyl)adenine (NCS-613), N-(3,5-Dichloro-4-pyridinyl)-8-methoxyquinoline-5-Carboxamide (D-4418), 3-[3-(Cyclopentyloxy)-4-methoxybenzyl]-6-(ethylamino)-8-isopropyl-3H-purine hydrochloride (V-11294A), 6-[3-(N,N-Dimethyl-Carbamoyl)phenylsulfonyl]-4-(3-methoxyphenylamino)-8-methylquinoline-3-Carboxamide hydrochloride (GSK-256066), 4-[6,7-Diethoxy-2,3-bis(hydroxymethyl)-naphthalen-1-yl]-1-(2-methoxyethyl)pyridin-2(1 H)-one (T-440), (-)-trans-2-[3'-[3-(N-Cyclopropylcarbamoyl)-4-oxo-1,4-dihydro-1,8-naphthyridin-1-yl]-3-fluorobiphenyl-4-yl]-Cyclopropanecarboxylic acid (MK-0873), CDC-801, UK-500001, BLX-914, 2-Carbomethoxy-4-Cyano-4-(3-Cyclopropylmethoxy-4-difluroromethoxy-phenyl)-Cyclohexan-1-one, cis [4-Cyano-4-(3-Cyclopropylmethoxy-4-difluoromethoxyphenyl)-Cyclohexan-1-ol, CDC-801, 5(S)-[3-(Cyclopentyloxy)-4-methoxyphenyl]-3(S)-(3-methylbenzyl)piperidin-2-one (IPL-455903), ONO-6126 (Eur Respir J 2003, 22(Suppl. 45): Abst 2557) and the salts claimed in the PCT patent applications number WO03/097613, WO2004/058729, WO 2005/049581, WO 2005/123693 and WO 2005/123692.

Examples of suitable P38 inhibitors that can be combined with compounds of the present invention are dilmapimod, losmapimod, regorafenib, PH-797804 (from Pfizer), KC-706 (from Kemia), ARRY-614 (from Array Biopharma), TA-5493 (from Tanabe Pharma), ARRY-797 (from Array Biopharma), BMS-582949 (from Bristol-Myers Squibb), AKP-001 (from Aska Pharmaceutical), GSK-610677 (from GlaxoSmithKline) and UR-13870 (from Palau Pharma).

Examples of suitable IKK2 inhibitors that can be combined with compounds of the present invention are IMD-0354 (from Institute of Medicinal Molecular Design), MLN-0415 (from Millennium Pharmaceuticals) and SAR-113945 (from Sanofi-Aventis).

Examples of suitable A2a agonists that can be combined with the compounds of the present invention are binodenoson, apanedoson, regadenoson and sonedenoson.

Examples of suitable antagonists of the Pl3K□□ inhibitors that can be combined with the compounds of the present invention are 2-Methyl-2-[4-[3-methyl-2-oxo-8-(3-quinolinyl)-2,3-dihydro-1 H-imidazo[4,5-C]quinolin-1-yl]phenyl]propanenitrile (BEZ-235 from Novartis), CAL-1 01 (from Calistoga Pharmaceuticals) and N-Ethyl-N'-[3-(3,4,5-trimethoxyphenylamino)pyrido[2,3-b]pyrazin-6-yl]thiourea (AEZS-126 from Aeterna Zentaris).

Examples for suitable anti-infectives that can be combined with the compounds of the present invention are mupiricin, retapamulin, clotrimazole, ketoconazole and terbinafine.

The combinations of the invention may be used in the treatment of respiratory diseases, wherein the use of bronchodilating agents is expected to have a beneficial effect, for example asthma, acute or chronic bronchitis, emphysema, or Chronic Obstructive Pulmonary Disease (COPD).

The active compounds in the combination may be administered together in the same pharmaceutical composition or in different compositions intended for separate, simultaneous, concomitant or sequential administration by the same or a different route.

It is contemplated that all active agents would be administered at the same time, or very close in time. Alternatively, one or two actives could be taken in the morning and the other (s) later in the day. Or in another scenario, one or two actives could be taken twice daily and the other (s) once daily, either at the same time as one of the twice-α-day dosing occurred, or separately. Preferably at least two, and more preferably all, of the actives would be taken together at the same time. Preferably, at least two, and more preferably all actives would be administered as an admixture.

The active substance compositions according to the invention are preferably administered in the form of compositions for inhalation delivered with the help of inhalers, especially dry powder inhalers, however, any other form or parenteral or oral application is possible. Here, the application of inhaled compositions embodies the preferred application form, especially in the therapy of obstructive lung diseases or for the treatment of asthma.

Additional suitable carriers for formulations of the active compounds of the present invention can be found in Remington: The Science and Practice of Pharmacy, 20th Edition, Lippincott Williams & Wilkins, Philadelphia, Pa., 2000. The following nonlimiting examples illustrate representative pharmaceutical compositions of the invention.

### FORMULATION EXAMPLES

### Formulation Example 1 (Oral suspension)

| **Ingredient** | **Amount** |
|---|---|
| Active Compound | 3 mg |
| Citric acid | 0,5 g |
| Sodium chloride | 2,0 g |
| Methyl paraben | 0,1 g |
| Granulated sugar | 25 g |
| Sorbitol (70% solution) | 11 g |
| Veegum K | 1,0 g |
| Flavoring | 0,02 g |
| Dye | 0,5 mg |
| Distilled water | q.s. to 100 mL |

### Formulation Example 2 (Hard gelatine capsule for oral administration)

| **Ingredient** | **Amount** |
|---|---|
| Active Compound | 1 mg |
| Lactose | 150 mg |
| Magnesium stearate | 3 mg |

### Formulation Example 3 (Gelatin cartridge for inhalation)

| **Ingredient** | **Amount** |
|---|---|
| Active Compound (micronized) | 0,2 mg |
| Lactose | 25 mg |

### Formulation Example 4 (Formulation for inhalation with a DPI)

| **Ingredient** | **Amount** |
|---|---|
| Active Compound (micronized) | 15 mg |
| Lactose | 3000 mg |

### Formulation Example 5 (Formulation for a MDI)

| **Ingredient** | **Amount** |
|---|---|
| Active Compound (micronized) | 10 g |
| 1,1,1,2,3,3,3-heptafluoro-n-propane | q.s. to 200 ml |

## Claims

1. A compound of formula (I), or a pharmaceutically acceptable salt or N-oxide or solvate or deuterated derivative thereof: 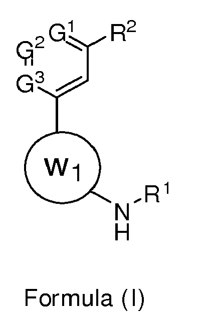 wherein:
● R¹ is a 5 to 6-membered monocyclic heteroaryl group comprising at least one N atom, wherein said heteroaryl group is optionally substituted by one or more substituents selected from the group consisting of a halogen atom, a linear or branched C₁-C₆ alkyl group, a -CN group, a -NR^{a}R^{b} group, a -CF₃ group, a C₁-C₄ alkoxy group and a (C₁-C₄ alkoxy)-(C₁-C₄ alkoxy) group;
● R² is selected from the group consisting of a -(CH₂)ₚ-CONR^{a}R^{b} group, a C₁-C₆ aminoalkyl group, a -NR^{a}-(CH₂)_{q}-NR^{b}R^{c} group, a -NR^{a}-(CH₂)_{q}-OH group, a -(CH₂)ₚ-COOH moiety, a N-Containing saturated or unsaturated 5 to 6-membered heterocyclyl group and a -NR^{d}R^{e} group, wherein
o R^{d} represents a hydrogen atom or a C₁₋₆ alkyl group, and R^{e} represents a hydrogen atom, a C₁₋₆ alkyl group or a monocyclic or polycyclic C₃-C₁₀ cycloalkyl group optionally substituted by a hydroxy group; or
o R^{d} and R^{e} together with the nitrogen atom to which they are attached form a 4-to 7-membered, mono- or poly-heterocyclic ring, optionally containing one additional nitrogen atom and optionally substituted by one or more substituents selected from the group consisting of a halogen atom, a hydroxy group, a -NH₂ group, a C₁-C₆ alkyl group, an oxo group, a -COR^{a} group, a -COOR^{a} group and a -CN group;
● W₁ represents a group of formula A, B, or C: 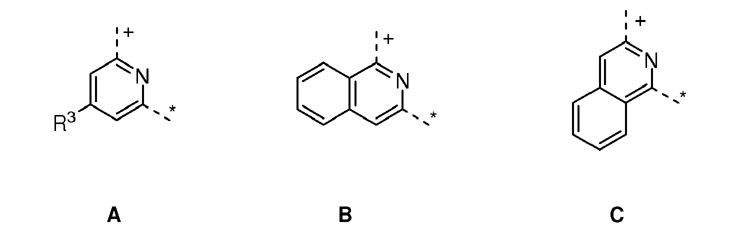 wherein the asterisk symbol indicates the bond that is linked to the -NH-R¹ moiety of Formula (I) and the plus symbol indicates the bond that is linked to the ring system of Formula (I);
● R³ is selected from the group consisting of a hydrogen atom, a ―OMe group, a -CN group, a -NR^{a}R^{b} group, a C₆-C₁₀ aryl group, a 5 to 10-membered heteroaryl group, a 3 to 10-membered heterocyclyl group and a ―L₁-L₂-W₂ group, wherein the aryl, heteroaryl and heterocyclyl groups are optionally substituted by one or more substituents selected from the group consisting of a halogen atom, a hydroxy group, a -NH₂ group, a C₁-C₆ alkyl group, an oxo group, a -COR^{a} group, a -COOR^{a} group and a -CN group;
● L₁ is selected from the group consisting of a -(CH₂)₀₋₁NR^{a}CO- group, a -NR^{a}CO₂- group, a -NR^{a}CONR^{b}- group, a -NR^{a}SO₂- group, a -NR^{a}- group, a -CONR^{a}- group, a -CH₂O- group and a -CO₂- group;
● L₂ is selected from the group consisting of a direct bond and a linear or branched C₁-C₆ alkylene group, wherein the alkylene group is optionally substituted by a carboxy group;
● W₂ is selected from the group consisting of a linear or branched C₁-C₆ alkyl group, a C₆-C₁₀ aryl group, a 5 to 10-membered heteroaryl group, a 3 to 10-membered heterocyclyl group, and a monocyclic or polycyclic C₄-C₁₂ cycloalkyl group, wherein the alkyl, aryl, heteroaryl, heterocyclyl and cycloalkyl groups are optionally substituted by one or more substituents selected from the group consisting of a halogen atom, a -CF₃ group, a ―OCF₃ group, a hydroxy group, a C₁-C₆ alkyl group, a C₁-C₆ alkoxy group, a C₆-C₁₀ aryl-C₁-C₄ alkoxy group, a -COR^{a} group, a -COOR^{a} group, a C₁-C₆ alkyl-COOR^{a} group, a -CONR^{a}R^{b} group and a -NR^{a}R^{b} group, the phenyl moiety optionally has two substituents which are in ortho position to each other and form together a methylenedioxy or ethylenedioxy ring;
● each R^{a}, R^{b} and R^{c} is independently selected from the group consisting of a hydrogen atom and a C₁-C₆ alkyl group;
● each p is an integer independently selected from 0 to 4;
● each q is an integer independently selected from 1 to 4;
● G¹ and G³ are independently selected from the group consisting of a N atom and a CH group;
● G² is selected from the group consisting of a N atom and a C-R⁴ group; and
● R⁴ is selected from the group consisting of a hydrogen atom, a linear or branched C₁-C₆ alkyl group, a halogen atom, a oxo group and a hydroxy group.

2. A compound according to claim 1, wherein R¹ represents a pyrazin-2-yl group, a pyridin-2-yl group, a pyrimidin-4-yl group or a 1*H-*pyrazol-3-yl group, wherein said groups are optionally substituted by one substituent selected from the group consisting of a halogen atom, a linear or branched C₁-C₆ alkyl group, a -CN group and a -CF₃ group, and preferably wherein R¹ represents an unsubstituted pyrazin-2-yl group or a pyridin-2-yl group, wherein said pyridin-2-yl group is optionally substituted by one substituent selected from the group consisting of a methyl group and a -CF₃ group.

3. A compound according to claim 1 or 2, wherein R² represents an C₁-C₆ aminoalkyl group, a -NR^{a}-(CH₂)ₚ-NR^{b}R^{c} group, a N-Containg saturated or unsaturated 6-membered heterocyclyl group or a -NR^{d}R^{e} group, wherein
o R^{d} and R^{e} together with the nitrogen atom to which they are attached form a 6-to 7-membered, mono- or poly-heterocyclic ring, containing one additional nitrogen atom and optionally substituted by one or more substituents selected from the group consisting of a hydroxy group, a -NH₂ group, a C₁₋₆ alkyl group, an oxo group, a -COR^{a} group, a -COOR^{a} group and a -CN group.

4. A compound according to claim 3, wherein R² represents an aminoethyl group, a -NH-(CH₂)₂-₃-NH₂ group, a 1,2,3,6-tetrahydropyridin-4-yl group, a piperidin-4-yl group, or a -NR^{d}R^{e} group, wherein
o R^{d} and R^{e} together with the nitrogen atom to which they are attached form a piperazin-1-yl group, a 1,4-diazepan-1-yl group and a 2,5-diazabicyclo[2.2.1 ]heptan-2-yl group, wherein said groups are optionally substituted by one or two substituents selected from the group consisting of a methyl group, a fluorine atom and a -CN group.

5. A compound according to claims 1 to 4, wherein R³ represents a hydrogen atom, a -NR^{a}R group, a phenyl group, a 5 to 7-membered heterocyclyl group or a -L₁-L₂-W₂ group, wherein the phenyl and heterocyclyl group are optionally substituted by a hydroxy group or a -COOR^{a} group, and preferably wherein L₁ represents -NHCO-, -CONH- or -NHCONH- and W₂ represents a phenyl group, a 5 to 6-membered heteroaryl group, a 6 to 10-membered heterocyclyl group or a cyclohexyl group, wherein the phenyl, heteroaryl, heterocyclyl and cyclohexyl groups are optionally substituted by one or two substituents selected from the group consisting of a fluorine atom, a -CF₃ group, a methyl group, a methoxy group, a benzyloxy group, a -COR^{a} group, a -COOR^{a} group or wherein the phenyl group optionally has two substituents which are in ortho position to each other and form together a methylenedioxy or ethylenedioxy ring.

6. A compound according to claims 1 to 5 wherein G³ represents a -CH group and/or G² is selected from a group consisting of a N atom and a -CH group.

7. A compound according to any one of the preceeding claims, wherein
● R¹ represents an unsubstituted pyrazin-2-yl group or a pyridin-2-yl group, wherein said pyridin-2-yl group is optionally substituted by one substituent selected from the group consisting of a methyl group and a -CF₃ group;
● R² represents an aminoethyl group, a -NH-(CH₂)₂-₃-NH₂ group, a 1,2,3,6-tetrahydropyridin-4-yl group, a piperidin-4-yl group, or a -NR^{d}R^{e} group, wherein
o R^{d} and R^{e} together with the nitrogen atom to which they are attached form a 6- to 7-membered, mono- or poly-heterocyclic ring, selected from a piperazin-1-yl group, a 1,4-diazepan-1-yl group and a 2,5-diazabicyclo[2.2.1 ]heptan-2-yl group, wherein said ring is optionally substituted by one or two substituents selected from the group consisting of a methyl group, a fluorine and a -CN group;
● R³ represents a hydrogen atom, a -NR^{a}R^{b} group, a phenyl group, a 5 to 7-membered heterocyclyl group or a ―L₁-L₂-W₂ group, wherein the phenyl and heterocyclyl group are optionally substituted by a hydroxy group or a -COOR^{a} group; and
● G³ represents a CH group and G² is selected from a group consisting of a N atom and a CH group

8. A compound according to claim 1, wherein
● R¹ represents a pyrazin-2-yl group, a pyridin-2-yl group, a pyrimidin-4-yl group or a 1*H-*pyrazol-3-yl group, wherein said heteroaryl groups are optionally substituted by one substituent selected from the group consisting of a fluorine atom, a methyl group, an amino group, a -CN group, a -CF₃ group, a -C(CH₃)₃ group and a -O(CH₂)₂OCH₃ group;
● R² represents a -NH₂ group, a -(CH₂)₀₋₁CONH₂ group, a -(CH₂)₁₋₂NH₂ group, a -NH(CH₂)₂₋₃NH₂ group, a -NH(CH₂)₂OH group, a -N(CH₃)(CH₂)₂₋₃N(CH₃)₂ group, a -CH₂CH₂COOH group, a -NH(CH₂)₂OH group, an adamantylamino group, a bicyclo[2.2.1]heptan-2-amino group, or a 4 to 7-membered monocyclic or polycyclic ring selected from the group consisting of a piperazin-1-yl group, an azetidin-1-yl group, a pyrrolidin-1-yl group, a piperidin-4-yl group, a piperidin-1-yl group, a 1,4-diazepan-1-yl group, a 1,2,3,6-tetrahydropyridin-4-yl group and a 2,5-diazabicyclo[2.2.1]-heptan-2-yl group, and wherein the adamantylamino group, bicyclo[2.2.1]heptan-2-amino group, or 4 to 7-membered monocyclic or polycyclic ring is optionally substituted by one or two substitutents selected from the group consisting of a methyl group, a hydroxy group, an oxo group, a -COOH group, a -COCH₃ group and a -NH₂ group;
● R³ represents a hydrogen atom, an ―OMe group, a -CN group, a NH₂ group, a -NHCH₃ group, a -NH(CH₂)₃CH₃ group, a -N(CH₃)₂ group, a phenyl group, a piperidin-1yl-4-ol group, a piperidin-1-yl-4-Carboxylic acid or a ―L₁-L₂-W₂ group, wherein
o L₁ represents a -NHCO- group, a -NHCONH- group, a -NHSO₂- group, a -NH- group, a -CH₂O group, a -CH₂NHCO group, or a -CONH- group;
o L₂ represents a direct bond or a linear or branched alkylene group selected from the group consisting of a -CH₂- group , a -(CH₂)₂- group , a -(CH₂)₃- group, a -CH(CH₃)-CH₂- group, a -CH₂-CH(CH₃)- group and a -CH₂CH(CH₃)CH₂- group, wherein said alkylene group is optionally substituted by a carboxy group;
o W₂ represents a methyl group, an ethyl group, an isopropyl group, a neopentyl group, a phenyl group, a pyridine group, a pyrimidine group, a quinoline group, a pyrazolyl group, a piperidinyl group, a 1,2,3,4-tetrahydroisoquinoline group, a cyclohexyl group, wherein said groups are optionally substituted by one or more, preferably one or two, substituents selected from the group consisting of a fluorine atom, a -CF₃ group, a ―OCF₃ group, a methyl group, a methoxy group, a benzyloxy group, a -COCH₃ group, a -COOH group, a -CH₂CH₂COOH group, a -CO₂CH₃ group, or wherein the phenyl group optionally has two substituents which are in ortho position to each other and form together a methylenedioxy ring;

9. A compound according to claim 1 which is one of:
*N*-{6-[3-(Aminomethyl)phenyl]pyridin-2-yl}-4-methylpyridin-2-amine;
*N*-{6-[3-(2-Aminoethyl)phenyl]pyridin-2-yl}-4-methylpyridin-2-amine;
*N*-[6-(3-Aminophenyl)pyridin-2-yl]-4-methylpyridin-2-amine;
3-{6-[(4-Methylpyridin-2-yl)amino]pyridin-2-yl}benzamide;
*N*-(3-{6-[(4-Methylpyridin-2-yl)amino]pyridin-2-yl}phenyl)ethane-1,2-diamine;
4-Methyl-N-{6-[3-(1,2,3,6-tetrahydropyridin-4-yl)phenyl]pyridin-2-yl}pyridin-2-amine;
4-Methyl-*N-*[6-(3-piperidin-4-ylphenyl)pyridin-2-yl]pyridin-2-amine;
*N*-{6-[3-(4-Aminopiperidin-1-yl)phenyl]pyridin-2-yl}-4-methylpyridin-2-amine;
*N*⁶-(4-Methylpyridin-2-yl)-2,4'-bipyridine-2',6-diamine;
*N*²'-(2-Aminoethyl)-*N*⁶-(4-methylpyridin-2-yl)-2,4'-bipyridine-2',6-diamine;
2'-(3-Aminopyrrolidin-1-yl)-N-(4-methylpyridin-2-yl)-2,4'-bipyridin-6-amine;
2'-(4-Aminopiperidin-1-yl)-N-(4-methylpyridin-2-yl)-2,4'-bipyridin-6-amine;
*N*²'-(3-Aminopropyl)-*N*⁶-(4-methylpyridin-2-yl)-2,4'-bipyridine-2',6-diamine;
*N*-(4-Methylpyridin-2-yl)-2'-piperazin-1-yl-2,4'-bipyridin-6-amine;
2-({6-[(4-Methylpyridin-2-yl)amino]-2,4'-bipyridin-2'-yl}amino)ethanol;
2'-(1,4-Diazepan-1-yl)-N-(4-methylpyridin-2-yl)-2,4'-bipyridin-6-amine;
*N*²'-[2-(Dimethylamino)ethyl]-*N*²'-methyl-*N*⁶-(4-methylpyridin-2-yl)-2,4'-bipyridine-2',6-diamine;
*N*-{6-[3-(2-Aminoethyl)phenyl]pyridin-2-yl}-4-*tert*-butylpyridin-2-amine;
*N*²'-(2-Aminoethyl)-*N*⁶-(4-*tert*-butylpyridin-2-yl)-2,4'-bipyridine-2',6-diamine;
*N*-{6-[3-(2-Aminoethyl)phenyl]pyridin-2-yl}-4-(trifluoromethyl)pyridin-2-amine;
*N*-{6-[3-(1,2,3,6-Tetrahydropyridin-4-yl)phenyl]pyridin-2-yl}-4-(trifluoromethyl) pyridin-2-amine;
*N*-[6-(3-Piperidin-4-ylphenyl)pyridin-2-yl]-4-(trifluoromethyl)pyridin-2-amine;
*N*-[6-(3-Piperazin-1-ylphenyl)pyridin-2-yl]-4-(trifluoromethyl)pyridin-2-amine;
2-[3-(6-{[4-(Trifluoromethyl)pyridin-2-yl]amino}pyridin-2-yl)phenyl]acetamide;
*N*²'-(2-Aminoethyl)-*N*⁶-[4-(trifluoromethyl)pyridin-2-yi]-2,4'-biyridine-2',6-diamine;
2'-Piperazin-1-yl-*N*-[4-(trifluoromethyl)pyridin-2-yl]-2,4'-bipyridin-6-amine;
*N*-{6-[3-(2-Aminoethyl)phenyl]pyridin-2-yl}-4-(2-methoxyethoxy)pyridin-2-amine;
*N*-{6-[3-(2-Aminoethyl)phenyl]pyridin-2-yl}pyrazin-2-amine;
*N*-{6-[3-(1,2,3,6-Tetrahydropyridin-4-yl)phenyl]pyridin-2-yl}pyrazin-2-amine;
*N-*[6-(3-Piperidin-4-ylphenyl)pyridin-2-yl]pyrazin-2-amine;
2-{3-[6-(Pyrazin-2-ylamino)pyridin-2-yl]phenyl}acetamide;
*N*-[6-(3-Piperazin-1-ylphenyl)pyridin-2-yl]pyrazin-2-amine;
*N*²'-(2-Amioethyl-*N*⁶-pyrazin-2-yl-2,4'-bipyridine-2',6-diamine;
2'-(4-Methylpiperazin-1-yl)-*N*-pyrazin-2-yl-2,4'-bipyridin-6-amine;
2'-Piperazin-1-yl-*N-*pyrazin-2-yl-2,4'-bipyridin-6-amine;
2'-(4-Acetylpiperazin-1-yl)-*N*-pyrazin-2-yl-2,4'-bipyridin-6-amine;
(4*r*,7*r*)-4-{[6-(Pyrazin-2-ylamino)-2,4'-bipyridin-2'-yl]amino}adamantan-1-ol;
*N*²'-[(1 *S*,2*S*,4*R*)-Bicyclo[2.2.1]hept-2-yl]-*N*⁶-pyrazin-2-yl-2,4'-bipyridine-2',6-diamine;
(4*r*,7*r*)-4-({6-[(4-Methylpyridin-2-yl)amino]-2,4'-bipyridin-2'-yl}amino)adamantan-1-ol;
6-[3-(2-Aminoethyl)phenyl]-*N*-(5-methyl-1*H-*pyrazol-3-yl)pyridin-2-amine;
*N*-(5-Methyl-1*H-*pyrazol-3-yl)-6-[3-(1,2,3,6-tetrahydropyridin-4-yl)phenyl]pyridin-2-amine;
*N*-(5-Methyl-1*H*-pyrazol-3-yl)-6-(3-piperidin-4-ylphenyl)pyridin-2-amine;
*N*-(5-Methyl-1*H*-pyrazol-3-yl)-6-(3-piperazin-1-ylphenyl)pyridin-2-amine;
*N*-(5-Methyl-1*H*-pyrazol-3-yl)-2'-piperazin-1-yl-2,4'-bipyridin-6-amine;
*N*-(2'-Piperazin-1-yl-2,4'-bipyridin-6-yl)pyrimidine-4,6-diamine;
3-[3-(2-Aminoethyl)phenyl]-*N*-[4-(trifluoromethyl)pyridin-2yl]isoquinolin-1-amine;
3-[3-(2-Aminoethyl)phenyl]-*N-*pyrazin-2-ylisoquinolin-1-amine;
*N*-(4-Methylpyridin-2-yl)-1-[3-(1,2,3,6-tetrahydropyridin-4-yl)phenyl]isoquinolin-3-amine;
*N*-Pyrazin-2-yl-1-[3-(1,2,3,6-tetrahydropyridin-4-yl)phenyl]isoquinolin-3-amine;
*N*-(5-Methyl-1*H-*pyrazol-3-yl)-1-[3-(1,2,3,6-tetrahydropyridin-4-yl)phenyl] isoquinolin-3-amine;
*N*-(5-Methyl-1*H-*pyrazol-3-yl)-1-(3-piperidin-4-ylphenyl)isoquinolin-3-amine;
*N*-(4-Methylpyridin-2-yl)-1-(2-piperazin-1-ylpyridin-4-yl)isoquinolin-3-amine;
1-(2-Piperazin-1-ylpyridin-4-yl)-N-pyrazin-2-ylisoquinolin-3-amine;
1-(2-Piperazin-1-ylpyridin-4-yl)-N-pyrimidin-4-ylisoquinolin-3-amine;
*N-*(5-Fluoropyridin-2-yl)-1-(2-piperazin-1-ylpyridin-4-yl)isoquinolin-3-amine;
6-{[1-(2-Piperazin-1-ylpyridin-4-yl)isoquinolin-3-yl]amino}nicotinonitrile;
(±)-1-{2-[(3R,5S)-3,5-Dimethylpiperazin-1-yl]pyridin-4-yl}-N-pyrazin-2-ylisoquinolin-3-amine;
1-[2-(2-Methylpiperazin-1-yl)pyridin-4-yl]-IV pyrazin-2-ylisoquinolin-3-amine;
1-12-[(1*S*,4*S*)-2,5-Diazabicyclo[2.2.1]hept-2-yl]pyridin-4-yl}-*N*-pyrazin-2-ylisoquinolin-3-amine;
(±)-1-12-[(2*S*,5*R*)-2,5-Dimethylpiperazin-1-yl]pyridin-4-yl)-*N*-pyrazin-2-ylisoquinolin-3-amine;
1-{4-[3-(Pyrazin-2-ylamino)isoquinolin-1-yl]pyridin-2-yl}azetidin-3-ol;
1-14-[3-(Pyrazin-2-ylamino)isoquinolin-1-yl]pyridin-2-yl}pyrrolidin-3-ol;
1-{4-[3-(Pyrazin-2-ylamino)isoquinolin-1-yl]pyridin-2-yl}piperidin-4-ol;
1-14-[3-(Pyrazin-2-ylamino)isoquinolin-1-yl]pyridin-2-yl}piperazine-2-Carboxylic acid;
4-{4-[3-(Pyrazin-2-ylamino)isoquinolin-1-yl]pyridin-2-yl}piperazin-2-one;
1-{4-[3-(Pyrazin-2-ylamino)isoquinolin-1-yl]pyridin-2-yl}piperazin-2-one;
2'-Piperazin-1-yl-6-(pyrazin-2-ylamino)-2,4'-bipyridine-4-Carbonitrile;
6-[3-(2-Aminoethyl)phenyl]-4-phenyl-*N*-[4-(trifluoromethyl)pyridin-2-yl]pyridin-2-amine;
*N*-{4-Phenyl-6-[3-(1,2,3,6-tetrahydropyridin-4-yl)phenyl]pyridin-2-yl} pyrazin-2-amine;
*N*-(5-Methyl-1*H-*pyrazol-3-yl)-4-phenyl-6-[3-(1,2,3,6-tetrahydropyridin-4-yl)phenyl]pyridin-2-amine;
4-Phenyl-2'-piperazin-1-yl-*N*-pyrazin-2-yl-2,4'-bipyridin-6-amine;
*N*-{2-(Pyrazin-2-ylamino)-6-[3-(1,2,3,6-tetrahydropyridin-4-yl)phenyl]pyridin-4-yl}benzamide;
3-[({2-(Pyrazin-2-ylamino)-6-[3-(1,2,3,6-tetrahydropyridin-4-yl)phenyl]pyridin-4-yl}amino)carbonyl]benzoic acid;
(2*S*)-2-Benzyl-4-oxo-4-{[6-(pyrazin-2-ylamino)-1",2",3",6"-tetrahydro-2,4':2',4"-terpyridin-4-yl]amino}butanoic acid;
*N*-[2'-Piperazin-1-yl-6-(pyrazin-2-ylamino)-2,4'-bipyridin-4-yl]benzamide;
*N-*[2'-Piperazin-1-yl-6-(pyrazin-2-ylamino)-2,4'-bipyridin-4-yl]-3-(trifluoromethyl) benzamide;
*N*-[2'-Piperazin-1-yl-6-(pyrazin-2-ylamino)-2,4'-bipyridin-4-yl]-1,3-benzodioxole-5-Carboxamide;
2-(3-Methoxyphenyl)-*N*-[2'-piperazin-1-yl-6-(pyrazin-2-ylamino)-2,4'-bipyridin-4-yl]acetam ide;
4-({[2'-Piperazin-1-yl-6-(pyrazin-2-ylamino)-2,4'-bipyridin-4-yl]amino}carbonyl) benzoic acid;
4-Fluoro-*N*-[2'-piperazin-1-yl-6-(pyrazin-2-ylamino)-2,4'-bipyridin-4-yl]benzamide;
4-Methoxy-*N*-[2'-piperazin-1-yl-6-(pyrazin-2-ylamino)-2,4'-bipyridin-4-yl]benzamide;
3-Phenyl-*N*-[2'-piperazin-1-yl-6-(pyrazin-2-ylamino)-2,4'-bipyridin-4-yl]propanamide;
1,3-Dimethyl-*N*-[2'-piperazin-1-yl-6-(pyrazin-2-ylamino)-2,4'-bipyridin-4-yl]-1 H-pyrazole-5-Carboxamide;
2-(2,4-Difluorophenyl)-*N*-[2'-piperazin-1-yl-6-(pyrazin-2-ylamino)-2,4'-bipyridin-4-yl]acetam ide;
*N*-[2'-Piperazin-1-yl-6-(pyrazin-2-ylamino)-2,4'-bipyridin-4-yl]nicotinamide;
*N*-[2'-Piperazin-1-yl-6-(pyrazin-2-ylamino)-2,4'-bipyridin-4-yl]quinoline-6-Carboxamide;
Methyl 3-({[2'-piperazin-1-yl-6-(pyrazin-2-ylamino)-2,4'-bipyridin-4-yl]amino} carbonyl)benzoate;
3-({[2'-Piperazin-1-yl-6-(pyrazin-2-ylamino)-2,4'-bipyridin-4-yl]amino}carbonyl) benzoic acid;
N-[2'-Piperazin-1-yl-6-(pyrazin-2-ylamino)-2,4'-bipyridin-4-yl]-2-pyridin-3-ylacetamide;
Methyl 4-(2-oxo-2-{[2'-piperazin-1-yl-6-(pyrazin-2-ylamino)-2,4'-bipyridin-4-yl]amino}ethyl)benzoate;
4-(2-Oxo-2-{[2'-piperazin-1-yl-6-(pyrazin-2-ylamino)-2,4'-bipyridin-4-yl]amino}ethyl)-benzoic acid;
*N*-[2'-Piperazin-1-yl-6-(pyrazin-2-ylamino)-2,4'-bipyridin-4-yl]piperidine-4-Carboxamide;
1-Acetyl-*N*-[2'-piperazin-1-yl-6-(pyrazin-2-ylamino)-2,4'-bipyridin-4-yl]piperidine-4-Carboxamide;
4-Oxo-4-{[2'-piperazin-1-yl-6-(pyrazin-2-ylamino)-2,4'-bipyridin-4-yl]amino} butanoic acid;
*N* [2'-Piperazin-1-yl-6-(pyrazin-2-ylamino)-2,4'-bipyridin-4-yl]pyrimidine-5-Carboxamide;
5-({[2'-Piperazin-1-yl-6-(pyrazin-2-ylamino)-2,4'-bipyridin-4-yl]amino}carbonyl) pyridine-2-Carboxylic acid;
5-({[2'-Piperazin-1-yl-6-(pyrazin-2-ylamino)-2,4'-bipyridin-4-yl]amino}carbonyl) nicotinic acid;
(2*S*)-2-Benzyl-4-oxo-4-{[2'-piperazin-1-yl-6-(pyrazin-2-ylamino)-2,4'-bipyridin-4-yl]amino}butanoic acid;
*trans*-4-({[2'-Piperazin-1-yl-6-(pyrazin-2-ylamino)-2,4'-bipyridin-4-yl]amino} carbonyl)-Cyclohexanecarboxylic acid;
(3*S*)-3-Benzyl-4-oxo-4-{[2'-piperazin-1-yl-6-(pyrazin-2-ylamino)-2,4'-bipyridin-4-yl]aminolbutanoic acid;
*N*-[2'-Piperazin-1-yl-6-(pyrazin-2-ylamino)-2,4'-bipyridin-4-yl]acetamide;
*N*-(2'-Piperazin-1-yl-6-{[4-(trifluoromethyl)pyridin-2-yl]amino}-2,4'-bipyridin-4-yl)nicotinamide;
4-{[(2'-Piperazin-1-yl-6-{[4-(trifluoromethyl)pyridin-2-yl]amino}-2,4'-bipyridin-4-yl)amino]-Carbonyl}benzoic acid;
5-Oxo-3-phenyl-5-(2'-(piperazin-1-yl)-6-(pyrazin-2-ylamino)-2,4'-bipyridin-4-ylamino)-pentanoic acid;
(3*R*)-3-(Cyclohexylmethyl)-4-oxo-4-{[2'-piperazin-1-yl-6-(pyrazin-2-ylamino)-2,4'-bipyridin-4-yl]amino}butanoic acid;
2-({[2'-Piperazin-1-yl-6-(pyrazin-2-ylamino)-2,4'-bipyridin-4-yl]amino}carbonyl)-Cyclohexanecarboxylic acid;
2-({[2'-Piperazin-1-yl-6-(pyrazin-2-ylamino)-2,4'-bipyridin-4-yl]amino}carbonyl)benzoic acid;
(3*R*)-3-Methyl-4-oxo-4-{[2'-piperazin-1-yl-6-(pyrazin-2-ylamino)-2,4'-bipyridin-4-yl]-amino}butanoic acid;
(3*S*)-4-Phenyl-3-{[3-({[2'-piperazin-1-yl-6-(pyrazin-2-ylamino)-2,4'-bipyridin-4-yl]-amino}carbonyl)benzoyl]amino}butanoic acid;
3,3-Dimethyl-5-oxo-5-{[2'-piperazin-1-yl-6-(pyrazin-2-ylamino)-2,4'-bipyridin-4-yl]-amino}pentanoic acid;
2-Methyl-3-phenyl-*N*-[2'-piperazin-1-yl-6-(pyrazin-2-ylamino)-2,4'-bipyridin-4-yl]-propanamide;
5-Methyl-3-({[2'-piperazin-1-yl-6-(pyrazin-2-ylamino)-2,4'-bipyridin-4-yl]amino}-Carbonyl)hexanoic acid;
(3*R*)-4-Oxo-3-phenyl-4-{[2'-piperazin-1-yl-6-(pyrazin-2-ylamino)-2,4'-bipyridin-4-yl]-amino}butanoic acid;
(3*R*)-3-Benzyl-4-oxo-4-{[2'-piperazin-1-yl-6-(pyrazin-2-ylamino)-2,4'-bipyridin-4-yl]-amino}butanoic acid;
*N*-[2'-[(1*S*,4*S*)-2,5-Diazabicyclo[2.2.1]hept-2-yl]-6-(pyrazin-2-ylamino)-2,4'-bipyridin-4-yl]nicotinamide;
4-({[2'-[(1*S*,4*S*)-2,5-Diazabicyclo[2.2.1]hept-2-yl]-6-(pyrazin-2-ylamino)-2,4'-bipyridin-4-yl]amino}carbonyl)benzoic acid;
*N*-(2'-[(1*S*,4*S*)-2,5-Diazabicyclo[2.2.1]hept-2-yl]-6-{[4-(trifluoromethyl)pyridin-2-yl]-amino}-2,4'-bipyridin-4-yl)nicotinamide;
4-{[(2'-[(1*S*,4*S*)-2,5-Diazabicyclo[2.2.1]hept-2-yl]-6-{[4-(trifluoromethyl)pyridin-2-yl]amino}-2,4'-bipyridin-4-yl)amino]carbonyl}benzoic acid;
*N*-[2'-Piperazin-1-yl-6-(pyrazin-2-ylamino)-2,4'-bipyridin-4-yl]benzenesulfonamide
*N*-[2'-Piperazin-1-yl-6-(pyrazin-2-ylamino)-2,4'-bipyridin-4-yl]-3-(trifluoromethyl) benzenesulfonamide;
*N*-[2'-Piperazin-1-yl-6-(pyrazin-2-ylamino)-2,4'-bipyridin-4-yl]-3-(trifluoromethoxy) benzenesulfonamide;
(3S)-4-Phenyl-3-[({[2'-piperazin-1-yl-6-(pyrazin-2-ylamino)-2,4'-bipyridin-4-yl]amino}-Carbonyl)amino]butanoic acid;
*N*-[2'-Piperazin-1-yl-6-(pyrazin-2-ylamino)-2,4'-bipyridin-4-yl]-3,4-dihydroisoquinoline-2(1H)-Carboxamide;
4-(Benzyloxy)-*N*-[2'-piperazin-1-yl-6-(pyrazin-2-ylamino)-2,4'-bipyridin-4-yl]piperidine-1-Carboxamide;
*N*⁶-(4-Methylpyridin-2-yl)-2'-piperazin-1-yl-2,4'-bipyridine-4,6-diamine;
2'-[(1*S*,4*S*)-2,5-Diazabicyclo[2.2.1]hept-2-yl]-*N*⁶-(4-methylpyridin-2-yl)-2,4'-bipyridine-4,6-diamine;
2'-Piperazin-1-yl-*N*⁶-pyrazin-2-yl-2,4'-bipyridine-4,6-diamine;
*N*⁴-Methyl-2'-(piperazin-1-yl)-*N*⁶-(pyrazin-2-yl)-2,4'-bipyridine-4,6-diamine;
*N*⁴,*N*⁴-Dimethyl-2'-piperazin-1-yl-*N*⁶-pyrazin-2-yl-2,4'-bipyridine-4,6-diamine;
1-(2'-(Piperazin-1-yl)-6-(pyrazin-2-ylamino)-2,4'-bipyridin-4-yl)piperidin-4-ol;
1-[2'-Piperazin-1-yl-6-(pyrazin-2-ylamino)-2,4'-bipyridin-4-yl]piperidine-4-Carboxylic acid;
*N*⁴-Butyl-2'-piperazin-1-yl-*N*⁶-pyrazin-2-yl-2,4'-bipyridine-4,6-diamine; *trans*-4-{[2'-Piperazin-1-yl-6-(pyrazin-2-ylamino)-2,4'-bipyridin-4-yl]amino}-Cyclohexanecarboxylic acid;
4-{[2'-Piperazin-1-yl-6-(pyrazin-2-ylamino)-2,4'-bipyridin-4-yl]amino}cyclohexanol;
*N*⁴-(3-Phenylpropyl)-2'-piperazin-1-yl-*N*⁶-pyrazin-2-yl-2,4'-bipyridine-4,6-diamine;
3-Benzyl-4-{[2'-piperazin-1-yl-6-(pyrazin-2-ylamino)-2,4'-bipyridin-4-yl]amino}butanoic acid;
4-{[2'-Piperazin-1-yl-6-(pyrazin-2-ylamino)-2,4'-bipyridin-4-yl]amino}butanoic acid;
5'-(Piperazin-1-yl)-*N*⁶-(pyrazin-2-yl)-2,3'-bipyridine-4,6-diamine;
3-{3-[4-Amino-6-(pyrazin-2-ylamino)pyridin-2-yl]phenyl}propanoic acid;
3-{3-[4-Amino-6-(pyrazin-2-ylamino)pyridin-2-yl]phenyl}propanamide;
(3S)-4-Phenyl-3-(2'-(piperazin-1-yl)-6-(pyrazin-2-ylamino)-2,4'-bipyridine-4-Carboxamido)butanoic acid;
2'-Piperazin-1-yl-6-(pyrazin-2-ylamino)-*N*-pyridin-3-yl-2,4'-bipyridine-4-Carboxamide;
N-{[2'-Piperazin-1-yl-6-(pyrazin-2-ylamino)-2,4'-bipyridin-4-yl]carbonyl}-L-phenylalanine;
(3*S*)-3-Benzyl-4-oxo-4-({[2'-piperazin-1-yl-6-(pyrazin-2-ylamino)-2,4'-bipyridin-4-yl]-methyl}amino)butanoic acid;
3-(4-{[2'-Piperazin-1-yl-6-(pyrazin-2-ylamino)-2,4'-bipyridin-4-yl]methoxy}phenyl)-propanoic acid; and
4-Methoxy-2'-piperazin-1-yl-*N*-pyrazin-2-yl-2,4'-bipyridin-6-amine.

10. A compound according to any one of claims 1 to 9 for use in the treatment of the human or animal body by therapy.

11. A compound according to any one of claims 1 to 9 for use in the treatment of a pathological condition or disease selected from asthma, chronic obstructive pulmonary disease, allergic rhinitis, allergic conjunctivitis, allergic dermatitis, atopic dermatitis, seasonal allergies, rheumatoid arthritis, multiple sclerosis, systemic lupus erythematosus, B cell lymphoma and immune thrombocytopenic purpura.

12. A pharmaceutical composition comprising a compound as defined in any one of claims 1 to 9 in association with a pharmaceutically acceptable diluent or carrier.

13. Use of a compound as defined in any one of claims 1 to 9 for the manufacture of a medicament for the treatment of a pathological condition or disease as defined in claim 11.

14. A method for treating a subject afflicted with a pathological condition or disease as defined in claim 11, which comprises administering to said subject an effective amount of a compound as defined in any one of claims 1 to 9.

15. A combination product comprising (i) a compound according to anyone of claims 1 to 9, and (ii) one or more active ingredients selected from the group consisting of a β2-adrenergic agonist, a corticosteroid, an anti-Cholinergic agent, an anti-allergic agent, an anti-inflammatory agent, an immunosuppressant, a PDE4 inhibitor, a topically acting p38 inhibitor, an IKK2 inhibitor, an A_{2A} agonist, a Pl3Kδγinhibitor and an anti-infective agent, for simultaneous, separate or sequential use in the treatment of the human or animal body.
